# EUROPEAN PATENT APPLICATION

(11) **EP 3 904 879 A1**
(43) Date of publication of application: **03.11.2021**
(21) Application number: 20171474.8
(22) Date of filing: 27.04.2020
(51) Int. Cl.: G01N 33/569

(54) **A METHOD FOR DISTINGUISHING HEALTHY INDIVIDUALS FROM INDIVIDUALS HAVING INFECTIOUS OR INFLAMMATORY CONDITIONS**

(71) Applicant: Abacuslabs Ltd., Sligo (IE)
(72) Inventor: ANHOLD, Heinrich, Sligo (IE); CANDON, Ruth, Sligo (IE)
(74) Representative: Purdylucey Intellectual Property

(57) **Abstract**

A method of determining a health status of an individual, the method comprising the steps of assaying a biological sample from the individual for the expression of at least genes, or proteins encoded by said genes, wherein the at least two genes are selected from SAA, CRP, Hp and MxA, wherein the expression of the at least two genes, or the proteins encoded by said genes, above or below a specified threshold level correlates with the health status of the individual.

SEQUENCE LISTING

<110>  ABACUSLABS LTD.

<120>  A method for distinguishing healthy individuals from individuals
       having infectious or inflammatory conditions

<130>  P13016EP00

<160>  12

<170>  PatentIn version 3.5

<210>  1
<211>  122
<212>  PRT
<213>  Homo sapiens

<400>  1

<210>  2
<211>  369
<212>  DNA
<213>  Homo sapiens

<400>  2
atgaagcttc tcacgggcct ggttttctgc tccttggtcc tgggtgtcag cagccgaagc      60

ttcttttcgt tccttggcga ggcttttgat ggggctcggg acatgtggag agcctactct     120

gacatgagag aagccaatta catcggctca gacaaatact tccatgctcg ggggaactat     180

gatgctgcca aaaggggacc tgggggtgtc tgggctgcag aagcgatcag cgatgccaga 240

gagaatatcc agagattctt tggccatggt gcggaggact cgctggctga tcaggctgcc 300

aatgaatggg gcaggagtgg caaagacccc aatcacttcc gacctgctgg cctgtctgag 360

aaatactga 369

<210> 3
<211> 10717
<212> DNA
<213> Homo sapiens

<400> 3
ttttcacagt tcatttacgg ccctctttcc aacacattca ctgccaatac tcttattgac 60

aataactgta ttgttgaacc ttccagtatc ctgcattccc ggatcaaggc cccctcaaag 120

ccctgatatg caaatatctg ggaaaagaat gttccagagg aaaggaacag ctaatccgag 180

gcccctaggg taagatgtgc ctgggggttt ggagaccagt gtggccagag caaaatgagc 240

aggaggagag aattggatga tgaggtacga gaggaaggag ttaggacagt ttgagtaaag 300

tttgaaaacc attataaggg ctttgacttc aactatgagt ggaagtggaa tcctccggag 360

agttttgaat ggagagtgat agaagttgtc ttgtgttgta acagtctggc tgctatactg 420

aaaagagact agttggcggc aaagggggaa atgtggaagc cagttaagaa gccatcataa 480

cccagaaggt gatgcctaat aacatctctc tgggagcagc ggagagatga taagggtttg 540

ccttctgaat atgttttttg acaattaatg taaacatttc aagtaggctg agattttatt 600

gcatattaac aatgtccatg ttcactcgcg gcagccgccc ccttctgcgc ggtcatgccg 660

agccagcacc tgggcctgga actgggccgc agcccccagc ttcacccacc acctccctac 720

catggacccc tgcaaagtga acgagcttcg ggcctttgtg aaaatgtgta agcaggatcc 780

gagcgttctg cacaccgagg aaatgcgctt cctgagagag tgggtggaga gcatgggagg 840

taaagtacca cctgctactc agaaggctaa atcagaagaa aataccaagg aagaaaaacc 900

tgatagtaag aaggtggagg aagacttaaa ggcagacgaa ccatcaactg aggaaagtga 960

tctagaaatt gataaagaag gtgtgattga accagacact gatgctcctc aagaaatggg 1020

agatgaaaat gtggagataa cggaggagat gatggatcag gcaaatgata aaaaagtggc 1080

tgctattgaa gtcctaaatg atggtgaact ccagaaagcc attgacttat tcacagatgc 1140

catcaagctg aatcctcgct tggccatttt gtatgcaaag agggccagtg tcttcgtcaa 1200

attacagaag ccaaatgctg ccatccaaga ctgtgacaga gccattgaaa taaatcctga 1260

ttcagctcag ccttacaagt ggcgggggaa agcacacaga cttctaggcc actgggaaga 1320

agcagcccat gatcttgcct ttgcctgtaa attggattat gatgaagatg ctagtgcaat 1380

gctgaaagaa gttcaaccta gggcacagaa aattgcagaa cattggagaa agtatgagcg 1440

aaaacatgaa gagcgagaga tcaaagaaag aatagaacga gttaagaagg ctcaagaaga 1500

gcaggagaga gcccagaggg aggaagaagc cagacgacag tcaggagctc actatggccc 1560

ttttccaggt ggctttcctg gtggaatgcc tggtaatttt cccggaggaa tgcctggaat 1620

gggaggggac atgcctggaa tggccggaat gcctggactc aatgaaattc ttagtgatcc 1680

agaggctctt gcagccatgc aggatccaga agttatggtg gccttccagg atgtggctca 1740

gaacccagca aatatgtcaa aataccagag caacccaaag gttatgaatc tcatcagtaa 1800

attgtcagcc aaatttggag gtcaagcata atgcccttct gataaataaa gccctgctga 1860

aggaaaagca acctagatca ccttatggat gtcgcaataa tacaaaccaa cgtacctctg 1920

accttctcat caagagagct ggggtgcttt gaagataatc cctacccctc tcccccaaat 1980

gcagctgaag cattttacag tggtttgcca ttagggtatt cattcagata atgttttcct 2040

actaggaatt acaaacttta aacacttttt aaatcttcaa atatttaaaa caaatttaaa 2100

gggtctgtta attcttatat ttttctttac taatcattgt ggatttttcc ttaaattatt 2160

gggcagggaa tatacttatt tatggaagat tactgctcta atttgagtga aataaaagtt 2220

attagtgcga ggcaaacata aaaaaaaaaa gtccatgttc atctctaaat gacatcattg 2280

ttccaaagct tttccattct tcttaacctt ccacctgtca atctatagga gatgacttct 2340

cctacttcac tcatgcattg actccttcaa tcaataaaag tgactaagaa cctgctacag 2400

gtgaggtgct gtgtttggtg ttaaagtgac aacagttatc tgtcaataag cctgacaagg 2460

ttcctatccc tgtgttttgt gcactctggg tcaaactcag aaatgcaaac aggtggagag 2520

cgatgagttc tatgactggt aaagaaaagg gcctgctggt ttccctcagg atctctgtcc 2580

ttcatctcaa aatgcatctt ccttgttatc gttcctctcc ttcctgtctc agaggaagac 2640

ctgctcctgc tacactctgg gcaaccttgt ccccgtggcc ctgtggcccc ttggttgttg 2700

aagtctatgt tatgccctat cttttaccct cagtcactct ctctgttaac attctccctg 2760

tgccctgtaa ccctccctca tctttaaata aatcctcctc ctttgacctt cgcatgtatt 2820

cagtcatgca actcaacaag catttattgc acagtgatat tcaatttgcc acttgctaaa 2880

agtctgaacc ttggcagctg aatgtgatca gaaaaaaagc acgactgcta tgactagtct 2940

cactttaaat tcatggtcgt tgaccaagag ctaccataca atccactacc tttctcaagt 3000

tcagtcacat tcttcctttc ctagatgtct gctttctact tctcttctct tctgaaactt 3060

cccacaactc ctcgttcatt ctcttctcag ttgacaactt tgcttcctat ttcactgaaa 3120

aatagaagca atcagatatg aacttctggc tgggcatggt agctcatgcc tataatctca 3180

gcactttggg aggccaaggc aggaggactg caggttagga atttgagacc agcctgggca 3240

acatggtgaa actcccactg tactaaaaat tttaaaaatt actcaaacat attggcaaac 3300

aactgcagtc ccagctactt gggaggttga gatgcaagga tcacttaaac ctgggaggct 3360

gaggctgcag tgagccatga ttgcaccact gcactccagc tcaggcaaca gagcaagacc 3420

ctgtcttgag aggagaggag aagagaggag gggaggggag ggcaggggag gggaggggag 3480

gggaagggag aggggagggg agaggggagg agagagggga ggggagggga ggggagggga 3540

ggggaggaga ggaggatcag gtgaggagta tgccaaggag tgtttttaag acttactgtt 3600

ttctctttcc caacaagatt gtcatttcct ttaaaaagta gttatcctga ggcctatatt 3660

catagcattc tgaaagaaag aaaagaaaag aggaaagaaa gagagaggaa ggaaggaagg 3720

agaaagagag aggaaggaag gagaaagaga gaggaaggaa gggaggaaga gaagaaggga 3780

ggaagaaaag aaggaaggaa ggagggaggg agggaaggga gggagggaaa gaggaagaaa 3840

ggagggaaag aaggaaggaa gagagagagg aaggaaggag gaagagagaa gaaggaagga 3900

ggaagacaga gagggagtaa ggaaggaagg aaggagaaag agagaggaag gaagaaatga 3960

aggaaggaag gaaagaaaga aaaaataaaa gagtgaaaac ggactggaga agaagaaacc 4020

acagttgctg ctatatccac cagcctctct gcatgtcctg gcctcagccc tgctgggctc 4080

tggtactgac cacttccttc cttcctaatt tcctaattga ctaggccagc tgagcagggc 4140

ttttctgtgc tgaggaggta aatctctgga tatctagact gaggggtgga aggagccttc 4200

cagggcacac atgagacatg gcaggggtag gctgctagtt ttattttgtt ttcttttaga 4260

cacagggtct tgctctgtta accaggctgg agtgcagtgg cgtgattata gctcactgca 4320

gccttgacct cctgggtctc ccacaatcct tccgcttcag cctcttgagt agctgggact 4380

gcaggtgcac actaccacac ccggtccatt tatttttata tttcgtagag acaagatctt 4440

acagttttgc acagagtgat cttaaactct tgaccccaag tgatcctcct gccttggcct 4500

ccaaaagcat tgggattata ggagtgagcc actgtgctgg acctagtctg tcagctttga 4560

agctttagat atgaactcag agggacttca tttcagaggc atctgccatg tggcccagca 4620

gagcccatcc tgaggaaatg actggtagag tcaggagctg gcttcaaagc tgccctcact 4680

tcacaccttc cagcagccca ggtgccgcca tcacggggct cccactctca actccgcagc 4740

ctcagccccc tcaatgctga ggagcagagc tggtctcctg ccctgacagc tgccaggcac 4800

atcttgttcc ctcaggttgc acaactggga taaatgaccc gggatgaaga aaccactggc 4860

atccaggaac ttgtcttaga ccgttttgta ggggaaatga cctgcaggga ctttccccag 4920

ggaccacatc cagcttttct tcgctcccaa gaaaccagca gggaaggctc agtataaata 4980

gcagccaccg ctccctggca ggcagggacc cgcagctcag ctacagcaca gatcaggtga 5040

ggagcacacc aaggagtgat ttttaaaact tactctgttt tctctttccc aacaagatta 5100

tcatttcctt taaaaaaaat agttatcctg gggcatacag ccataccatt ctgaaggtgt 5160

cttatctcct ctgatctaga gaggtaagca gggtcgggcc tggtagtact tggatgggag 5220

aacacctggg aataccaggt gctaaaggct ttaagaataa aaaataatga tcctgctttg 5280

tgtttatccc atgttgagtt ctgtgcgggg cagagggaac acacggtaaa tgcgttatgg 5340

ggaattatag gctacttgag ggagtgacag tctggtggta actcctgcct tcctccatca 5400

gtgccacgtt ggcatcctct tatgcagtca ggcttcaggg ctgatgggtt cagaaccgag 5460

ggcttctggc tctgagtgag gtcctgctgc aaggtttcct agatgagcca ctgagactct 5520

aataagatcc agtggaaata accaggctct cgtcggaata taagtcccaa gggaagctgt 5580

gccagtcttg tgggcgactg cctgacttct cctttcattt cagcaccatg aagcttctca 5640

cgggcctggt tttctgctcc ttggtcctgg gtgtcagcag ccgaagcttc ttttcgttcc 5700

ttggcgaggc ttttgatggt aaggcttcag aaggtttgca ggatttctga agagaaacat 5760

caccctggac ctgataaact ggggaaaatg atgctttcgg aaggctgctt ttgaaccaca 5820

gagttgctag tgtctgcgtt gctgaggcct gccaggaact agggtttgct gggttgcctg 5880

tctcgagtct ttcagagctg ctgggaatat cccctttccc cgtagtgcag cttctcagga 5940

tgtgttaagt ggatggatca catttcagaa gccgctgcaa ggtgtatcaa aaacacatct 6000

cctgagccgt aagggacggg gcatccagta acaacgcaca cggggtattt ttgggcttcc 6060

ttaagatttg agccgctgcc ttaggttgtg ctgcccaatg tgcctgggga gctgctaaac 6120

agattagaga gtcgaggatt gttgtcagtt actcagagaa agaacaatca tcctttccag 6180

gagcacctga gctgtttgtt ttgcgtagaa gatgcaaaat aaggcctgca atgggtataa 6240

aatgtccctc agcataaatc gcataggagt atgactaagg ctgttgactc ttctgtcttc 6300

tttctccttc ctccttcgat ttcctagttg gataatgtac agggctcttt agcctcgctc 6360

tgtcaggggc tcccttcctg gtttgttctg tttccattct tccttctcca gccttcttga 6420

caagagctgg gaactaacgt gcctcaagcc cccacaagga ccacagcatt ttctcattta 6480

gtttcagaat gactctgtga cgcaatcttc ctctcttgga aggtgagaaa gctgatcttg 6540

gaaggtgaga aagctgagac ttagagcagc tgaagccaat gcccagggac ttactgccag 6600

tcagcaggtg gcagggcaga ggtttgagcc cggctgtgct tgaggtcagg gctcttgcca 6660

ggtagacgca tcactgacca cctcctagag gttgatggtt atgaatctca ggcacacctt 6720

ggcatcacct gaaataccca tgccttcaac tccccagcag agtctgcaga aactggcctg 6780

gggtgtggcc tgggcactgg gactttcagt ttctctctgg gtgattagaa agtgcagcca 6840

aggctcacgc ctgtaattcc agcactttgg gaggccaagg tggatgaatc acttgaggtc 6900

atgagttccg gagcagcctg gccaacatgg tgaaaccccg tctctactaa aaatactaaa 6960

atgtagccag gcgtggtggc aggcacctgt aatcccagct actcaggagg ctgaagcacg 7020

agaatcactt gaacccgaga agcagaggtt gcagtgacta gagatcgcac cagtgtcctc 7080

caacctgggt gacagagcga gactccatct aaaaaaaatg aaaaagaaag tgcagccaag 7140

gcagagcacc actgccctat tgcttcctca agcaacccac agcatcagta cagcctacta 7200

agaaagtatt tagggacttt tatgctccta acagtcactg gaactcacgt cacaatgacg 7260

tgtattccat ttgcaagaat atatacttta ggtcggggtg cggtggctca cgcctgtaat 7320

cccagcactt tgggaggcca aggcaggggg atcacgaggt caggagttcg agaccagcct 7380

gaccaacatg gtgaaatccc cgtctctact aaaaatacaa aaattagcca ggcgtgatgg 7440

cgcatgcctg taatctcagc tactcaggag gctgaggcag aagaatctct tgaacctggg 7500

aggtggaggt tgcgatgagc tgagatagca ccactgcact ccagcctggg cgacagagca 7560

agactctgtc taaaaaaaaa aaaaaaaaaa aaaaaaaaaa aaagaatata aactttagta 7620

gtcagggcag aagtactctg tgtctgccac ctttctcagc atcagtattc catgtcacta 7680

cctcattcat acacactcct ggatcttatc ataggcagct tcattctata gcagtggctc 7740

ttcaccaggg cacttgaaga agccaactag gataaaggaa tgtgcttctc aacccatggt 7800

atccaaggct gctatgatca caggctgaaa gcttgaagtc agtggaagat ttgtccttcc 7860

tcattcccct ctaaggtgtt gttggagtct ttatgttctc ctgatgtccc ttctgccttt 7920

cctttccttt ccaggggctc gggacatgtg gagagcctac tctgacatga gagaagccaa 7980

ttacatcggc tcagacaaat acttccatgc tcgggggaac tatgatgctg ccaaaagggg 8040

acctgggggt gcctgggctg cagaagtgat caggtaactg gagctcctgg gacgttaggg 8100

ctgggtgagc agagcttgcc tgccttggac agtcaggagg gagacgagct ccttgtggag 8160

aagttagagg ctgcggcccc tcctcctctt gccctctctc tgcctctgtg ctcagtgtga 8220

ggtctgagtg gatggtagga gtgagtgatt cctcatcctc cctctctggg tgctgttcat 8280

ccagcctagg ggtgcccagc ctggctgaat ggggtggtgc ccagtgtttt catccctcct 8340

tccttggcct ttctgggctc ctctctgagc cctcccttgg aacagggaga atgggagggt 8400

gggctattgc tcactggcct gattattaat ctccttcttg cctgccttga ttacagcgat 8460

gccagagaga atatccagag attctttggc catggtgcgg aggactcgct ggctgatcag 8520

gctgccaatg aatggggcag gagtggcaaa gaccccaatc acttccgacc tgctggcctg 8580

cctgagaaat actgagcttc ctcttcactc tgctctcagg agatctggct gtgaggccct 8640

cagggcaggg atacaaagcg gggagagggt acacaatggg tatctaataa atacttaaga 8700

ggtggaattt gtggaaactg ggtgttatac tttgtggtat agactgcctg tttagtatga 8760

aggggcgatc catgcacatc taagtgaacg tggaggctgg gtgggtggga gacgactcct 8820

gggcacacag ggcatcctgg gcatccctga ggcaaggaca tgatgagttc agtggccacc 8880

cccacaggat cccaggggct tcagcagatc ccacccctta ccccatgtga gcagctgccc 8940

agtgagtctg taggaacccg agccacattc ccagtgagtt caactgcacc ccggcacgtt 9000

ttgctagcac ctcaatggag agctccttgc ttgcagcttt ggcttgtggc acccagcaaa 9060

agcttcctgc cacccagtgg ctacagccac acactctcca gcaagattta atctcagcct 9120

tgtgaggagc cctttcccaa atttatttct ttctgtgttt tttatccctt agtagctaat 9180

ctcatgttag ccattaataa ctctctatgt taaacccttc cttttgtatc tgcggctaca 9240

ttgatcaatt gtctcacacc gctcacccac cccctctccc tggtcatgca gaggcctcac 9300

cagtcatttt attgctattc ccaggcctct gtgtgcccag atctcttcac tgccctgtca 9360

gttgtgtcct gtccccttct cgacctcctg gccttgtcct caatgatgtt tctatgaggc 9420

tttggaaagc ctcatcccaa gagtcctggc aactgataca ttagtctcac caacactagc 9480

ccctactcct gatctcattt taaaatttta ttttcttatt ttattattat tatttttagg 9540

gacatcaccc tgcttctgac tgacccaatt tttaaagttt ctctttattc tccttaaaga 9600

atggctctcc catttgtttc cctcatcttt cttttcactg acttagaatt caggtccaga 9660

caaaaattcc acttccttga agagccttcc cagtagccat gaaccacact ctggggcaga 9720

gtttgtggct cccaagcact ttgttcacac ctgctcgttc atctttaccc cctcctctca 9780

gaattggttt tgtatgtcag ttcccctgct ggactggaag cccctgtaac ataactagca 9840

tttgaacagt tcataaaaac actttcattt ctattgtcct gtgtaactgt tcacaagata 9900

cattagtctc actcattgtc gtttgacaat gtttattgtt ctaaagagaa agcgagatat 9960

gtaggtagac acaacacaat aagagcctca atacaggcac acactggtgg cagtccaaac 10020

tctgctggaa tatggaaggc cagtgaatag taactagaat cgctgccatc ttttacactg 10080

caattgcaag actgtgtcct atactttaca tgctttatcg gattgacact gcagagaagc 10140

ctttgatgtt ctcggttgga taaacaggtt tagaggtatg tgttcaggcc ttctccaagg 10200

tcacacatgt aacaggtgca gagccaagac tgcaggctgg tttgtctgac tccagaacct 10260

gtgatacact gaacaaggcc aagagattca gcagtggaga cctcctctct tcttcaggaa 10320

gatagggaac atggtaagag cagtgaattc cataagcatg ggaccccgtt atacttcatt 10380

ttttgtaaaa ttagttcctt gatcagaagc aacgcctttg ggtctctcgc tattgagtta 10440

agtgtggatg tgccactctc cctagtaccc cagagtgtag ttggaggctt gcctagagat 10500

tagaggttga ggatccttca gtagttccta taccaagcca taagttgtgc tgcatctgca 10560

ggtagggtca gggagctccg ctgaacagat gtggaggaca tcagagtggg aggaaaagga 10620

agcaagtcgt gtggggagag aagacccagc ctgcaatgat gactggttaa ctactgtttc 10680

ttacctaata cacactcaga taacagacta agtctag 10717

<210> 4
<211> 224
<212> PRT

<213>  Homo sapiens

<400>  4

Met Glu Lys Leu Leu Cys Phe Leu Val Leu Thr Ser Leu Ser His Ala
1               5                   10                  15

Phe Gly Gln Thr Asp Met Ser Arg Lys Ala Phe Val Phe Pro Lys Glu
            20                  25                  30

Ser Asp Thr Ser Tyr Val Ser Leu Lys Ala Pro Leu Thr Lys Pro Leu
        35                  40                  45

Lys Ala Phe Thr Val Cys Leu His Phe Tyr Thr Glu Leu Ser Ser Thr
    50                  55                  60

Arg Gly Tyr Ser Ile Phe Ser Tyr Ala Thr Lys Arg Gln Asp Asn Glu
65                  70                  75                  80

Ile Leu Ile Phe Trp Ser Lys Asp Ile Gly Tyr Ser Phe Thr Val Gly
                85                  90                  95

Gly Ser Glu Ile Leu Phe Glu Val Pro Glu Val Thr Val Ala Pro Val
            100                 105                 110

His Ile Cys Thr Ser Trp Glu Ser Ala Ser Gly Ile Val Glu Phe Trp
        115                 120                 125

Val Asp Gly Lys Pro Arg Val Arg Lys Ser Leu Lys Lys Gly Tyr Thr
    130                 135                 140

Val Gly Ala Glu Ala Ser Ile Ile Leu Gly Gln Glu Gln Asp Ser Phe
145                 150                 155                 160

Gly Gly Asn Phe Glu Gly Ser Gln Ser Leu Val Gly Asp Ile Gly Asn
                165                 170                 175

Val Asn Met Trp Asp Phe Val Leu Ser Pro Asp Glu Ile Asn Thr Ile
            180                 185                 190

Tyr Leu Gly Gly Pro Phe Ser Pro Asn Val Leu Asn Trp Arg Ala Leu
        195                 200                 205

Lys Tyr Glu Val Gln Gly Glu Val Phe Thr Lys Pro Gln Leu Trp Pro
    210                 215                 220

<210>  5
<211>  1307
<212>  DNA

<213>  Homo sapiens

<400>  5
aaggcaagag atctaggact tctagcccct gaactttcag ccgaatacat cttttccaaa      60

ggagtgaatt caggcccttg tatcactggc agcaggacgt gaccatggag aagctgttgt     120

gtttcttggt cttgaccagc ctctctcatg cttttggcca gacagacatg tcgaggaagg     180

cttttgtgtt tcccaaagag tcggatactt cctatgtatc cctcaaagca ccgttaacga     240

agcctctcaa agccttcact gtgtgcctcc acttctacac ggaactgtcc tcgacccgtg     300

ggtacagtat tttctcgtat gccaccaaga gacaagacaa tgagattctc atattttggt     360

ctaaggatat aggatacagt tttacagtgg gtgggtctga aatattattc gaggttcctg     420

aagtcacagt agctccagta cacatttgta caagctggga gtccgcctca gggatcgtgg     480

agttctgggt agatgggaag cccagggtga ggaagagtct gaagaaggga tacactgtgg     540

gggcagaagc aagcatcatc ttggggcagg agcaggattc cttcggtggg aactttgaag     600

gaagccagtc cctggtggga gacattggaa atgtgaacat gtgggacttt gtgctgtcac     660

cagatgagat taacaccatc tatcttggcg ggcccttcag tcctaatgtc ctgaactggc     720

gggcactgaa gtatgaagtg caaggcgaag tgttcaccaa accccagctg tggccctgag     780

gcccagctgt gggtcctgaa gtgctttctt aattttatgg ctcttctggg aaactcctcc     840

ccttttccac acgaaccttg tggggctgtg aattctttct tcatccccgc attcccaata     900

tacccaggcc acaagagtgg acgtgaacca cagggtgtcc tgtcagagga gcccatctcc     960

catctcccca gctccctatc tggaggatag ttggatagtt acgtgttcct agcaggacca    1020

actacagtct tcccaaggat tgagttatgg actttgggag tgagacatct tcttgctgct    1080

ggatttccaa gctgagagga cgtgaacctg ggaccaccag tagccatctt gtttgccaca    1140

tggagagaga ctgtgaggac agaagccaaa ctggaagtgg aggagccaag ggattgacaa    1200

acaacagagc cttgaccacg tggagtctct gaatcagcct tgtctggaac cagatctaca    1260

cctggactgc ccaggtctat aagccaataa agcccctgtt tacttga                  1307


<210>  6
<211>  2480
<212>  DNA
<213>  Homo sapiens

<400>  6
tttgcttccc ctcttcccga agctctgaca cctgccccaa caagcaatgt tggaaaatta      60

tttacatagt ggcgcaaact cccttactgc tttggatata aatccaggca ggaggaggta     120

gctctaaggc aagagatctg ggacttctag cccctgaact ttcagccgaa tacatctttt     180

ccaaaggagt gaattcaggc ccttgtatca ctggcagcag gacgtgacca tggagaagct     240

gttgtgtttc ttggtcttga ccagcctctc tcatgctttt ggccagacag gtaagggcca     300

ccccaggcta tgggagagtt ttgatctgag gtatgggggt ggggtctaag actgcatgaa 360

cagtctcaaa aaaaaaaaaa aaagactgta tgaacagaac agtggagcat ccttcatggt 420

gtgtgtgtgt gtgtgtgtgt gtgtgtgtgg tgtgtaactg gagaaggggt cagtctgttt 480

ctcaatctta aattctatac gtaagtgagg ggatagatct gtgtgatctg agaaacctct 540

cacatttgct tgtttttctg gctcacagac atgtcgagga aggcttttgt gtttcccaaa 600

gagtcggata cttcctatgt atccctcaaa gcaccgttaa cgaagcctct caaagccttc 660

actgtgtgcc tccacttcta cacggaactg tcctcgaccc gtgggtacag tattttctcg 720

tatgccacca agagacaaga caatgagatt ctcatatttt ggtctaagga tataggatac 780

agttttacag tgggtgggtc tgaaatatta ttcgaggttc ctgaagtcac agtagctcca 840

gtacacattt gtacaagctg ggagtccgcc tcagggatcg tggagttctg ggtagatggg 900

aagcccaggg tgaggaagag tctgaagaag ggatacactg tgggggcaga agcaagcatc 960

atcttggggc aggagcagga ttccttcggt gggaactttg aaggaagcca gtccctggtg 1020

ggagacattg gaaatgtgaa catgtgggac tttgtgctgt caccagatga gattaacacc 1080

atctatcttg gcgggccctt cagtcctaat gtcctgaact ggcgggcact gaagtatgaa 1140

gtgcaaggcg aagtgttcac caaaccccag ctgtggccct gaggccagct gtgggtcctg 1200

aaggtacctc ccggtttttt acaccgcatg ggccccacgt ctctgtctct ggtacctccc 1260

gcttttttac actgcatggt tcccacgtct ctgtctctgg gcctttgttc ccctatatgc 1320

attgaggcct gctccaccct cctcagcgcc tgagaatgga ggtaaagtgt ctggtctggg 1380

agctcgttaa ctatgctggg aaatggtcca aaagaatcag aatttgaggt gttttgtttt 1440

catttttatt tcaagttgga cagatcttgg agataatttc ttacctcaca tagatgagaa 1500

aactaacacc cagaaaggag aaatgatgtt ataaaaaact cataaggcaa gagctgagaa 1560

ggaagcgctg atcttctatt taattcccca cccatgaccc ccagaaagca ggagcattgc 1620

ccacattcac agggctcttc agtctcagaa tcaggacact ggccaggtgt ctggtttggg 1680

tccagagtgc tcatcatcat gtcatagaac tgctgggccc aggtctcctg aaatgggaag 1740

cccagcaata ccacgcagtc cctccacttt ctcaaagcac actggaaagg ccattagaat 1800

tgccccagca gagcagatct gctttttttc cagagcaaaa tgaagcacta ggtataaata 1860

tgttgttact gccaagaact taaatgactg gtttttgttt gcttgcagtg ctttcttaat 1920

tttatggctc ttctgggaaa ctcctcccct tttccacacg aaccttgtgg ggctgtgaat 1980

tctttcttca tccccgcatt cccaatatac ccaggccaca agagtggacg tgaaccacag 2040

ggtgtcctgt cagaggagcc catctcccat ctccccagct ccctatctgg aggatagttg 2100

gatagttacg tgttcctagc aggaccaact acagtcttcc caaggattga gttatggact 2160

ttgggagtga gacatcttct tgctgctgga tttccaagct gagaggacgt gaacctggga 2220

ccaccagtag ccatcttgtt tgccacatgg agagagactg tgaggacaga agccaaactg 2280

gaagtggagg agccaaggga ttgacaaaca acagagcctt gaccacgtgg agtctctgaa 2340

tcagccttgt ctggaaccag atctacacct ggactgccca ggtctataag ccaataaagc 2400

ccctgtttac ttgagtgagt ccaagctgtt ttctgatagt tgctttagaa gttgtgacta 2460

acttctctat gacctttgaa 2480

<210> 7
<211> 406
<212> PRT
<213> Homo sapiens

<400> 7

Met Ser Ala Leu Gly Ala Val Ile Ala Leu Leu Leu Trp Gly Gln Leu
1               5                   10                  15

Phe Ala Val Asp Ser Gly Asn Asp Val Thr Asp Ile Ala Asp Asp Gly
            20                  25                  30

Cys Pro Lys Pro Pro Glu Ile Ala His Gly Tyr Val Glu His Ser Val
        35                  40                  45

Arg Tyr Gln Cys Lys Asn Tyr Tyr Lys Leu Arg Thr Glu Gly Asp Gly
    50                  55                  60

Val Tyr Thr Leu Asn Asp Lys Lys Gln Trp Ile Asn Lys Ala Val Gly
65                  70                  75                  80

Asp Lys Leu Pro Glu Cys Glu Ala Asp Asp Gly Cys Pro Lys Pro Pro
                85                  90                  95

Glu Ile Ala His Gly Tyr Val Glu His Ser Val Arg Tyr Gln Cys Lys
            100                 105                 110

Asn Tyr Tyr Lys Leu Arg Thr Glu Gly Asp Gly Val Tyr Thr Leu Asn
        115                 120                 125

Asn Glu Lys Gln Trp Ile Asn Lys Ala Val Gly Asp Lys Leu Pro Glu
    130                 135                 140

Cys Glu Ala Val Cys Gly Lys Pro Lys Asn Pro Ala Asn Pro Val Gln
145                 150                 155                 160

Arg Ile Leu Gly Gly His Leu Asp Ala Lys Gly Ser Phe Pro Trp Gln
                165                 170                 175

Ala Lys Met Val Ser His His Asn Leu Thr Thr Gly Ala Thr Leu Ile
            180                 185                 190

Asn Glu Gln Trp Leu Leu Thr Thr Ala Lys Asn Leu Phe Leu Asn His
        195                 200                 205

Ser Glu Asn Ala Thr Ala Lys Asp Ile Ala Pro Thr Leu Thr Leu Tyr
    210                 215                 220

Val Gly Lys Lys Gln Leu Val Glu Ile Glu Lys Val Val Leu His Pro
225                 230                 235                 240

Asn Tyr Ser Gln Val Asp Ile Gly Leu Ile Lys Leu Lys Gln Lys Val
                245                 250                 255

Ser Val Asn Glu Arg Val Met Pro Ile Cys Leu Pro Ser Lys Asp Tyr
            260                 265                 270

Ala Glu Val Gly Arg Val Gly Tyr Val Ser Gly Trp Gly Arg Asn Ala
        275                 280                 285

Asn Phe Lys Phe Thr Asp His Leu Lys Tyr Val Met Leu Pro Val Ala
    290                 295                 300

Asp Gln Asp Gln Cys Ile Arg His Tyr Glu Gly Ser Thr Val Pro Glu
305                 310                 315                 320

Lys Lys Thr Pro Lys Ser Pro Val Gly Val Gln Pro Ile Leu Asn Glu
                325                 330                 335

His Thr Phe Cys Ala Gly Met Ser Lys Tyr Gln Glu Asp Thr Cys Tyr
            340                 345                 350

Gly Asp Ala Gly Ser Ala Phe Ala Val His Asp Leu Glu Glu Asp Thr
        355                 360                 365

Trp Tyr Ala Thr Gly Ile Leu Ser Phe Asp Lys Ser Cys Ala Val Ala
    370                 375                 380

Glu Tyr Gly Val Tyr Val Lys Val Thr Ser Ile Gln Asp Trp Val Gln
385                 390                 395                 400

Lys Thr Ile Ala Glu Asn
                405

<210>  8
<211>  38542

<212>  DNA
<213>  Homo sapiens

<400>  8
tctagaagct gccatcatac tgaaggtaat cttctgaaac ttgcggtttt ctttcaaaaa      60

ttatgtttat aagatgatcc atgttcttgc agagtttatt cattttatgc tgtataatat     120

tccattatat ccacatacaa tgcagtattg acccttcctc ctgttgatgg gcatttgtct     180

tgtttctagt tactttgcta ttatatcagt gtcaccatga ttatccaaaa gtaattcttt     240

tgtacactct aatttaagaa caactaaccc tttttaatga ataaatcaac cttgtattga     300

gttgctacta agtttcagtt gactagtacc tgggatacac acaggtgcag acatttgact     360

gagacatatt gatttttctc atctgcctat ttaggctaat caccagacta taaaaccatg     420

agaaccactg ccattgagta tagtctgtgt cagtctacac tatagcttta actagttgtg     480

tgatttcttg caaagagcaa tcagagaaga cacaataaac acatttactg atttcaggct     540

ggagagcttt taagcaatag ggagatggcc acacacaagg tggagaaaat tactgtgaaa     600

aggaagtact ttctttagag ccccacctaa gctaggctgc agaaatgtct acaatgggtt     660

tgaaaaaact caaaatgagc ctttctgcag tgtgaaaatc ctccaagata aagagacaga     720

ttgatggttc ctgccgccgc cctgtcctgc ccagttgctg atttcaggaa atactttggc     780

aggtttgtgg gtcatagagt tgccaggttt cttgggattt gtaatagaac atcacaagaa     840

aatcaagtgt gaagcaagag ctcaactctt aacaggggta ttgtttgtgg ttttgttact     900

ggaaaagata gtgaccttac cagggccaaa gtttgtagac acaggaatta cgaaatggag     960

aaggggagaa agtgagctag tggcagcata aaaagaccag cagatgcccc acagcactgc    1020

tcttccagag gcaagaccaa ccaagatgag gtgggtccac agctttccct cctgcctttc    1080

ctctggttct ttatttcagt cttttttgca tacatcggta gagatgcaga aatagaacaa    1140

agaaacgggc aaatgggcta aattatagtg aaccaaaggg cttagtgtgt taaatcttct    1200

ccttttctgc atccatagaa gacagtgctg ctgtctttcc caggagataa gatttactct    1260

caggagtgtc tttttccttc aggttacatt tttgacttta tagggtatgt catcagctcc    1320

cgtggtaggc ttcctggcat cctgagtata tttattagca gatattttcc tctttaaaaa    1380

tgtacaataa ggaagactaa tagtaacaca tttgaatgac acaattaatt gactagtacc    1440

tgggatacac actaatacct gggatacatc taattaaggc acttagatct tataaaaata    1500

aacacttttt gaaatgttga aataataaga ctagaaactt tttttttttt tgagatggag    1560

tctcgctttg tcaccaggct gcagtgcagt ggcatgatct cggctcactg caacctccac    1620

ctcccaggtt caagtgattc tcctgcctca gcctcccagg tagctgggac tacaggcgtg    1680

cgccactatg cccacctaat tttgtatttt ttagtagaga cgggctttca ccatgttggc    1740

caggatgacc tcgatctctt gacctcgtga tctgcctgcc ttggcctccc aaagtgctgg    1800

gattacaggc atgagccacc gtgtctggcc tagaaactat tttaatagaa gcaagtagtg 1860

cccgaatggt tggcatttgt tagtgagatg gtgaactggc agacggcacc tgtgggtcaa 1920

tgcccatggc caccgtcctg cttttggaca ccagttctct tccaggtaac cttctggcat 1980

tttggggttt cagataccat ttcctaaagg tgaattatta taaaatacta gaaataccca 2040

cgtgtttaaa attatatatt taaggaacct tttattacgg aaaatatcaa gaagtagagg 2100

aatagcacag taagcccaaa gttcccatct ctgagttatc tacattttat taccactatc 2160

tttgcggtgt ctgagggagg tttctctttc ctggagggct cctgtattat tgccaatgta 2220

ctttcctgaa tgcagccaga aactgagccc acccctccac ctatgtgcct ttctatccct 2280

ctctgaagct tctgcagaat tcccagcagg acagggcttg ctggaagctt ggtatgctca 2340

gaagctgcta aagtgtgtat gggcaggtgt gggggcaatt tcttggtcct agcacttcca 2400

tatatcgact ttcttttctg gctgctaagt gggaggagtg tgtgtgtatg catgtgtgtg 2460

tgtgtgtgtg tacatgcatg tgtgtgtgga tgcatgcatg tgctgtgaag cagggagact 2520

agctttccac tcctccttgt cttctctctg cagtgccctg ggagctgtca ttgccctcct 2580

gctctgggga cagctttttg cagtggactc aggcaatgat gtcacggata tcgcaggtca 2640

gtctttggtt gggtaggagt gtgcatccca ctctgaccct ctcgggtctg cactctctct 2700

gagaacaccc aattccccct tcttatctcg acctctgggc tttcaggacc ataaagaaca 2760

ttggggttcc tgccagaaat gaggggagct tgcctttcca ttggcttcta ttcggggtgg 2820

aaggagattg atgtgcagag cagctcccgc tcatctgact tttcacggtt cactgggaac 2880

aatttccaaa tagcaaactc tctggcttct ctctctttgc agatgacggc tgcccgaagc 2940

cccccgagat tgcacatggc tatgtggagc actcggttcg ctaccagtgt aagaactact 3000

acaaactgcg cacagaagga gatggtaaga tgtggacaac tgtctccatg ccctacatac 3060

aacccccttc tctgacattt ccatgatggg tggtgctgag gtgattcgcc agaaagttcg 3120

ttgctctcct tggagccagg agatttagat tctaataagc gttttgtcgc cagtagccat 3180

ggccctttgg gcagactaac ttttgtcagc ctcaagtttt ctgttttgtt aaggggaggc 3240

gatgccatgc agcctacctc atgtaaatct cagagtcaga tttacatctc cagcagatgt 3300

gggaaaagaa ggaatgctga tgatgatgtc accctcacct agtgagtctt gctgtcctgg 3360

cactgctcta agggctttat acttatttgc tcacttagtc ctcacagtat ccctctgaac 3420

agagtttatt gttttcactt tgctgataag gaaactgagg cacagacagg ttgagtatct 3480

tgcccaaatt caggcagcct gtaagaggca gagtcaggat ttgaaccctg agccctccct 3540

gtactgcttg gctgtgaccg ccatgaccac agtgtgttct gctgggctta actggtgtcc 3600

aggcacttgg cttccagcac agcactcttt cccttcctcc ttctcatatt ctctctcctt 3660

tctcccttcc tgtctgcctc ctttcttctt cttcttttta attcttctcc ttaaatgcct 3720
tctcactctg ctctgggtgc agacttgact tttcctttgg ctcatttctt gccttttgtt 3780
tcaggagtat acaccttaaa tgataagaag cagtggataa ataaggctgt tggagataaa 3840
cttcctgaat gtgaagcagg tgggtgctga gcactgagca cttaagagag caggcaggcg 3900
tccagcgggg aacgtcctag aggcacagcc ttccagtgcg gcttcctctg agcacacaag 3960
agccaggagg agggatgtgg gagaaccgca gctggccagg gagagactta agcagttagg 4020
tgatgactcc ctaagggtca ccaagggtct tgttcattgg ggcctgaagg gcactggctg 4080
aatccactgt cggcactgcc cacagatcag gagagcctgt gcatacagag agcctgctag 4140
aaagccctgg gtctaaggag aagcaagctc cagggagaac aagtcaagga atgacataaa 4200
atcttaatcc atggaagcct agcaggaggc tggacatggg ctggaactcc tgcttctcgt 4260
tattaggagg agctgttgct ctctcctttc attctcagaa ccagaggcaa agacccagcc 4320
tcttctgctc ttactggtgt ggaaatgcca acctgcctcg tattaactgc accatctaca 4380
aaatctgagc tccagccagt gctgctctag attcatcttt ctttagagag aatgaattat 4440
tgtagcccct agccctttca atgaatttca gggaattgtg gaaattcctt tattgggata 4500
attgtttaaa tataatacag ttcgcgagct tctattcggg gtggaaggag attgatgtgc 4560
agagcagctc ccgctcatct gacttttcac ggttcactgg gaacaatttc caaatagcaa 4620
actctctggc ttctctctct ttgcagatga cggctgcccg aagccccccg agattgcaca 4680
tggctatgtg gagcactcgg ttcgctacca gtgtaagaac tactacaaac tgcgcacaga 4740
aggagatggt aagatgtgga caactgtctc catgccctac atacaacccc cttctctgac 4800
atttccatga tgggtggtgc tgaggtgatt cgccagaaag ttcgttgctc tccttggagc 4860
caggagattt agattctaat aagcgttttg tcgccagtag ccatggccct ttgggcagac 4920
taacttttgt cagcctcaag ttttctgttt tgttaagggg aggcgatgcc atgcagccta 4980
cctcatgtaa atctcagagt cagatttaca tctccagcag atgtgggaaa agaaggaatg 5040
ctgatgatga tgtcaccctc acctagtgag tcttgctgtc ctggcactgc tctaagggct 5100
ttatacttat ttgctcactt agtcctcaca gtatccctct gaacagagtt tattgttttc 5160
actttgctga taaggaaact gaggcacaga caggttgagt atcttgccca aattcaggca 5220
gcctgtaaga ggcagagtca ggatttgaac cctgagccct ccctgtactg cttggctgtg 5280
accgccatga ccacagtgtg ttctgctggg cttaactggc atccaggcac ttggcttcca 5340
gcacagcact ctttcccttc ctccttctca tatactctct ccttttcccc ttcctttttg 5400
tccccttttc ctcttccttt tagttcttct ctttaaatgc cttctcactc tgcacggggt 5460
ctagacttga cttctccttt ggctcacttc ttgccttttg tttcaggagt gtacacctta 5520
aacaatgaga agcagtggat aaataaggct gttggagata aacttcctga atgtgaagca 5580

ggtgggtgct gagcacttaa gagagcaggc aggcgtccag cggggaacgt cctagaggca 5640

cagccttcca gtgcggcttc ctctgagcac acaagagcca ggaggaggga tgtgggagaa 5700

ccgcagctgg ccagggagag acttaagcag ttaggtgatg actccctaag ggtcaccaag 5760

ggtcttgttc attagggcct gaagggcact ggctgaatcc attgtctaca tcgcccacag 5820

attaggagag cctgtgcata cagagagcct gctagagagc cctgggtcta aggagaagca 5880

agctccaggg agaacaagtc aaggaatgac ataaaatctt aatccatgga agcctagcag 5940

gaggctggac atgggctgga actcctgctt ctcgttatta ggaggagctg ttgctctctc 6000

ctttcattct cagaacaaga ggcaaaggcc cagcctcttc tgctcttact ggtgtggaaa 6060

tgccaacctg cctcgtatta actgcaccat ctacaaaatc tgagctccag ccagtgctgc 6120

tctagattca tctttcttta gagagaatga attattgtag cccctagccc tttcaatgaa 6180

tttcagggaa ttgtggaaat tcctttattg ggataattgt ttaaatataa tacagttcac 6240

cagccagggc tcaaaaatct cagtatttcc cacttccttt gttagaaaag tgggaaatag 6300

agctttttgt aatgtaaaca atttaaaaaa cagaattatt ttaaaactgc aactattgga 6360

aatgagatca gcaggtggta agggcaaagc atttaaatct ttctacttta cgcagcagtg 6420

acagccgccc atgctttcac ccctttctca gatggaaagg ctcttgcaca tttccactca 6480

cgagtgtctt gctctccttg acagtatgtg ggaagcccaa gaatccggca aacccagtgc 6540

agcggatcct gggtggacac ctggatgcca aaggcagctt tccctggcag gctaagatgg 6600

tttcccacca taatctcacc acaggtgcca cgctgatcaa tgaacaatgg ctgctgacca 6660

cggctaaaaa tctcttcctg aaccattcag aaaatgcaac agcgaaagac attgccccta 6720

ctttaacact ctatgtgggg aaaaagcagc ttgtagagat tgagaaggtt gttctacacc 6780

ctaactactc ccaggtagat attgggctca tcaaactcaa acagaaggtg tctgttaatg 6840

agagagtgat gcccatctgc ctaccttcaa aggattatgc agaagtaggg cgtgtgggtt 6900

atgtttctgg ctgggggcga aatgccaatt ttaaatttac tgaccatctg aagtatgtca 6960

tgctgcctgt ggctgaccaa gaccaatgca taaggcatta tgaaggcagc acagtccccg 7020

aaaagaagac accgaagagc cctgtagggg tgcagcccat actgaatgaa cacaccttct 7080

gtgctggcat gtctaagtac caagaagaca cctgctatgg cgatgcgggc agtgcctttg 7140

ccgttcacga cctggaggag gacacctggt atgcgactgg gatcttaagc tttgataaga 7200

gctgtgctgt ggctgagtat ggtgtgtatg tgaaggtgac ttccatccag gactgggttc 7260

agaagaccat agctgagaac taatgcaagg ctggccggaa gcccttgcct gaaagcaaga 7320

tttcagcctg gaagagggca aagtggacgg gagtggacag gagtggatgc gataagatgt 7380

ggtttgaagc tgatgggtgc cagccctgca ttgctgagtc aatcaataaa gagctttctt 7440

ttgacccatt tctgtgttgt gttcagtctt gagtcttttt tatttgctcc tttatggtcc 7500

agggtagtca gaaggtatag agtctactgg gagtatggca gaaaacaccc taaacccact 7560

ggaaatcccg aaggtgatac aaactcttcc accttaggga atcatgctca ctgattgagt 7620

gcctattgaa tgctaggtcc cagaaagtta actgttgtcc ttgttttaca gacaaggaaa 7680

cagagactca gagatggtaa gtgagttgct taaggttaca tagctatgaa acagggaagc 7740

agaactttga acccaggtct gtttgataca aactcagagg tcctttcact gcatgctgtt 7800

gcctcctcaa agtgaattag gagaaaaggc atgggcctgg ttgaggaaga ggctagctca 7860

aaatgggatg ggaaaaaagt gttttaacac agacagtaat tcagagtttg ggatctaacc 7920

taccagcact ccagaaaaca aaagacaatg atgtaaaggc aggatctctg gacttactga 7980

gtccaaatca cagaatggcc cataaatttg ctaggtaatt taggtttcct ggccatcagt 8040

tcctggtctc ttccatgaag gacttgagcc aggcgatggc taatgtccct atcagcaata 8100

accttctggt attccataaa gagtggggag gtagagtccc tgcatcagta gaagctgctt 8160

ttcctactgg gaacaaaccc accctgcacc aggaatcaaa tgcaggctgt ctgagcctcc 8220

tctcctgatg gcaggtcatg ctattaaggg tctcccagaa gaaaggttct ggaactctga 8280

gaaacagaag ctcttctaac agaacttttt cttgctataa gctttcaata ccaccaatca 8340

ccagtgcacc ttcttctcat gccaaaatac ggcccctcct ttgtagggtg taaaacgaca 8400

ctgatgtcac tctctctcac caaggtctgg ccatctctgt gaagctctcc tcacaggcac 8460

cctgcagatt catcaataaa cttcaacagg ttagcgccag taggccaata agatgcacac 8520

ttatattcta gaacctgtga aagaagtaat agccagaaac tggaaaataa cagtcaaact 8580

cttaaaaaaa tgcctctcag aacacagtca cttacacctt ttcctctggt agttctcttc 8640

agcaccaagt ttcctggaag aacaagctac tccccagttt tcttaaagaa ttgtctgatt 8700

gtattaagta agtggcttct ggtaaaataa tggtaagaga aaagctctgg cattctcttg 8760

atcactcatc ttctctgcct tcctccctag caacatctcc tggcctcagc ctgtgggctt 8820

cagcaacacc aaactccatg ttgctctctg acaagcccgg ccgtctccag ttcttgggct 8880

ttatttcctc gggggccctc tgctgaaatt cccttcccct tgctgtatta gcacctccta 8940

cccgccttca acatgcaggc actgcctcct ctaaggagcc atctgtactt acgtagctgt 9000

gagcatagga tggggcatac agcaggcact taacaaatac ttgtggaatg gaaagaaact 9060

aagataggat caaaaataca aatcaagatg tgagagaaag acgatcaaaa gccctcacga 9120

gggggtcggg cgtgatggct cacacctgta atctcaacac tttgggaggc taaggcgggt 9180

ggatcacttg agggtgggag tttgagatca gccagagcaa catggagaaa cctcgtctct 9240

actaaaaata caaaaaatta gccaggcatg gtgatatatg cctgtaatcc cagctactcg 9300

ggaggctgag gcaggagaat tgcttgaacc tcggaggtag aagttgtggt gagctgagat 9360

catgccattg tactccagcc tgggcaacag gagtgaaact ctgtctcaaa aaaaacaaaa 9420

aacaaaaaag aacaccacgg gaaaatcaag tgtgaagcaa gagctcaact cttaacaggg 9480

gcaatgtttg gagttttgtt actggaaaag atagagacct taccaggtcc aaagtttgta 9540

gacacaggaa ttacgaaatg gagaaggggg agaagtgagc tagtggcagc ataaaaagac 9600

cagcagatgc cccacagcac tgctcttcca gaggcaagac caaccaagat gaggtgggtc 9660

cacagctttc cctcctgcct ttcctctggt tctttatttc agtctttttt gcatacattg 9720

gtacagatgc agaaatagaa caaagaaaca gggcaaatgg gctaaattat agtgaaccaa 9780

agggcttagt gtgttaaatc ttctcctttt ctgcatccat agaagacagt gctgctgtct 9840

ttcccaggag ataagattta ctctcaggag tgtctttttc cttgaggtta cgtttttgtc 9900

tttgtagggt atgtcatcag ctcccgtggt aggcttcctg gcattcggaa tatatttact 9960

agcagatatt ttcctcttta aaaatgtata ataagaaaga ctaatagtaa cacatttgaa 10020

tgacacaatt aattgactag tacttgggat acacactagt acctgggata catctaatta 10080

agacacttag atcttaaaaa ataaaaagac tttttgaaat gttgaaataa taagactaga 10140

aacttttttt tttgtgagac agagtctcac tctgtcacca ggatggagtg agtggcgcga 10200

tctcagctca tcgcaacctc cccctccccg gttcaagcaa ttctcctgcc tcagcctccc 10260

aagtagctag gactacaggc gtgtgccacc acgcccagct aatttttgta tttttagtag 10320

cgatgtggtt caccatgttg gcccggatga tctcgatctc ttgacctcgt gatccacctg 10380

tctcggcctc ccaaagtgtt gggattacag gtgtgagcca ccgcaccctg cctagaaact 10440

attttaatag aagcaaatag tgcctgaatg gtgggcgtct gttagcgaga tggtgaactg 10500

gcagatggcg cctgtgtgtc aatgcccatg gccaccgtcc tgctttcaga caccagttct 10560

cttccaggta accttctggc attttggggt ttgagatacc atttcctaaa ggtgaattat 10620

cacaaaatac tagaaataac cacataagtg tttaaaatta ttgttaaata cagtaagaaa 10680

ttcttcttca aaagtttagc ctgcttaagt ttccttgtcc tttgtttcct gctttcaagg 10740

ccagacttcc ttactctctg tgtttcccct accctggtaa acaaccttcc tgccagtcct 10800

tacccataca gcccacattc cacatctgct acccactctg tgatttacct ctcccgtcgc 10860

aatagcctct cccaccaaaa ctgatcttcc cgccttccca ccagtgcaac cacattcctg 10920

cactttgcaa gttagccaac cgggttcgga ttgtgcagtc caactccagc caatggagtc 10980

aggacacagt agcagggaca agctgcgtta gacataaaaa cctctgcttt cctttgttta 11040

gggtgctctc gtggcaacca gacttaccag gagctctatt ctgcaaaagt aaatttgcct 11100

tgctgagaga ccctttgtcc tttggctcag tgttggttct tctttgcagc accgagcatt 11160

tgtttccaac aaatttggtg gcccatacag ggaaaacatt gtcctccggg aaagggttct 11220

ttgatcatcc tcttgagagg agaacacatc ccactgtcct tgttgcggtg gcctcatgag 11280

taggaatcga gacccacctg tctgatgaat aaccccagac tctcaacaac gtggggagaa 11340

aaagacttgc aacactatgg tggccaggta actctgtgcg cagaccaagg taagaaatgt 11400

cgcaggagtg acaaagtact tccttggtgg tcactatatt ctggtggctg aaagttcatg 11460

aatggtaaca agtgctactg ctgtgtggag tgaatgagtc caatctgtgg gtctatggtt 11520

acctcatacg gcttagcctt ctctgaagga tcctgatgtt ggggtttata tagtcctccc 11580

aatgctaagc gggacttaaa atattcctag gaggaaagtg gccagagtgg atgaagcaaa 11640

aggagaagag tgtgaagaac ctccaggagg tggggctaaa agataggcaa gaaatctcta 11700

atacgaggga ttgagccaca gaaagctcca gacagataaa aaagaaattc ctaatatgag 11760

gaactgagcc acagcaagcc tccagcaggc aacaaatccc taatatgagg gattgagcct 11820

agctaagacc caatatggga aataccccaa gaaagacaga gaataagaag gatgaaaata 11880

gtaacaagga tatacccctt gatagtcccc taggtttcat gttaaaatac tggaaagata 11940

atgagaggac taagcataag aagaaggagc aaatgataaa atattgctgt ttcatttgga 12000

cccaaggtcc catcctcaaa ccctcaatct tctggccaaa gtatgggtcg aatgagggtg 12060

taatgagtca actcctaatc caatatgtta atgataaaag tctggtttct caagaagaac 12120

tagactatgc tctttgttgg aagcagggac ctgtcctcct ctttccctta aagacaacta 12180

gggaagaatc cgatccagca tctcaaattg agaagtcaga caagccgact cccacaccta 12240

aagtcagcac atgggatccc ctagactgtc ttcctttgct tactgcccca atcctaaccc 12300

ccctactcct caggcagctg ctgctgcccc agatcccatc ccagattctt ccccaactca 12360

tgatgttcct cccgcttaca accctgactc tcaggggcag tcgtcccaag agcctgttca 12420

ttgccaacct aaatatcctt ccttaaaagg gctccaacat gaaatagagc agtgtaaaaa 12480

ggacattcag aactcccctt ttctttccac acctaaggag tcagccataa ctttcttccc 12540

tttaaaaggg gtatcacaag gaggggaagt cattggtttt gtaaatgctc tcttgactat 12600

ttcagaagtc tgaggtctga agaaagaact taagccactg ctagatgacc cctatggagt 12660

ggcagatcaa gttgatcaat tcttagggac ctcagttata cacttgggtc gagcaaatgt 12720

ctatcctagg ctcttctttt caggggagga aagaaagcat gatccgtagg gctgctatgg 12780

caatttggga acatgaacac cctcctggtc aaaacgttcc tactgtggac caaacatttg 12840

ctgcccaagc aagactcctg gtgggacaac agcaatgcag cccactgaga aaacatgcag 12900

gacctaaggg aaataataat aaaaggaatc agtgaatcca tacccagaac tcaaaagctc 12960

tctaaagcat ttgatataca acaggagaaa gatgagggac ctataagatt cctagacaga 13020

ctaaaggagc aaatgaagca atatacaggt ctgaatttag aagatcccct tgggcaaagg 13080

atgttaaaga tccattttgt cactaaaggc tggccagatg tttcaaaaaa gttacaaaaa 13140

ttggaggact gagaaaaccg acctctaaga gaacttctca gagaagctca aaaggtgtat 13200

gtgaggaggg acagggaaaa acaaaaacag aagtcaaaac caatgttatc tactttccag 13260

caggtggctc caaacccata tgctactaaa tgaggcttcc agggagccag aaactataaa 13320

aggtcccaag cctcccaaac ccagtttaga gaaaccaaac cttcagctag aggacccaag 13380

tctacatttc ccaggccccc taaagagcat agaaaagcaa gaccaaaaaa tcccaaaact 13440

gagagagggg aaggacaaga taagtgttac aaatgtggaa ggacagcccc cttcaaaaga 13500

gaatgtccca aattagaaaa ggagagagaa gcccttccac tcacgacctt tgaagaggaa 13560

cagggaagtc aggggctctg tctatattat cttgagtccc accaggagcc cttgataaat 13620

ttggaggtgg gacctacaca tgagcttatc acatttttgg ttgattcagg agcggcctgt 13680

tcctctgttt gtttcccctc atctaatgtt gcctgctctt cagaagaact tatagtctct 13740

gagataaaag gggaaggatt tacggtgaga atcttagaaa atacagaagt caagtaccaa 13800

gactaaacaa cccaggttca atttttgtta atccctgaag cagcaactag tttgttagga 13860

agagacttaa tgttaaagtt aggcataggc ctacaagtca gcccaaaggg attccttact 13920

tcattaaact tactcaccac ggcggatgag aaatacattc atcctgatgt ttggtcaagg 13980

gaagaaaact gaggaaagct tcgaattctc ccaatccaca tcaagctaaa caccccgcac 14040

tgggaagtag tgaggaggaa gcaattcccc attcccttag agggcatgct agggctaaaa 14100

cctataattg aaagtctcat taatgatggg cttcttgaac cctgtatgtc tccttataac 14160

accccaatac tgcctgtcaa aaaatcagat gggtcatacc ggctggtgaa agacctcaga 14220

gccattaacc aaacagtcca gaccactaac cctgttgtcc ccaaccctta caccattctc 14280

agcaaaattc catataatca tcaatggttt actgtaatag atttaaagga tgcttttggg 14340

gcatgtccct ggctgaagag agccgagaca catttgcctt tgagtgggaa gatccccagt 14400

tagggtgaaa acaatggtat caatggacag tcttgcctca ggggttcatg gattcaccca 14460

accttttggg tcaaatttta gaacaagtgc tagacaaagt ttctgttcca aaacaattat 14520

gcctgcttca atatgtcgat gatattctca tatctggtga ggatatagag aaagaagctg 14580

gcttctctac acatattttt gaccatctac agttcgaggg gttacgggtc tcaaaggaaa 14640

agcttcagtg tatggaacct aaagttaaat atttaggccg cttaataagt gcgggcaagc 14700

gaaggatagg gcctgaatgg gtcgaaggaa tcgtgtcctt acccttgcct cagactaaac 14760

aggaactcag gaaattttta gggttagttg gatactgccg cttaaggatt aactcacatg 14820

ccctaaacag taaactttta taccaaaaac ttgcccaggg aaaacctgag catctcctgt 14880

ggacttctaa agaggtcgat caggtcaaag agctaaaagg aatagcttat aactgctctt 14940

gccctagcct taccttccct agaaaataca cttcaccttt tcgtcagcgt gaaaaatggg 15000

gtggctttag gggtgcttat ccaagagcac agaggctgct ggcagcccat ggccttcctg 15060

tcaaaaattt tggacctggt cacctgtgga tggcctcagt gcatccaatc cattgcagct 15120

acagcagtat tagttgaaga gagtagaaaa ttaacctttg ggtggagatt aacagtaagc 15180

acaccccacc aagttagaga gctattttaa ataaaaaagc aggaaggcga ctaactgact 15240

ccagaatctt aaaatatggg gctattctac taaaaaaaga tgagagaaca cctatgtcta 15300

gatttaattg actaccaaac aaaactcagg ccagatctag gaaagatccc tttcaaaaca 15360

ggacggcact tatttataga tggttcctcc cagctgattg agggaaaaag acacaacggg 15420

tatccagtaa tcgatggaga aattctctat gtataagaac agagtcaaga aaattgccta 15480

ataattggtc tgcccaaact tgtgaactgt ttgcactcag ccaagcctta aagcacttgc 15540

aaaaccagga aggaaccatc tatactgatt ctaagtatgc ctttggagtg gctcatacat 15600

ttggaaaaag ttggactgaa tgtggcctca ctaatagtaa aggtcatgac cttgttcata 15660

aggagttaat catccaagta ctggataacc ttcagttgcc agaagaaata gctattgtcc 15720

gtgtacccgg gcaccagaaa agcctttctt ttgaaagtcg aggaaataac ctaacaaacc 15780

agatagccaa acaagctgct gtttcctccg aaacacctat gtttcactta actccttgtc 15840

ttccttcccc tactgcaatt tcctttttct cttccattga aaaagaagaa taaagatagg 15900

agccaaagga gaagaccaga atgaaaacgg ctgttactag accaaaagga aatgttatcc 15960

aagcccgtta tgtggaagat ctagtctcaa ctatatctga ggacacactg gggatcccaa 16020

gccatgtgcg atgcagttct cagggtctat ggatgtataa gaatttacac cctagccaaa 16080

caagttacag atagttgctt aatatgtaaa aagactagta agcagattct aagaaaaccg 16140

ccccttggag aaagagattc agggctaaga ccatttccaa gtgttcaatt aattatactg 16200

aaatgccccc aattggtcat ttaaaatact tattagtaat aatagaccac tttacctact 16260

gggtagagac tatcccacac tcaaatgcaa ccaccagtaa cgtagttaag gcattaattg 16320

aaaacattgt acccagattt ggactaatac aaagcactga ttcagacaat ggaacccatt 16380

tcactgcata tgtcattaaa aagttagccc aggtactaga cataaaatgg aaaaaccata 16440

tcccttggca tctctcctcc tcaagaagag tagaaaggat gaatcagact ctaaaaagcc 16500

acttaactaa attagttcta gaaacttgat tgccatggac taaatgtctt cctattgcct 16560

tgttaagaat ccgaactgct cctcagagag atactggcct ttccccttat gagatgctct 16620

atggattgcc ctatttatac tccactgcta acattcctac attcaaaata aaagatcagt 16680

tcctcaaaaa ttatatactt ggtccatact ctactttctc ttcccttaag actaaaggtc 16740

tcctagcaca ggcgccaccg ctggagtttc cagcacatta gcgtcagcct ggagaccatg 16800

tcctcataaa agggtggaag gaaggcaaac tcaaaccagc ttgggaagga ccctacttgg 16860

tgctcctaaa tactaagact gcagtccgaa cagcagaatg aggatggact gatcacaccc 16920

gcgtcaaaaa ggcgctgcca cctccaggat cataaaccgt cactccaggg ctcaccccaa 16980

ccaaattaac tctaaaaagg gcttaataat cacttgttta tttttctttt tctttccaac 17040

agaaggtcat cttgtcatca atgtaacttg ggctaaccat cctttaatcc ttcagtttga 17100

tgcttgttca gtcatcctgt gtggagacaa gcaagctcaa aggaagctgt ctcatgtaga 17160

taagtaccta tgtccatacc ataaaaagtc aaccaagtat aagtatagaa ccttaaaaag 17220

tccctgtggt gactggacag atgtttggaa accactcagt atggagggtg gacagccagg 17280

cccccttttc aaataagtta tggggactaa aacagaaact ccaactaatt catggtccca 17340

ccccaccaaa ctgtaagcca ctgcagtgta acccctcatt gctaattata gctaaccccc 17400

aaacaatggc ccaagaaccc actatattca aacagtatgg attaggagca aatgttacaa 17460

aacaaaatcc cataaaaatc ttcaaccact ggttaacaat gataattaaa aaaaaatcca 17520

cacccagagt ctgggaaacg agtggaatct aacactctct ccaagtatag cgagctcagc 17580

gtcctcctcc catctccaaa acaacccaac taaagtaacg gttgtaaagg taaaaaattt 17640

aaagcagact atagcccaac taaagtaacg gttgtaaagg taaaaaattt aaagcagact 17700

atagccctag agacaggata tcaagatgca aagtcttggc taaaatggat taaatattcc 17760

atctgcactt taaacaaata tgaatgttat gcttgtgctc acggtagacc agaggcccag 17820

attgtcccct ttccactcag atggtcttct aactgaccgg gcataagctg tatggtagtt 17880

ctcttccaag atcacacagc ctggggtgac aaatcatgtc aagctctttc tctgctgttc 17940

cctgaagctc aacaccctga gggtcagccc ccgagggcca tcagcttccg tttcccaatg 18000

ccaattttac tttgtgtctc tcacaacagg gagaaaactt ggtgttcctt ggagccttaa 18060

tgggatgcag tgagcttaag tccttccaag agcttaccca tcagtctgcc cttagtcatc 18120

ctcgagcgga tgtatggtgg tattgtggtg gaccattact ggacactgcc gagtaactgg 18180

agcagaacat gcactctaat tccattggct atccctttca ccctggcatt tcatcaacca 18240

gaaaagatag aaaccaaaca ctgtaaaaca agagaggccc ctcatgggtc tgttgactcc 18300

cacatttata tagattccac tggagtccaa cgaggagtgc cagatgaatt taaggcccaa 18360

aatcagacaa ctgcaggatt tgaatccacg ttcttctggt ggttaactat aaataaaaat 18420

gtgaattaga taaactacat ttattacaac caatgatgat tttttttttt ttgacggagt 18480

tttgctcttg ttgcccaggc tggaatgcaa cggcatgatc tcggctcacc acaacttctg 18540

cctcctgggt ccaagcaatt cttctgcctc ggcctcccga gtagctggga ttataaacat 18600

gtgccaccac gcctggctaa ttttgtattt ttagtagaga caggtttctc ccatgatggt 18660

caggctggtc tcaaactccc gacctcaggt tatctgcctg cctcggcctc ccaaagtgct 18720

gggattacag gcatcagcca ctgtgcccag cctatgattt gttaattata ctagagatgc 18780

tcttaaaggg acagctgaac aattaagacc caccagtcaa atggcctggg aaaataaaat 18840

agcattagac atgataccag cgaaagaagg tggagattgt gtcattatcg gaacccgatg 18900

ctatactttt atccctaata atactactcc aggtgggatc acaccaaaag cactacaagg 18960

tcttaccgcc ttatcaaatg agctagccaa aaattctgga ctaaacgacc ccttcacaaa 19020

tttaatggga aattctttgg caaatggaag ggatttatgt cctcaatcct catgtctctt 19080

gccatcagaa tagggttgct tattcttgta ggatgctgtg tcataccctg tgccctagga 19140

ttaatacaaa agctcattga aacagctctc cccaaaacct ccctcaatcc tcctcctcct 19200

cgttccagtg agctttttct tttagaagac ccaagtagaa taacagagcc aaattatgtt 19260

aaaagggttt aagaaaaaat actgtaaaat atttaacagg gggattrtgt taaatacagt 19320

aagaaattct tcttcaaagg ttcagcttgc ttaagtttcc ttgttctttg ctccctgctt 19380

tcaaggccag acttccttac tctctgtgtt ctccctgcgc tggtaaacaa ccttcccgcc 19440

agtcgttatc tatagagccc acattccaca tctgctaccc actctgtgaa ttacccctcc 19500

catcacaatg gcttctcccg gcaaaactga tcttcctgcc ttcccagcag tgtaatcaca 19560

ttcctgcact tttcaagtta gccaaccggg ttcagcttag attgtacggt ccaactctag 19620

tcaatggagt caggatacag tagcagggac aagctgcgtt agacataaaa acctctgctt 19680

tcctttgttc ggggtgctct cgtggcaacc gaacctatga ggagcaacct tatgcaaaag 19740

taaatttgcc ttgctgagag accctttgtc ctttgtctca gtgctagttc ttctttgcag 19800

caccaagcat ttgttttcaa caattatata tttaaggaat ctttgattat ggaaaatttc 19860

aagaagtaga gggaatagca tactaagccc tctgttccca tctttgagtt atctacattt 19920

tattagaagt atctttgggg tgactgaggg aggtttctct ttcctggagg gcttctgaat 19980

tattgccaat gtaccttcct gactgcagcc agaaactgag gcccctatac ctatgtacct 20040

ttctatccct ctctgaagct tctgcagaat tcccagcaag acagagcttg ctggaagctt 20100

ggtatgctca gaagcagcta aagcgtgtat gtggggcgga gggtgggggc aacttcttgg 20160

tcctagcact tccatatatt gatttctttt tctggctgct tagtgggagg agtgtgtgtg 20220

tatgcatgtg tgtgtgtgcg tgtgtgtgtg tgtgtacatg cctgtgtgtg tggatgcatg 20280

catgtgctgt gaagcaggga gaccagcttt ccgttccttc ttgttttctc tctgcagtga 20340

cctgggagct gtcatttccc tcctgctctg gggacgacag ctttttgcac tgtactcagg 20400

caatgatgtc acggatattt caggtcagtc tttgagttgg gtaggagcat gcatccctgg 20460

cactgccaca tcccactctg actctctcgg gtctgcattc tttctttgag aacacacagt 20520

tccccattct tatcctgacc tctgggcttt caggactgcc aagaacattg gggatcctgc 20580

cagaaatgag gggagcttga gctttcgttg gcttctattt ggggtagaag gagattgata 20640

tgcagagcag cttccactca tctgactttt catgggtctc tgggaacaat ttccaaatgg 20700

taaactctct ggcttctctc tctttgcaga tgaccgcttc ccgaagcccc ctgagattgc 20760

aaatggctat gtggagcact tgtttcgcta ccagtgtaag aactactaca gactgcgcac 20820

agaaggagat ggtaagacct ggacaactat ctcctgtgct ctacctacaa cccctgctct 20880

gacatttcca tgatgggtgg tgctgaggtg atttgccaga aagttcgttg ctctccttgg 20940

agccaggaga tttagattct gataagcgtt ttgtcgccag tagccatggc cctttgggca 21000

gactaacttt tgtcagcctc aagttttctg ttttgttaag gggaggtgat gccatgcagc 21060

ctacctcatg taaatctcag agtcagattt acatctccag cagatgtggg aaaagaagga 21120

atgctgatga tgatgtcacc ctcacctagt gagtcttgct gtcctggcac tgctctaagg 21180

gctttatatt tatttgctca cttagtcctt acagtttacc tctgaacaga ggttattatt 21240

ctcactttgc cgataaggaa acagaggcac cgacaggttg agtatcttgc ccaaattcag 21300

gcggcctgta agaggcagag tcaggatttg aaccctgagc cctccctgta ctgcttggct 21360

gtgaccgcca tcaccacagt gtgttctgct gggcttaact ggtgtccagg cacttggctt 21420

ccagcacagc actctttccc ttcctccttc tcgtattctc tctcctttct cccttcctgt 21480

ctgcctcctt tcttcttctt ctttttaatt cttctcctta aatgccttct cactctgctc 21540

tgggtgcaga cttgactttt cctttggctc atttcttgcc ttttgtttca ggagtataca 21600

ccttaaatga taagaagcag tggataaata aggctgttgg agataaactt cctgaatgtg 21660

aagcaggtgg gtgctgagca ctgagcactt aagagagcag gcaggcgtcc agcggggaac 21720

gtcctagagg cacagccttc cagtgcggct tcctctgagc acacaagagc caggaggagg 21780

gatgtgggag aaccgcagct ggccagggag agacttaagc agttaggtga tgactcccta 21840

aggatcacca gggtcttgtt cattggggcc tgaagggcac tggctgaatc cactgtcggc 21900

attgccacag atcaggagag cctgtgcata cagagagcct gctagagagc cctgggtcta 21960

aggagaagca agctccaggg agaacaagtc aagggataac ataaaatctt aatccatgga 22020

agcctagcag gaggctggac atgggctgga actcctgctt ctcgttatta ggaggaactg 22080

ttgctctctc ctttccatct cagaaccaga ggcaaaggcc cagcctcttc tgctcttact 22140

ggtgtggaaa ggccagcctc ttctgctgtg agtggtgtgg aactaccaac ctgcctcgta 22200

ttaactgcaa catctataaa gtatgagctc cagccaatgc tgctctagat tcctctttct 22260

tcagagatga tgaattattg tagctcctag ccctttcttt tttctttctt tctttttttt 22320

tttttttgag acagagtttt gctctcgtcg cctaggctgg agtgcagtgg tgctaattcc 22380

tgacctcagg tgatccaact gcctcagcct cccaaagtgc tgggattaca ggcgtgagcc 22440

accgcatctg gcccctagcc ctttcaatga atttcaggga attgtgaaaa ttcctttgtt 22500

gagataattg tttaaatata atatagttca ccagccaggc ctcaaaaaac tcagtatttc 22560

tcatttcctt atttagaaat agagcttttt gtaatgtaaa caattaaaaa aattatttta 22620

aaactgcaac tattggaaat gagatcagca ggtggtaagg acaaagcatt taaatctttc 22680

cagtttatgc agcagtgaca gccgccaatg ctttcacccc tttctcagat ggaaaggctc 22740

ttgcacattt ccactcacga gtgtcttgct ctccttgaca gtatgtggga agcccaagaa 22800

tccggcaaac ccagtgcagc ggatcctggg tggacacctg gatgccaaag gcagctttcc 22860

ctggcaggct aagatggttt cccaccataa tctcaccaca ggggccacgc tgatcaatga 22920

acaatggctg ctgaccacgg ctaaaaatct cttcctgaac cattcagaaa atgcaacagc 22980

gaaagacatt gcccctactt taacactcta tgtggggaaa aagcagcttg tagagattga 23040

gaaggtggtt ctacacccta actaccacca ggtagatatt gggctcatca aactcaaaca 23100

gaaggtgctt gttaatgaga gagtgatgcc catctgccta ccttcaaaga attatgcaga 23160

agtagggcgt gtgggttacg tgtctggctg gggacaaagt gacaacttta aacttactga 23220

ccatctgaag tatgtcatgc tgcctgtggc tgaccaatac gattgcataa cgcattatga 23280

aggcagcaca tgccccaaat ggaaggcacc gaagagccct gtaggggtgc agcccatact 23340

gaacgaacac accttctgtg tcggcatgtc taagtaccag gaagacacct gctatggcga 23400

tgcgggcagt gcctttgccg ttcacgacct ggaggaggac acctggtacg cggctgggat 23460

cctaagcttt gataagagct gtgctgtggc tgagtatggt gtgtatgtga aggtgacttc 23520

catccaggac tgggttcaga agaccatagc tgagaactaa tgcaaggctg gccggaagcc 23580

cttgcctgaa agcaagattt cagcctggaa gagggcaaag tggacgggag tggacaggag 23640

tggatgcgat aagatgtggt ttgaagctga tgggtgccag ccctgcattg ctgagtcaat 23700

caataaagag ctttcttttg acccatttct gtgttgtgtt cagtcttgag tcttttttat 23760

ttgctccttt atggtccagg gtagtcagaa ggtatagagt ctactgggag tatggcagaa 23820

aacaccctaa acccactgga aatcccgaag gtgatacaaa ctcttccacc ttagggaatc 23880

atgctcactg attgagtgcc tattgaatgc taggtcccag aaagttaact gttgtccttg 23940

ttttacagac aaggaaacag agactcagag atggtaagtg agttgcttaa ggttacatag 24000

ctatgaaaca gggaagcaga actttgaacc caggtctgtt tgatacaaac tcagaggtcc 24060

tttcactgca tgctgttgcc tctttaaagc gaattaggag aaaaggcatg ggccttggtg 24120

aggaagaggc tagctcaaaa tgggatgggg aaaaagtgtt ttaacacaga cagtacttca 24180

gagtttggga tctaacctac cagcactcca gaaaacaaaa gatgatgatg taaaggcagg 24240

atctctggac ttgctgagtc caaatcatgg acttggccca aacatttgct aagtaattta 24300

ggcttcctgg ccattagttc ctggtctctt ccatgaagga cttgagccag ggtaacgtcc 24360

ctatcagcaa taagcttctg ggactctgta aagagtgggg aggtagagtc cctgcctcag 24420

tagaagctgc ttttcctact tggaacaaac ccacccttca ccaggaatca aaagcaagct 24480

gtctgagcct cctctcctga tggcaggtca tgctattaag ggtctcccag aagaaagctt 24540

ctggagctct gagaaacaga agctcttcta acagaacttt ttcttgctat aagccttcaa 24600

taccaccaat caccaatgtg ccttcttctc atgcgaaaat acggcccctc ctttgcaggg 24660

tgtaaaatga cactgatgtc actctctctc accagggtct ggccatctct gtgaagctct 24720

cctcacaggc accctgcaga ttcatcaata aacttcaaca ggttagcgct agtaggccaa 24780

taagacctat attctagaac ctgtgaaaga gataatagcc agaaactggc aaacaccagt 24840

caaactcttt aaaaaaatgc ctcttagaac acagtcactt acacattttc ctagatctct 24900

tcagcaccaa gtttcttgga agaacaaggc actctgcagt tttcttaaag aattgtctga 24960

ttgtattaag tggcttctgg taaaacaaca gtaagagaaa agctctggca ttctcttgat 25020

cactcatctt ctctgccttc ctccccagca ccacctcctg gcctcagcct gtgggcttct 25080

gcaaagtcag gtatcaggac taattagcta gcatcaaatt cctgttaatt ccagaagcag 25140

ggacaaacct gttggggagg gacttaatga caaaattagg tataggtcta tacgttaatc 25200

aaggaaaatt ctcaacttcc ttaaacttac ttaccaccct agaggagagc caaatccact 25260

ctgatgacgt ttggtcatag gaaggaaatt ggggaaaatt acaagtttct ccaatccatg 25320

taaaattaag aaatcctggg gaagttgtaa agagaaaaca gtaccctatt cccttgaaag 25380

gcagaatagg tttataactt ataatcgaag gtctccttca ggaaggactt cttgaacccc 25440

atatgtcctc ttacaacact ccaatattgc ctgtaaaaaa gtcggatggg tcatagcggc 25500

tactgcgaga ccttcgagcc attaaccaaa tagtccagac cacccaccct gtcattccta 25560

acccttacac cattatcaag caagatccct catgactgcc agtggttcac agtaatagac 25620

ctcaatgatg acttctgggc ttgcccctta gctgtggata gctgggacat atttgctttt 25680

gaatgggaag accctcattc cagttggaag caatagtagt gatggacagt tctaccccaa 25740

gggtttacag actctccaaa cctattagtc aaagtttgga acaagtccta gagaatttcc 25800

ctctttcatc atccttatgt ctactccaat atgtggatga cctgctcatt tccagaggca 25860

ccaaagacca agtaaccgca attttaatta gctttctaaa tttcctaagg gaacaatggt 25920

tacgggtctc aaaaagtaaa ctccagcttg tagaacctga ggtaaaacac atggggcact 25980

taataagcaa aggtaagcgg aagatagggc ccgaacaaat ggaagggatc atatccctgc 26040

cactgcctga gacaaaacaa gaacttagaa aattcttcag gctagctgga aattattgcc 26100

tgtggattga ctcttatgcc ttaaaaacaa aacctctata tctaaaactt acccaagaag 26160

ggcctgaccc ccttctttgg accccacaag aagtccagca agttgaggaa ctaaaacatc 26220

tacttataac tgcccctgtc ttagctctgc catccttaga acagccattt cacttttttg 26280

ttaatataag caatggggca gctataggag tactcactca aaagcatggg gccattgcca 26340

gcccacagcc tttctgtcaa acattcttga cgtggtaacc tgtgggtggc ccgaatgtat 26400
ccaatctaca gcagcaactg ttttattgac agaggaaagt agaaaaataa cctttggggg 26460
aagtctcatc ctaagcacac cccatcaggt taaaaccatt cttagccaaa aagcagggag 26520
atggcttaca gattcaagaa tcctaaaata tgaagctatc ctattagaaa aaggtgattt 26580
aaccctaacc actgacaatg tgctcaaccc cgccaccttc cttttttttt tttttttttg 26640
agatggagtt tcactcttat tgcccatgct agagtgcaat ggcacaatct cggctcaccg 26700
caacctttgc ctcctgggtt caagcaattc tcctgcctca gcctaccaag tagctgggat 26760
tacaggcatg ctaccatgcc tggctaattt ttttgtattt ttagtagaga tggggtttct 26820
ccatgttggt caggctggtc ttgaactcct gtcctcaggt gatctgcctg ccttggcctc 26880
ccaaagtgtt gggattacag gcgtgagcca ctgtgcccgg cctaccccgc caccttcctg 26940
acaggaaatc caaacgctgg agaccccaag cacaaatgtt tagacttaat catcaaacta 27000
aagtaaggcc tgatctaagt gagacccctt taaaaacagg gcaccatctc tttgtagaca 27060
gctcctctcg ggtaattgaa gagaaaaaac ataatggcta ctcagtggtt gatggagaaa 27120
ctctcacaga agtagaatca gggagacttc ccaataactg gtctaaccaa acatgtgagt 27180
tgttcgcact aaatcaagcc ttaaaatagc tgcagaatca agaagggact atttacacca 27240
attccaaata tgcttttgga gtggcccata cctttgggaa gatttggact gaacaaggcc 27300
ttattactag caagggccaa aaccttgtcc acaaagagtt aatcatgcaa gtattagaaa 27360
accttcagct accagaggac atagccattg ttcatgtccc aggacaccat aaaggtctct 27420
ctttcaagag ttacttccca agaagcaccc attttccact gaaccccttg tctcccttcc 27480
ccatctgcaa cccccatctt ctcccaagca gacaaagaaa aattaaagaa aataggggct 27540
aacaaaaact cagaagagaa acggatactg ccagataaca aagaaatgtt gtccaaaccc 27600
ctaatgacag aaatcttaac acaacttcac caagggaccc attggggtcc ccaagccatg 27660
tgtgatgcag tccttagaac ctatgggtgt acagggattt ataccctcac caggcaagtc 27720
acagacagtt gtatggtatg cagaaaaacc aataaacaaa ctttaagaaa actgcccttc 27780
ggcggaagaa atcctgggct aaggcattcc aaagtgtcca aattgactta taccgaaatg 27840
ccccaaatag gctgcctcaa atgtttgtca gttatagtgg accatctcac tcactgggtg 27900
gaagccattc acctgccaag tgcaactgcc aataatgtag ttaaagtact aatagaagat 27960
accataccca gattcagatt aatagagaac attgactcag acaatggaac ccattttact 28020
gcacaggtca ttaaaggatt aacccagaca ctagagataa aatgggaata tcatactccc 28080
tggcacccac cctcatcagg aaaagcagaa agaatgaatc aaaataaata aatcacctaa 28140
ctaaactaat cttggaaacc cgactgccat gaactaagta cctccctgtc gccttactaa 28200
gaattcgtat cgccccttga aaagatatcg gcctgtcccc ttatgaaacg ctctacagat 28260

tgccatgctt aaatccacca ctgacatccc tacatttgaa acaaaacatc aatttcttaa 28320

aaactatata cgtggtctat cttccaccct tttttccctc aggacccaag gcctcttagc 28380

acaaatgccg cctctagagt ttccagtaca cccacatcag cctggggatt atgtgctcat 28440

caaaagctgg agggaggaaa actcgaacca tcgtgggaag gaccctatct aatactccta 28500

acaactgaaa cagcagcccg aacggctgaa caggggtgga ctcatcacac ttgggtaaaa 28560

aggaccccac cccttatgga atcatggacc atcactccag gaccaactcc ctcaaaatta 28620

acattcaaaa aggtctaatt tgtctcttct tccccctaat gcccaggggc atttcatttt 28680

tttttttttt tgagatggag tctcgctctg tgcccaggct ggagtgcagt ggcaagatct 28740

ccactcactg caagctctgc ctcctgggtt cacaccattc tcctgcctca gcctcccgag 28800

tagctgggac tacaggtgcc tgccaccatg cccggctaat tttttgtatt tttagtagag 28860

acggggtttc acagtgttag ccaggatggt tgatctcctg accttgtgaa ctgcccgcct 28920

cggcctccca aagtcctggg attacaggca tgagtcaccg tgcccagccc gcatttcatt 28980

gttaatgtaa ccaggtcagc ctcccctcaa gctgttgtgt ttgatgcttg cctagtcata 29040

ccttgcagag acctacaaag tcaaaggcaa ctagcctctt cagaaaagta tctttgccct 29100

tggtactcac cccaccctca atctgatcct tgcaagattc cctataattg gctaccttgc 29160

cattcctggt acgatattct ctggaccact cagtcccaag gctggacttc ttcagcaggc 29220

tgcacctccc taaaacctta tatccatttt actaaacaaa ataactcctc taactgccaa 29280

cacctccgat gtaacctggt gcatatttct attactactt caacctccac taaccatatt 29340

cccaccctaa gtcgctttta tggtatgggg cagatgttag cgggaaagac cccatagggt 29400

cctttgagat tcgttttgtc gcccctccag caccccttcc tttccgaaga tctcctccaa 29460

atgaaacaac tatcttgcca ctacctaatg acaaaactaa agtgcacata gtggaagtca 29520

aagacctaaa acaaaccctg gccatcgaga ccggacacca agaaatgaat gcctggctga 29580

aatggatcaa atattcagtt tgcactttaa ataaaagcga ctgttatgcc tgtgcaatgg 29640

gcaggccaga aatccaaata gttcccttcc tgcttggatg gtctaaccag ccgggcatgg 29700

actgtacagt agctctcttc cagcacccca cagcctgggg taataagtca tgcaccagtc 29760

tctcactgct tttcccaaaa gtcaaagact tccctgtggg tcagccccca aggccatctg 29820

gctcctggcc tctgatgcca attttacctc ttgtctctca tgacggggaa aacttacatc 29880

tttcaggaat ctaacgggat gcagtgaatc cgagcctttt cgagagctca ccaatcagac 29940

tgcccttgtc tatccacaag cagatgtttg gtggtattgt ggaggacctc tactgggtat 30000

gctgccaaat aattggagca gcacttgcac tctaatccaa ctggccatct ctttcaccct 30060

ggcatttcat cagtccgaca aaaagtcacc ctcttgggaa gaaagagaag ctcctcaagg 30120

gtcttttgac ccccatgtct atatagaggc aattggagta ccacgagtag ccgccaatga 30180

atttaaagct cgaaatcaaa tagctgccag gtttgagtca gtcttatttt ggtggtctac 30240

tataaacaaa aacgtagact ggataaacta tatatattac aatcaacaag gatttgttaa 30300

ttataccaga gataccattt ggggatagcc gagcaatttg gtcccaccag ccaaatggcc 30360

tgggaaaact ggatagctct tgacatgaca ttagcagaaa acggcagagt ttgtgtaatc 30420

atcagagccc aatgttgtac tttgatccca aataacacag ctcccgatgg aaccactacc 30480

aaggctctac aaggcctcac caccctagca aatgagctgg ctgaaaattc tggaatagat 30540

gaccccttca caggtttaac caaaagatgg tttggtaaat ggaaaggact tatggcctct 30600

atcctcactt cccttgccat tgttacaagt gcactcatcc ttgtaggctg ttgcatccac 30660

cttgtgtttg tgggttagtt caaagactca tagaaatggc ttgcactaaa accttctctg 30720

tctctccccc tccatattca gaccaacttt tactcctaga caatcaagaa gagcaacaaa 30780

gccaaatctt actaaaataa tttgaagagg aagaattata acaacaaaaa ggggaaattt 30840

gtcagaataa aagaaaggtt cctcttcaaa gatccaactt tcttgtcata ggctgtaaac 30900

cgccttgctc caaaccttaa cctgtaacct ttaccctctg ccttatttga gaaagattca 30960

agcgcatagc caattggagt cagcctagac tgtgcggtcc aacccagcca atggggaaag 31020

acaaagaagc aggaactgcg ttagggttag aaacctgttc tcctttatta ggtgtgcttt 31080

tgcgactgct ataggcgcgg gcagcaccct tctgcagaag taaactttgc cttgctgaga 31140

aaacttttct tctgagtgct cctcttcctc tgcggcactc agaaataaga gaacttattt 31200

ctaacaggag gaagaacatg ataattttgc acacgcaggt tctgagtttt ggctggtgcg 31260

ctgagtgctt accataccgc aaatactgtg tgatctgggc tgaagaacag aaaacccaac 31320

taacagtgac ttaggcaaaa agaaatttta ttctttcaca gaagtctgtg gcaggcaggt 31380

ccagagttca tgcaggagct caataacatc agagattggg ctctttttgt tcttcttagc 31440

atatcgatgt tttacgtgat gctgggcacc tcggggacct aagatggctg ttgtagctcc 31500

atgcatcatg attaaaccag agacaggaag tgtacgatgg aagaggccag aggtgaatgg 31560

cctcctcctc tccttttgtc ttattcctgg agtgactccc tcctcttatc agcagggata 31620

caagcagtgc ctggcagcct tccctctctc ctgtatgagc caaaagcaag actcatggca 31680

cccctccctg caggaaaggg gtggaatggg cccctggcaa agggggctgg acttgcagtg 31740

acagtgctgc tctgaacagg actaggcttc tgttggagag gtggtcagcg gctgctaggc 31800

aggtcacaag ctgcctcacc catggctctc cagctctgca cctaccctct ccttctaata 31860

aatctgtttc aataaccacc acgaggttta aagaggagca gtaacttgcc cacggttgcg 31920

ccaggcaccc ctagaagagc aagaattcct tacaaagtta ttaaaacagg caaatcccat 31980

ataggtgaca tttctatcca acaatgctga catctctttt agttttgagc cttagtaatc 32040

caattttctg cctcaggaag aggcagcagc ttctgggaaa aatctgctcc tgctaaggtg 32100

gctcattcat tgttttacct gaatagagtg gtggatggtg aaggttcaaa ttctcacttt 32160

ccatattaaa ttttataaca taaaaaataa ttaaatgggg tgcgcgcggt ggctcacgcc 32220

tataatccca gcacctggga ggctgaggag ggcagatcac gaggtcagga gatcgagacc 32280

atcttggcta acacggtgaa actgcgcctc tataaaaata caaaaaatta gctgggcatg 32340

gtggcgggcg cctgtagtcc cagctactca ggaggctgaa gcaggaaaat ggcatgaacc 32400

caggaggtgg agcttgcagt gagccgagat cgcgccactg cactccagcc tgggtgacaa 32460

gagtgactcg gtctcaaaaa aaaaaaaaaa aattaaatga gccaaccagg acaatgttta 32520

aaagccagag ccaaaaaggc tccaaaataa aacaaacttg tctgctcaat taattatttg 32580

aagagaacct acagtaatac agactggaga tgggaaagca cgagggatgt atgagtcgag 32640

gaaccggcaa aggggtgtgg gggtgtgtgt gtgtgtgtgt gtgcacacac atgcacccag 32700

ctagggtaga aaataagtac ttttgcttct atgggaagtg actttccagt tcacttgtgt 32760

atcaaatgaa catgatgaaa tcaagttgta ctagccaatg tttgggaggg ttctgtatta 32820

aagggataaa gaaaaacatt agaaagagaa gttacaaaaa tcactgtggg gaaaatgaac 32880

accaatgact tggctgctct gaggcttgca gggagtagac agttaggcat cccaggtcat 32940

cagagaacca gtggggtctt ttcctcaggg gccgggtttt ctacacgcaa gtcaaacctc 33000

ttctcctctg ggacacatgt ttccaaagga ctccagaaac acagcacagc tggattcagg 33060

tactgtgtca agatgtgcct tcttcttcat ctttgacagc aattaatgta ctaaatgtct 33120

tgatagagaa ggtcatattt gggaatattc ttgggatcaa taggactttg gacaataagc 33180

ttccccctca ttgctcctcg atagattact tcaatcaaat ctatgaagtc ttgtttggtt 33240

ttgaagcttc ccacaaactt agtgtgatct ggagatctag acagcataga agagaaacaa 33300

ctgctctaag aactttgagt aatcacttgt tgaagacttt caggataaac aaaatgaaaa 33360

acagctttgt tattttcttc ttgtcaatga gacttcagaa ataaagccca acattaattt 33420

ttcaatcatt tttttccatt tagtaaatga ctcaacccag tgctgtgggc cactagcatg 33480

aaagctctgt catgtgcaaa gctttagctt caaaagctat tgtaatcaca aaatctattt 33540

ttatcacttc tcaaatcatc aagcagcttt caccaagtgt tggagtgcac attgtttctt 33600

cgtttgctgt tgttgctttt gcctgggcat tgtgcaaagc ccattcaata ttcatggcct 33660

tttgatgttg gtaggtatta aaaaattgat tttttagaaa agtatctcaa acaccaggta 33720

tgttactgta aaattttaaa gaaataaata aaatcacgga tgaggccaga ggccagaggt 33780

cacttttcag tgtctatcct tccaatcttg tgtgtgtgtg tgtgtgtgtg tgtgtgtgtg 33840

tgtgtgtgtg tatgtgcaga tacaaacttc ttttttccca acattgggaa ttatgtcata 33900

caaattattc agtaatatgc ttttttctag ctgacatata tttctttcat ttgttaaatg 33960

tgcatctgca acatcatatt aaagtcctgg aagtgtaatt actgggttaa aaagtttaaa 34020

aactttaaac tatttgatac ataatgttga actggccccc ataaaaggtt gtaccaaatt 34080

aatacttaca tcaaatgtgt tccagtagaa taaacagctt taaaatactt ttgtgctttt 34140

tttttgttgt tgttgttgtt gttttgcgat ggagccttgc tctgtcacca gactggagtg 34200

tagtggtgcg ttctctgctc actgcaacct ccacctcctg ggctcaagcg gaatctcctg 34260

cctcagcctc ctgagtagtt gggattacag gcacccacca tcatgcctgg ctaatctttt 34320

ttttgagacg gcagctctgt tgcccaggat ggagtgcagt ggcgcgctct cggctcactg 34380

caagctccgc ctcctgggtt catgccattt ttctgcctca gcctccagag tagccgggac 34440

tacatgcgcc cggccaccac gcccagctaa ttttttgtat ttttagtaga gacagggttt 34500

cactgtgtta gccaggatgg tctcgatctg ctgacctcat aatctgcctg cctcggcttc 34560

ccaaagtgct gggattacac gcgtgagcca ctgcgcctgc catgcctggc taatttttgt 34620

atttttagta gagatggggt ttcactatgt tggccatgct ggttttgaac tcctgacctc 34680

aaatgatccg cctgccttgg cctcccaaag tgctgggatt acaggcgtga gccactatgc 34740

ccagcctaaa acactttaaa acagataata tttcacatac tttcaaataa agacaaaaag 34800

ggtacacaga tctagataag tggacacatc aaccacagta agaaatgatc agaatcatgg 34860

atgcacccaa tcatcaatat taagtattga gatggtaatt ggatggagtg gaaggcacta 34920

aagaggacat catgttactt attcttgaca ggctttgttg aagaatgatt taaaattcaa 34980

tggcgaaaag caggaaactt tgcaagagag aagcacatct tagtttgcta gaaggaatgt 35040

ttctttcata aatgccactt accagtccaa aagcttaaga aaaactacat gttttgtttc 35100

aaatgaaatt cctgggaaaa ttttaagagt tctttattta gctaatatgg ctattttata 35160

ttttatagac ttatggatgg tagtgctttg ccacctgtac tatttcttca tacttaagtc 35220

ggaaattaaa atttgacaaa ccatgtgtat gcactattaa aaaaataaat aaaatttgac 35280

aatcatacca gaagcactct cattttgtag caaggttttg cataaaaaat tagagagtgc 35340

gtcaattggg gaaacagtac tgatataaga ctcacagagc caacatcaca tggaaatagg 35400

caagctactg aaaatgctta ccaaggaaac ttacaaggtt gcaattaact tcagatcaac 35460

tgcacttacc cataatccac tttcatatgc tgcccattga agaaaaagac agtagatgga 35520

atataactga tgtcaaaata ctgtgtataa actgcagttt ggtccacatc taccaggtat 35580

atagcagcca ttttacttaa gtcagaagag gtcttagaaa gctgcaaatg acgagagaga 35640

caaaggttac aatatgagag gcagcttaat gtttcttctg tgaaacttgt tactacaaca 35700

gagtgttttg tttgaaaaaa ctcctgggcc aacaatgcag cccatgtggc tcccacatta 35760

gacaaagcat aaccccatcc tccagagtct cattcactgc aacggggtta ggagggaatc 35820

ttggggaaaa ctgtatgagg ttaggaaggg accttgggga aaactgtgtg aggttagaag 35880

gggaccttgg ggaaaactgt gtgagtttag gagggtgaca gcatgggagt attcctgtct 35940

attcattgtg gagagagacc taacccagaa ctaagacctc tacccgccat ttctactgct 36000

gccatcccgt gttaagagct gagcatacaa atccttggga aaactgtgtg aatctgtttc 36060

ttcctttaat ttctttcttt gaatgctttt ataaaagtag cccatgctca tttaaaacaa 36120

cacaacttag aaatatgtta tgtggatact gaaattccct aacgtctcct tcctcccaac 36180

cctcccagaa atgaaaacca cgataattag tagcagacac ttatataaca ttactatgtg 36240

gtaactgtcc taagtgctta acatttgtta actcatttaa tccttacatc attcttataa 36300

ggtatatgct atcagagtcc ccattttata gatagggaaa ctgaggcaca ggaaggtaaa 36360

gtaatttgcc ccaggtcaca cagctcctga gcagtagggc tggggtttga actttggcag 36420

tgtggcttca gtataccact gacctctgtg gtatttcctc ccgcctatga ctgagggtag 36480

agttctctgg cccttttttc cccaagttaa cacactgcac cttcattgca attatggaaa 36540

cgaggatttg tacgctcagc tcttacacgg gatggtagca gtagaaatgg cggtaagagg 36600

tcttagttct gggttaggtc tctctctgca tcaggcacat actggaggca gcacactaga 36660

aaagattatt tgcagctaca gaaaactgag ttcaaatctc aactctgcaa tatagtggat 36720

ctgcacctgt gggaaattac ttgacctctc tggttttttt tttcctactg ggaaatgggg 36780

ctactattat cccactattg cgagcactga aaagatagtg ttcacagatg cttgttaatt 36840

agttttctgc tcttccatca ccttgttggt gaaacagcat tgacagcttc tagtgggtaa 36900

ggacatccaa gacagattca gcacacacag atttgggggc ttggttcagg gcaggcacac 36960

aatcagctct tagtgaacta acaaatgaac agctatatac ccaaccctga ctactccctc 37020

tcatgtacta ctcagatgct gaagtgagaa tatacagatt attaataaaa accaattatt 37080

ttagacattc acttacaata tcatctagct gcagacagac aggatcttca tctctcccaa 37140

acctgagaac caacaccttc tcagcagtac tttttatcgc ctggtctact tcctttttgc 37200

tagtcagctt gggcagtagg aagctcatct tgaaataacc caaatgtgaa tcctcactgt 37260

cactcctgaa ataaaggtgc aatgtttaaa gacagtttag attaagtgcg tgagccctca 37320

ttaaaaaaaa ttaattcaca gagtagctaa tgtattcaca tggtatgaaa gaaacatcat 37380

agaaaggtaa cactgtggag tttccctctc tttgtccctg ctccttggaa ccagttttca 37440

ttgttccctc ccccgggcca gataactgct ttcattagtt tctcatgtat ctttcagagt 37500

tttgttatgc attcaccaaa aaatacaaat atgcgttccg atattttttt tctctctctc 37560

cccaccccca aaagatagcc tattgtatca gtgttccagc aggaaagcga tgacactctc 37620

aactttggta atttgaaggc agtttaatta aggcaggtgt agaaaaacaa tacggtaaag 37680

tgtactactt tggggctagt aacagcggga tgctgtaccc atccctaggc catgggagga 37740

agaagccacg gtaggaagca gttataagaa ttgtgtggca gaaagaggtg agtggagagg 37800

acagtgtata ggagggcagc ctgctgttag gtgttgcagc cactctcctc cctccctctg 37860

acctttgctc atgcaaccac cggctgactc caactggaac cagagggcag gggcctgttg 37920

aggcaggcaa acctcccttg tcccagagca gtctaagagg aggttgaaga atggctgtgg 37980

agggaccaat ggcagatgtt cagcacacac actttatcta ctcttcagca cttttcatta 38040

tttcaaacct aacctatgaa gattgcccca tatctctata tagagaacat ccatattctc 38100

tgtcatggca gcatattatt tcttgcatgt gttacacttt ttaaacagtc ctttattgta 38160

tactttatat tattacaagt cctggcttaa agattcttcg tgtgcacctt actaggtgca 38220

cctcattaca cggcaaacag atcaattaag agtgtgtgtg aaaactacaa taccataaac 38280

aaataataaa atactgaggt acaagggacc taatacaacc ccgtcatttt acaaactagc 38340

ttgcttacag ttgcactttc tactacaatg acaaggacta gaatacaggc caccaaattt 38400

ctagtccagg ggtccttcta ctacaataat ctattttcat gattggtaga aatttcattt 38460

ttgagaaact ctttgacaaa gaagcttagt tttgtggtca tctataagag actccattga 38520

gatggtcggt attttggaat tc 38542

<210> 9
<211> 22250
<212> DNA
<213> Homo sapiens

<220>
<221> misc_feature
<222> (11552)..(11651)
<223> n is a, c, g, t or u

<400> 9
aactgccatc atactgaagg taatcttctg aaacttgcgg ttttctttca aaattatgtt 60

tataagatga tccatgttct tgcagagttt attcatttta tgctgtataa tattccatta 120

tatccacata caatgcagta ttgacccttc ctcctgttga tgggcatttg tcttgtttct 180

agttactttg ctattatatc agtgtcacca tgattatcca aaagtaattc ttttgtacac 240

tctaatttaa gaacaactaa ccctttttaa tgaataaatc aaccttgtat tgagttgcta 300

ctaagtttca gttgactagt acctgggata cacacaggtg cagacatttg actgagacat 360

attgattttt ctcatctgcc tatttaggct aatcaccaga ctataaaacc atgagaacca 420

ctgccattga gtatagtctg tgtcagtcta cactatagct ttaactagtt gtgtgatttc 480

ttgcaaagag caatcagaga agacacaata aacacattta ctgatttcag gctggagagc 540

ttttaagcaa tagggagatg gccacacaca aggtggagaa aattactgtg aaaaggatac 600

gtacttttct ttagagcccc acctaagcta ggctgcagaa atgtctacaa tggctttgaa 660
aaaactcaaa atgagccttt ctgcagtgtg aaaatcctcc aagataaaga gacagattga 720
tggttcctgc cgccgccctg tcctgcccag ttgctgattt caggaaatac tttggcaggt 780
ttgtgggcat agagttgcca ggtttcttgg gatttgtaat agaacatcac aagaaaatca 840
agtgtgaagc aagagctcaa ctcttaacag gggtattgtt tgtggttttg ttactggaaa 900
agatagtgac cttaccaggg ccaaagtttg tagacacagg aattacgaaa tggagaaggg 960
ggagaagtga gctagtggca gcataaaaag accagcagat gccccacagc actgctcttc 1020
cagaggcaag accaaccaag atgaggtggg tccacagctt tccctcctgc ctttcctctg 1080
gttctttatt tcagtctttt ttgcatacat cggtagagat gcagaaatag aacaaagaaa 1140
cgggcaaatg ggctaaatta tagtgaacca aagggcttag tgtgttaaat cttctccttt 1200
tctgcatcca tagaagacag tgctgctgtc tttcccagga gataagattt actctcagga 1260
gtgtcttttt ccttcaggtt acatttttga ctttataggg tatgtcatca gctcccgtgg 1320
taggcttcct ggcatcctga gtatatttat tagcagatat tttcctcttt aaaaatgtac 1380
aataaggaag actaatagta acacatttga atgacacaat taattgacta gtacctggga 1440
tacacactaa tacctgggat acatctaatt aaggcactta gatcttataa aaataaacac 1500
tttttgaaat gttgaaataa taagactaga aacttttttt ttttttgaga tggagtctcg 1560
ctttgtcacc aggctgcagt gcagtggcat gatctcggct cactgcaacc tccacctccc 1620
aggttcaagt gattctcctg cctcagcctc ccaggtagct gggactacag gcgtgcgcca 1680
ctatgcccac ctaatttttg tatttttagt agagacgggc tttcaccatg ttggccagga 1740
tgacctcgat ctcttgacct cgtgatctgc ctgccttggc ctcccaaagt gctgggatta 1800
caggcatgag ccaccgtgtc tggcctagaa actattttaa tagaagcaag tagtgcccga 1860
atggttggca tttgttagtg agatggtgaa ctggcagacg gcacctgtgg gtcaatgccc 1920
atggccaccg tcctgctttt ggacaccagt tctcttccag gtaaccttct ggcattttgg 1980
ggtttcagat accatttcct aaaggtgaat tattataaaa tactagaaat acccacgtgt 2040
ttaaaattat atatttaagg aaccttttat tacggaaaat atcaagaagt agaggaatag 2100
cacagtaagc ccaaagttcc catctctgag ttatctacat tttattacca ctatctttgc 2160
ggtgtctgag ggaggtttct ctttcctgga gggctcctgt attattgcca atgtactttc 2220
ctgaatgcag ccagaaactg agcccacccc tccacctatg tgcctttcta tccctctctg 2280
aagcttctgc agaattccca gcaggacagg gcttgctgga agcttggtat gctcagaagc 2340
tgctaaagtg tgtatgggca ggtgtggggg caatttcttg gtcctagcac ttccatatat 2400
cgactttctt ttctggctgc taagtgggag gagtgtgtgt gtatgcatgt gtgtgtgtgt 2460
gtgtgtacat gcatgtgtgt gtggatgcat gcatgtgctg tgaagcaggg agactagctt 2520

tccactcctc cttgtcttct ctctgcagtg ccctgggagc tgtcattgcc ctcctgctct 2580

ggggacagct ttttgcagtg gactcaggca atgatgtcac ggatatcgca ggtcagtctt 2640

tggttgggta ggagtgtgca tcccactctg accctctcgg gtctgcactc tctctgagaa 2700

cacccaattc ccccttctta tctcgacctc tgggctttca ggaccataaa gaacattggg 2760

gttcctgcca gaaatgaggg gagcttgcct ttccattggc ttctattcgg ggtggaagga 2820

gattgatgtg cagagcagct cccgctcatc tgacttttca cggttcactg ggaacaattt 2880

ccaaatagca aactctctgg cttctctctc tttgcagatg acggctgccc gaagcccccc 2940

gagattgcac atggctatgt ggagcactcg gttcgctacc agtgtaagaa ctactacaaa 3000

ctgcgcacag aaggagatgg taagatgtgg acaactgtct ccatgcccta catacaaccc 3060

ccttctctga catttccatg atgggtggtg ctgaggtgat tcgccagaaa gttcgttgct 3120

ctccttggag ccaggagatt tagattctaa taagcgtttt gtcgccagta gccatggccc 3180

tttgggcaga ctaacttttg tcagcctcaa gttttctgtt ttgttaaggg gaggcgatgc 3240

catgcagcct acctcatgta aatctcagag tcagatttac atctccagca gatgtgggaa 3300

aagaaggaat gctgatgatg atgtcaccct cacctagtga gtcttgctgt cctggcactg 3360

ctctaagggc tttatactta tttgctcact tagtcctcac agtatccctc tgaacagagt 3420

ttattgtttt cactttgctg ataaggaaac tgaggcacag acaggttgag tatcttgccc 3480

aaattcaggc agcctgtaag aggcagagtc aggatttgaa ccctgagccc tccctgtact 3540

gcttggctgt gaccgccatg accacagtgt gttctgctgg gcttaactgg tgtccaggca 3600

cttggcttcc agcacagcac tctttccctt cctccttctc atattctctc tcctttctcc 3660

cttcctgtct gcctcctttc ttcttcttct ttttaattct tctccttaaa tgccttctca 3720

ctctgctctg ggtgcagact tgacttttcc tttggctcat ttcttgcctt ttgtttcagg 3780

agtatacacc ttaaatgata agaagcagtg gataaataag gctgttggag ataaacttcc 3840

tgaatgtgaa gcaggtgggt gctgagcact gagcacttaa gagagcaggc aggcgtccag 3900

cggggaacgt cctagaggca cagccttcca gtgcggcttc ctctgagcac acaagagcca 3960

ggaggaggga tgtgggagaa ccgcagctgg ccagggagag acttaagcag ttaggtgatg 4020

actccctaag ggtcaccaag ggtcttgttc attggggcct gaagggcact ggctgaatcc 4080

actgtcggca ctgcccacag atcaggagag cctgtgcata cagagagcct gctagaaagc 4140

cctgggtcta aggagaagca agctccaggg agaacaagtc aaggaatgac ataaaatctt 4200

aatccatgga agcctagcag gaggctggac atgggctgga actcctgctt ctcgttatta 4260

ggaggagctg ttgctctctc ctttcattct cagaaccaga ggcaaagacc cagcctcttc 4320

tgctcttact ggtgtggaaa tgccaacctg cctcgtatta actgcaccat ctacaaaatc 4380

tgagctccag ccagtgctgc tctagattca tctttcttta gagagaatga attattgtag 4440

cccctagccc tttcaatgaa tttcagggaa ttgtggaaat tcctttattg ggataattgt 4500

ttaaatataa tacagttcgc gagcttctat tcggggtgga aggagattga tgtgcagagc 4560

agctcccgct catctgactt ttcacggttc actgggaaca atttccaaat agcaaactct 4620

ctggcttctc tctctttgca gatgacggct gcccgaagcc ccccgagatt gcacatggct 4680

atgtggagca ctcggttcgc taccagtgta agaactacta caaactgcgc acagaaggag 4740

atggtaagat gtggacaact gtctccatgc cctacataca acccccttct ctgacatttc 4800

catgatgggt ggtgctgagg tgattcgcca gaaagttcgt tgctctcctt ggagccagga 4860

gatttagatt ctaataagcg ttttgtcgcc agtagccatg gccctttggg cagactaact 4920

tttgtcagcc tcaagttttc tgttttgtta aggggaggcg atgccatgca gcctacctca 4980

tgtaaatctc agagtcagat ttacatctcc agcagatgtg ggaaaagaag gaatgctgat 5040

gatgatgtca ccctcaccta gtgagtcttg ctgtcctggc actgctctaa gggctttata 5100

cttatttgct cacttagtcc tcacagtatc cctctgaaca gagtttattg ttttcacttt 5160

gctgataagg aaactgaggc acagacaggt tgagtatctt gcccaaattc aggcagcctg 5220

taagaggcag agtcaggatt tgaaccctga gccctccctg tactgcttgg ctgtgaccgc 5280

catgaccaca gtgtgttctg ctgggcttaa ctggcatcca ggcacttggc ttccagcaca 5340

gcactctttc ccttcctcct tctcatatac tctctccttt tccccttcct ttttgtcccc 5400

ttttcctctt ccttttagtt cttctcttta aatgccttct cactctgcac ggggtctaga 5460

cttgacttct cctttggctc acttcttgcc ttttgtttca ggagtgtaca ccttaaacaa 5520

tgagaagcag tggataaata aggctgttgg agataaactt cctgaatgtg aagcaggtgg 5580

gtgctgagca cttaagagag caggcaggcg tccagcgggg aacgtcctag aggcacagcc 5640

ttccagtgcg gcttcctctg agcacacaag agccaggagg agggatgtgg gagaaccgca 5700

gctggccagg gagagactta agcagttagg tgatgactcc ctaagggtca ccaagggtct 5760

tgttcattag ggcctgaagg gcactggctg aatccattgt ctacatcgcc cacagattag 5820

gagagcctgt gcatacagag agcctgctag agagccctgg gtctaaggag aagcaagctc 5880

cagggagaac aagtcaagga atgacataaa atcttaatcc atggaagcct agcaggaggc 5940

tggacatggg ctggaactcc tgcttctcgt tattaggagg agctgttgct ctctcctttc 6000

attctcagaa caagaggcaa aggcccagcc tcttctgctc ttactggtgt ggaaatgcca 6060

acctgcctcg tattaactgc accatctaca aaatctgagc tccagccagt gctgctctag 6120

attcatcttt ctttagagag aatgaattat tgtagcccct agccctttca atgaatttca 6180

gggaattgtg gaaattcctt tattgggata attgtttaaa tataatacag ttcaccagcc 6240

agggctcaaa aatctcagta tttcccactt cctttgttag aaaagtggga aatagagctt 6300

tttgtaatgt aaacaattta aaaaacagaa ttattttaaa actgcaacta ttggaaatga 6360

gatcagcagg tggtaagggc aaagcattta aatctttcta ctttacgcag cagtgacagc 6420

cgcccatgct ttcacccctt tctcagatgg aaaggctctt gcacatttcc actcacgagt 6480

gtcttgctct ccttgacagt atgtgggaag cccaagaatc cggcaaaccc agtgcagcgg 6540

atcctgggtg gacacctgga tgccaaaggc agctttccct ggcaggctaa gatggtttcc 6600

caccataatc tcaccacagg tgccacgctg atcaatgaac aatggctgct gaccacggct 6660

aaaaatctct tcctgaacca ttcagaaaat gcaacagcga aagacattgc ccctacttta 6720

acactctatg tggggaaaaa gcagcttgta gagattgaga aggttgttct acaccctaac 6780

tactcccagg tagatattgg gctcatcaaa ctcaaacaga aggtgtctgt taatgagaga 6840

gtgatgccca tctgcctacc ttcaaaggat tatgcagaag tagggcgtgt gggttatgtt 6900

tctggctggg ggcgaaatgc caattttaaa tttactgacc atctgaagta tgtcatgctg 6960

cctgtggctg accaagacca atgcataagg cattatgaag gcagcacagt ccccgaaaag 7020

aagacaccga agagccctgt aggggtgcag cccatactga atgaacacac cttctgtgct 7080

ggcatgtcta agtaccaaga agacacctgc tatggcgatg cgggcagtgc ctttgccgtt 7140

cacgacctgg aggaggacac ctggtatgcg actgggatct taagctttga taagagctgt 7200

gctgtggctg agtatggtgt gtatgtgaag gtgacttcca tccaggactg ggttcagaag 7260

accatagctg agaactaatg caaggctggc cggaagccct tgcctgaaag caagatttca 7320

gcctggaaga gggcaaagtg gacgggagtg gacaggagtg gatgcgataa gatgtggttt 7380

gaagctgatg ggtgccagcc ctgcattgct gagtcaatca ataaagagct ttcttttgac 7440

ccatttctgt gttgtgttca gtcttgagtc tttttatttt gctcctttat ggtccagggt 7500

agtcagaagg tatagagtct actgggagta tggcagaaaa caccctaaac ccactggaaa 7560

tcccgaaggt gatacaaact cttccacctt agggaatcat gctcactgat tgagtgccta 7620

ttgaatgcta ggtcccagaa agttaactgt tgtccttgtt ttacagacaa ggaaacagag 7680

actcagagat ggtaagtgag ttgcttaagg ttacatagct atgaaacagg gaagcagaac 7740

tttgaaccca ggtctgtttg atacaaactc agaggtcctt tcactgcatg ctgttgcctc 7800

ctcaaagtga attaggagaa aaggcatggg cctggttgag gaagaggcta gctcaaaatg 7860

ggatggggaa aaagtgtttt aacacagaca gtaattcaga gtttgggatc taacctacca 7920

gcactccaga aaacaaaaga caatgatgta aaggcaggat ctctggactt actgagtcca 7980

aatcacagaa tggcccataa atttgctagg taatttaggt ttcctggcca tcagttcctg 8040

gtctcttcca tgaaggactt gagccaggcg atggctaatg tccctatcag caataacctt 8100

ctggtattcc ataaagagtg gggaggtaga gtccctgcat cagtagaagc tgcttttcct 8160

actgggaaca aacccaccct gcaccaggaa tcaaatgcag gctgtctgag cctcctctcc 8220

tgatggcagg tcatgctatt aagggtctcc cagaagaaag gttctggaac tctgagaaac 8280

agaagctctt ctaacagaac tttttcttgc tataagcttt caataccacc aatcaccagt 8340

gcaccttctt ctcatgccaa aatacggccc ctcctttgta gggtgtaaaa cgacactgat 8400

gtcactctct ctcaccaagg tctggccatc tctgtgaagc tctcctcaca ggcaccctgc 8460

agattcatca ataaacttca acaggttagc gccagtaggc caataagatg cacacttata 8520

ttctagaacc tgtgaaagaa gtaatagcca gaaactggaa aataacagtc aaactcttaa 8580

aaaaatgcct ctcagaacac agtcacttac accttttcct ctggtagttc tcttcagcac 8640

caagtttcct ggaagaacaa gctactcccc agttttctta aagaattgtc tgattgtatt 8700

aagtaagtgg cttctggtaa aataatggta agagaaaagc tctggcattc tcttgatcac 8760

tcatcttctc tgccttcctc cctagcaaca tctcctggcc tcagcctgtg ggcttcagca 8820

acaccaaact ccatgttgct ctctgacaag cccggccgtc tccagttctt gggctttatt 8880

tcctcggggg ccctctgctg aaattccctt ccccttgctg tattagcacc tcctacccgc 8940

cttcaacatg caggcactgc ctcctctaag gagccatctg tacttacgta gctgtgagca 9000

taggatgggg catacagcag gcacttaaca aatacttgtg gaatggaaag aaactaagat 9060

aggatcaaaa atacaaatca agatgtgaga gaaagacgat caaaagccct cacgaggggg 9120

tcgggcgtga tggctcacac ctgtaatctc aacactttgg gaggctaagg cgggtggatc 9180

acttgagggt gggagtttga gatcagccag agcaacatgg agaaacctcg tctctactaa 9240

aaatacaaaa aattagccag gcatggtgat atatgcctgt aatcccagct actcgggagg 9300

ctgaggcagg agaattgctt gaacctcgga ggtagaagtt gtggtgagct gagatcatgc 9360

cattgtactc cagcctgggc aacaggagtg aaactctgtc tcaaaaaaaa caaaaaacaa 9420

aaaagaacac cacgggaaaa tcaagtgtga agcaagagct caactcttaa caggggcaat 9480

gtttggagtt ttgttactgg aaaagataga gaccttacca ggtccaaagt ttgtagacac 9540

aggaattacg aaatggagaa gggggagaag tgagctagtg gcagcataaa aagaccagca 9600

gatgccccac agcactgctc ttccagaggc aagaccaacc aagatgaggt gggtccacag 9660

ctttccctcc tgcctttcct ctggttcttt atttcagtct tttttgcata cattggtaca 9720

gatgcagaaa tagaacaaag aaacagggca aatgggctaa attatagtga accaaagggc 9780

ttagtgtgtt aaatcttctc cttttctgca tccatagaag acagtgctgc tgtctttccc 9840

aggagataag atttactctc aggagtgtct ttttccttga ggttacgttt ttgtctttgt 9900

agggtatgtc atcagctccc gtggtaggct tcctggcatt cggaatatat ttactagcag 9960

atattttcct ctttaaaaat gtataataag aaagactaat agtaacacat ttgaatgaca 10020

caattaattg actagtactt gggatacaca ctagtacctg ggatacatct aattaagaca 10080

cttagatctt aaaaaataaa aagacttttt gaaatgttga aataataaga ctagaaactt 10140

ttttttttgt gagacagagt ctcactctgt caccaggatg gagtgagtgg cgcgatctca 10200

gctcatcgca acctccccct ccccggttca agcaattctc ctgcctcagc ctcccaagta 10260

gctaggacta caggcgtgtg ccaccacgcc cagctaattt ttgtattttt agtagcgatg 10320

tggttcacca tgttggcccg gatgatctcg atctcttgac ctcgtgatcc acctgtctcg 10380

gcctcccaaa gtgttgggat tacaggtgtg agccaccgca ccctgcctag aaactatttt 10440

aatagaagca aatagtgcct gaatggtggg cgtctgttag cgagatggtg aactggcaga 10500

tggcgcctgt gtgtcaatgc ccatggccac cgtcctgctt tcagacacca gttctcttcc 10560

aggtaacctt ctggcatttt ggggtttgag ataccatttc ctaaaggtga attatcacaa 10620

aatactagaa ataaccacat aagtgtttaa aattattgtt aaatacagta agaaattctt 10680

cttcaaaagt ttagcctgct taagtttcct tgtcctttgt ttcctgcttt caaggccaga 10740

cttccttact ctctgtgttt cccctaccct ggtaaacaac cttcctgcca gtccttaccc 10800

atacagccca cattccacat ctgctaccca ctctgtgatt tacctctccc gtcgcaatag 10860

cctctcccac caaaactgat cttcccgcct tcccaccagt gcaaccacat tcctgcactt 10920

tgcaagttag ccaaccgggt tcggattgtg cagtccaact ccagccaatg gagtcaggac 10980

acagtagcag ggacaagctg cgttagacat aaaaacctct gctttccttt gtttagggtg 11040

ctctcgtggc aaccagactt accaggagct ctattctgca aaagtaaatt tgccttgctg 11100

agagaccctt tgtcctttgg ctcagtgttg gttcttcttt gcagcaccga gcatttgttt 11160

ccaacaaatt tggtggccca tacagggaaa acattgtcct ccgggaaagg gttctttgat 11220

catcctcttg agaggagaac acatcccact gtccttgttg cggtggcctc atgagtagga 11280

atcgagaccc acctgtctga tgaataaccc cagactctca acaacgtggg gagaaaaaga 11340

cttgcaacac tatggtggcc aggtaactct gtgcgcagac caaggtaaga aatgtcgcag 11400

gagtgacaaa gtacttcctt ggtggtcact atattctggt ggctgaaagt tcatgaatgg 11460

taacaagtgc tactgctgtg tggagtgaat gagtccaatc tgtgggtcta tggttacctc 11520

atacggctta gccttctctg aaggatccgg gnnnnnnnnn nnnnnnnnnn nnnnnnnnnn 11580

nnnnnnnnnn nnnnnnnnnn nnnnnnnnnn nnnnnnnnnn nnnnnnnnnn nnnnnnnnnn 11640

nnnnnnnnnn nagcgtcctc ctcccatctc caaaacaacc caactaaagt aacggttgta 11700

aaggtaaaaa atttaaagca gactatagcc ctagagacag gatatcaaga tgcaaagtct 11760

tggctaaaat ggattaaata ttccatctgc actttaaaca aatatgaatg ttatgcttgt 11820

gctcacggta gaccagaggc ccagattgtc ccctttccac tcagatggtc ttctaactga 11880

ccgggcataa gctgtatggt agttctcttc caagatcaca cagcctgggg tgacaaatca 11940

tgtcaagctc tttctctgct gttccctgaa gctcaacacc ctgagggtca gcccccgagg 12000

gccatcagct tccgtttccc aatgccaatt ttactttgtg tctctcacaa cagggagaaa 12060

acttggtgtt ccttggagcc ttaatgggat gcagtgagct taagtccttc caagagctta 12120

cccatcagtc tgcccttagt catcctcgag cggatgtatg gtggtattgt ggtggaccat 12180

tactggacac tgccgagtaa ctggagcaga acatgcactc taattccatt ggctatccct 12240

ttcaccctgg catttcatca accagaaaag atagaaacca aacactgtaa aacaagagag 12300

gcccctcatg ggtctgttga ctcccacatt tatatagatt ccactggagt ccaacgagga 12360

gtgccagatg aatttaaggc ccaaaatcag acaactgcag gatttgaatc cacgttcttc 12420

tggtggttaa ctataaataa aaatgtgaat tagataaact acatttatta caaccaatga 12480

tgattttttt ttttttgacg gagttttgct cttgttgccc aggctggaat gcaacggcat 12540

gatctcggct caccacaact tctgcctcct gggtccaagc aattcttctg cctcggcctc 12600

ccgagtagct gggattataa acatgtgcca ccacgcctgg ctaattttgt atttttagta 12660

gagacagggt ttctccatga tggtcaggct ggtctcaaac tcccgacctc aggttatctg 12720

cctgcctcgg cctcccaaag tgctgggatt acaggcatca gccactgtgc ccagcctatg 12780

atttgttaat tatactagag atgctcttaa agggacagct gaacaattaa gacccaccag 12840

tcaaatggcc tgggaaaata aaatagcatt agacatgata ccagcgaaag aaggtggaga 12900

ttgtgtcatt atcggaaccc gatgctatac ttttatccct aataatacta ctccaggtgg 12960

gatcacacca aaagcactac aaggtcttac cgccttatca aatgagctag ccaaaaattc 13020

tggactaaac gaccccttca caaatttaat gggaaattct ttggcaaatg gaagggattt 13080

atgtcctcaa tcctcatgtc tcttgccatc agaatagggt tgcttattct tgtaggatgc 13140

tgtgtcatac cctgtgccct aggattaata caaaagctca ttgaaacagc tctccccaaa 13200

acctccctca atcctcctcc tcctcgttcc agtgagcttt ttcttttaga agacccaagt 13260

agaataacag agccaaatta tgttaaaagg gtttaagaaa aaatactgta aaatatttaa 13320

cagggggatt gttaaataca gtaagaaatt cttcttcaaa ggttcagctt gcttaagttt 13380

ccttgttctt tgctccctgc tttcaaggcc agacttcctt actctctgtg ttctccctgc 13440

gctggtaaac aaccttcccg ccagtcgtta tctatagagc ccacattcca catctgctac 13500

ccactctgtg aattacccct cccatcacaa tggcttctcc cggcaaaact gatcttcctg 13560

ccttcccagc agtgtaatca cattcctgca cttttcaagt tagccaaccg ggttcagctt 13620

agattgtacg gtccaactct agtcaatgga gtcaggatac agtagcaggg acaagctgcg 13680

ttagacataa aaacctctgc tttcctttgt tcggggtgct ctcgtggcaa ccgaacctat 13740

gaggagcaac cttatgcaaa agtaaatttg ccttgctgag agaccctttg tcctttgtct 13800

cagtgctagt tcttctttgc agcaccaagc atttgttttc aacaattata tatttaagga 13860

atctttgatt atggaaaatt tcaagaagta gagggaatag catactaagc cctctgttcc 13920

catctttgag ttatctacat tttattagaa gtatctttgg ggtgactgag ggaggtttct 13980

ctttcctgga gggcttctga attattgcca atgtaccttc ctgactgcag ccagaaactg 14040

aggcccctat acctatgtac ctttctatcc ctctctgaag cttctgcaga attcccagca 14100

agacagagct tgctggaagc ttggtatgct cagaagcagc taaagcgtgt atgtggggcg 14160

gagggtgggg gcaacttctt ggtcctagca cttccatata ttgatttctt tttctggctg 14220

cttagtggga ggagtgtgtg tgtatgcatg tgtgtgtgtg cgtgtgtgtg tgtgtgtaca 14280

tgcctgtgtg tgtggatgca tgcatgtgct gtgaagcagg gagaccagct ttccgttcct 14340

tcttgttttc tctctgcagt gacctgggag ctgtcatttc cctcctgctc tggggacgac 14400

agctttttgc actgtactca ggcaatgatg tcacggatat ttcaggtcag tctttgagtt 14460

gggtaggagc atgcatccct ggcactgcca catcccactc tgactctctc gggtctgcat 14520

tctttctttg agaacacaca gttccccatt cttatcctga cctctgggct ttcaggactg 14580

ccaagaacat tggggatcct gccagaaatg aggggagctt gagctttcgt tggcttctat 14640

ttggggtaga aggagattga tatgcagagc agcttccact catctgactt ttcatgggtc 14700

tctgggaaca atttccaaat ggtaaactct ctggcttctc tctctttgca gatgaccgct 14760

tcccgaagcc ccctgagatt gcaaatggct atgtggagca cttgtttcgc taccagtgta 14820

agaactacta cagactgcgc acagaaggag atggtaagac ctggacaact atctcctgtg 14880

ctctacctac aacccctgct ctgacatttc catgatgggt ggtgctgagg tgatttgcca 14940

gaaagttcgt tgctctcctt ggagccagga gatttagatt ctgataagcg ttttgtcgcc 15000

agtagccatg gccctttggg cagactaact tttgtcagcc tcaagttttc tgttttgtta 15060

aggggaggtg atgccatgca gcctacctca tgtaaatctc agagtcagat ttacatctcc 15120

agcagatgtg ggaaaagaag gaatgctgat gatgatgtca ccctcaccta gtgagtcttg 15180

ctgtcctggc actgctctaa gggctttata tttatttgct cacttagtcc ttacagttta 15240

cctctgaaca gaggttatta ttctcacttt gccgataagg aaacagaggc accgacaggt 15300

tgagtatctt gcccaaattc aggcggcctg taagaggcag agtcaggatt tgaaccctga 15360

gccctccctg tactgcttgg ctgtgaccgc catcaccaca gtgtgttctg ctgggcttaa 15420

ctggtgtcca ggcacttggc ttccagcaca gcactctttc ccttcctcct tctcgtattc 15480

tctctccttt ctcccttcct gtctgcctcc tttcttcttc ttctttttaa ttcttctcct 15540

taaatgcctt ctcactctgc tctgggtgca gacttgactt ttcctttggc tcatttcttg 15600

ccttttgttt caggagtata caccttaaat gataagaagc agtggataaa taaggctgtt 15660

ggagataaac ttcctgaatg tgaagcaggt gggtgctgag cactgagcac ttaagagagc 15720

aggcaggcgt ccagcgggga acgtcctaga ggcacagcct tccagtgcgg cttcctctga 15780

gcacacaaga gccaggagga gggatgtggg agaaccgcag ctggccaggg agagacttaa 15840

gcagttaggt gatgactccc taaggatcac cagggtcttg ttcattgggg cctgaagggc 15900

actggctgaa tccactgtcg gcattgccac agatcaggag agcctgtgca tacagagagc 15960

ctgctagaga gccctgggtc taaggagaag caagctccag ggagaacaag tcaagggata 16020

acataaaatc ttaatccatg gaagcctagc aggaggctgg acatgggctg gaactcctgc 16080

ttctcgttat taggaggaac tgttgctctc tcctttccat ctcagaacca gaggcaaagg 16140

cccagcctct tctgctctta ctggtgtgga aaggccagcc tcttctgctg tgagtggtgt 16200

ggaactacca acctgcctcg tattaactgc aacatctata aagtatgagc tccagccaat 16260

gctgctctag attcctcttt cttcagagat gatgaattat tgtagctcct agccctttct 16320

tttttctttc tttctttttt tttttttttg agacagagtt ttgctctcgt cgcctaggct 16380

ggagtgcagt ggtgctaatt cctgacctca ggtgatccaa ctgcctcagc ctcccaaagt 16440

gctgggatta caggcgtgag ccaccgcatc tggcccctag ccctttcaat gaatttcagg 16500

gaattgtgaa aattcctttg ttgagataat tgtttaaata taatatagtt caccagccag 16560

gcctcaaaaa actcagtatt tctcatttcc ttatttagaa atagagcttt ttgtaatgta 16620

aacaattaaa aaaattattt taaaactgca actattggaa atgagatcag caggtggtaa 16680

ggacaaagca tttaaatctt tccagtttat gcagcagtga cagccgccaa tgctttcacc 16740

cctttctcag atggaaaggc tcttgcacat ttccactcac gagtgtcttg ctctccttga 16800

cagtatgtgg gaagcccaag aatccggcaa acccagtgca gcggatcctg ggtggacacc 16860

tggatgccaa aggcagcttt ccctggcagg ctaagatggt ttcccaccat aatctcacca 16920

caggggccac gctgatcaat gaacaatggc tgctgaccac ggctaaaaat ctcttcctga 16980

accattcaga aaatgcaaca gcgaaagaca ttgcccctac tttaacactc tatgtgggga 17040

aaaagcagct tgtagagatt gagaaggtgg ttctacaccc taactaccac caggtagata 17100

ttgggctcat caaactcaaa cagaaggtgc ttgttaatga gagagtgatg cccatctgcc 17160

taccttcaaa gaattatgca gaagtagggc gtgtgggtta cgtgtctggc tggggacaaa 17220

gtgacaactt taaacttact gaccatctga agtatgtcat gctgcctgtg gctgaccaat 17280

acgattgcat aacgcattat gaaggcagca catgccccaa atggaaggca ccgaagagcc 17340

ctgtaggggt gcagcccata ctgaacgaac acaccttctg tgtcggcatg tctaagtacc 17400

aggaagacac ctgctatggc gatgcgggca gtgcctttgc cgttcacgac ctggaggagg 17460

acacctggta cgcggctggg atcctaagct ttgataagag ctgtgctgtg gctgagtatg 17520

gtgtgtatgt gaaggtgact tccatccagg actgggttca gaagaccata gctgagaact 17580

aatgcaaggc tggccggaag cccttgcctg aaagcaagat ttcagcctgg aagagggcaa 17640

agtggacggg agtggacagg agtggatgcg ataagatgtg gtttgaagct gatgggtgcc 17700

agccctgcat tgctgagtca atcaataaag agctttcttt tgacccattt ctgtgttgtg 17760

ttcagtcttg agtctttttt atttgctcct ttatggtcca gggtagtcag aaggtataga 17820

gtctactggg agtatggcag aaaacaccct aaacccactg gaaatcccga aggtgataca 17880

aactcttcca ccttagggaa tcatgctcac tgattgagtg cctattgaat gctaggtccc 17940

agaaagttaa ctgttgtcct tgttttacag acaaggaaac agagactcag agatggtaag 18000

tgagttgctt aaggttacat agctatgaaa cagggaagca gaactttgaa cccaggtctg 18060

tttgatacaa actcagaggt cctttcactg catgctgttg cctctttaaa gcgaattagg 18120

agaaaaggca tgggccttgg tgaggaagag gctagctcaa aatgggatgg ggaaaaagtg 18180

ttttaacaca gacagtactt cagagtttgg gatctaacct accagcactc cagaaaacaa 18240

aagatgatga tgtaaaggca ggatctctgg acttgctgag tccaaatcat ggacttggcc 18300

caaacatttg ctaagtaatt taggcttcct ggccattagt tcctggtctc ttccatgaag 18360

gacttgagcc agggtaacgt ccctatcagc aataagcttc tgggactctg taaagagtgg 18420

ggaggtagag tccctgcctc agtagaagct gcttttccta cttggaacaa acccaccctt 18480

caccaggaat caaaagcaag ctgtctgagc ctcctctcct gatggcaggt catgctatta 18540

agggtctccc agaagaaagc ttctggagct ctgagaaaca gaagctcttc taacagaact 18600

ttttcttgct ataagccttc aataccacca atcaccaatg tgccttcttc tcatgcgaaa 18660

atacggcccc tcctttgcag ggtgtaaaat gacactgatg tcactctctc tcaccagggt 18720

ctggccatct ctgtgaagct ctcctcacag gcaccctgca gattcatcaa taaacttcaa 18780

caggttagcg ctagtaggcc aataagacct atattctaga acctgtgaaa gagataatag 18840

ccagaaactg gcaaacacca gtcaaactct ttaaaaaaat gcctcttaga acacagtcac 18900

ttacacattt tcctagatct cttcagcacc aagtttcttg gaagaacaag gcactctgca 18960

gttttcttaa agaattgtct gattgtatta agtggcttct ggtaaaacaa cagtaagaga 19020

aaagctctgg cattctcttg atcactcatc ttctctgcct tcctccccag caccacctcc 19080

tggcctcagc ctgtgggctt ctgcaaagtc aggtatcagg actaattagc tagcatcaaa 19140

ttcctgttaa ttccagaagc agggacaaac ctgttgggga gggacttaat gacaaaatta 19200

ggtataggtc tatacgttaa tcaaggaaaa ttctcaactt ccttaaactt acttaccacc 19260

ctagaggaga gccaaatcca ctctgatgac gtttggtcat aggaaggaaa ttggggaaaa 19320

ttacaagttt ctccaatcca tgtaaaatta agaaatcctg gggaagttgt aaagagaaaa 19380

cagtacccta ttcccttgaa aggcagaata ggtttataac ttataatcga aggtctcctt 19440

caggaaggac ttcttgaacc ccatatgtcc tcttacaaca ctccaatatt gcctgtaaaa 19500

aagtcggatg ggtcatagcg gctactgcga gaccttcgag ccattaacca aatagtccag 19560

accacccacc ctgtcattcc taacccttac accattatca agcaagatcc ctcatgactg 19620

ccagtggttc acagtaatag acctcaatga tgacttctgg gcttgcccct tagctgtgga 19680

tagctgggac atatttgctt ttgaatggga agaccctcat tccagttgga agcaatagta 19740

gtgatggaca gttctacccc aagggtttac agactctcca aacctattag tcaaagtttg 19800

gaacaagtcc tagagaattt ccctctttca tcatccttat gtctactcca atatgtggat 19860

gacctgctca tttccagagg caccaaagac caagtaaccg caattttaat tagctttcta 19920

aatttcctaa gggaacaatg gttacgggtc tcaaaaagta aactccagct tgtagaacct 19980

gaggtaaaac acatggggca cttaataagc aaaggtaagc ggaagatagg gcccgaacaa 20040

atggaaggga tcatatccct gccactgcct gagacaaaac aagaacttag aaaattcttc 20100

aggctagctg gaaattattg cctgtggatt gactcttatt cttaaaaaca aaacctctat 20160

atctaaaact tacccaagaa gggcctgacc cccttctttg gaccccacaa gaagtccagc 20220

aagttgagga actaaaacat ctacttataa ctgcccctgt cttagctctg ccatccttag 20280

aacagccatt tcactttttt gttaatataa gcaatggggc agctatagga gtactcactc 20340

aaaagcatgg ggccattgcc agcccacagc ctttctgtca aacattcttg acgtggtaac 20400

ctgtgggtgg cccgaatgta tccaatctac agcagcaact gttttattga cagaggaaag 20460

tagaaaaata acctttgggg gaagtctcat cctaagcaca ccccatcagg ttaaaaccat 20520

tcttagccaa aaagcaggga gatggcttac agattcaaga atcctaaaat atgaagctat 20580

cctattagaa aaaggtgatt taaccctaac cactgacaat gtgctcaacc ccgccacctt 20640

cctttttttt tttttttttt gagatggagt ttcactctta ttgcccatgc tagagtgcaa 20700

tggcacaatc tcggcttatc gtaatctttg cctcctgggt tcaagcaatt ctcctgcctc 20760

agcctattaa gtagctggga ttacaggcat gctaccatgc ctggctaatt tttttgtatt 20820

tttagtagag atggggtttc tccatgttgg tcaggctggt cttgaactcc tgtcctcagg 20880

tgatctgcct gccttggcct cccaaagtgt tgggattaca ggcgtgagcc actgtgcccg 20940

gcctaccccg ccaccttcct gacggaaatc caaacgctga agaccccaag cacaaatgtt 21000

tgaacttaat catcaaacta aagtaaggcc tgatctaagt gagacccctt taaaaacagg 21060

gcaccatctc tttgtagaca gctcctctcg ggtaattgaa gagaaaaaac ataatggcta 21120

ctcagtggtt gatggagaaa ctctcacaga agtagaatca gggagacttc ccaataactg 21180

gtctaaccaa acatgtgagt tgttcgcact aaatcaagcc ttaaaatagc tgcagaatca 21240

agaagggact atttacacca attccaaata tgcttttgga gtggcccata cctttgggaa 21300

gatttggact gaacaaggcc ttattactag caagggccaa aaccttgtcc acaaagagtt 21360

aatcatgcaa gtattagaaa accttcagct accagaggac atagccattg ttcatgtccc 21420

aggacaccat aaaggtctct ctttcaagag ttacttccca agaagcaccc attttccact 21480

gaaccccttg tctcccttcc ccatctgcaa cccccatctt ctcccaagca gacaaagaaa 21540

aattaaagaa aataggggct aacaaaaact cagaagagaa acggatactg ccagataaca 21600

aagaaatgtt gtccaaaccc ctaatgacag aaatcttaac acaacttcac caagggaccc 21660

attggggtcc ccaagccatg tgtgatgcag tccttagaac ctatgggtgt acagggattt 21720

ataccctcac caggcaagtc acagacagtt gtatggtatg cagaaaaacc aataaacttt 21780

aagaaaactg cccttcggcg gaagaaatcc tgggctaagg ccatttccaa agtgtccata 21840

ttgacttata ccgaaatgcc ccaaataggc tgcctcaaat gtttgtcagt tatagtggac 21900

catctcactc actgggtgga agccattcac ctgccaagtg caactgccaa taatgtagtt 21960

aaagtactaa tagaagactc catacccaga ttcagattaa tagagaacat tgactcagac 22020

aatggaaccc attttactgc acaggtcatt aaaggattaa cccagacact agagataaaa 22080

tgggaatatc atactccctg gcacccaccc tcatcaggaa aagcagaaag aatgaatcaa 22140

aataaataaa tcacctaact aaactaatct tggaaacccg actgccatga actaagtacc 22200

tccctgtcgc cttactaaga attctcatgt ttgtccttat catcgataag 22250

<210> 10
<211> 662
<212> PRT
<213> Homo sapiens

<400> 10

Met Val Val Ser Glu Val Asp Ile Ala Lys Ala Asp Pro Ala Ala Ala
1               5                   10                  15

Ser His Pro Leu Leu Leu Asn Gly Asp Ala Thr Val Ala Gln Lys Asn
            20                  25                  30

Pro Gly Ser Val Ala Glu Asn Asn Leu Cys Ser Gln Tyr Glu Glu Lys
        35                  40                  45

Val Arg Pro Cys Ile Asp Leu Ile Asp Ser Leu Arg Ala Leu Gly Val
    50                  55                  60

Glu Gln Asp Leu Ala Leu Pro Ala Ile Ala Val Ile Gly Asp Gln Ser
65                  70                  75                  80

Ser Gly Lys Ser Ser Val Leu Glu Ala Leu Ser Gly Val Ala Leu Pro
                85                  90                  95

Arg Gly Ser Gly Ile Val Thr Arg Cys Pro Leu Val Leu Lys Leu Lys
            100                 105                 110

Lys Leu Val Asn Glu Asp Lys Trp Arg Gly Lys Val Ser Tyr Gln Asp
        115                 120                 125

Tyr Glu Ile Glu Ile Ser Asp Ala Ser Glu Val Glu Lys Glu Ile Asn
    130                 135                 140

Lys Ala Gln Asn Ala Ile Ala Gly Glu Gly Met Gly Ile Ser His Glu
145                 150                 155                 160

Leu Ile Thr Leu Glu Ile Ser Ser Arg Asp Val Pro Asp Leu Thr Leu
                165                 170                 175

Ile Asp Leu Pro Gly Ile Thr Arg Val Ala Val Gly Asn Gln Pro Ala
            180                 185                 190

Asp Ile Gly Tyr Lys Ile Lys Thr Leu Ile Lys Lys Tyr Ile Gln Arg
        195                 200                 205

Gln Glu Thr Ile Ser Leu Val Val Val Pro Ser Asn Val Asp Ile Ala
    210                 215                 220

Thr Thr Glu Ala Leu Ser Met Ala Gln Glu Val Asp Pro Glu Gly Asp
225                 230                 235                 240

Arg Thr Ile Gly Ile Leu Thr Lys Pro Asp Leu Val Asp Lys Gly Thr
                245                 250                 255

Glu Asp Lys Val Val Asp Val Val Arg Asn Leu Val Phe His Leu Lys
            260                 265                 270

Lys Gly Tyr Met Ile Val Lys Cys Arg Gly Gln Gln Glu Ile Gln Asp
        275                 280                 285

Gln Leu Ser Leu Ser Glu Ala Leu Gln Arg Glu Lys Ile Phe Phe Glu
    290                 295                 300

Asn His Pro Tyr Phe Arg Asp Leu Leu Glu Glu Gly Lys Ala Thr Val
305                 310                 315                 320

Pro Cys Leu Ala Glu Lys Leu Thr Ser Glu Leu Ile Thr His Ile Cys
                325                 330                 335

Lys Ser Leu Pro Leu Leu Glu Asn Gln Ile Lys Glu Thr His Gln Arg
            340                 345                 350

Ile Thr Glu Glu Leu Gln Lys Tyr Gly Val Asp Ile Pro Glu Asp Glu

355                 360                 365

Asn Glu Lys Met Phe Phe Leu Ile Asp Lys Val Asn Ala Phe Asn Gln
    370                 375                 380

Asp Ile Thr Ala Leu Met Gln Gly Glu Glu Thr Val Gly Glu Glu Asp
385                 390                 395                 400

Ile Arg Leu Phe Thr Arg Leu Arg His Glu Phe His Lys Trp Ser Thr
                405                 410                 415

Ile Ile Glu Asn Asn Phe Gln Glu Gly His Lys Ile Leu Ser Arg Lys
            420                 425                 430

Ile Gln Lys Phe Glu Asn Gln Tyr Arg Gly Arg Glu Leu Pro Gly Phe
        435                 440                 445

Val Asn Tyr Arg Thr Phe Glu Thr Ile Val Lys Gln Gln Ile Lys Ala
    450                 455                 460

Leu Glu Glu Pro Ala Val Asp Met Leu His Thr Val Thr Asp Met Val
465                 470                 475                 480

Arg Leu Ala Phe Thr Asp Val Ser Ile Lys Asn Phe Glu Glu Phe Phe
                485                 490                 495

Asn Leu His Arg Thr Ala Lys Ser Lys Ile Glu Asp Ile Arg Ala Glu
            500                 505                 510

Gln Glu Arg Glu Gly Glu Lys Leu Ile Arg Leu His Phe Gln Met Glu
        515                 520                 525

Gln Ile Val Tyr Cys Gln Asp Gln Val Tyr Arg Gly Ala Leu Gln Lys
    530                 535                 540

Val Arg Glu Lys Glu Leu Glu Glu Glu Lys Lys Lys Lys Ser Trp Asp
545                 550                 555                 560

Phe Gly Ala Phe Gln Ser Ser Ser Ala Thr Asp Ser Ser Met Glu Glu
                565                 570                 575

Ile Phe Gln His Leu Met Ala Tyr His Gln Glu Ala Ser Lys Arg Ile
            580                 585                 590

Ser Ser His Ile Pro Leu Ile Ile Gln Phe Phe Met Leu Gln Thr Tyr
        595                 600                 605

Gly Gln Gln Leu Gln Lys Ala Met Leu Gln Leu Leu Gln Asp Lys Asp
    610                 615                 620

Thr Tyr Ser Trp Leu Leu Lys Glu Arg Ser Asp Thr Ser Asp Lys Arg
625                 630                 635                 640

Lys Phe Leu Lys Glu Arg Leu Ala Arg Leu Thr Gln Ala Arg Arg Arg
                645                 650                 655

Leu Ala Gln Phe Pro Gly
            660

<210>  11
<211>  2828
<212>  DNA
<213>  Homo sapiens

<400>  11
atctgattca gcaggcctgg gttcgggccc gagaacctgc gtctcccgcg agttcccgcg      60

aggcaagtgc tgcaggtgcg gggccaggag ctaggtttcg tttctgcgcc cggagccgcc     120

ctcagcacag ggtctgtgag tttcatttct tcgcggcgcg gggcggggct gggcgcgggg     180

tgaaagaggc gaagcgagag cggaggccgc actccagcac tgcgcaggga ccggaattct     240

gtggccacac tgcgaggaga tcggttctgg gtcggaggct acaggaagac tcccactccc     300

tgaaatctgg agtgaagaac gccgccatcc agccaccatt ccaagcttac tttgcaaaga     360

aggaagatgg ttgtttccga agtggacatc gcaaaagctg atccagctgc tgcatcccac     420

cctctattac tgaatggaga tgctactgtg gcccagaaaa atccaggctc ggtggctgag     480

aacaacctgt gcagccagta tgaggagaag gtgcgcccct gcatcgacct cattgactcc     540

ctgcgggctc taggtgtgga gcaggacctg gccctgccag ccatcgccgt catcggggac     600

cagagctcgg gcaagagctc cgtgttggag gcactgtcag gagttgccct tcccagaggc     660

agcgggatcg tgaccagatg cccgctggtg ctgaaactga agaaacttgt gaacgaagat     720

aagtggagag gcaaggtcag ttaccaggac tacgagattg agatttcgga tgcttcagag     780

gtagaaaagg aaattaataa agcccagaat gccatcgccg gggaaggaat gggaatcagt     840

catgagctaa tcaccctgga gatcagctcc cgagatgtcc cggatctgac tctaatagac     900

cttcctggca taaccagagt ggctgtgggc aatcagcctg ctgacattgg gtataagatc     960

aagacactca tcaagaagta catccagagg caggagacaa tcagcctggt ggtggtcccc    1020

agtaatgtgg acatcgccac cacagaggct ctcagcatgg cccaggaggt ggaccccgag    1080

ggagacagga ccatcggaat cttgacgaag cctgatctgg tggacaaagg aactgaagac    1140

aaggttgtgg acgtggtgcg gaacctcgtg ttccacctga agaagggtta catgattgtc    1200

aagtgccggg gccagcagga gatccaggac cagctgagcc tgtccgaagc cctgcagaga    1260

gagaagatct tctttgagaa ccacccatat ttcagggatc tgctggagga aggaaaggcc 1320

acggttccct gcctggcaga aaaacttacc agcgagctca tcacacatat ctgtaaatct 1380

ctgcccctgt tagaaaatca aatcaaggag actcaccaga gaataacaga ggagctacaa 1440

aagtatggtg tcgacatacc ggaagacgaa aatgaaaaaa tgttcttcct gatagataaa 1500

gttaatgcct ttaatcagga catcactgct ctcatgcaag gagaggaaac tgtaggggag 1560

gaagacattc ggctgtttac cagactccga cacgagttcc acaaatggag tacaataatt 1620

gaaaacaatt ttcaagaagg ccataaaatt ttgagtagaa aaatccagaa atttgaaaat 1680

cagtatcgtg gtagagagct gccaggcttt gtgaattaca ggacatttga gacaatcgtg 1740

aaacagcaaa tcaaggcact ggaagagccg gctgtggata tgctacacac cgtgacggat 1800

atggtccggc ttgctttcac agatgtttcg ataaaaaatt ttgaagagtt ttttaacctc 1860

cacagaaccg ccaagtccaa aattgaagac attagagcag aacaagagag agaaggtgag 1920

aagctgatcc gcctccactt ccagatggaa cagattgtct actgccagga ccaggtatac 1980

aggggtgcat tgcagaaggt cagagagaag gagctggaag aagaaaagaa gaagaaatcc 2040

tgggattttg gggctttcca gtccagctcg gcaacagact cttccatgga ggagatcttt 2100

cagcacctga tggcctatca ccaggaggcc agcaagcgca tctccagcca catccctttg 2160

atcatccagt tcttcatgct ccagacgtac ggccagcagc ttcagaaggc catgctgcag 2220

ctcctgcagg acaaggacac ctacagctgg ctcctgaagg agcggagcga caccagcgac 2280

aagcggaagt tcctgaagga gcggcttgca cggctgacgc aggctcggcg ccggcttgcc 2340

cagttccccg gttaaccaca ctctgtccag ccccgtagac gtgcacgcac actgtctgcc 2400

cccgttcccg ggtagccact ggactgacga cttgagtgct cagtagtcag actggatagt 2460

ccgtctctgc ttatccgtta gccgtggtga tttagcagga agctgtgaga gcagtttggt 2520

ttctagcatg aagacagagc cccaccctca gatgcacatg agctggcggg attgaaggat 2580

gctgtcttcg tactgggaaa gggattttca gccctcagaa tcgctccacc ttgcagctct 2640

ccccttctct gtattcctag aaactgacac atgctgaaca tcacagctta tttcctcatt 2700

tttataatgt cccttcacaa acccagtgtt ttaggagcat gagtgccgtg tgtgtgcgtc 2760

ctgtcggagc cctgtctcct ctctctgtaa taaactcatt tctagcagac aaaaaaaaaa 2820

aaaaaaaa 2828

<210> 12
<211> 45622
<212> DNA
<213> Homo sapiens

<400> 12
atcattcttc acagaactag aaaaaacatt cttaacattc atatggaacc aaaaaaggag 60

cccgcatagc caaagcaaaa ctaagcaaaa agaataaacc tggaggcatc atattaccca 120

acttcaaact atactacaag gctatagtcc ccaaaacagc atgatactga tataaaaata 180

ggcacataga ccaatggaac agaatacata acccagaaat aaagccaaat acttatagct 240

gactgaactt tgacaaagca aacaaaagca taaagtgggg aaaggacacc ctattcaaca 300

catggtgctg ggataattgg caagctacat gtagaagaat gaaactggat cctcatatct 360

tgccttatac aaaagtcaac tcaagatgga tcaaagactt aaatctagga cctaaaacca 420

taaaaattct agaagataac attggaaaaa cccttgtaga cattggctta ggcaaagagt 480

tcatgaccaa gaacccaaaa gcaaatgcaa caaaaatgaa gacaaatgga tcggacttaa 540

ttaagctgaa aagcttctac acagcaaaag aaataattag cagagtaaat agacaacata 600

cagagtggga gaaaattttg caaactatgc agccaacgaa gggctaatat ccagaatcta 660

caaggaactc aaacaaatca gcaagaagaa aacaaatact ccctcacaaa gtgggctaag 720

gatgtgaata gacaattctc aaaagaagat atacaaatgg ccaacatata aaaaaaaatc 780

aacatcacta attattagga aaatgcaaat caaaaccaca atgggatacc accttactcc 840

tgcaagaagg gccacaatca agaaatcaaa aaataataga tgttggcatt aatgtggtga 900

aagggatcac ttttacactg ctggcaggaa tgtaaactag tacaaccaat ggaaaacagt 960

agagtttcct taaataactg aaagtagatc taccatttga tccaacaatc ccactagtgg 1020

gtatttagcc agtggaaaat aagtcattat atgaaaaaga cacttgcaca cacatgttta 1080

tagcagcaca attccccaag tgcccaccaa tcaacaagtg gacaaaatgt tatatatata 1140

tgtgtatata tatgtatata tatataatgt gatatatatg tatatatatg tatatatata 1200

taatgtgata tatatgtata tatataatgt gatatatatg tatatataat aatgtgatat 1260

atatgtgtat atataatgtg atatatatgt atatatatat aatgtgatat atatgtatat 1320

atatataatg tgatatatat gtgtgtgtgt atatatataa tgtgatatat atatgtgaat 1380

actactacta ctactactac tactactaat actcagccat aaaaaggaat gaaataatgg 1440

cattcacagc aacctggatg gagttggaga ccattattct aagtgaagta actcgggaat 1500

aaaaaccaaa cattgtatat tctcactcat aagtgagatt atacaagcta tgaggatgca 1560

aagtcataag aatgatacaa taaacattgg gaactcaggg ggaagggtga gggtggggtg 1620

agggataaaa gactagacat cgggtacagt atacactgct tgggtgatgg gtgcaccaaa 1680

atcccagaaa tcacgattaa agaacttgtc aatgtaacca aacaccacct gttcctcaaa 1740

aactattgaa ataaaaaata aatacataaa agaagttgag gttaaattaa tctaagtagg 1800

gagtgcattt gggccaagct tgaggattgc aacgttggag cacagattta agttgccctg 1860

aatctacact ctgataagtg gcatttataa gtggattttt aaaggcaaaa gggggagaca 1920

gggagagggc tgatacaaag ctgtttgtca ggaattctta ttggtttaca gaaataacat 1980

tgataagtgc ttattagtcc attttcatgc tgctgataaa gacatacctc agactgggca 2040

atttacaaaa gaaagaggtt tattggacct atagttccac atggctgagg aggcctcaca 2100

atcatggtgg aaggcaagga ggagcaagtc acatcttaca tggatgacag caggaaaaaa 2160

gagagagctt gtgcagagaa actccctttt ttaaaaccat cagatcttgt gagacccgtt 2220

caggatcatg agaacagcac aggaaagacc cacccccaag attcaatcat ctcccaccgg 2280

gtctctccca caacatgagt gaattatggg agctacagga tgagatttgg gtgaggacac 2340

agagccaaac catatcagag tgattgacta tacattgtta agctacaggg tgtggattac 2400

agcgtccagt gtggcattat taggttaatc catagctatt aatacctgtg gcaatagtaa 2460

gaggtttcaa gagatgaaca cacagctgaa agtggggagt agggagtggt tgctgtctta 2520

ttttaatgtc tctctgggcc tgataatttt aaaagccttg cattcttcgg atacaagttc 2580

ctttcttttt ttcaagaaca acagagaaag gctgcctggc tacctttacc tgcagcacct 2640

ctgatagtgg ctgcttggca gctggttagg taaacccaat ttctactgga taaacttcat 2700

ggacattcac ccatgttagt actggggaat gctctgaaat agcaaacttg tccatacagc 2760

tcccttgctg ctcagagggt ctggagagga acagcagagg cccagcccca aatacttctt 2820

gtaccggctt ctattatgag aggtgatggc acctttaggc acttatattt caggtagaag 2880

gagcaggaat aaagaagaga gggccacaat ctgaaatatc agttctggat ctccctgggg 2940

gcaagacact ggcccttagc tgagctattc tgtcatcact gtgagccctc atgctcgacc 3000

tcaccggagc acccctcgat catgtgctga atgcctagca cgcgcctggc actgtggggt 3060

acagaaatct agagcacaga gctgagtttt cagggcttag ctgtcctcac atctgtgaca 3120

caaacaagga tcaaggaaag ttttgttttt gttattttta ttttttttta agttggggcc 3180

ttactctgta gcccaagcta gagtgcagtg gtgcaaacat agctcattgc accctcgaac 3240

tcctgggctc aagtcatcct cccaccttgg cctccagagt agctggaagc ccagctcaag 3300

gaaagcatta acatgttcta cttttcacca taactcctgt gtattgggaa atggaaaatg 3360

agcttttgaa gtggcctatt cgcttcctcc atgagtactt cctgaacact ttcctcttgg 3420

ccactgctcc caagaccccc tcaaagtctc tctcctggac ttccagcttc tggaatgagc 3480

cccaagagtg gcacttagct ccagctccta gtagctcact ccttccttgc tcacctgtgc 3540

cccccacctg tctccttagg ggcagtggct ttgtcttact catttgcata tcacaaatgt 3600

ccagcataga agccagcatg gggcccagta agtgtttgtg gagaaagaaa gggaagaggt 3660

gaagaaggga ggagacggag ggagaaaagg agggcaagag aggggatgtc atgtcatgtc 3720

atgtcatgtc atgtcagttc tcccttccta gatctggaca agggaaagaa aagcaagcaa 3780

gcctgaccgc ctcagtgcag accctctaag atgcaagcct tccccaggta agacagttcc 3840

gcagggtgct tctgtgggct ggctttctga gcagccatct caaagtatgc gaaagaaata 3900

gattttgggc cgggcacggt ggctcatgcc tgtaatccca gcactttagg aggccgaggc 3960

gtgtggatca cctgaagtca ggagttcaag accagcctgg ccaacatggt gagaccttgt 4020

atctactaaa aatacaaaaa ctagccgggc ttggtggcgc atgcctgtag tcccagctac 4080

tcaggaggct gaggcaggag aatcgcttga acccaggagg taaatgttgc agtaagccga 4140

gattgcccca ctgcactcca gcctggatga caagagtgag actccatctc aaaaaaaaaa 4200

aaaaaaaaag attttagggg taaaatattt tgctttcctt cagtggctgg gccacctgta 4260

cacaatgcct gaacccaggt cctgcagccc ccagcttggc tctccaaagc tcaccagtat 4320

caaaggattt cagcttctgc tcctctccgc agggctctct ggtgggcccc agacagagat 4380

gaaggaaaag tctggccttg accttgagga ccaaaagcga ccacgtcaga cgccagcagt 4440

ctcgtgccca gttgccaagg gatcacctga ggaaggccct taaaatacag gtggtaatgc 4500

aggggtcttg ggatggcccc ggaccgttca tttctaataa tttcccaggt gaagccaatg 4560

ctgctgttcc aggaaccacc ctttgagaag cacgggtcta taccacacgc acagaagagg 4620

aaacgaaacg aggcaaagga aaccagtccc tttactcgcg cccaggcagg acaaagcggg 4680

caagggtggg ggcgggggcg cgggcgcggg cgcgggcagg cggacggact tgcaggaaat 4740

gcagccggga ggatgggcag gttgtgggat gctccgccgt tccgcgacca cccgcctccc 4800

cggcccagca gaaccgggtc caggctgggg gaggtgctag cggtgtatgt gagtaacact 4860

gccccctcgt cgtggcaccg ccccctcttt ccctgccccc ttccccgccc cctggattct 4920

gaagtctgaa gtcatgcaat ttgacttttc ctttctggaa accagcgagc agaaatgaaa 4980

ccgaaactga attgtccggg aaattcgcgg tgggggcgga gagcgcaggg agaagtaagc 5040

ccagtgcagg atcctgaggc ccgtgtttgc aggaccaggg ccggccttcc gattccccat 5100

tcattccaga agcaccgaac cacgctgtgc ccggatccca agtgcagcgg cacccagcgt 5160

gggcctgggg ttgccggttg acccggtcct cagcctggta gcagaggcca ggccagtgcc 5220

acaaggtcag gctccactgg gccctgcccg cccaggagcc tgtggtgtca ggcacctgaa 5280

ggggtgggtt gcccctccac acctgtgggt gtttctcgtt aggtggaagg agagacttgg 5340

aaaagaaaaa gacacagaga caaagtatag agaaagaaat aaggggaccc agggaaccag 5400

cgttcagcat atggaggatc ccgccagcct ctgagttccc ttagtattta ttgatcattt 5460

gtgggtgttt ctccgagagg gggatgtgtc agggtcacaa gacaatagtg gggagagagt 5520

cagcagacaa acacgtgaac aaaggtcttt gcatcataga cagggtaaag aatcaagtgc 5580

tgtgctttta gatatgcata cacataaacg tctcaatgct ttacaaagca gtattgctgc 5640

ctgcatgtcc cacctccagc cttaaagtgg tttttcccta tctcagtaga tggaacgtac 5700

aatcgggttt tataccgaga cattccattg cccagggacg ggcaggagac agatgccttc 5760

ctcttgtctc aactgcaaga ggcatgcctt cctcttatat taatcctcct cagcacagac 5820

cctttatggg tgtcggactg ggggacggtc aggtctttcc cttcccacga ggccgtattt 5880

cagactatca catggggaga aaccttggac aatacctggc tttcctaggc agaggtccct 5940

gcggccttcc gcagtgtttg tgtccctggg tacttgagat tagggcgtgg tgatgactct 6000

taaggagcat gctgccttca agcatctgtt taacaaagca catcttgcac cgcccttaat 6060

ccatttaacc ctgagtggac acagcacatg tttcagagag catggggttg ggggtaaggt 6120

tatagattaa cagcatccca aggcagaaga atttttctta gtacagaaca aaatggagtc 6180

tcctatgtct acttctttct acacagacac agtaacaatc tgatctctct tgcttttccc 6240

cacaggcacc taagtccacc tgggcctgga gcaggacagg ttgcaaaaga aaatatctcg 6300

ggacccccaa actccttatg ctaagggaaa catcgagcct gggaactgag ccatcaacgc 6360

tgccattctt tttcccaaac agaaccctgt tgtcagaggt acacccagag caactccaca 6420

ccgggtgcat gccacagcaa ctccatctta aataggagct ggtaaaacga ggctgatacc 6480

tactgggctg cattcccaga cgggtaaggc attctaagtc acagggtgag ataggaggtc 6540

agcacaaggt cataaaggtc ataaagacct tgctcataaa acaggttgca ataaagaagc 6600

tggttaaaac tcaccacaac caagatggcc acgagagtga cctgtggttg tcctcatttt 6660

tacactccca ccagcgcatg acagtttaca aatgccatgg caatgtcagg aagttaccct 6720

acatgtatgt ctaaaacggg gaggcatgaa taatccaccc cgtgtttagc atatcatcaa 6780

gaataaccac aaaaatgggc aaccagcagc cctcagggtt gctctgtgga gtagccattc 6840

tcttactcct tcacttcagg caggggagat gaatttccct ttgagcctgt ggacagaggg 6900

cagcctggca gagggacagg catcaacaaa gcctctgagt gtgacctggg tcctgagttc 6960

agggatggca atggggtaac tgagactgga tcagaagttg cctggaagaa gagaggccca 7020

ttatcagagt ataagagtgt gaaacttatc tggtaggaaa aaaaaaagag taactgctgt 7080

tcgtttaata gagcacacat cctctgcaat tcaggtggga ggttgtgagc ttcttaagta 7140

cagaagctgc ctcttgattt cagcatagcg aggaggtgct gaagagcgca ggtttggaga 7200

atgatcacct ggattggaac catagctcta ccaatatgga acccagctcc ttaggcctcg 7260

gtcttctcat ggagaacatg gtgtgataat cctactcctc tgggagggtg gctgttaagg 7320

tgagtgattg cacacgagtc cctctagctg atgttaccga gcggtccttg tttttagagc 7380

tcccaagagg gtggcgggcc acttccaaga tggcggcaag cctcttgttc tctgacctgg 7440

ggttcttggc ctcacagatt ccaaggaatg gaatcttggg gcatgtggtg agtgttctca 7500

ttaggacgaa cccaggcact tagacatgcg ggaacaatgg cgagccttta gcccgaatgg 7560

gagcggcaat gggtgcctcg ctggatcagg agcacagcgg acacactgcc ggatccagag 7620

gggtggacat cagtggtggg tctgtgatgg cggcaaacaa cagcagacag caaatggcag 7680

tggtggatgg caagcgaaag ctcagctcca gccataacaa acacggacca gaagagtgtg 7740

cagttgcaag atttaacaga gtgaaaacag acgtcccata caaaggaggg gaacccaaag 7800

ggggttgcca ttgctgggtc gaatgcctgg gtttctgtct caatcactgt ccctccccct 7860

gtgctctcag gcaatagatg attggctatt tctttacttc ctgtttttgc ctaattagta 7920

ttttagtgag ctctctttac tacctgattg gtcgggtgtg agctaagttg caagccctgt 7980

gtttaaaggt ggatgtggtc accttcccag ctaggcttag ggattcttag tcggcctggg 8040

acatccagct cgtcccgtct ctcactgaca cctaacacat cctacatgct cagtagacag 8100

gagctattat tgtatgactg actgctgaat tacaccccaa aacaaccacc tatactcccc 8160

aaggccctgc catatcatat gcatattaga ggcaatgaag caccaaaatc taaggccagg 8220

gaaggactgc ggtggacaag ggaagtgcct agggcgttaa aacttaagga ggcattgcac 8280

caactgggag tgagctgtgt gtgctgtgtt tcctgggcag ggcgcctcac tgcccttccg 8340

agtcctgccc tggctgaggt tgtgcctcta tctggttatc agttgcctgc agctctttct 8400

gattttgttc gtgaacaagg gcccttaggg aagcctcccg ctgttggaaa cagcagcagc 8460

atcacccaga gtgaagtctc tgttcacatg agtgccagct ctgctcacac ctaccctgcg 8520

ttcccaaggc actgttcctg cttcagaaag catcagccca gcctgctcac agtcccccag 8580

gatgtgctgt tgcctgggtt cttgactagg aagtaaaagg cccagagggg ttgagtgtgt 8640

gtgtgtgtgt gtgtgtgtgt gtgtgtgtgt taaagtgagg tcattgctaa ttcctgggag 8700

aacttgtgtt ccccttggat gaaccccttg gatgaagaac cccttggatg gggttgccca 8760

gccctaggag tggatcctgc aaagcaaaat agatttcagc acagggagct ggctgggggg 8820

ctcctccccc tcttgtaggt gcagcagtat tgaatttgat tccatccagg tttcccctga 8880

ccccaggacc taaagctggg gcaggttctg ggccttgagg ttggttgggg gtgacccttc 8940

ctaatcatgg aagtctttag cgggggcttg taaggactgg tgagctgagc taaggagaat 9000

ggaggtgggg tgccaaccgc tcccaaaggg aaatgccatc tcccactcac agccagttag 9060

ccgagaatgg gagctcccaa agggaggacc acccatgggt ctgcttgact cagccctccc 9120

caaccccttt cacctttgca gtaaaactct agccaaggaa gacaaagaga cctttggaga 9180

ccaaaacaga acttttaatt cgggccaaca gcaggctcat gcccaaaatg gctgccaacc 9240

ccaacaaaga aagcagctag cttatatgtc gtttgagatg ggaaaaacaa ggtaggatac 9300

aggtttcaga caaagacagt aaattactta acccgtgaca attctgagga aactggcaat 9360

tcagttatat tgactagtca tcctccaagc tggactaggg ttggaggctg gggtcccgag 9420

gcaggtgata agcttttgag ataagcttgc atctgcaact tgttacaatg ctgggagggg 9480

ctgcttaaaa ttttagccta tgtgttactt ctaaatagct tatactaaat gttaactgtt 9540
ttcatgtgcg tccatgtgaa gagaccacca aacaggcttt gtgtgagcaa catggctgtg 9600
tatttcacct gggtgcaggc gggctgagtc cgaaaagaga gtcagcgaag ggagataggg 9660
gtggggccgt tttataggat ttgggaaggt aatggaaaat tacagtcaaa gggggttgtt 9720
ctctggtggg caggggtgaa tctcacaaag tacattctca agggtgggga gaattacaaa 9780
taaccttctt aagggtgggg aagattacaa agtacattga tcagttaggg tggggcagga 9840
acaaatcaca atggtggaat gtcatcagtt aaggctgttt ttacttcttt tgtggatctt 9900
cagttacttt aggccatctg gatgtatacc tgcaagtcac aggggatgcg atggcctggc 9960
ctgggatgcg atggcctggc ctgacaacta ttacctatgt tatgtttatt attttaagct 10020
ttattattac tattttattt attttatttt attttccttc cacacacccg tttccaccct 10080
ggagaggcca gatgagccag actccaggga ggcctagaag tgggcaaggg gaaacgggaa 10140
aggaggaaga tggtatgggt gtgcctggtt aggggtggga gtgctggacg gagttcggga 10200
caagaggggc tctgcagcca ttggcacaca atgcctggga gtccctgctg gtgctgggat 10260
catcccagtg agccctggga gggaactgaa gacccccaat taccaatgca tctgttttca 10320
aaaccgacgg ggggaaggac atgcctaggt tcaaggatac gtgcaggctt ggatgactcc 10380
gggccattag ggagcctccg gagcaccttg atcctcagac gggcctgatg aaacgagcat 10440
ctgattcagc aggcctgggt tcgggcccga gaacctgcgt ctcccgcgag ttcccgcgag 10500
gcaagtgctg caggtgcggg gccaggagct aggtttcgtt tctgcgcccg gagccgccct 10560
cagcacaggg tctgtgagtt tcatttcttc gcggcgcggg gcggggctgg gcgcggggtg 10620
aaagaggcga agcgagagcg gaggccgcac tccagcactg cgcagggacc ggtgagtgtc 10680
gcttctgggg gcagcgccca gtaaccgcgc taggagcgcg gagaagggca ttgggagagc 10740
ggcgttcgtg gcggagacta gcgctccgga gcacgggcac gacgggggca ccttctcggc 10800
tgctagtaac taacaataat aataatcata atcatagcaa gggcgctgat gggcgggctc 10860
ggagcacgcc tgattctggt tcccaccagg ctgcccaggc tcctgatgac gcatcagaaa 10920
catcccccta acccgcggcc ttcctgcagg agaggttggg aaggggtggg ggacggggct 10980
cgggggaggt ctccgaggga ctctagtaag cggggaaggg cgccgggaaa gtttcagatc 11040
cacggctgcg cgggccacga gcccacccga acgccgacca ctgctttccg tcgacttcta 11100
tttcctggga acgcgcgaaa gcaaacccaa gtcagactgc ggaggtcgct ggggagggaa 11160
ggttcaagga gttctcgccg atcctgctga ataaaggggg ttccgagctg ggccgagatg 11220
gggcatgcgc gggaagaccc ctgcccgctg ttccccccca ccgccccagt ggatgccatg 11280
cctggggcct ccccggcgcg tggggctgac gcaccctcgg ggtccatcgt agttggccgg 11340
gatcgtggag tgggtgcggt ggacgaaggg aggcaggaca gtcccggggg tggcagaagg 11400

agcccgggca cagctgagac ctgcgctccc atcccaccaa cactcacagc aggtgctgcc 11460

gagctgggca attgggatgg cccaagttat ttggttaaat tttaaatcac gtttgttact 11520

gggaagtaga gtccagtgat gctaaccgcg cctctacctc caccaccggt gtcagtccca 11580

aagggctcct aaaatggctg tgtcatcttt cagccttgga ccgcagttgc cggccaggaa 11640

tcccagtgtc acggtggaca cgcctccctc gcgcccttgc cgcccacctg ctcacccagc 11700

tcaggggctt tggtaggtag cagtgcattt ggtctaaagg gcaagatgtt ctctctttta 11760

ttcataacaa atttaaatac cagcagggtt tggggggaaa aacgctttca gaagaaaagg 11820

tgaatgtcag tcctgcaaga gttagtttta aaactagact gaattggcac atgtatacct 11880

atgtaacaaa cctgcacgtt ctgcacatgt accccagaac ttaaaagctt ataaaaaaag 11940

aaaaaactag actggattat gttgggaaag tgtagcctct tccatcttag gcatttccta 12000

gaacgtaggc agtaggtggt ccttattagg agttttggga gaggaagggg gctgaatcct 12060

acctcccatc cctgctcctc tatggggtct gagctgagga agcttcacca caaggagaga 12120

accccctgac aaccctggat gccaccttta ccctcactgc aggaattctg tggccacact 12180

gcgaggagat cggttctggg tcggaggcta caggaagact cccactccct gaaatctgga 12240

gtgaagaacg ccgccatcca gccaccattc caaggtaagg cagaaatgaa gtgggccgtt 12300

gggttctttc ttttctttct ttcttttttt tgagacaagg tctcactctg tcgcccaggc 12360

tggagtgcag tggcgccatc tcagctcact gcaacccccg cctcccaggt tcaagcgatt 12420

ctggtgcctc agtctcctga gtagctggga ttacaggcac acatcaccac acctggctaa 12480

tttgtatgtg tttagtagag acagcatttc accatattgg ccaggctggt ttcaaactcc 12540

tggcctcaaa tgatccaccc gccttggctt cccaaagtac tgggattaca ggcacgagcc 12600

actgcaccca gtcaggttca ttttagttgt tatgttaacc aggtttcctg cacctgtgcg 12660

ctaactttca ctttcccaaa aggtttcagg gtgacccagc aggcaatgag tgattctcaa 12720

attcaggatt tattgtgaga gattcacaca cacaattgag cagacattca cagtacaatg 12780

attaaaggga gtgatagggt aaggacccac agtggaggct ctggaggcca gcccactgac 12840

agccactcca gggagtccag aagtcccgct ctagtgctgg gtggtggagg gaaatctgtt 12900

cctccaggga cctcgtcctc ggctgcccag ctgccaaagt caggaataag ctttcagaaa 12960

tctcactgcc aagattccga aaacgcttca gacattgcta gtcccttgtc gcttttgcga 13020

tcctccacag gtgtgcgtgc cactgggtcc ttattcactg gggtctctgg tggcattggg 13080

ccacagcaag tgttccctca tccccttagt ctaccacaca catgcttacc actttgaaga 13140

aaaacccctt tactatgagc gaaagtgaga aacacgtatg tttattgttt ctaaagaaag 13200

aaacttaata tgggcttaat gctacctagt gagtgcctcc attttgagac attagggtca 13260

caagtcatta ttatatatca tgggcacaaa cctgccctgg gcagggacgg aaggaagccc 13320

ctgcacaggg gcagttgctc aggatgtgag aagagcctgg tgcataaccc catccatgcc 13380

cacctaacat ctcaggctct gaccagtggg gctgtgcagt agcgagtgga tggagggctg 13440

gaaccctgca gcctcctctc caaacacagg gtgcagccaa gacattttag gagcaatttg 13500

ggatggagag ctaggagtcg ccacctcttg gctcttccaa ggccggaact ggtgcctgca 13560

ctcagttcag tttgaagact gcagctggat gccaagttcc atggaggagt aagaaaccgg 13620

tttgaactcc cgagattgcc ctgcccctga aatccaaact gatgttccga atgatcaggg 13680

aaaggtacaa acgtttatgg tttacagaca aaacccataa ggtttagctt tcagagaatc 13740

tcattttatg aagcaaatta gggaagggaa tctactcacc aagtcctgtt tcagctgatt 13800

gagtggaacc tgtggtcatg tggtacaagt cctggtctca atgatgctcc ttatctggct 13860

gcagaaaggc caactgaggc aaccatagcc cagaagactg gtactcctga gaggcagatg 13920

aagtggtggt ctttgatatc gagcctggga tgccctgggc acatgaggta tttccaaagg 13980

catgggagtt ttagggaata aattcccaga ttgtcagact ccataagtac cgtttacaat 14040

ggattacctt ttataaccat cccaatccta cctgacaaaa gaggtgggca gattacgagg 14100

tcaggaaatt gagaccatcc tggctaacac tgtgagaccc catctctact aaataaatac 14160

aaaaaattag ccgggtctgg tggcgtgcac ctgtagtccc agctacttgg gagcctgagg 14220

caggataatc gcttgaaccc gggaggtgga ggttgcagtg agccaagatt gcgccactgc 14280

tctccagcct ggtgacaaag cgagactgtc tcaaaaaaaa aaaaaaaaag aaagaaatac 14340

atccctttct tcccttccaa atcgagcaag gatgcctgcc ctggaagtgt ataaacccgg 14400

ggaagggaga cagaaaagga tagttttaag tattggtgtt ggggacgtgt tctttagcca 14460

aggcagcatg aacccatggc agcacttccc aaccttcctg acatgggcgt ttctgtgaac 14520

tccagtgtga tggagaaatg gcattggctc aggtgtgcag ctagatatgt tacagagcag 14580

ggtgacaggc aggggtgatg agttttgttt taacaacctg tcccttcaac cctcatggta 14640

ctgacaaaga tcacatggct ctcgggggag attcctgcga ggggaagcaa ggagagcatc 14700

cttacatatt attgatccag gcagcagatt tgcagcaaag ctctgtgctt tattcatctg 14760

ttaaaatagt taaaatagtc aaaacatagg aaaaggattc tgggaagtca gaatcggctt 14820

cagagcacac ccctcctgca cttgcccggt tctcagactt gggaatggga ctgggtgggt 14880

gggtactctc gggtgttccg cgggtttggg tcttacttgt acactttgct tgatttcaag 14940

gaggtgcagg agaacagctc tgtgatacca tttaacttgt tgacattact tttatttgaa 15000

ggaacgtata ttagaggtaa gttggtgcat gctattttct gtaacattta ttttgagtca 15060

taggagaaag attttcagtt acttttatcc aagattatta gacactgtaa aatttcatat 15120

ttaggcactt gtcctacaac attttaaaaa tgaatttcaa atacatacgt gtgtatttgt 15180

aatgcagaca agtataaggc agtcagttac atgctttcaa gagtaaaatg aatgacattt 15240

catttccccc atttgtggga gtaaaagaat gacaatatga aattgatgat caaaagaaag 15300

agcataaaag atttagagct cacgtgtttt ttaaactaaa ggtttgggta tcaaattacc 15360

gtaatatttg gattctcttg gctacattgg aaacagttct ataacaattt tatttttaaa 15420

tgtaaagttt ttgtttgttg ttgtttttaa gacggggtct cgctctgtcg cccaggctgg 15480

agtgcagtgg tgcgatctcg gctcactgca accttcacct cccaggttca agtgattctc 15540

ctgcctcagc ctcccaagta gctgggacta caaacacaca ccaccacttc cagctaattt 15600

ttgtatattt agtagagatg gggtttcact atgttggcca agctcgcttc gaactcctga 15660

cctcaggtga tccactcacc ttggtctccc aaagtgctgg gtgggataca ggcgtgagct 15720

actgtgccca gcctttaaat gtaaactttt taattgtatt acaactgcat cagaagttgt 15780

atactttgca actattcaaa tttatactga aaacgttttt gaagttcaac ctaaaattat 15840

gacaggagat agttttagaa aatattttgg ggaacagagg catattctat tttttttttt 15900

tgagacagag tcttgctctg ttgcccagac tggggtgcag tggcgtgatc tcagctgact 15960

gcatcctctg cctgccaggt tcaagcaatt ctttgcctca gcctcctgag tagttgggat 16020

tacaggtgcc cgccaccatg cctggctaat ttttttgtat ttttagtaga gacaagtttt 16080

accatcttgg ccagggtggt attgaactcc tgacctcatg atctacctgc ctcagcctcc 16140

caaagtgctg agattacagg cgtgagcccc ggggcccggc ctggagggat attcttgaag 16200

cgccttgagc agggaggcag cgtttgtctt tatctgacct tggcttcttt gggtcactct 16260

gtttctcttt ccgtgaataa aaagccagtg agcacacact gtgtcccagg cactcttcta 16320

cgctctgggg acatcaccat gaacaactag tcagagtccc cacctccagg ggccttccgt 16380

tctggtggtg ggtttggttc catggttgaa tgcacccagc tgcttatctg ttcaataggc 16440

atctgcttta ttttaagctt actttgcaaa gaaggaagat ggttgtttcc gaagtggaca 16500

tcgcaaaagc tgatccagct gctgcatccc accctctatt actgaatgga gatgctactg 16560

tggcccagaa aaatccaggc tcggtaagtt gctctctgaa agtcgctatc catgtgacat 16620

gagccatgcc cattcgaggc tgcccttttc tacagcggtg ctactgctgc ccagatatgc 16680

ctgctctctc gcctctcctg tgccaggacg cagatcctga ccctctactt gccagctgac 16740

aaccatgtat agactcgttg ccttagctca ccgtgggtgg aggtgctgct ggggttgggg 16800

acactggctg agctgtcgat gggttcagct catctatact agaaggaact ggcccaggcc 16860

ctgggttcaa ggagcactag gtttgttttt cctgcccaca tcatgattca gtctcaagct 16920

acaaaccctg gggcatcatt aagatattat ctttgtaggg aagccacgtg gtgaattttt 16980

tgcccagaat caagacatat ttttggtggg aaccaatcat ggcccctgga atcagtccaa 17040

cctcagttgg tcccgacgct gacactctca gctgtgtgat tgtgggtagt tattcctcct 17100
gttataagcc tggttttcca tatctaaaat gagaataatg gtctttgctt tatatggctg 17160
agagcaaagt gcagggagtg tccaggtact cagagtgctc agtttcttat taccgtggat 17220
cactggtctg tttcaggctg gctctgtttt cctaggcaat gttaaacaat ttttcaaaca 17280
atattcaaaa aacattgaag cgtttagaaa aatacagagg accaccacct ttccaccacc 17340
ctgataccac cgtttacatt tttctgcatt tccgtccctg gtgcgtctct tgtctggctg 17400
taagagatgt aattatgtca gggtatgggg caggaggaac tctcacccac tcatacttgc 17460
gtgctttgga agcagcttgg ccttttccag tcaaggagaa cagagtgtgt ctcgtgatct 17520
ggcagttcca cttctgggga tagactgtgg ttctcaaagt gtggccccca gcccagccca 17580
ttagcatcac ctgggaagtt gactgaaatg caaatgacca gcccctcctc ccactcttag 17640
acctggtgaa ttggacactc tggggcaggg cccaccccct gtgctttacc aggctctcca 17700
ggtgattctg ctgtgtgctg gagtttgagg accatgtgta aacccttgca catccccctg 17760
ggagccacat gcatagcagc actgtttata acagcaaaac tcagaccctg tctacgtgcc 17820
tgtcatggtg gaatggatac tgggagtgtg gtatgttcat gtagcagaat tctatacagt 17880
agtaaagagg aatgaactag agttccaggt atcaaaatgg ctgcatcaaa tgaacaacgt 17940
tgaaccaaag aatcaagtta caggaaatat acaaaatgat tccacttacg taaaattccc 18000
caacaggcag caataggcaa tataccattg tgggaaaaac atataggtgg caaaactata 18060
aagaaaagca aggatgtgat gattgcaagc ctcaggatag agggaccctc tggaggtgaa 18120
ggagttgacc cagggaggtg tgtgcaatgt cctaagtact gggagtgttt gtgttcttaa 18180
cccaagtggt tggtacatgc atatttgctt tattatgttt gacacacttt tgtaaataga 18240
tagtataaat aaatgaaaac aaacaaaaag ttataaggtg aactaagacc gaggctacca 18300
actgtattca tgcatttggt aaggctgtgg ttctttctca gtcagggccc attttgctcc 18360
ctggaacttg tggccaggtc tagagacatc ttggttgtca caactcaggg tgaatagtga 18420
atagaggcca ggtatttggc taaacctcct acaatgtgta gggtagcccc tgcaacaaac 18480
aatcttctaa ccccaaatat gaatagcttc ttgtcctgtt ataaagaagc tattctagta 18540
aaaacgtctg tctatgatga agcatgcaca aaaatagtca ttagaaagag gtaaaagaca 18600
aaatgatttt ctcatatttt cttcctgaac ctcaatcagc ccactttagg aaaattgcac 18660
ccagctgctg gtaggtaggc aggaccgagt gtgaagtctg ctgctttctc tgtttttatg 18720
caagtacttc actattttga ttactttagt tttatagtaa ttatggaaat taggtaatgc 18780
tagtcctctg actttgttct tctttttcaa agtcattttg actaagaata tttagatcat 18840
ttctatttaa tgtgactggt aatataatta gatttgagaa tatcatcttg ctatttgttt 18900
tctatttgtc ccgtctgttc ttcgttcccc ctttcgtctt tttctgcctt ctcttggatt 18960

attttttatg attccatcat gtctcctttg ttgtcttatt aagtataact cttggtgttt 19020

ttagtatata tctttaactt aataagtcaa ccttcaagtg atagtctgtc acttcatgta 19080

tagtgcgaga accttagaat agtgtatttc catctctctg ctctgagcct tcatactatt 19140

tctatcatgc atcttttaca tacatcataa accccacaat acattgttat tattgatgtt 19200

caaacattca attatctttt aaataaagat agcaaaggaa tacaaaaaag tgtagtagtt 19260

accccttcta gtatagactc ctttgtatag atccagattt ccattcagta tcattttcct 19320

tctacctaaa gaacttcttt aacatttcct gtagtgcagg tctgctggta atgaattagt 19380

taagcttttg aatggctaaa aaagtctttg ttttgccttc atttttaaaa gttatttttg 19440

ctgggtatag aattctagat tgatggtgtt tttcagtact ttaaaaatac tgcttcactg 19500

tcttctcgct tgttattgct tctgataaga ttgacagcag atttctcatt tgtgtccctc 19560

tgctcacact gtatcattct ctggctgctc ctaacatttt ctctttatta ctggttttga 19620

gcaatttgac cctcttatgg cttgatgatg tttatgtttg ctgtgcttaa tgtctgttga 19680

gtttctggga tctttgggtt tatggttttc attaagtttg agggaattgt atgtattatt 19740

tcttcaaata tttttttctg tctctcttcc attctctttt ggggattcca gtaacctgtg 19800

tattagactt attgaagttg gccgtcttta atggagtgta ttggttcatt ctcacactgc 19860

tataaagaac tgcctgaaac taggtaattt ataaagaaaa gaggtttaat tgactcacag 19920

tccacatggc tggggaggcc tcaggaaact tacaatcatg gaggaaggca tctcttcaca 19980

aggtggcagg agagagaatg actgaaggag gaacttgcca aacacttata aaaccatcag 20040

acctcatgaa aactcactat catgagaaca gcatggggga aacctccccc acaatccaat 20100

tacctccacc tggtctctcc cttgacacgt agggattatg gggattacaa ttcgagatga 20160

gatttgggta gggacacaga accaaaccat atcatgagca tgatttgcag gccatgaaga 20220

attctccatt tttgtttcct ccaggtggct gagaacaacc tgtgcagcca gtatgaggag 20280

aaggtgcgcc cctgcatcga cctcattgac tccctgcggg ctctaggtgt ggagcaggac 20340

ctggccctgc cagccatcgc cgtcatcggg gaccagagct cgggcaagag ctccgtgttg 20400

gaggcactgt caggagttgc ccttcccaga ggcagcggta agaacttaca ttctgtgtta 20460

gtctgctcag gctgccataa caaaatacca cagacagggt ggcttataca acaaaagttt 20520

attttctcac agttctggag actggaagtc caaaatcagg gtttagcttc tcctgaggcc 20580

tttctccatg gcttgcagat ggccacctcc tcaccgctcc cccatgcggc cttccttcca 20640

cacacaagca tctctgctgt ctcttcccct tcttcataag ggcaccagtc atattggatt 20700

ggggcctacc ctaatgacct catttaacct taattgcctc tttaaaggcc ttatcttcaa 20760

atacagtccc attaggggtt agggcttcaa cataggaatt tggagggaac accatttcat 20820

aacgctatct cattgcacat ttttttcaca taagtcatat gtaatctaca gtttgaggag 20880

aatctgaata aacacatttg ggtcccccag ttcagaacta tatcagtgag gtctcaaaga 20940

gttcaggcct gggacgggtc ttgcaataca gatcaggtgt ggtaggaaga atatggaagg 21000

agtttacagt aggaggactg ttgtaaggtg gcctggtagc agcaggatgc tttcctaatg 21060

ggggtagagt gtgtatgctg gggggataat ggaagatgtt cggatgagtt tcgggggatt 21120

cccaatgtgg tctgccacct gagctgatgg cagaacactg ggatgaggca ggaagccaaa 21180

agtggtggct ttcaagcgtt agtaagcaaa aactcacctg ggttacatac tcagaatgca 21240

tgttcttgag gtcacccaga cacagtgcga tgtccccgca tatcagaggg taagaccaga 21300

aagtttccag ttttaaatgt ctccccatat gattgtataa aagtttgaga accatgggcc 21360

taaggcgcta tgtaggtctt ttaagagcaa agtggagcac tgatgtgggc gtggcctcct 21420

agggatcgtg accagatgcc cgctggtgct gaaactgaag aaacttgtga acgaagataa 21480

gtggagaggc aaggtcagtt accaggacta cgagattgag atttcggatg cttcagaggt 21540

agaaaaggaa attaataaag gtgagtaccc cctgtttgga tgcctggtca agccttctga 21600

catgcatggg gtctgtttgt aactgttcat actcccacct ccctgggcct gtgctgtcag 21660

gacacctttc tcctgcacat caggccacgg ttccttctac ttcttttacc tcattatgac 21720

cagcacgctt ggatatcagc atctgatagc aatcatttat ttctggccag gcacagtggc 21780

ttgtgcctgt aatcccagct ctttgggagg ctgacacggg gggattgctt gagcctagga 21840

gttcaagacc agccagggca acatagtgag accccgtctc ttaaaaaaaa aaaattaaaa 21900

tagctgggca gggtggcatg cacctgtggt cccagctatt caggaggctg aggtgggaga 21960

gttgcttgag cctgggaagt caaggctgca gtgagccgtg atcttgctac ggcactctag 22020

cctgacaaca gagtgagacc ctgtctcaaa aacacatgta ttgcttatta tgtaagtatc 22080

tagaataatg tgaattttaa aatgtcccca catatggatg atctgtccct tattcaaggg 22140

cttccctact tagatctggc aggaagagga gccagatatg ggggtgaggg agctcctccc 22200

cctgttcctt tgtacaagga actccacatt gtggacagga tcgtcactga accccactca 22260

gaaccagcac ccttttctaa gaaagaggag tgactgtgtt tgcataatcc cagcttaggc 22320

taattcatgg caggcctcca taaatgcaaa ccacaaggat caatttgaag tgtctgcaag 22380

gggaaatgac accagcagtg tagacagggt agagtagctg acaaagaaca gcctctgtga 22440

gatgcatgga taacatcttc ctatcgacct tcatgttttt ctggcatgtc acatgtttaa 22500

gtttcattca cactgggaag gtactgaaga gacatgaact aatgcccagc agtaggaagg 22560

gacgggttta gcatttgtaa agatggagca ttaatcacat ttgttgactg tttaaagaaa 22620

gataaataat gttattgaca aaccgtgatt ttgaattagt tgggattagg ttggctgctt 22680

gcagcagaaa actcaaaata actgtgtgcc ttagcccttg ctgtataaca aacccatctt 22740

aaaactaatg gcttgaccac ttttatttct catgatttga tggaccagct gggcagttct 22800

tctctgggct agctgggctg gggctgatgg tccaggatgg cccttggctg gagtgaccgg 22860

gccttctgtc tgtgtggtct ctcaccctcc agaaggctaa gctggactta tcaatgtggg 22920

tggaggggtt cccagcagca agagagggca agacccgaca tctaacacct ttcggtctct 22980

cctgacgtgg cactgtgtaa catcccctta gccagagaaa gtcacgtggc caagcccaat 23040

ttcaagggac cgattttctc tctctccatg gaagtgacga aggcaccctg taaagtagcg 23100

tgcatacagg gatggaaggg aatgtggatg ccattgtgcc agcaagttgc tgagacagcg 23160

gtctaaacaa tgtagaggct ttctgtccct ctcatataag tctgaaggcg ggcagaccag 23220

agctgattgg ggattccaat gtcaggaacc aagatttatt cttctcccat ttcttcattg 23280

ggtgtggcct ctgtttccaa ggcccccccg tgacttagtc agcaagtccg ctttgaagcc 23340

agctggacca tggcaagggg catatctctg cctttgtaag cacactttct ggaagttgca 23400

cataacattt tcacatggcc cattggccag aacccgatct catgaccaca tggcaggtac 23460

atggatattg ggggaacaat tagtggacca taaccactga tatttcctaa gttctaaatt 23520

gatatcaaac atcccaaaaa ggcattctag atttagaaaa gagtaaagtg gtgttagcca 23580

acaatttgat gaaacaaatt catatcctaa aattcattaa ggaggaagga gcaaaataaa 23640

atctcttaat gggatgttaa cagccagtgc ttatcttagc taaaataagc acatttcccc 23700

atataatttt ccagtttata ttttaggcat ttccatatat ttttatttgt ttttattttg 23760

cttggttgct aatttcctac tgacatcaat gagaaggatt taggaatgct accaggaaga 23820

acttcttgcc tccgcccagc tttggactgg tctaagtggg tgtcactcat ggtgacgttc 23880

tcacaaggtc tctctacaca cagtgctggc caacagcagg gaaaatactg agttatcctt 23940

tgagatctct tttatcccaa tcacagaaaa ttgaatctgc tccaaatatg cttttatcca 24000

tgactcgcag agaggagaag atgctttcag agtattcacc atcatgagat ccgtttatcc 24060

taagctctgt ttgggtttga ttttccctgt ctcttttcta gcccagaatg ccatcgccgg 24120

ggaaggaatg ggaatcagtc atgagctaat caccctggag atcagctccc gagatgtccc 24180

ggatctgact ctaatagacc ttcctggcat aaccagagtg gctgtgggca atcagcctgc 24240

tgacattggg tataaggtca gacttcagac ccattctgac cttggccgtg gcgtggggat 24300

gggggagtgg aggggtggga ggagaaagag ggtactgtat tagagtaacc gtgagtccag 24360

agctgagttt tggagttagt atttggaggt gtgagtgggg aatttagaga gcccgttggt 24420

cacagtctgt tctgtcaagt tgaatggaag cttctttgga gaaagtgagg ccagtgggca 24480

cagttggaaa tgtgttctgt gtatttgttt tatgttttat gcaatgactt gtttttggtt 24540

atatacattt tgcagcatat ctaaagtgct gtgtattagg aaggggtctt atgtgggaag 24600

agagcattaa aaataagtat aatgggccac acacagtggc tcagtcctat aatcccagca 24660

ctttgggagg ctgaggcagg aggattcctt gagcccaaga gtttgacaga aacctgagca 24720

acatagtgag acccccttct ctataaaaga aaggttaaaa aattagccag gtatggtggc 24780

gtgcacctgt cagctactag gaggattgct tgaaccaggg aggctgtgat gagccgtgat 24840

tgtgccactg cactccagcc tgggcaacag agcaagaatc tgtctcaaaa caaaaaacaa 24900

aacaaaacaa gcaagaaaga aataggtata atgatatttt agtatcagtg aatctcactt 24960

tacagattaa agatttaggg gtgaagtggg gttttttggc caccattttt cattgtgacc 25020

atcagatctg aggtcttagg ggttaattat ctgaaacttc atggttttcc ctgagcctat 25080

agctctgctt ctgccacaga taatttattt tctcataatt ccagcttggt acctccaggg 25140

ttgtgtttgt gggttcattt ctccaaagtt acttcttttg ggggaaatac ccctgggact 25200

cttagggcct aaagcaagtg caaggtcagg acttgtctca cctctcactt gcctttgcca 25260

tactcacgag tcacctcctc tcatttcctt acagatcaag acactcatca agaagtacat 25320

ccagaggcag gagacaatca gcctggtggt ggtccccagt aatgtggaca tcgccaccac 25380

agaggctctc agcatggccc aggaggtgga ccccgaggga gacaggacca tcggtgagag 25440

tgggggagcc ccactgtgct cagtgagaat gggggagccc gcctgtgctc ggtgagaatg 25500

ggggagccca cctgtgctcg gtgagaatgg gggagcccgc ctgtgctcgg tgagaatggg 25560

ggagcccgcc tgtgctcggt ggtctgccag tgggcaagcg tccctccagt ctccatgggc 25620

tttgctcagt ggggacctgc ctccactaag acctgctaag ggagcaggtt tggtgcccac 25680

caaggccaag tgaaatgagc tgcttttgac tctcactggc taggttgcct tgtaagcctt 25740

atctacttgc tcagaaaggc acagtgggct cggaagcagg tcaaactcag gaggcacatg 25800

gtactcatta agaatgcatt tgagatggga tgtccataac tcaagggata aacaaaacgt 25860

ggcgtgttct acagtggacc cgggtgaagg agcttgggga gagccacatg ctgttctggg 25920

aggcatccct gccttcacgc ggcttgtcgt ggagttcttt tctggagcgg ggctccactg 25980

cccccatggt tctgcagggg ctatggcctg tcctcaagca aggatgggag gaaaccctgg 26040

gaggccgggg gcgtgagcag ttgttcgttc acctctgcct cgtgactgag cacgttctct 26100

ccccaaatac atctggctcg caggaatctt gacgaagcct gatctggtgg acaaaggaac 26160

tgaagacaag gttgtggacg tggtgcggaa cctcgtgttc cacctgaaga agggttacat 26220

gattgtcaag tgccggggcc agcaggagat ccaggaccag ctgagcctgt ccgaagccct 26280

gcagagagag aagatcttct ttgagaacca cccatatttc aggtgcgctt gcctgggttt 26340

catcatggat cagtccaagc ccaggatgtc aggccttcca ggggacagtg gcagccgtcc 26400

cacagatgtg tggagtgtgt gtgtgtgtgt gtgcgtgtgt gtgtgtgcgc gtgtgtgtgt 26460

gactatgctt gttccccaac aaggactatg gaattcacct agaagaatag gaaggggatt 26520

acaaaatact gccaagaaaa aaaaaactaa aaaccaatca aaatagggag agaacaatgt 26580

acaataattt acgtagcatg gtgctggaac catattttat aaaaacataa atagaagaga 26640

ataggaaaaa agtagaaagc ccagaaatag acctagatat atatatttga cacatgataa 26700

atgcagcatt tcaaatcaaa tagtggacta tatcagcttg agtatctatt agtggtgtta 26760

ggataattaa tatttgggaa aaaactaaaa tactgccctt acttatctca ttataccaaa 26820

atagttaaag tttaaagtta aatattaaga ggaagcaata agcatctcag aagaaaggta 26880

ggtgaatagt ttataagatt tttctatccc tcctaccaaa agtgacattt tttaaaagga 26940

aaagactgac aaattggtaa gatttaaaat gatgagacta tgtagagttg taaacattct 27000

tacattcagt tctcccagaa gcctacagag agccattact cagaattcca ggaatatcaa 27060

atggaaactt acatcctgtt ctgcacattc acaattgcca gaagatgaga tgattcagtg 27120

tccattgatg gatggatgca gaaagcaatg tggtctgtac aaaaacatgg aatactttca 27180

gccttagaaa ggaaagacat tctgacacat gctacaacat ggatgaagct tgggaacatt 27240

ctactaagtg aaagaaccca gtcgtaagag gacggatact gtctgattcc acttagctga 27300

ggtccctgga gtagtcagat tcatagagac agaaagaatg atgggcacca ggggctggga 27360

gagagagaat gggcaggtag tgtttaatgg ggccattgtt tcagtttggg aatatgaaaa 27420

gttctgaaga cagacagtgg tgatggttgc ataatagtgt gaatgtactt aatgccactc 27480

aagtgtactc ttaaagatgg ttaaatggtc aattttatat gtagtttacc acaatttaga 27540

aaaattgaca gagaaactga agcttaggta tgagtatact cacaaaaagg cacagaaact 27600

catgcttcac tgctgccttt atcctaaaat gtcctataaa atgtgggaaa ccctgtaata 27660

actcactctg tgagcacaaa tttggatcga gtgagaagat acttgacttc cttcctccag 27720

gcagcccatg gtttaagttt ttatcttgga caagatatct tgtgtctctt ctcctcagtg 27780

ttctgctacc catttatctc aatatgcttc aatgtatttg tatgaagata tgtctgtatc 27840

cattatgatc acctacacat attacacata gaagggggta tgtgttataa aaacatatct 27900

atacatgtct gtgtattttt gtgatgacca agtctatagt cagacaccat gatacacatt 27960

tattatatca gctggaagag ctcattccat atcattgtgg aaatatccta gattgctaaa 28020

attcagtcat aatcctattc aatccagttc tgagtatttg ttgggtacca actgcaagac 28080

attccatcca gttgtaagca actgaaattt gccttgactt tccccaacag caaaaaggca 28140

gacatgcgtg ttctggctac atcaaggtgg aaatcggtcc tgtgttctct tctagggatc 28200

tgctggagga aggaaaggcc acggttccct gcctggcaga aaaacttacc agcgagctca 28260

tcacacatat ctgtgtaagc acgggcagag ctgtgggttc tctaaaaaga atactacgac 28320

cgcagagctg aaccttgctg gcttcttaaa catcactgta cacacagatc ttctgaggat 28380

cttgttaaga tgcagtttca gattctgtgg gtctggagtg gggcctagaa ttctgcattt 28440

ccagcaagcc cccagacaat gtggatattc cttttcaggg gaccacagtc agggggaatg 28500

ctgatagact atatctactg ggccaaaata aaaattaaaa tcttatgcac aagctactaa 28560

ctcttccttt ctcattgaca accactatta taatgtctta gtcattctaa tgaacatatt 28620

ttttaacttc taaaagcttt gtaaaagctc tctgtggttc tttttaaaag tctgcctgaa 28680

tatagtgtct tcctttttca aattttcttt tcttttcttt tctttctttt tttttttttt 28740

tttttttttt ttgagacaga gtctcactct gttgcccagg ctggagtgca gtggtgtgat 28800

ctcagctcac tgcaacctct gcctcctggg ttcaagcaat tctcctacct cagcctcctg 28860

agtagctggg attacaggtg cccaccacca tgcctggcta atttttgtat ttttagtaga 28920

gacagggttt caccatgttg accagactgg cctttttcaa attttcaact cagcaccaga 28980

gtgcaaggtc ttccacgtgg tccccaggaa tgcgggtgca taacagggtt gtttccagcc 29040

gaccatgatg agtgcagagc tctctggggt cccactgtat gcagaaagag gatgcttcct 29100

tattagattc cccacctcga gcaagcccat ggggattgat tttttgcctc tgcaccaagt 29160

caggttcata agttcccgtt cgaattttct tacctagaca gatgcccttg tggctgagcc 29220

gggcttcatt gctgccttct ccttgagccc ctgcctggcc actgttactg gggctggcct 29280

ctgactaccc ctcactaact tgtgagtcca ccgatacatt taaaggtgca gctttcacat 29340

gtcagctggc atttttagat gtttgccgtg gaagggtgag ccagcatatg gcgtcaaccg 29400

tattgttaaa aacataagtc tctgatcact ttttattgat tgcaagcaac ataaaagttg 29460

ttgaatctca aattgctcca aatgccactt tttcagaacc tactagacaa gtggatctct 29520

ccagtctccc tccagagagt ttacctaata tgaccacaga ggaactgctc ccgggtcact 29580

ctgccggggc ctaggaccca tgcacagtgg gtgccacagt gctgctcatg aggctgctgt 29640

cgcaggagtg gggaaggggg aagacctggg cagaaaacag tgcccccagt gtgtgccccc 29700

ctgcacctcc cccgggtctg gaaaagcttc cttttagagg aagccaggaa gtcaaatggc 29760

ccacacaact cctctgcaga gggaggcccg ggacctcctt ttcattctct gttcatcttt 29820

acacatttcc attattttct ctccattttc ctcagaaatc tctgcccctg ttagaaaatc 29880

aaatcaagga gactcaccag agaataacag aggagctaca aaagtatggt gtcgacatac 29940

cggaagacga aaatgaaaaa atgttcttcc tgatagatgt gagtgttgcc agctgcatgg 30000

agctggagaa gcacatgtca tggtcaaaaa agggaccctg ggccttatgc acttccttct 30060

tcactccccc aaggctgatc caaagacatc tggcccgtag cactcaaagg gtggacaggg 30120

ctgagggagg cagggcaggg agtgcagatg tgggggtgga gtcagcagcg agggatgctc 30180

aggctgcgtt gtgcctactc tgtcacgagc atcacccaga tccctaaggc agtaaggggt 30240

gggataggat tttctagtgc caaaacctct tctttcccct gatccacagt gtcccataag 30300

aaagcaaaga atgactcccc accctccaca taggcacggc ctccaaatga ccttgacact 30360

tggatttgaa gtctatccac ttatactgat gtttttcttc ttgacagaaa gttaatgcct 30420

ttaatcagga catcactgct ctcatgcaag gagaggaaac tgtaggggag gaagacattc 30480

ggctgtttac cagactccga cacgagttcc acaaatggag tacaataatt gaaaacaatt 30540

ttcaagaagg tgagtgtctt agtcccttct tttgggctgc tacaaccgaa tacctgagac 30600

tgggtcattt ataaacagta gaaacttatt gctcattgtt ctggaggtga gaaatctatt 30660

cttaaggaat caggaaattt ggtgtctggt gagagcttgt tctctgcttc aaagatggca 30720

ccttctagct gtgtcctctc atgggataag ggacgaacaa gcttcctcgg acctcttttt 30780

tacaggggta ccacaggcat acctcagaga tattgtgggt tcagttccag acaaaaagaa 30840

tattgcaata atgcaagtca tataaacttc ttggttcctg gtgcataaac agttcattta 30900

tgccctactg cagtctatta agtatgtaat agcattaggc ctaaaaaata tgtatgtacc 30960

ttagtttaaa acaccttatt gctaaaaaat tgctggtaca gaaacaaaaa gtgagcatgt 31020

gctactggaa aaaaatggtg ctgatttgct tgacatggga ttgccacaga ctttcaattt 31080

gtaaaaaata cggtatcagt gaagtgcaat aaaacaagat atgcctggaa tgccattatg 31140

cgggcagagt gctcataacc caatccttcc taaaggtctc ctctgttgat accatcacac 31200

tggggattaa gtttcaacat aggaattttt aggggacacc aacatgtaga ccatagcaat 31260

gagtcaatac cgtggtaaac ctgatacgtt ggcttaagac agagaagagt ggggcagttg 31320

gggaggatgg tcaggataag gagctagtga caactaaagc catgtttgct ctcttctata 31380

tcactgaacc caaatgacca tccactgatg aattgataaa ccaactggct tgtgtctgtg 31440

tgtagcggtt ggctttggct gtcataacaa agtaccacag acttgggggg gcttaaacag 31500

tagaaattta ttttcttaca gttctggagg ctggaagtcc aagatcaaga tgttggtgga 31560

gctggttcct tctaaggcct ctctccttgc cttgcagatg gcctcttctc attgggtcct 31620

catgtggttg tccctcagtg tgtgtgtcct catctcctcc cacaaggaca ctaggcagat 31680

gggatgaggg cccaccctag tgacctgatt tcaatttaat tacctctttg cctgtctcca 31740

aacacagtca gattctgagt ttctgagggt taggacttca acattggagt ttgaagaggt 31800

cacaactcaa gctcagcccg taacaccaag tcctggaata tttccagcca cagacaggca 31860

cagagtgctg gtccacagca ccccatggat gaaccttgaa aatgtcatgc tgagtgaaag 31920

aagccagcca caaaggccac acggtctatg attccattga tagaaaatgg ctagaacagg 31980

caaacccagg caggcagaaa gcagaatagt ggctgccagg ggctggggag ggaaaagtgg 32040

gaagttatca ctgatgggtg atggatgtgg ggtttgggag ttatgtctgg ggatggtcgc 32100

acaactttgt gaatatacta aaattcactc acccatacac tttttttttt ctttttcttt 32160

ggagacaggg tctcactctg ttgcccaggc tggggtgcag tggctcagtc tcggctcact 32220

gcaccctctg cctcccagat tcaagcgatt ctcctgcttc agcctccacc tccctagtag 32280

ctgggattat aggcacctgt ctaatttttg tatttttagt agagatgggg tttcaccatg 32340

ttggccaggc tggtctcgac ctcctgacct caggtgatca ctggcctcag cctcccagag 32400

tgttgggatt acgggcgtga gccactgtgc ctggcctgaa ccatatattt ttaacagagt 32460

gaatgttata ctatgtaaat gacatctcaa ttagaaaaat ccttatggga aaatatttcc 32520

tgactaaaaa aagtgttcta gattaccact caaaaaggaa ctcaaaccct ctgaacttct 32580

gatggggcta actctctcta gtgtggattg ttgggagtac aaatcattcc aaaagtttaa 32640

agaaaaatgc agcatcttac acagtgaaca gtgctactgt atcacattca tacaagttga 32700

tgtgcctggt ttactctgtt atcccatttg acttgtaaac actttctaca catggcaata 32760

ctttcgacac atgaatatgt gatgtattgt taattccaga aagtgttcat gctcatttct 32820

aatgggcatg cagttgaggg caaggagtgt attatggtac aatttctttg gtaaaactaa 32880

aattggattc acaaaacttc atactcgagt acgtttttaa gaaggggtct tggccgggca 32940

cggtggctca cgcctgtaat cccagcactt tgggaggccg aggcaggtgg atcatgaggt 33000

caggaaattg agaccatcct ggctaacacg gtgaaacccc gtctctacta aaaatacaaa 33060

aaaattagcc gggcgtggtg gcgggtacct gtagtcccag ctacttggga ggctgaggca 33120

gaagaatggc gtgaacccgg gaggcagagc ttgcagtgag ctgagatcac gccactgcac 33180

tccagcctgg gtgacagagc gagactctgt cacaaacaaa caaatgaaaa aaaagggtct 33240

tactcgaagt ttctgcgtat gtgggttcct ggcatcgtac ctggctctgc actccccttc 33300

ctgagatgac taaggaaaat tatcttcaga tctggttttg tgtgtgtgtg tgtgtgtgtg 33360

tgtgtgtgtg tgtgtgtgtg tgtgtgtaat ccctggatat ttttagttta ccagttagat 33420

ttgatttgat accacttttt cttgccattt atattttcag aaaatttaga atggtattgt 33480

gtttagaaaa atgtgcaaga ttatttttgt aaaataattt agaggttttt ttttcctgct 33540

ataggccata aaattttgag tagaaaaatc cagaaatttg aaaatcagta tcgtggtaga 33600

gagctgccag gctttgtgaa ttacaggaca tttgagacaa tcgtgaaaca gcaaatcaag 33660

gcactggaag agccggctgt ggatatgcta cacaccgtga cgggtgagtg ctcagtttca 33720

cctctgagca ttgatttcta aagaaaggaa aggttcgaac caaagccagc accaaacttc 33780

agcactttcc tcctggggtg catcccacac caacgagcaa acctctcatt ctccagatgc 33840

caagttggta ttcaacaatt caattcaatt ctgacactaa ctaccctcag tcagtgtgga 33900

ccccatagct taagggctca gttccacaac actggcccca actacaaatg ccggtcacaa 33960

gtcccagacc tcctattctt ctgatggact gtttataaat caaggttctt gcgacccatt 34020

cctcaggtca accaagaact ctggaacaca cttcactgac atttactggt ctattagaaa 34080

ggatttgata aggggcacaa atgaagctgt tggagaggca catagtaggg gcctgaacac 34140

agaagcttct gtccccacgg ggttgggggc accatcctca tggcacagag atgtggtcat 34200

caaccaggga gctcttggaa cctcaccgcg gagaaggttt tatggaggcc tcatcatgaa 34260

ggcatgatgg aggattgact caatctccag gccctccctc ctctgtggag ctggaagttc 34320

taagtttcta gccaaggctt ggtctttcta gtgcccggcc ccaatcctga agctatgtag 34380

gggcccacca ggcatcatct cttcaaaaca cgagatactc ctaaggctgg acattccaag 34440

agatgtaggg gctctatgtt aggaaatggg gacaaagaca aaatatttat attttcccta 34500

tcacaccaca cctcccccct gctccactgc tatgctggct tcacactcaa aatcggctgt 34560

ttatttgaaa tctccgagga gtaaagccaa tggttccata actgcacgtg tagatgtgtt 34620

tggaaccttt tggagtgctg taggaatcta ggtgtgtcac ggataggtag gaaactagat 34680

cctactgtgg atccactccc ttcttgaaat gctttgcttt cttggttttc caggtattaa 34740

atctctattc ttcatcctct ccttgactga cagtatcctt actcacactt cagctgcctc 34800

atcttagcag taattaataa tcactcatgg atccatgaac taaggagctg gagatagcct 34860

cagaacagct cattcagagg tgtatttcca gtaaaattga ccttttagcc ctgataatca 34920

tataccaaaa cctgcaatca tgttgttttg gtccattgta gactcttaac tcattccaga 34980

ggaaagttta taatacttag agccttatag tcataaaaat caacatagat atacctattt 35040

ctttttcaga aatgtatgac atggagatca ataagaggtt ttcaatcata aagatactat 35100

accttgtatt acaataaaat tctgtgagga agtagaatag aaatgagttt caaaaataaa 35160

agataaataa tataaatttt ttaatctaag agcttgttct tgtatttttt tcaaatggat 35220

aatgtagaca ctcaaattcc attgatatat ttaagagtga tttgacttat attaagagtt 35280

gtattataaa atattaatat ttataattta aaagaaatta cattctttgc agctatttag 35340

gataaaaagt ttaaatatca aataaatgta tgccaggggt catttgcttt taagattctt 35400

ccagcaaatt attaagcaaa aagagcatgc cttgcttttt catggtaaag agaagaaggg 35460

agcggggaga ggggaaactt tacttcatac catttgatcc tcatattttt ttgcatctta 35520

agaagagaac aaatgatcct accaatattg aactattttt ctctctttga ttagatatgg 35580

tccggcttgc tttcacagat gtttcgataa aaaattttga agagtttttt aacctccaca 35640

gaaccgccaa ggtaaaacca accatgtgtt gtttaaaaaa aaaaaagaaa agaaattaag 35700

cttgacacta gaaaatagat ttcttggatg aggattattt caactttatt gtatactttt 35760

agaacagcaa ataacatcac tcactagtgc ttcttctgat gttaccggtg atgtctggtt 35820

aaaagcaata aaggagggag tgcttaaacg cacagaacaa gagatccaca gttagcggag 35880

aagattatca catctaaggg caatggctcc aaatccagaa actcactgag gaaactacat 35940

ataaaaatag aatatttctg gcccgagtgg gcatgatgag cctgtaatcc cagcactttg 36000

ggaggctgag gccggtagat gacttaaagc caggagtttg agaccagcct ggcccacatg 36060

gcaaaacccc atctctacta aaaatacaaa aaagtagctg gacgtggtgg tgcatgcctg 36120

taatcccagc tacttgggag gctgacactt tagaattgat tgagcccagg aggtggaagt 36180

tgcagtgagc caacattgca tcactgcact ctagcctagg cgatggagcg agaccccgtc 36240

tcaaaaaaaa aaaaaaaaac aaacaaaaaa actttccatc cagagtgagg aaagagccta 36300

caggaaatga gcctggggga cagactgggc caagagacca gacttagcca ctcttagaaa 36360

taggtgtccc cggcacagat gaggagcctg gccccatgat tcaccagctg gaggccttgg 36420

gatgtgccac ttccagcctg tgcccctgac tcctcattca taaaagaaga ctgataaggc 36480

cttcctcaga aggttgagat ggacgtggag taagatgttt aggatgcacc tgccactgtg 36540

cactgtgcct ctcctcaagg cctggagggt ccaggggtga agtttctcct cctcaggttt 36600

tggcaaccag tttctctaaa ccccgggaac ataaaacata attttctgac ttaaacatgg 36660

ctttcctgct catccctgtg gattatctga tggatatgac aatcctcgcc atcagatata 36720

gaagccccta aaagagaaag gaaagaagct gagttacggg gcctgaaagc aagcctgtgc 36780

aggtccccag gccccgggat gggggtccgg cccatctgtg gctcaagcct cctgggaagc 36840

tctgaccctc agccagggct agaaacctgc cttagataca ccagggcgcg gcccagaggg 36900

ctgttccagg aaacgtgctg tttcactcac gttgggtaac ctggtattta cggacttctt 36960

acctactttc ctgtgactca ggaatttgtg tcttgaggga aactgtattt atttattttt 37020

tactgtagtc caaaattgaa gacattagag cagaacaaga gagagaaggt gagaagctga 37080

tccgcctcca cttccagatg gaacagattg tctactgcca ggaccaggta tacaggggtg 37140

cattgcagaa ggtcagagag aaggagctgg aagaagaaaa gaagaagaaa tcctgggatt 37200

ttggggcttt ccagtccagc tcggcaacag actcttccat ggaggagatc tttcagcacc 37260

tgatggccta tcaccaggta cgtcttcgcg tggttcagga tgccagcttc cattctttcc 37320

ttttcttctg aacgcctctc tctttagtct tgctctctct gtaggtgacg ttggtcagct 37380

ctgtcgttta cctccttgtt agcctcctgt attagtccat tttcatgctg ctgataaaga 37440

catacctgag actgggcaat ttacaaaaga aagaggttta acggacttac agttccacat 37500

ggctggggag gccttctacc atcacggcag aaggcaatgg gcacttctta cctggcggcg 37560

gtggcaagag agagaatgag agccaagtga aaggggtttc cccttatcaa accatcagat 37620

ctcatgagag ttactcacta ccatgagaac agtattggag aaactgcccc catgattcag 37680

ttatctcccc ctgagtccct cccacaacag gtgggaatta tgggagtaca attcaagatg 37740

agatttgggt ggggacacag agccaaacca tatcgccttc gtagaagcag ctcaacctca 37800

gacagagaga tggtggctta gagccagtga catctggttt tgatggctgt ctagctctgg 37860

ccaagttact taacctctct gagcctcagc tttctttgta aaatggtgtc tcctcataga 37920

ttctagtgca tattccagga gacgagtgtg gatgatgata atggattgct aatggaaaaa 37980

ccaaactctg ttaaaatatt tgaaagaggt ttattctgag ccaaatatga gggaccatgg 38040

ctctgggaac agtctcagga ggtcctgagg aagtgtgcct gaggctgtca ggatgcagtt 38100

tgattttata catttcagag aggcaggaat tgtaggtaaa atcataaatc aatacatggg 38160

aggtgtactt gccctcccta aagaggcagg acaccttgaa ggagggggag cttaccggtc 38220

ataggtgggt tcagagattt tctggttgac gattgcttga aagagttaaa ctttgtctac 38280

aaacttgaca tcaatagaaa gaaatgcctg agttaaggca gtgttagagg ccaaaggtat 38340

gtagatgaag actctgggta gcagccttca gagagaataa atggtaaatg tttcttttca 38400

ggccttagag gcagcaggct ctcagttaat ctctcctaga ttcagggaag gcctagaagg 38460

ggagaggtct gactgcatta atggagattc tctacaggtg caaattcccc ccccacaaaa 38520

catggcaggg ccatttcaat ctgttggtcc tgttacagcc gtttcaaaat atgtccacaa 38580

aatatatttt taggtaaaat atttgtattt cctttagggt ctgcaatctg tcttgtgatg 38640

ctataccaga gtcgggttgg aaagtaagcc attttatact gagttcatgg aaactcatcc 38700

aaggagattt catggtttgt ggggtgtgtg tgacttaacc cctgcctcac atgactttat 38760

aatatggtat cttactactc cagagtcttt ttggccaacc ttatgatctc aatttcaacc 38820

taaactccaa aagggcctgg cttctcttcc tgttacggcc aggaattcag attttcaggt 38880

ttctctgggg tccacttggc caagaggggg tctgttgagt tggctggaag gcataggatt 38940

ttatttctgg tttacaacaa tttccttagt gcagcattgg aatgcaatgg tagcagacta 39000

aatggaagct atcgcgtaga cacatgcttt ggttgatact gcacgattca gttaacctga 39060

agtacaatct aattcatcct agggaaggag gcagtgaaca cagacacaac tcaggtagag 39120

cccttgggat gtgtaaacac ctgaggaggt aaagcaaatt gtaatctctc ggtttatcag 39180

atgtccccat tgccttacta tttggatgct ttaaagcagg gcctctcaaa ctccacccag 39240

cacagaggcc tcctgggatc ttgtggaaat gcagatgctg attgcaggtc aggatgaagc 39300

tgagattctg cctttctttt tttttttttt ttgaaaccga gtctcactcc attacccagg 39360

ctggagtgca gtggcacaat ctcagctcac tgcaacctct acatcctggg ttcaagcaat 39420

tcacctgcct cagcctccca agtagctggg attacaggct taccttgcca ccatgcctag 39480

ctattttttc tatttttagt agagatggac ttttaccatg ttggccaggc tggtcttgaa 39540

ctcctgacct caagtgatct gcctgcctcg gcctcccaaa gtgctgggat tacaggcgtg 39600

agccaccatg cctggcctct gcatttctaa cgggctctca ggggtcacca tactactgga 39660

tagaggccac acttggagga gcaaggctct aaaccgaggg tcaacatcca ttcctccaga 39720

cactgggagc tgcatgcacg tgagtgaagc cagttaaggg gaagacaggc atgcacatca 39780

gcttctcctg cagccaagct cacacctgtc cgctgcttcc actgcctcct agaatgaaca 39840

gttaccttga gagtaggtga ggcatataca tgcacagaat ccaaacaata ggatgagtga 39900

caatggcaga ggagtctccg agccaagcag ctccctggac agaagcagcc cttctccggg 39960

ttcatttctg tcctccgagg ctgactcatg cactcaaaag ctcccatgca tatacatttt 40020

ataatggttt ttacacaaag gttagcagag gagtggacgt gctgctctgt accctgcctc 40080

ttttgctgta cctgggagat tgttctgcct cagttctgat ggggctgcct tgttcttttc 40140

aatggctgct gagtatccca ttttatggat gtggtatatt gagccagctc cctttaagcg 40200

aacagtttgt ttgcagtctt ttgctaatgc aggtgtgttg ctgtgaatag gtttgtttgt 40260

atatcatgta tctggaagca tcaattccta gaaatgagat tcctggtata ttaggattgt 40320

gcagggaaac agaaccacag atatatgtat gtaaagaagt atatttcagc caggcatggt 40380

ggctcatgcc tgtaatcccg gcactctgga aggctgaggt gggtggatct cttgaggcca 40440

ggagtttgag accagcctgg ccaacatggc gaaacccggt ctctactaaa aatacaaaaa 40500

aattagctgt gcatggtggc ccatgcctat agtcccagct acttgggaag ctgagatatg 40560

agaattgctc gaacctggga ggcagaggtt gcagtgagcc aagatcacac cactgcactc 40620

cagcctgggt gacagagcga gactccatct caaaacaaac aaacaaacaa aaaacaaaac 40680

aaaacaaaaa acagaaggaa agaaagaaat atatgtatat ttcaaggaat tggcttctgc 40740

cattttggga gctggcaagt ccaaaatccc agggcaggcc agcaggaaga gcaggccaga 40800

aattgcagca ggagctaagg ctgagtccac aggcggaatt tcttcttttt agggaaacct 40860

catttttctt cttaagaccc tcaactgatt ggatgaggcc catccacatc attgagaatg 40920

gtctccttca cttaaagtca gtgggttaca catgttaccc acatctacag aatacctccg 40980

cagcaatacc tagattcgtg tttgatggaa tcactgggga ctcgagccta gccaagctaa 41040

cacatgaaac acaccatcac agctggggaa aggatggctt attttagact gataaagatg 41100

acccagagaa ggcctgctcc atccacactg gccgctttag tctgcactaa agttgttggt 41160

tttttttgtt tgtttggttt ttttttggtg acagagtctc actctgtcgc ccaggctgga 41220

gtgcagtggc gcagtctcag ctcactgcaa cctctgcatc ctgagatcaa gcgattctcc 41280

tgcctcagcc tcctgagtag ctgggactac aggcacgtgc caccacactc ggctaatttt 41340

tgttttttca gtagagacgg ggtttcacca tattggccag gctggtcttg aacgcctgac 41400

catgtgatcc atccgcctca gtctaccaaa gtgctgggtt tacaggcgtg agccaccacg 41460

cccggccttg ttggggtttt ttgacagcct aataggtgaa aatgacatct cattacaatc 41520

ttaattggca ttctcttatg acaacaagct ggtacatctt tttgtgtgtt gagggttatt 41580

tctatttctt gctcagcaaa cagttcatcc aggaagagct tcttggtgag atagtagacc 41640

tctgcgattt ctgttgcaga cgatctacat tttgtcattt gctttgtcat ttttgtctat 41700

ggtggtttta gactatgcgt aagttttcta gagcagaaac tcaagttgga tttgggcctc 41760

agtggttatt gccatacttt aaaaggactt tgtctccctg agatgataaa tgaggtggac 41820

aatattttct ttaagtaatt tcttatttta actgttacat gatacctttg gcccatttgg 41880

agttctttga tgtcaagaat gaggcaggat ccagatggca gcagaggtcc cagtcccatc 41940

ctggaagggt cgtctagttc ccactggtac tccacacgcc cactcaggca ctcacttccc 42000

ctctgcgttg ggtcttgtct gcaagactct cttatgtttt accatctagt gcagccagca 42060

cccccacatc accctcactt tttctttctt taaattgtgc agaaatattc atcatgtcta 42120

ttttgccatc ttaaccattt gggggtacat agttcagtgg cattaagtac attcatattg 42180

tgccagcatc accagcagcc atctccagga ccctatcacc ttcccacact gaaactctgt 42240

ccccattaaa cacattcccc attccccgcc cctgaatccc tgacagctac catcctactg 42300

tctgtctctg tgaattcaac taacctaagt acctcatagg agttgtgact ggcttgtttc 42360

atgcagtatg atgtcctcat ccaggtggta gcaagtgtca gagtttcacg cctatttatt 42420

tattattatg agacagagtc ttgctctgtc gcccagcctg gagtacaatg gcgcgatccc 42480

agctcactgc agcctccccc tgcctgggtt caaacaattc tcctgcctca gcctcccatg 42540

gtgtgccgcc acacctggct attttttgta tttttagtag agacgcggtt tcaccacgtt 42600

gaccaggctg gtctggaaat gcagtttttg cactgtctgc ctgcttacct ttatagagca 42660

tattttgccc tcttccatca gaattaccca tttaatggtc aggaaaagct gctgggaata 42720

tgactcatag ctgggacatt ctctgcactg tgcatagttc ctctctgcca ccaccatgga 42780

ggagattgat gggtttgaaa cccaggggaa ggtcattgcc ctgcgagggt ctccctcatt 42840

gagaatctgg atcccctcat gtgcacatgg tgaggtcaga gtcccctcct cacagtgtcc 42900

cctccaccct cccgtgaact gttctttcct tccaggaggc cagcaagcgc atctccagcc 42960

acatcccttt gatcatccag ttcttcatgc tccagacgta cggccagcag cttcagaagg 43020

ccatgctgca gctcctgcag gacaaggaca cctacagctg gctcctgaag gagcggagcg 43080

acaccagcga caagcggaag ttcctgaagg agcggcttgc acggctgacg caggctcggc 43140

gccggcttgc ccagttcccc ggttaaccac actctgtcca gccccgtaga cgtgcacgca 43200

cactgtctgc cccgttcccc gggtagccac tggactgacg acttgagtgc tcagtagtca 43260

gactggatag tccgtctctg cttatccgtt agccgtggtg atttagcagg aagctgtgag 43320

agcagtttgg tttctagcat gaagacagag ccccaccctc agatgcacat gagctggcgg 43380

gattgaagga tgctgtcttc gtactgggaa agggattttc agccctcaga atcgctccac 43440

cttgcagctc tccccttctc tgtattccta gaaactgaca catgctgaac atcacagctt 43500

atttcctcat ttttataatg tcccttcaca aacccagtgt tttaggagca tgagtgccgt 43560

gtgtgtgcgt cctgtcggag ccctgtctcc tctctctgta ataaactcat ttctagcaga 43620

cactgctctg ccatgttttg tattttggcg agaagcctga aactagcagg tagggtgcag 43680

tggagcagtg gacgtaaagc tgccctctgt ggcggggcca ggtaggagca agcaaaagaa 43740

cagggtctga tgatttccta gagactggag ggaaatgggg gaacacactt taaaaaatga 43800

aacaatatga agccaacggt gctaacttaa taatgtgcat tgatcaaatg tccaatttgt 43860

tgagtcactg ggcacctggc atggagttga aaagcacaag gcccagtcag gaagcggttg 43920

ccaggccagg tgcgatggct cacgctgtaa tcccttcact ttgggaggcc gaggcgggtg 43980

gatcacctga ggtcaggagt tcgggaccag cctgaccaat atggtgaaac cctgtctcta 44040

ctaaaaatac aaaaattagc tgggtgtggt ggcgtgtgcc tatagtccca gctactcagg 44100

aggctgagac atgagaattg cttaaacccg ggaggtggag gttgcagtga gcccagatcg 44160

tgccactgca ctccagcctg ggctacagag caagactcca tctcagggaa aaaaaaaggt 44220

ttcccagtgg gactttacct gcagaggctg tggctggggg agcggtctgg gaagcagaga 44280

aggcggaaca ctaaccttgg aggagaacct gactgaagtt ctctggtcta gcctggttgg 44340

gggagactcg agccacactc ctacgccctg actctgagaa ccatggctcc caatcagtgc 44400

tgctgctggg cctcctcacc atgaggccag ggcaggctca agggcaggaa atcgaacagt 44460

ggactagatg gatggggcag gagaggaccg gacactctgt cccttaaggg gagagttgaa 44520

ggatcaggat tgccccagcc taggaaagag aaggccaaaa ggcaacaaga gagatgaagt 44580

gtgagcacca aggtgtgaca tcctctgggg aggtatattc tagatgatca gtgtggggaa 44640

tgggggtact ttctacagag gggcccccgg atggcatggc ttcctcaaga gctgtgaggt 44700

cccaggcagt gggggcatca ggccaagctg cacagctgct tgtcaggttg ggggtggggg 44760

ctggaccagc aacaccatcc tctcccctct ggaccacgcc cccccatccc agatctcttc 44820

tgactcttgg gactgtgtaa ttttcaattc ttcgggaagt ttgagaatac atttataaga 44880

gaggatgtcc ggagagcagt ggacatttca caagcccagg tcagcagctg ccgctgggca 44940

gcaactttat ggttccccca ggtgctgaag tgcacctgca ggttgacttg atctactgtt 45000

aacagagggt gatcagtttg actcatgctt aagggagaga cgacaccaac acacacacag 45060

aggtttgcaa gagtaaggca gggtggatcc aaacattctt aattcaatac taggcacgcg 45120

ttctcgcagt ggacatggcc caggtcctct ggctgtgttg gtgcatcaga cagctcggcc 45180

cttccatgag gcaggtgccc tctcgtggca ggcctgggaa acttctccac tacaagacgt 45240

cccttccttc caccaccacc caaatcagtc ttccacttgc cttaagtttc tgctgcacgg 45300

gaaacagagt cagaggtgtc aaagtgttgc acaggattct attttgctgt tgttagaatt 45360

ttgctagtga taaccagaga ggatggagga agaaagttga cagtatgtgt cccagagaac 45420

agcaatttca atttccccag cagagtgaac ctgctgggga tgccccaagg tgcccgcaag 45480

gatgttcctt tcagccaaaa tgaacatgtg tggtgatccc ccaggcctgt cctaactagt 45540

catccagagc cgagagggga gtggccccag cggccagaac tgggagagag ccctggagac 45600

cactttgtgt gactccttcc tg 45622

## Description

### Field of the Invention

The invention relates to measuring levels of biomarkers in a biological sample to determine the health status of an individual by distinguishing whether the individual is healthy, has an infectious condition or has an inflammatory condition.

### Background to the Invention

Infectious disease in mammals is typically characterized by the invasion of host tissues by infectious agents, including bacteria and viruses, and encompasses the host response to these agents and the resulting inflammatory response. Other conditions lacking an infectious element, such as autoimmune and degenerative diseases are also driven by inflammatory processes initiated by less characterised means. Mechanisms of the host immune system, led by an initial innate response frequently associated with inflammation, are activated when the body encounters infectious agents. Soluble and cellular defences including the complement cascade, collectin, and phagocytic cells are activated to destroy and dispose of the invading stimulus. This non-specific innate response is vital to control the early stages of an infection and is responsible for many of the familiar hallmarks of inflammation (pain, swelling and fever). This is followed by the adaptive immune response wherein specific antibodies, cell mediated immunity and immunological memory are developed.

Successful clearance of the infectious stimulus leads to a resolution of the inflammatory response and typically a return to the normal physiological state.

The host immune response is often complimented by the administration of selected antibiotics or anti-virals in cases of bacterial and viral infections, respectively. However, bacteria and viruses have proved adaptable and many strains have overcome the mechanisms of inhibition of antimicrobials and have become resistant to treatment. This has serious implications for the healthcare of individuals and healthcare systems, particularly in a clinical setting. Several organizations now recommend that antimicrobials are not administered unless there is clear evidence of infection.

Despite advances in diagnostic methods, medical professionals are often required to employ antimicrobial interventions before the presence of an infection can be determined with accuracy in order to reduce risk of negative patient outcomes. A useful clinical tool would provide means of early and sensitive differentiation between healthy and inflammatory or infectious conditions and would more accurately dictate the requirement for antimicrobial intervention by providing clear evidence of infection.

Such a clinical tool may offer additional insight when infection is detected by further differentiating infectious conditions into viral or bacterial categories. Bacterial and viral symptoms are often similar and difficult to differentiate without engaging pathogen-specific detection techniques, which cannot be performed rapidly, and/or at the patients/users' side. A need therefore remains for a point-of-care tool which can rapidly differentiate between healthy individuals, and inflammatory, viral, and/or bacterial infection in a patient.

When considering judicious use of antimicrobials, it is important to identify the causative agent, as viral infections do not respond to antibiotic treatment. Viral infections reportedly frequently affect more than one bodily system at a time and are less frequently associated with inflammation and pain when compared to bacterial infection. However, these observations are the limit of differentiation that can be made without further *in vitro* assessment

A clinical tool that leads to a reduction in the number of viral conditions treated unnecessarily, and/or an increase in the number of bacterial infections identified correctly immediately, provides a valuable solution to over-prescription of anti-microbial treatments.

In certain settings, for example in a hospital, when the presence of infection is confirmed in a patient, prudent antimicrobial treatment is an important part of patient management and overall outcome. Multiple studies indicate that health care associated infection is among the most common types of adverse events affecting hospitalized patients. The US centre for Disease Control and Prevention has determined that almost 1.7 million hospitalised patients annually acquire healthcare associate infections while being treated for other issues. More than 98,000 patients die due to these infections.

Access to biomarkers that accurately monitor a patient's response to antimicrobial therapy allows the clinician to withdraw treatment at an earlier stage thereby restricting the unnecessary use of antibiotics further. This also allows the clinician to change or augment the therapy or monitoring regime if the patient's physiological response is not deemed satisfactory *i.e.* if a decrease in the concentration or ratio of the biomarker(s) is not observed within a certain period of time or observation of a sudden elevation which might indicate an escalation in disease severity. Biomarkers with the ability to differentiate between healthy, infectious and inflammatory conditions have the potential to be employed in any scenario where antibiotics are used to treat known or suspected bacterial infections, as well as in conjunction with post-operative antimicrobial therapy. Such biomarkers would have the potential create positive behavioural change by supporting the clinician in choice of treatment and improve treatment outcomes by decreasing unnecessary exposure to antibiotics and the side effects associated with overuse whilst ensuring condition appropriate therapies are engaged for healthy patients and those with non-infectious inflammatory illness.

Inflammation is an important part of the host response to infectious agents and is triggered by immune cell recognition of highly conserved pathogen associated molecular patterns (PAMPs). However, chronic inflammation triggered in the absence of infectious agents causes significant harm to tissues and organs. This chronic inflammation is often associated with the release of damage associated molecular patterns (DAMPs) which activate specific immune pathways. For example, the endogenous protein β-amyloid is associated with Alzheimer's disease by acting as an inflammatory stimulus. DAMPs have also been associated with disease severity and prognosis in conditions such as infection, asthma, ischemic heart disease, and cancer and they may prove to be useful tools in distinguishing between infectious versus non-infectious inflammation.

Although there is an array of acute phase biomarkers and test methods now available in the field to identify the onset of the innate immune response, these tests lack the sensitivity and specificity to contribute in a meaningful way to differentiation between healthy, inflammatory and infectious disease and thus, do not provide information at the point of care about what therapy should be pursued. Consequently, they are not compelling enough to create behaviour change and promote better patient care through more judicious use of antimicrobials and administration of condition appropriate therapies.

People of skill in the field are routinely interested in identifying and improving features such as the clinical utility and performance. Single biomarker tests that differentiate between infectious and inflammatory conditions have been challenging to establish to date. Furthermore, there does not appear to be a method available that allows differentiation between these infectious and inflammatory groups and healthy conditions. Such a method would be of great benefit in a home setting, in a clinical setting, as screening protocols including screening during disease outbreak or within immunocompromised and vulnerable populations.

It is an object of the present invention to overcome at least one of the above-mentioned problems.

### Summary of the Invention

The main objective of this invention is to determine and monitor an individual's health status by distinguishing between healthy, infectious and inflammatory conditions, together with monitoring the efficacy of any treatment being administered to the individual.

In a home testing setting, particularly where testing is engaged as a screen or as a monitoring tool, such a test may be employed as a self-test to guide a home users decision making as to whether or when to present to a clinician for medical intervention. Users who suspect the onset of illness *e.g*. after exposure to infection, may use the test to differentiate between healthy and non-healthy status and furthermore to differentiate between infectious and non-infectious causes. The user may further differentiate between viral and bacterial infection, as well as the identification of non-infectious inflammation (for example, sterile inflammatory diseases such as atherosclerosis and lung diseases; and autoimmune inflammatory conditions such as Alzheimer's disease and multiple sclerosis).

Infectious and inflammatory disease often cause the same symptoms and are difficult to distinguish. From the point of view of the clinician, it is important to identify the causative agent, as inflammatory conditions do not respond to antibiotic treatment and would benefit from other types of therapy such as NSAIDS, corticosteroids or a monitoring regime.

A clinical or prognostic method of assessing and monitoring inflammatory status would be extremely beneficial in non-infectious inflammatory conditions. Significantly, particularly as these groups are often considered as immunocompromised and vulnerable groups, early detection of the onset of pathogen associated infection in such groups enables rapid clinical decision making, assessment of disease severity and prediction of clinical outcome.

The inventors have devised a method with much improved clinical utility and performance indicators (sensitivity, specificity, positive and negative predictive values) for determining an individual's health status by measuring SAA, CRP, Hp and MxA concentrations in a sample and using those values to determine the health status of the individual by distinguishing between: healthy and unhealthy (non-infectious inflammatory and infectious conditions); healthy and non-infectious inflammatory status; non-infectious inflammatory and Infectious inflammatory status; bacterial Infection and viral infection; and further differentiating between non-infectious inflammation and bacterial infection, or further differentiating between non-infectious inflammation and viral infection in the individual.

There is provided, as set out below and in the appended claims, a method of determining a health status of an individual, the method comprising the steps of assaying a biological sample from the individual for the positive expression of a gene, or protein encoded by said genes, selected from SAA, CRP, Hp and MxA, and wherein the positive expression of the at least one gene, or a protein encoded by said gene, above or below a specified threshold level correlates with the health status of the individual.

A method of monitoring the effectiveness of treatment of an infectious disease in an individual with an infectious disease, the method comprising the step of assaying a biological sample from the individual with an infectious disease for the positive expression of at least one gene, or a protein encoded by said gene, selected from SAA, CRP, Hp, and MxA, wherein the positive expression of the at least one gene, or a protein encoded by said gene, above or below a specified threshold level correlates with the effectiveness of treatment of the infectious disease in the individual.

A method of determining the progression of treatment of an individual with an inflammatory disease, the method comprising the step of assaying a biological sample from the individual with the inflammatory disease for the positive expression of at least one gene, or a protein encoded by said gene, selected from CRP, Hp and MxA, and negative expression of SAA, wherein the positive expression of the at least gene, or a protein encoded by said gene, above or below a specified threshold level, and negative expression of SAA, correlates with the effectiveness of treatment of the inflammatory disease or disorder in the individual.

A method of identifying an individual with an infection or an infectious disease that is suitable for treatment with a therapy for treating the infectious disease, the method comprising the steps of assaying a biological sample from the individual with the infection or infectious disease for the positive expression of at least one gene, or a protein encoded by said gene, selected from SAA, CRP, Hp and MxA, wherein the positive expression of the at least one gene, or protein encoded by said gene, above or below a specified threshold level correlates with the effectiveness of treatment of the infection or infectious disease in the individual.

A method of treating an infection, an infectious disease, an inflammatory condition, or an autoinflammatory condition in a subject, the method comprising: receiving the results of an assay that indicates that the level of at least one gene, or a protein encoded by said gene, in a sample obtained from the subject is above or below a threshold level; and administering a treatment for the disease or condition.

It should be understood that where there is positive expression of one of said two genes, for example SAA, and the previously positively expressing second gene but which is now negative for expression, the user should then retest (for example, after 6 to 12 hours) to confirm the presence of infection, as other biomarkers are expected to also be elevated by then. For example, Hp may be elevated alone in the case of non-infectious inflammation but the elevated levels of SAA may have decreased or is not being detected. Thus, there is a way to monitor the progress of treatment or the progress of the condition by determining whether the expression levels of the already detected genes (or proteins encoded by said genes) decrease below the threshold levels over time.

A method of determining a health status of an individual, the method comprising the steps of assaying a biological sample from the individual for the positive expression of at least two genes, or proteins encoded by said genes, selected from SAA, CRP, Hp and MxA, and wherein the positive expression of the at least two genes, or proteins encoded by said genes, above or below a specified threshold level correlates with the health status of the individual.

In one aspect, the at least two genes, or proteins encoded by said genes, are SAA and one selected from CRP, Hp and MxA. In one aspect, the at least two genes, or proteins encoded by said genes, are SAA and CRP. In one aspect, the at least two genes, or proteins encoded by said genes, are SAA and Hp. In one aspect, the at least two genes, or proteins encoded by said genes, are SAA and MxA. In one aspect, the at least two genes, or proteins encoded by said genes, are CRP and Hp.

In one aspect and for all embodiments described herein, the threshold levels for SAA protein expression levels are between about 10 ug/ml to about 30 ug/ml; preferably between about 15 ug/ml to about 25 ug/ml; and ideally about 20 ug/ml. In one aspect, the threshold levels for Hp protein expression levels are between about 400 ug/ml and 550 ug/ml; preferably between about 425 ug/ml and about 525 ug/ml; more preferably between about 450 ug/ml and about 500 ug/ml, and ideally about 475 ug/ml. In one aspect, the threshold levels for CRP protein expression levels are between about 3 ug/ml and 6 ug/ml; preferably between about 3.5 ug/ml and 5.75 ug/ml; more preferably between about 4 ug/ml and 5.25 ug/ml; even more preferably between about 4.25 ug/ml and 4.8 ug/ml; and ideally about 4.7 ug/ml.

In one aspect, the threshold levels for SAA protein expression levels are 20 ug/ml, the threshold levels for Hp protein expression levels are 475 ug/ml and the threshold levels for CRP protein expression levels are 4.7 ug/ml.

In one aspect, when the protein expression levels of SAA are less than 20 ug/ml (between 0 ug/ml and 19.99 ug/ml) and wherein when the protein expression levels of Hp are less than 475 ug/ml (between 0 ug/ml and 474.99 ug/ml), the individual is healthy.

In one aspect, wherein when the protein expression levels of SAA are less than 20 ug/ml and wherein when the protein expression levels of Hp are greater than 475 ug/ml, the individual is experiencing an inflammatory event or is suffering from an inflammatory condition.

In one aspect, wherein when the protein expression levels of SAA are greater than 20 ug/ml and wherein when the protein expression levels of Hp are greater than 475 ug/ml, the individual is experiencing an infection.

In one aspect, when the protein expression levels of SAA are about 19 ug/ml to about 21 ug/ml, and wherein when the protein expression levels of Hp are about 470 ug/ml to 480 ug/ml, the individual is at risk of onset of an autoimmune and/or an autoinflammatory condition.

In one aspect, there is provided a method of monitoring the effectiveness of treatment of an infectious disease in an individual with an infectious disease, the method comprising the step of assaying a biological sample from the individual with an infectious disease for the positive expression of at least two genes, or proteins encoded by said genes, selected from SAA, CRP, Hp, and MxA, wherein the positive expression of the at least two genes, or proteins encoded by said genes, above or below a specified threshold level correlates with the effectiveness of treatment of the infectious disease in the individual.

In one aspect, the at least two genes, or proteins encoded by said genes, are SAA and one selected from CRP, and Hp, and MxA. In one aspect, the at least two genes, or proteins encoded by said genes, are SAA and CRP. In one aspect, the at least two genes, or proteins encoded by said genes, are SAA and Hp. In one aspect, the at least two genes, or proteins encoded by said genes, are SAA and MxA. In one aspect, the at least genes, or proteins encoded by said genes, are CRP and Hp.

In one aspect, wherein the threshold levels for SAA protein expression levels are 20 ug/ml, the threshold levels for Hp protein expression levels are 475 ug/ml and the threshold levels for CRP protein expression levels are 4.7 ug/ml.

In one aspect, wherein when the protein expression levels of SAA are less than 20 ug/ml and wherein when the protein expression levels of Hp are less than 475 ug/ml, the individual is responding to treatment.

In one aspect, wherein when the protein expression levels of SAA are greater than 20 ug/ml and wherein when the protein expression levels of Hp are greater than 475 ug/ml, the individual still has the infection and is not responding to treatment.

In one aspect, there is provided a method of determining the progression of treatment of an individual with an inflammatory disease, the method comprising the step of assaying a biological sample from the individual with the inflammatory disease for the positive expression of at least two genes , or proteins encoded by said genes, selected from SAA, CRP, Hp and MxA, and negative expression of SAA, wherein the positive expression of the at least two genes, or proteins encoded by said genes, above or below a specified threshold level, and negative expression of SAA, correlates with the effectiveness of treatment of the inflammatory disease or disorder in the individual.

In one aspect, the at least two genes, or proteins encoded by said genes, are SAA CRP and one selected from Hp and MxA. In one aspect, the at least two genes, or proteins encoded by said genes, are CRP and Hp. In one aspect, the at least two genes, or proteins encoded by said genes, are MxA and Hp. In one aspect, the at least two genes, or proteins encoded by said genes, are CRP and MxA.

In one aspect, wherein the threshold levels for SAA protein expression levels are 20 ug/ml, the threshold levels for Hp protein expression levels are 475 ug/ml and the threshold levels for CRP protein expression levels are 4.7 ug/ml.

In one aspect, wherein when the protein expression levels of SAA are less than 20 ug/ml and wherein when the protein expression levels of Hp are less than 475 ug/ml, the individual is responding to treatment.

In one aspect, wherein when the protein expression levels of SAA are less than 20 ug/ml and wherein when the protein expression levels of Hp are greater than 475 ug/ml, the individual is experiencing an inflammatory response and is not responding to treatment.

In one aspect, there is provided a method of identifying an individual with an infection or an infectious disease that is suitable for treatment with a therapy for treating the infectious disease, the method comprising the steps of assaying a biological sample from the individual with the infection or infectious disease for the positive expression of at least two genes, or proteins encoded by said genes, selected from SAA, CRP, Hp and MxA, wherein the positive expression of the at least two genes, or proteins encoded by said genes, above or below a specified threshold level correlates with the effectiveness of treatment of the infection or infectious disease in the individual.

In one aspect, the at least two genes, or proteins encoded by said genes, are SAA and one selected from CRP, Hp, and MxA. In one aspect, the at least two genes, or proteins encoded by said genes, are SAA and CRP. In one aspect, the at least two genes, or proteins encoded by said genes, are SAA and Hp. In one aspect, the at least two genes, or proteins encoded by said genes, are SAA and MxA. In one aspect, the at least genes, or proteins encoded by said genes, are CRP and Hp.

In one aspect, the threshold levels for SAA protein expression are 20 ug/ml, the threshold levels for Hp protein expression are 475 ug/ml and the threshold levels for CRP protein expression are 4.7 ug/ml.

In one aspect, wherein when the protein expression levels of SAA are less than 20 ug/ml and wherein when the protein expression levels of Hp are less than 475 ug/ml, the individual is responding to treatment.

In one aspect, wherein when the protein expression levels of SAA are greater than 20 ug/ml and wherein the protein expression levels of Hp are greater than 475 ug/ml, the individual is not responding to treatment.

In one aspect, the individual is a mammal. Preferably, the individual is a human.

In one aspect, there is provided a kit for determining the health status of a subject, the kit comprising a control oligonucleotide and a set of oligonucleotides for detecting SEQ ID NO: 2, 3, 5, 6, 8, 9, 11 and 12, wherein detection of one or more of SEQ ID NO: 2, 3, 5, 6, 8, 9, 11 or 12 above a threshold in the sample is indicative of the health status of the subject, and wherein the sensitivity of the assay for detecting the at least one sequence from SEQ ID NO: 2, 3, 5, 6, 8, 9, 11 or 12 is at least 80%.

In one aspect, the kit further comprises a support having at least one oligonucleotide selected from group SEQ ID NO: 2, 3, 5, 6, 8, 9, 11 and 12 anchored thereon.

In one aspect, the kit comprises a support having two, three, or four oligonucleotides anchored thereon selected from SEQ ID NO: 2, 3, 5, 6, 8, 9, 11 and 12.

In one aspect, there is a method of treating an infection, an infectious disease, an inflammatory condition, or an autoinflammatory condition in a subject, the method comprising: receiving the results of an assay that indicates that the level of at least two genes, or proteins encoded by said genes, in a sample obtained from the subject is above or below a threshold level; and administering a treatment for the disease or condition.

When the method, assays, kits or tests show negative expression of all of the genes, or proteins encoded by said genes, below the threshold level, the result indicates a healthy status in the subject, and the treatment, if being administered, can be stopped.

In one aspect, the method is carried out on individuals with known sterile inflammatory conditions to monitor health status, disease severity and/or early onset of pathogen-related infection for which the individual may be more at risk for.

In one aspect, the method is carried out on individuals with a suspected infection.

In one aspect, the method is used to predict the worsening of a condition by monitoring the individual over time. For example, every hour, every 2 hours, every 6 hours, every 12 hours, every 18 hours, every 24 hours, every 2 days, every 3 days, every 4 days, every 5 days, every 6 days, once a week, one a fortnight, once a month, and the like.

In one aspect, individuals with progressively decreasing expression levels of SAA are likely to have a better prognosis.

In one aspect, individuals with progressively decreasing protein expression levels for SAA and Hp are likely to have a better prognosis from disease state.

In one aspect, the method is carried out by a home user to determine their or an individual's health status. Measurements may be taken at additional/multiple time points.

In one aspect, threshold levels for biomarkers described herein are engaged to distinguish between healthy, inflammation and infectious conditions.

In one aspect, an individual diagnosed with a disease or condition may be selected for therapy associated with the disease or condition.

In one aspect, the method is used to detect onset of a condition before the onset of symptoms is detected.

In one aspect, there is provided a method for distinguishing if a pathogen-associated infection has been eradicated following a treatment step, the method comprising the step of assaying a biological sample from the individual for the positive expression of at least two genes, or proteins encoded by said genes, selected from SAA, CRP, Hp and MxA, and wherein the positive expression of the at least two genes, or proteins encoded by said genes, above or below a specified threshold level correlates with whether the pathogen-associated infection has been eradicated following the treatment step.

In one aspect, there is provided a method of monitoring an individual diagnosed with a sterile inflammatory disease, the method comprising the step of providing the individual with a treatment, assaying a biological sample from the individual for the positive expression of at least two genes, or proteins encoded by said genes, selected from SAA, CRP, Hp and MxA, and wherein the positive expression of at least two genes, or proteins encoded by said genes, above or below a specified threshold level correlates with whether the patient diagnosed with the sterile inflammatory disease is responding to the treatment step.

As described herein, levels of at least two genes, or proteins encoded by said genes, selected from SAA, CRP, Hp and MxA, can be increased in infectious diseases, inflammatory diseases, autoinflammatory diseases, cancer and/or in subjects with heart disease. In some embodiments of any of the aspects, the level of at least two genes, or proteins encoded by said genes, selected from SAA, CRP, Hp and MxA, can be increased in infectious diseases and/or in subjects with inflammatory diseases. Accordingly, in one aspect of any of the embodiments, described herein is a method of treating an infectious diseases in a subject in need thereof, the method comprising administering a suitable antibiotic to a subject determined to have an expression level of at least two genes, or proteins encoded by said genes, selected from SAA, CRP, Hp and MxA, above a specified threshold level. In one aspect of any of the embodiments, described herein is a method of treating an inflammatory disease in a subject in need thereof, the method comprising: a) determining the expression level of at least two genes, or proteins encoded by said genes, selected from SAA, CRP, Hp and MxA, in a sample obtained from a subject; and b) administering an anti-inflammatory drug, such as non-steroidal anti-inflammatory drugs (NSAIDs), to the subject if the expression level of at least two genes, or proteins encoded by said genes, selected from SAA, CRP, Hp and MxA, is above or below a specified threshold level.

In some embodiments of any of the aspects, the method comprises administering a suitable treatment as set out in Table 1 to a subject previously determined to have an expression level of at least two genes, or proteins encoded by said genes, selected from SAA, CRP, Hp and MxA, that is above or below a specified threshold level. In some embodiments of any of the aspects, described herein is a method of treating a diseases or condition set out in Table 1 in a subject in need thereof, the method comprising: a) first determining the expression level of at least two genes, or proteins encoded by said genes, selected from SAA, CRP, Hp and MxA, in a sample obtained from a subject; and b) then administering a suitable treatment as set out in Table 1 to the subject if the expression level of at least two genes, or proteins encoded by said genes, selected from SAA, CRP, Hp and MxA, is above or below a specified threshold level.

In one aspect of any of the embodiments, described herein is a method of treating a disease or condition as set out in Table 1 in a subject in need thereof, the method comprising: a) determining if the subject has an expression level of at least two genes, or proteins encoded by said genes, selected from SAA, CRP, Hp and MxA, above or below a threshold level; and b) administering a suitable treatment as set out in Table 1 to the subject if the expression level of at least two genes, or proteins encoded by said genes, selected from SAA, CRP, Hp and MxA, is above or below a threshold level. In some embodiments of any of the aspects, the step of determining if the subject has an expression level of at least two genes, or proteins encoded by said genes, selected from SAA, CRP, Hp and MxA, above or below a threshold level can comprise i) obtaining or having obtained a sample from the subject and ii) performing or having performed an assay on the sample obtained from the subject to determine/measure the expression level of at least two genes, or proteins encoded by said genes, selected from SAA, CRP, Hp and MxA, in the subject. In some embodiments of any of the aspects, the step of determining if the subject has a specified threshold expression level of at least two genes, or proteins encoded by said genes, selected from SAA, CRP, Hp and MxA, can comprise performing or having performed an assay on a sample obtained from the subject to determine/measure the expression level of at least two genes, or proteins encoded by said genes, selected from SAA, CRP, Hp and MxA, in the subject. In some embodiments of any of the aspects, the step of determining if the subject has an expression level of at least two genes, or proteins encoded by said genes, selected from SAA, CRP, Hp and MxA, above or below a threshold level can comprise ordering or requesting an assay on a sample obtained from the subject to determine/measure the expression level of at least two genes, or proteins encoded by said genes, selected from SAA, CRP, Hp and MxA, in the subject. In some embodiments of any of the aspects, the step of determining if the subject has an expression level of at least two genes, or proteins encoded by said genes, selected from SAA, CRP, Hp and MxA, above or below a threshold level can comprise receiving the results of an assay on a sample obtained from the subject to determine/measure the expression level of at least two genes, or proteins encoded by said genes, selected from SAA, CRP, Hp and MxA, in the subject. In some embodiments of any of the aspects, the step of determining if the subject has positive expression of at least two genes, or proteins encoded by said genes, selected from SAA, CRP, Hp and MxA can comprise receiving a report, results, or other means of identifying the subject as a subject with an expression level of SAA, CRP, Hp and/or MxA above or below a threshold level.

In one aspect of any of the embodiments, described herein is a method of treating a disease or condition as set out in Table 1 in a subject in need thereof, the method comprising: a) determining if the subject has a positive expression of at least two genes, or proteins encoded by said genes, selected from SAA, CRP, Hp and MxA is above or below a threshold level; and b) instructing or directing that the subject be administered a suitable treatment as set out in Table 1 if the level of at least two or more genes, or proteins encoded by said genes, selected from SAA, CRP, Hp and MxA is above or below a specified level. In some embodiments of any of the aspects, the step of determining if the subject has a specified expression level above or below a specified threshold of at least two or more genes, or proteins encoded by said genes, selected from SAA, CRP, Hp and MxA can comprise i) obtaining or having obtained a sample from the subject and ii) performing or having performed an assay on the sample obtained from the subject to determine/measure the level of at least two genes, or proteins encoded by said genes, in the subject. In some embodiments of any of the aspects, the step of determining if the subject has positive expression of at least two genes, or proteins encoded by said genes, selected from SAA, CRP, Hp and MxA, can comprise performing or having performed an assay on a sample obtained from the subject to determine/measure the expression level of at least two genes, or proteins encoded by said genes, selected from SAA, CRP, Hp and MxA, in the subject. In some embodiments of any of the aspects, the step of determining if the subject has a specified threshold level of expression level of at least two genes, or proteins encoded by said genes, selected from SAA, CRP, Hp and MxA, can comprise ordering or requesting an assay on a sample obtained from the subject to determine/measure the expression level of at least two genes, or proteins encoded by said genes, selected from SAA, CRP, Hp and MxA, in the subject. In some embodiments of any of the aspects, the step of instructing or directing that the subject be administered a particular treatment can comprise providing a report of the assay results. In some embodiments of any of the aspects, the step of instructing or directing that the subject be administered a particular treatment can comprise providing a report of the assay results and/or treatment recommendations in view of the assay results. In some embodiments of any of the aspects, the step of instructing or directing that the subject undergoing a particular treatment as set out in Table 1 for a particular disease or condition as set out in Table 1 is to cease receiving that particular treatment can comprise providing a report of the assay results. In some embodiments of any of the aspects, the step of instructing or directing that the subject stop being administered a particular treatment can comprise providing a report of the assay results and/or treatment recommendations in view of the assay results.

**Table 1: Diseases and Conditions, and their prescribed treatments***

| **Infectious Disease** | **Common Treatment** |
|---|---|
| Sepsis | Intravenous broad-spectrum antibiotics (selected from ceftriaxone, azithromycin, ciprofloxacin, vancomycin, piperacillin-tazobactam); Intravenous fluids; and/or vasopressors |
| Meningitis | Intravenous antibiotics (selected from cephalosporins, various penicillin-type antibiotics, aminoglycoside drugs such as gentamicin); and/or corticosteroids. |
| Eye Infection | Antibiotic eye drops and/or aminoglycosides and cephalosporins. |
| Bacterial Pneumonia | Antibiotics (macrolides, fluoroquinolones, tetracyclines), cough medicine, fever reducer/pain reliever, acetaminophen/NSAIDs, or combinations thereof. |
| Tuberculosis | Antibiotics such as isoniazid, rifampin (Rifadin®, Rimactane®), ethambutol (Myambutol®), pyrazinamide. |
| Viral Pneumonia | Anti-virals (such as oseltamivir, zanamivit, peramivir, Ribavirin), cough medicine, fever reducer/pain reliever, acetaminophen/NSAIDs. |
| Gastritis | Antibiotics (such as clarithromycin, amoxicillin, metronidazole), |
| | proton pump inhibitors (PPIs), histamine blockers, and/or antacids. |
| Nail Infection | Antibiotics such as amoxicillin, clavulanic acid, clindamycin, or combinations thereof. |
| Skin Infection | Antibiotics such as dicloxacillin, erythromycin, tetracycline; or combinations thereof. |
| Influenza Pneumonia | Antivirals (such as oseltamivir, zanamivit, peramivir), cough medicine, fever reducer/pain reliever, acetaminophen/ NSAIDs, or combinations thereof. |
| Rhinovirus Pneumonia | Antivirals (such as ribavirin), cough medicine, fever reducer/pain reliever, acetaminophen/NSAIDs, or combinations thereof. |
| Whooping cough | Antibiotics (such as erythromycin), cough medicine, fever reducer/pain reliever, acetaminophen/NSAIDs, or combinations thereof. |
| Gastrointestinal ∘ Rotavirus ∘ Norovirus | Fever reducer/pain reliever, acetaminophen/ NSAIDs, or combinations thereof. |
| Urinary Tract Infection | Antibiotics (such as Levaquin® (levofloxacin), ciprofloxacin, Keflex® (cefalexin), Zotrim® (sulfamethoxazole, trimethoprim, phenazopyridine), Bactrim® (rimethoprim/sulfamethoxazole)), fever reducer/pain reliever, acetaminophen/NSAIDs, or combinations thereof. |

| **Inflammatory Disease** | |
|---|---|
| Acne | Topical ointment (benzoyl peroxide), hormonal treatment, or bomcinations thereof |

| **Autoimmune disorders** | |
|---|---|
| Inflammatory bowel disease | Anti-inflammatories (corticosteroids, aminosalicylates), immunosuppressors (azathioprine, mercaptopurine, cyclosporine, methotrexate, TNF-alpha inhibitors (infliximab, adalimumab, golimumab)), anti-diarrheal medication, pain relievers (acetaminophen, NSAIDs), iron supplements, calcium & Vitamin D supplements, or combinations thereof. |
| Lupus | Non-steroidal anti-inflammatory drugs (NSAIDS) (e.g. Ibuprofen, naproxen), anti-malarial drugs, BLyS-specific inhibitors, immunosuppressive agents, chemotherapy, or combinations thereof. |
| Rheumatoid Arthritis | Anti-inflammatories (NSAIDs), corticosteroids, disease-modifying antirheumatic drugs (DMARDs), biologic agents (abatacept, adalimumab, anakinra, baricitinib, certolizumab, etanercept, golimumab, infliximab, rituximab, sarilumab, tocilizumab, tofacitinib), or combinations thereof. |
| Multiple Sclerosis | Corticosteroids, plasma exchange, ocrelizumab, beta interferons, glatiramer acetate, fingolimod, dimethyl fumarate, teriflunomide, siponimod, ocrelizumab, natalizumab, alemtuzumab, mitoxantrone, or combinations thereof. |
| Type 1 diabetes | Diet management, insulin, or combinations thereof. |
| Chronic Inflammatory demyelinating polyneurophathy | Corticosteroids, immunosuppressant drugs, plasmapheresis, intravenous immunoglobulin therapy, or combinations thereof. |
| Guillian-Barre syndrome | Plasmapheresis, intravenous immunoglobulin therapy, pain relievers, laxatives, or combinations thereof. |
| Psoriasis | Topical treatments (corticosteroids, Vitamin D and analogues thereof, anthralin, topical retinoids, calcineurin inhibitors, salicylic acid, coal tar), light therapy, oral/intravenous medication (retinoids, methotrexate, cyclosporin), or combinations thereof. |
| Vasculitis | Corticosteroids, steroid-sparing medication (methotrexate, azathioprine, mycophenolate, cyclophosphamide), surgery, o combinations thereof. |
| Grave's disease | Radioactive iodine therapy, anti-thyroid medication, beta-blockers (propranolol, atenolol, metoprolol, nadolol), surgery, corticosteroids, or combinations thereof. |
| Myasthenia gravis | Cholinesterase inhibitors, corticosteroids, immunosuppressants, plasmapheresis, intravenous immunoglobulin therapy, monoclonal antibodies, surgery, or combinations thereof. |
| Hashimoto's thyroiditis | Synthetic hormones (such as levothyroxine) |

| **Autoinflammatory conditions** | |
|---|---|
| Cystic Fibrosis | Medication targeting gene mutation (cystic fibrosis transmembrane conductance regulator (CTFR) modulators); antibiotics (to treat and prevent infection); anti-inflammatories; mucus-thinning drugs (for example, hypertonic saline); bronchodilators; oral pancreatic enzymes; stool softeners; acid-reducing medication; vest therapy; pulmonary rehabilitation; surgery; lung/liver transplant; or combinations thereof. |
| Chronic Obstruction Pulmonay Disease (COPD) | Smoking cessation; short-acting bronchodilators (albuterol, Ipratropium, Levalbuterol); long-acting bronchodilators (aclidinium, arformoterol, formoterol, indacaterol, tiotropium, salmeterol, umeclidinium); inhaled steroids (fluticasone, budesonide); combination inhalers; oral steroids (corticosteroids); phosphodiesterase-4 inhibitors; theophylline; Antibiotics (if infection present); lung therapies (oxygen therapy, pulmonary rehabilitation program); surgery; lung transplant, or combinations thereof. |
| Alllergy | Antihistamines, corticosteroids, decongestants, NSAIDs, or combinations thereof. |
| Asthma | Corticosteroids, albuterol, or combinations thereof. |
| Celiac disease | Diet management, anti-inflammatories, or combinations thereof. |
| Glomerulonephritis | Dialysis |
| Hepatitis | Anti-virals (entecavir, tenofovir, lamivudine, adefovir, telbivudine), interferon injections, surgery (liver transplant), or combinations thereof. |
| Chronic Prostatitis | NSAIDs, alpha blockers, or combinations thereof. |
| Diverticulitis | Pain relievers (acetaminophens), antibiotics (if infectious), surgery, or combinations thereof. |
| Cancer | Surgery, radiotherapy, chemotherapy, immunotherapy, hormone therapy, targeted drug therapy, cryoablation, radiofrequency ablation, or combinations thereof. |
| Heart disease | ACE inhibitors, aldosterone inhibitors, angiotension II receptor blockers, beta-blockers, vasodilators, or combinations thereof. |

| | |
|---|---|
| *other diseases which are associated with those listed above are also described below. | |

### Definitions

In the specification, the term "healthy" should be understood to mean where the individual or patient has no underlying medical condition, or does not have or is not suffering from an infection, an inflammatory response, event, disorder, condition or otherwise.

In the specification, the term "health status" should be understood to mean whether a subject is healthy (see above), or is found to be, or is, suffering from one or more from an infection, an infectious disease, an infectious condition, an inflammatory disease, an inflammatory condition, an autoinflammatory condition, an autoimmune condition, inflammatory bowel disease, heart disease, cancer, or combinations thereof.

In the specification, the term "infection" or "infectious condition" should be understood to mean infections or diseases caused by microorganisms or microbes such as viruses, bacteria, fungi, protozoa and helminths. Examples of infections include septicaemia, meningitis, eye infections, tuberculosis, upper respiratory tract infections, pneumonia, gastritis, nail and skin infections and the like. Common bacteria that cause disease and infections include *Staphylococcus, Streptococcus, Pseudomonas, Clostridium difficile, Escherichia coli* and *Listeria monocytogenes.*

In the specification, the term "inflammatory condition" should be understood to mean immune-related conditions resulting in allergic reactions, myopathies and abnormal inflammation and non-immune related conditions having causal origins in inflammatory processes. Examples include as acne, autoimmune conditions, autoinflammatory condition, chronic prostatitis, diverticulitis, cancer, heart disease, and the like.

In the specification, the term "autoinflammatory condition" should be understood to mean a group of diseases characterised by seemingly unprovoked episodes of fever and inflammation of skin, joints, serosal surfaces and other organ involvement including the nervous system. Examples of autoinflammatory conditions include allergy, asthma, autoimmune conditions, celiac disease, glomerulonephritis, hepatitis, cryopyrinopathies or cryopyrin-associated periodic syndromes (CAPS) (a group of three rare autoinflammatory diseases that includes familial cold autoinflammatory syndrome (FCAS), Muckle-Wells syndrome (MWS) and chronic infantile neurologic cutaneous articular syndrome (CINCA)), and the like.

In the specification, the term "autoimmune condition" should be understood to mean a condition in which your immune system mistakenly attacks your body. Examples of autoimmune conditions include asthma, inflammatory bowel diseases, lupus, rheumatoid arthritis, multiple sclerosis, Type 1 diabetes, Chronic inflammatory demyelinating polyneuropathy, Guillian-Barre syndrome, psoriasis, vasculitis, Grave's disease, myasthenia gravis, Hashimoto's thyroiditis, and the like.

In the specification, the term "inflammatory bowel disease" should be understood to mean disorders that involve chronic inflammation of the digestive tract. Examples of inflammatory bowel disease include colitis, ulcerative colitis, and Crohn's disease.

In the specification, the term "cancer" should be understood to mean a cancer selected from the group comprising node-negative, ER-positive breast cancer; early stage, node positive breast cancer; multiple myeloma, prostate cancer, glioblastoma, lymphoma, fibrosarcoma; myxosarcoma; liposarcoma; chondrosarcoma; osteogenic sarcoma; chordoma; angiosarcoma; endotheliosarcoma; lymphangiosarcoma; lymphangioendotheliosarcoma; synovioma; mesothelioma; Ewing's tumour; leiomyosarcoma; rhabdomyosarcoma; colon carcinoma; pancreatic cancer; breast cancer; ovarian cancer; squamous cell carcinoma; basal cell carcinoma; adenocarcinoma; sweat gland carcinoma; sebaceous gland carcinoma; papillary carcinoma; papillary adenocarcinomas; cystadenocarcinoma; medullary carcinoma; bronchogenic carcinoma; renal cell carcinoma; hepatoma; bile duct carcinoma; choriocarcinoma; seminoma; embryonal carcinoma; Wilms' tumour; cervical cancer; uterine cancer; testicular tumour; lung carcinoma; small cell lung carcinoma; bladder carcinoma; epithelial carcinoma; glioma; astrocytoma; medulloblastoma; craniopharyngioma; ependymoma; pinealoma; hemangioblastoma; acoustic neuroma; oligodendroglioma; meningioma; melanoma; retinoblastoma; and leukemias. Also included are metastases selected from the group comprising: bone metastases; lung metastases; liver metastases; bone marrow metastases; breast metastases; and brain metastases.

In the specification, the term "heart disease" should be understood to mean cardiovascular disease selected from the group comprising: ischemic heart disease, cardiac hypertrophy; myocardial infarction; stroke; arteriosclerosis; and heart failure.

In the specification, the term "subject", "individual" or "patient" should be understood to mean all mammals, for example, a human, primates, non-human primates, farm animals (such as pigs, horses, goats, sheep, cows (including bulls, bullocks, heifers *etc*.), donkey, reindeer, *etc*.), veterinary mammals (such as dogs, cats, rabbits, hamsters, guinea pigs, mice, rats, ferrets, *etc*.), and mammals kept in captivity (such as lions, tigers, elephants, zebras, giraffes, pandas, rhino, hippopotamus, *etc*.), and other mammals and higher mammals for which the use of the invention is practicable.

In this specification, the term "biological sample" should be understood to mean blood or blood derivatives (serum, plasma *etc*.), urine, saliva or cerebrospinal fluid.

In the specification, the term "treatment" should be understood to mean prohibiting, preventing, restraining, and slowing, stopping or reversing progression or severity of a disease or condition associated with inflammatory or non-inflammatory diseases, conditions or infections.

Serum Amyloid A (SAA) is small protein primarily associated with the innate response and is predominantly secreted by hepatocytes. The protein responds very quickly to the onset of a challenge with levels increasing 1000-fold or more within 24hours. The SAA sequence is remarkably conserved across many species indicating an important role. The literature widely reports SAA as being associated with non-specific inflammatory responses.

Haptoglobin (Hp) is a glycoprotein composed of 2α and 2β subunits. It is primarily engaged in haemolysis where it limits haemoglobins oxidative activity but additionally levels are seen to elevate during infectious and inflammatory episodes. The protein is produced by hepatocytes with some reports of production at other sites such as the lung and kidney.

C Reactive Protein (CRP) is another acute phase protein whose levels are seen to increase in response to acute and chronic challenges. CRP levels are widely reported as non-specific but clinically useful markers for many types of inflammation. CRP is a member of the pentraxin family of plasma protein and are described as stably conserved in vertebrate evolution.

Myxovirus Resistance Proteins (Mx) are innate intracellular GTPases activated by the hosts interferon system (IFN) in response to viral challenge. They are understood to play an important role in the host immune response. Myxovirus Resistance Protein A (MxA), the best understood of the Mx family, accumulates in the cytoplasm of cells and in human peripheral blood mononuclear cells (PBMC), and macrophages are the principle producers. MxA is evolutionarily conserved and present in almost all vertebrates.

In the specification, the term "test platform" in relation to determining the levels of the biomarkers should be understood to mean determining biomarker concentrations by immunoassay-based methods such as lateral flow immunoassays (LFAs), enzyme-linked immunosorbent assay (ELISA), immunoturbidimetric assays, quantitative polymerase chain reaction (PCR), reverse transcriptase PCR (RT-PCR), quantitative real time RT-PCR (qRT-PCR), Western Blot, protein determination on polyacrylamide gels, and the like.

In this specification, the term "at least two" should be understood to mean and encompass that at least two of or all of the proteins can be selected from the group consisting of SAA, Hp, CRP and MxA.

In the specification, the term "variant" should be understood to mean proteins having amino acid sequences which are substantially identical to wild-type SAA, CRP, Hp and MxA proteins, respectively. Thus, for example, the term should be taken to include proteins or polypeptides that are altered in respect of one or more amino acid residues. Preferably such alterations involve the insertion, addition, deletion and/or substitution of 5 or fewer amino acids, more preferably of 4 or fewer, even more preferably of 3 or fewer, most preferably of 1 or 2 amino acids only. Insertion, addition and substitution with natural and modified amino acids is envisaged. The variant may have conservative amino acid changes, wherein the amino acid being introduced is similar structurally, chemically, or functionally to that being substituted. Generally, the variant will have at least 70% amino acid sequence identity, preferably at least 80% sequence identity, more preferably at least 90% sequence identity, and ideally at least 95%, 96%, 97%, 98% or 99% sequence identity with wild-type human proteins described herein. In this context, sequence identity is a polypeptide sequence that shares 70% amino acid identity with wild-type human protein is one in which any 70% of aligned residues are either identical to, or conservative substitutions of, the corresponding residues in the wild-type human proteins. It should be noted that this is the amount of characters which match exactly between two different sequences. Hereby, gaps are not counted, and the measurement is relational to the shorter of the two sequences.

The term "variant" is also intended to include chemical derivatives of the wild-type protein, *i.e.* where one or more residues of Cf5 is chemically derivatized by reaction of a functional side group. Also included within the term variant are Cf5 molecules in which naturally occurring amino acid residues are replaced with amino acid analogues.

The protein sequences encoded by the human genes of SAA, CRP, Hp and MxA are provided in SEQ ID NO: 1, 4, 7 and 10, respectively. The Accession Numbers for the human proteins are also provided. The nucleotide sequences of the human genes of SAA, CRP, Hp and MxA are provided in SEQ ID NO: 2, 3, 5, 6, 8, 9, 11 and 12. The Accession Numbers for the human genes are also provided.

In the specification, the term "Youden Index (J)" should be understood to mean the sum of sensitivity and specificity minus one and reflects the overall capacity of an early warning model to detect outbreaks and non-outbreaks.

In some embodiments of any of the aspects, measurement of the level of a target and/or detection of the level or presence of a target, *e.g*. of an expression product (nucleic acid or polypeptide of one of the genes described herein) or a mutation can comprise a transformation. As used herein, the term "transforming" or "transformation" refers to changing an object or a substance, *e.g*., biological sample, nucleic acid or protein, into another substance. The transformation can be physical, biological or chemical. Exemplary physical transformation includes, but is not limited to, pre-treatment of a biological sample, *e.g*., from whole blood to blood serum by differential centrifugation. A biological/chemical transformation can involve the action of at least one enzyme and/or a chemical reagent in a reaction. For example, a DNA sample can be digested into fragments by one or more restriction enzymes, or an exogenous molecule can be attached to a fragmented DNA sample with a ligase. In some embodiments of any of the aspects, a DNA sample can undergo enzymatic replication, *e.g.,* by polymerase chain reaction (PCR).

Transformation, measurement, and/or detection of a target molecule, *e.g*. an SAA mRNA or polypeptide, can comprise contacting a sample obtained from a subject with a reagent (*e.g.* a detection reagent) which is specific for the target, *e.g.,* a target-specific reagent. In some embodiments of any of the aspects, the target-specific reagent is detectably labeled. In some embodiments of any of the aspects, the target-specific reagent can generate a detectable signal. In some embodiments of any of the aspects, the target-specific reagent generates a detectable signal when the target molecule is present.

Methods to measure gene expression products are known to a skilled artisan. Such methods to measure gene expression products, *e.g*., protein level, include ELISA (enzyme linked immunosorbent assay), western blot, immunoprecipitation, and immunofluorescence using detection reagents such as an antibody or protein binding agents. Alternatively, a peptide can be detected in a subject by introducing into a subject a labeled anti-peptide antibody and other types of detection agent. For example, the antibody can be labeled with a detectable marker whose presence and location in the subject is detected by standard imaging techniques.

For example, antibodies for the various targets described herein are commercially available and can be used for the purposes of the invention to measure protein expression levels, *e.g*. anti-SAA (Cat. No. ab687; Abcam, Cambridge MA). Other antibodies for each of SAA, CRP, Hp and MxA are available from Abcam, Lifespan Biosciencs, Novus Biological, Bio-Rad, Thermofisher, Abcepta, and the like. Examples of the available antibodies can be found in Table 2 below.

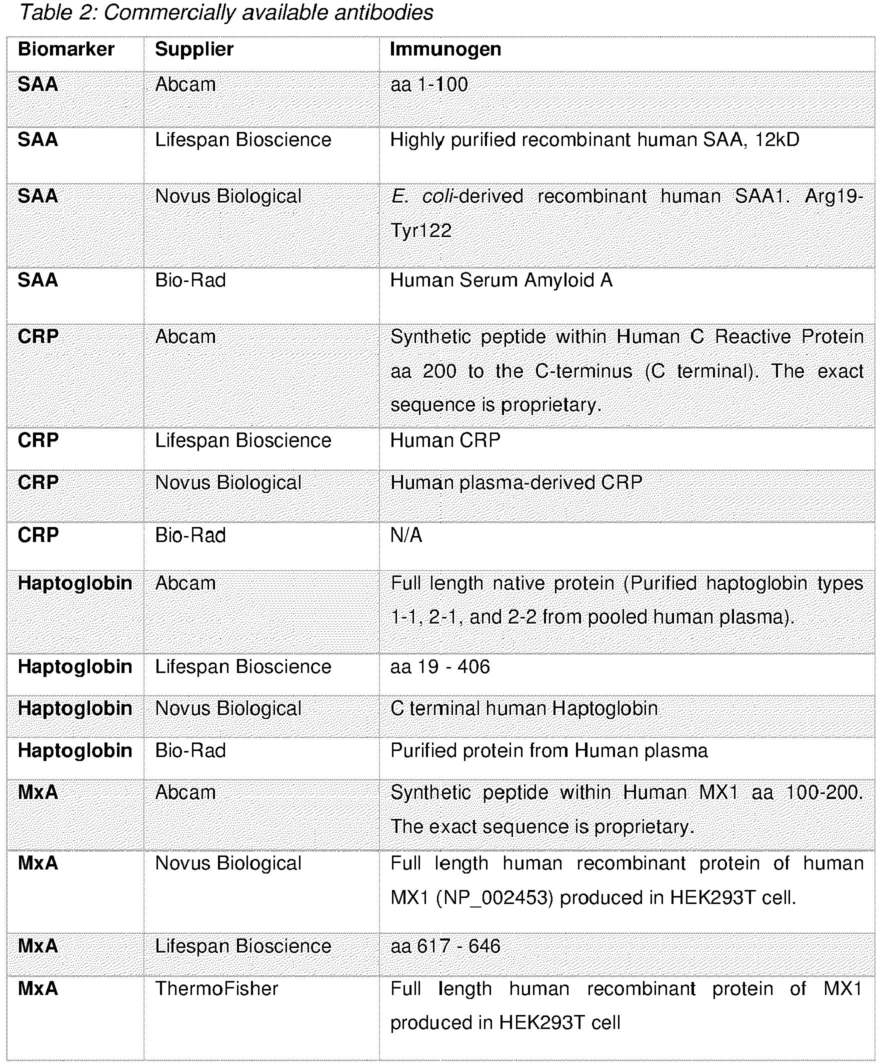

Alternatively, since the amino acid sequences for the targets described herein are known and publicly available at the NCBI website, one of skill in the art can raise their own antibodies against these polypeptides of interest for the purpose of the methods described herein.

The amino acid and nucleotide sequences of the polypeptides described herein have been assigned NCBI accession numbers for different species such as human, mouse and rat. In particular, the NCBI accession numbers for the amino acid sequence of human SAA, human CRP, human Hp and human MxA is included herein, *e.g*. SEQ ID NO: 1, 4, 7, and 10, respectively. The NCBI accession numbers for the nucleotide sequence of human SAA, human CRP, human Hp and human MxA is included herein, e.g. SEQ ID NO: 2, 3, 5, 6, 8, 9, 11 and 12.

In some embodiments of any of the aspects, immunohistochemistry ("IHC") and immunocytochemistry ("ICC") techniques can be used. IHC is the application of immunochemistry to tissue sections, whereas ICC is the application of immunochemistry to cells or tissue imprints after they have undergone specific cytological preparations such as, for example, liquid-based preparations. Immunochemistry is a family of techniques based on the use of an antibody, wherein the antibodies are used to specifically target molecules inside or on the surface of cells. The antibody typically contains a marker that will undergo a biochemical reaction, and thereby experience a change of color, upon encountering the targeted molecules. In some instances, signal amplification can be integrated into the particular protocol, wherein a secondary antibody, that includes the marker stain or marker signal, follows the application of a primary specific antibody.

In some embodiments of any of the aspects, the assay can be a Western blot analysis. Alternatively, proteins can be separated by two-dimensional gel electrophoresis systems. Two-dimensional gel electrophoresis is well known in the art and typically involves iso-electric focusing along a first dimension followed by SDS-PAGE electrophoresis along a second dimension. These methods also require a considerable amount of cellular material. The analysis of 2D SDS-PAGE gels can be performed by determining the intensity of protein spots on the gel or can be performed using immune detection. In other embodiments, protein samples are analyzed by mass spectroscopy.

Immunological tests can be used with the methods and assays described herein and include, for example, competitive and non-competitive assay systems using techniques such as Western blots, radioimmunoassay (RIA), ELISA (enzyme linked immunosorbent assay), "sandwich" immunoassays, immunoprecipitation assays, immunodiffusion assays, agglutination assays, *e.g*. latex agglutination, complement-fixation assays, immunoradiometric assays, fluorescent immunoassays, *e.g*. FIA (fluorescence-linked immunoassay), chemiluminescence immunoassays (CLIA), electrochemiluminescence immunoassay (ECLIA, counting immunoassay (CIA), lateral flow tests or immunoassay (LFIA), magnetic immunoassay (MIA), and protein A immunoassays. Methods for performing such assays are known in the art, provided an appropriate antibody reagent is available. In some embodiments of any of the aspects, the immunoassay can be a quantitative or a semi-quantitative immunoassay.

An immunoassay is a biochemical test that measures the concentration of a substance in a biological sample, typically a fluid sample such as blood or serum, using the interaction of an antibody or antibodies to its antigen. The assay takes advantage of the highly specific binding of an antibody with its antigen. For the methods and assays described herein, specific binding of the target polypeptides with respective proteins or protein fragments, or an isolated peptide, or a fusion protein described herein occurs in the immunoassay to form a target protein/peptide complex. The complex is then detected by a variety of methods known in the art. An immunoassay also often involves the use of a detection antibody.

Enzyme-linked immunosorbent assay, also called ELISA, enzyme immunoassay or EIA, is a biochemical technique used mainly in immunology to detect the presence of an antibody or an antigen in a sample. The ELISA has been used as a diagnostic tool in medicine and plant pathology, as well as a quality control check in various industries.

In one embodiment, an ELISA involving at least one antibody with specificity for the particular desired antigen (*e.g*., any of the targets as described herein) can also be performed. A known amount of sample and/or antigen is immobilized on a solid support (usually a polystyrene micro titer plate). Immobilization can be either non-specific (*e.g*., by adsorption to the surface) or specific (*e.g*. where another antibody immobilized on the surface is used to capture antigen or a primary antibody). After the antigen is immobilized, the detection antibody is added, forming a complex with the antigen. The detection antibody can be covalently linked to an enzyme or can itself be detected by a secondary antibody which is linked to an enzyme through bio-conjugation. Between each step the plate is typically washed with a mild detergent solution to remove any proteins or antibodies that are not specifically bound. After the final wash step the plate is developed by adding an enzymatic substrate to produce a visible signal, which indicates the quantity of antigen in the sample. Older ELISAs utilize chromogenic substrates, though newer assays employ fluorogenic substrates with much higher sensitivity.

In another embodiment, a competitive ELISA is used. Purified antibodies that are directed against a target polypeptide or fragment thereof are coated on the solid phase of multi-well plate, *i.e.,* conjugated to a solid surface. A second batch of purified antibodies that are not conjugated on any solid support is also needed. These nonconjugated purified antibodies are labeled for detection purposes, for example, labeled with horseradish peroxidase to produce a detectable signal. A sample (*e.g*., a blood sample) from a subject is mixed with a known amount of desired antigen (*e.g*., a known volume or concentration of a sample comprising a target polypeptide) together with the horseradish peroxidase labeled antibodies and the mixture is then are added to coated wells to form competitive combination. After incubation, if the polypeptide level is high in the sample, a complex of labeled antibody reagent-antigen will form. This complex is free in solution and can be washed away. Washing the wells will remove the complex. Then the wells are incubated with TMB (3,3',5,5'-tetramethylbenzidene) color development substrate for localization of horseradish peroxidase-conjugated antibodies in the wells. There will be no color change or little color change if the target polypeptide level is high in the sample. If there is little or no target polypeptide present in the sample, a different complex in formed, the complex of solid support bound antibody reagents-target polypeptide. This complex is immobilized on the plate and is not washed away in the wash step. Subsequent incubation with TMB will produce significant color change. Such a competitive ELSA test is specific, sensitive, reproducible and easy to operate.

There are other different forms of ELISA, which are well known to those skilled in the art. The standard techniques known in the art for ELISA are described in "Methods in Immunodiagnosis", 2nd Edition, Rose and Bigazzi, eds. John Wiley & Sons, 1980; and Oellerich, M. 1984, J. Clin. Chem. Clin. Biochem. 22:895-904. These references are hereby incorporated by reference in their entirety.

In one embodiment, the levels of a polypeptide in a sample can be detected by a lateral flow immunoassay test (LFIA), also known as the immunochromatographic assay, or strip test. LFIAs are a simple device intended to detect the presence (or absence) of antigen, *e.g*. a polypeptide, in a fluid sample. There are currently many LFIA tests used for medical diagnostics, either for home testing, point of care testing, or laboratory use. LFIA tests are a form of immunoassay in which the test sample flows along a solid substrate via capillary action. After the sample is applied to the test strip it encounters a colored reagent (generally comprising antibody specific for the test target antigen) bound to microparticles which mixes with the sample and transits the substrate encountering lines or zones which have been pretreated with another antibody or antigen. Depending upon the level of target polypeptides present in the sample the colored reagent can be captured and become bound at the test line or zone. LFIAs are essentially immunoassays adapted to operate along a single axis to suit the test strip format or a dipstick format. Strip tests are extremely versatile and can be easily modified by one skilled in the art for detecting an enormous range of antigens from fluid samples such as urine, blood, water, and/or homogenized tissue samples etc. Strip tests are also known as dip stick tests, the name bearing from the literal action of "dipping" the test strip into a fluid sample to be tested. LFIA strip tests are easy to use, require minimum training and can easily be included as components of point-of-care test (POCT) diagnostics to be use on site in the field. LFIA tests can be operated as either competitive or sandwich assays. Sandwich LFIAs are similar to sandwich ELISA. The sample first encounters colored particles which are labeled with antibodies raised to the target antigen. The test line will also contain antibodies to the same target, although it may bind to a different epitope on the antigen. The test line will show as a colored band in positive samples. In some embodiments of any of the aspects, the lateral flow immunoassay can be a double antibody sandwich assay, a competitive assay, a quantitative assay or variations thereof. Competitive LFIAs are similar to competitive ELISA. The sample first encounters colored particles which are labeled with the target antigen or an analogue. The test line contains antibodies to the target/its analogue. Unlabelled antigen in the sample will block the binding sites on the antibodies preventing uptake of the colored particles. The test line will show as a colored band in negative samples. There are a number of variations on lateral flow technology. It is also possible to apply multiple capture zones to create a multiplex test.

The use of "dip sticks" or LFIA test strips and other solid supports have been described in the art in the context of an immunoassay for a number of antigen biomarkers. U.S. Pat. Nos. 4,943,522; 6,485,982; 6,187,598; 5,770,460; 5,622,871; 6,565,808, U. S. patent applications Ser. No. 10/278,676; U.S. Ser. No. 09/579,673 and U.S. Ser. No. 10/717,082, which are incorporated herein by reference in their entirety, are non-limiting examples of such lateral flow test devices. Examples of patents that describe the use of "dip stick" technology to detect soluble antigens via immunochemical assays include but are not limited to US Patent Nos. 4,444,880; 4,305,924; and 4,135,884; which are incorporated by reference herein in their entireties. The apparatuses and methods of these three patents broadly describe a first component fixed to a solid surface on a "dip stick" which is exposed to a solution containing a soluble antigen that binds to the component fixed upon the "dip stick," prior to detection of the component-antigen complex upon the stick. It is within the skill of one in the art to modify the teachings of this "dip stick" technology for the detection of polypeptides using antibody reagents as described herein.

Other techniques can be used to detect the level of a polypeptide in a sample. One such technique is the dot blot, an adaptation of Western blotting (Towbin et al., Proc. Nat. Acad. Sci. 76:4350 (1979)). In a Western blot, the polypeptide or fragment thereof can be dissociated with detergents and heat and separated on an SDS-PAGE gel before being transferred to a solid support, such as a nitrocellulose or PVDF membrane. The membrane is incubated with an antibody reagent specific for the target polypeptide or a fragment thereof. The membrane is then washed to remove unbound proteins and proteins with non-specific binding. Detectably labeled enzyme-linked secondary or detection antibodies can then be used to detect and assess the amount of polypeptide in the sample tested. A dot blot immobilizes a protein sample on a defined region of a support, which is then probed with antibody and labelled secondary antibody as in Western blotting. The intensity of the signal from the detectable label in either format corresponds to the amount of enzyme present, and therefore the amount of polypeptide. Levels can be quantified, for example by densitometry.

In some embodiments of any of the aspects, the level of a target can be measured, by way of non-limiting example, by Western blot; immunoprecipitation; enzyme-linked immunosorbent assay (ELISA); radioimmunological assay (RIA); sandwich assay; fluorescence in situ hybridization (FISH); immunohistological staining; radioimmunometric assay; immunofluoresence assay; mass spectroscopy and/or immunoelectrophoresis assay.

In certain embodiments, the gene expression products as described herein can be instead determined by determining the level of messenger RNA (mRNA) expression of the genes described herein. Such molecules can be isolated, derived, or amplified from a biological sample, such as a blood sample. Techniques for the detection of mRNA expression is known by persons skilled in the art, and can include but not limited to, PCR procedures, RT-PCR, quantitative RT-PCR Northern blot analysis, differential gene expression, RNAse protection assay, microarray based analysis, next-generation sequencing; hybridization methods, *etc.*

In general, the PCR procedure describes a method of gene amplification which is comprised of (i) sequence-specific hybridization of primers to specific genes or sequences within a nucleic acid sample or library, (ii) subsequent amplification involving multiple rounds of annealing, elongation, and denaturation using a thermostable DNA polymerase, and (iii) screening the PCR products for a band of the correct size. The primers used are oligonucleotides of sufficient length and appropriate sequence to provide initiation of polymerization, i.e. each primer is specifically designed to be complementary to a strand of the genomic locus to be amplified. In an alternative embodiment, mRNA level of gene expression products described herein can be determined by reverse-transcription (RT) PCR and by quantitative RT-PCR (QRT-PCR) or real-time PCR methods. Methods of RT-PCR and QRT-PCR are well known in the art.

In some embodiments of any of the aspects, the level of an mRNA can be measured by a quantitative sequencing technology, e.g. a quantitative next-generation sequence technology. Methods of sequencing a nucleic acid sequence are well known in the art. Briefly, a sample obtained from a subject can be contacted with one or more primers which specifically hybridize to a single-strand nucleic acid sequence flanking the target gene sequence and a complementary strand is synthesized. In some next-generation technologies, an adaptor (double or single-stranded) is ligated to nucleic acid molecules in the sample and synthesis proceeds from the adaptor or adaptor compatible primers. In some third-generation technologies, the sequence can be determined, e.g. by determining the location and pattern of the hybridization of probes, or measuring one or more characteristics of a single molecule as it passes through a sensor (e.g. the modulation of an electrical field as a nucleic acid molecule passes through a nanopore). Exemplary methods of sequencing include, but are not limited to, Sanger sequencing, dideoxy chain termination, high-throughput sequencing, next generation sequencing, 454 sequencing, SOLiD sequencing, polony sequencing, Illumina sequencing, Ion Torrent sequencing, sequencing by hybridization, nanopore sequencing, Helioscope sequencing, single molecule real time sequencing, RNAP sequencing, and the like. Methods and protocols for performing these sequencing methods are known in the art, see, *e.g*. "Next Generation Genome Sequencing" Ed. Michal Janitz, Wiley-VCH; "High-Throughput Next Generation Sequencing" Eds. Kwon and Ricke, Humanna Press, 2011; and Sambrook et al., Molecular Cloning: A Laboratory Manual (4 ed.), Cold Spring Harbor Laboratory Press, Cold Spring Harbor, N.Y., USA (2012); which are incorporated by reference herein in their entireties.

The nucleic acid sequences of the genes described herein have been assigned NCBI accession numbers for different species such as human, mouse and rat. For example, the human SAA nucleotide sequences (*e.g*. SEQ ID NO: 2 and 3) is known. Accordingly, a skilled artisan can design an appropriate primer based on the known sequence for determining the mRNA level of the respective gene.

Nucleic acid and ribonucleic acid (RNA) molecules can be isolated from a particular biological sample using any of a number of procedures, which are well-known in the art, the particular isolation procedure chosen being appropriate for the particular biological sample. For example, freeze-thaw and alkaline lysis procedures can be useful for obtaining nucleic acid molecules from solid materials; heat and alkaline lysis procedures can be useful for obtaining nucleic acid molecules from urine; and proteinase K extraction can be used to obtain nucleic acid from blood (Roiff, A et al. PCR: Clinical Diagnostics and Research, Springer (1994)).

In some embodiments of any of the aspects, one or more of the reagents (*e.g*. an antibody reagent and/or nucleic acid probe) described herein can comprise a detectable label and/or comprise the ability to generate a detectable signal (*e.g*. by catalyzing reaction converting a compound to a detectable product). Detectable labels can comprise, for example, a light-absorbing dye, a fluorescent dye, or a radioactive label. Detectable labels, methods of detecting them, and methods of incorporating them into reagents (*e.g*. antibodies and nucleic acid probes) are well known in the art.

In some embodiments of any of the aspects, detectable labels can include labels that can be detected by spectroscopic, photochemical, biochemical, immunochemical, electromagnetic, radiochemical, or chemical means, such as fluorescence, chemifluoresence, or chemiluminescence, or any other appropriate means. The detectable labels used in the methods described herein can be primary labels (where the label comprises a moiety that is directly detectable or that produces a directly detectable moiety) or secondary labels (where the detectable label binds to another moiety to produce a detectable signal, *e.g*., as is common in immunological labeling using secondary and tertiary antibodies). The detectable label can be linked by covalent or non-covalent means to the reagent. Alternatively, a detectable label can be linked such as by directly labeling a molecule that achieves binding to the reagent via a ligand-receptor binding pair arrangement or other such specific recognition molecules. Detectable labels can include, but are not limited to radioisotopes, bioluminescent compounds, chromophores, antibodies, chemiluminescent compounds, fluorescent compounds, metal chelates, and enzymes.

In other embodiments, the detection reagent is label with a fluorescent compound. When the fluorescently labeled reagent is exposed to light of the proper wavelength, its presence can then be detected due to fluorescence. In some embodiments of any of the aspects, a detectable label can be a fluorescent dye molecule, or fluorophore including, but not limited to fluorescein, phycoerythrin, phycocyanin, o-phthaldehyde, fluorescamine, Cy3™, Cy5™, allophycocyanine, Texas Red, peridenin chlorophyll, cyanine, tandem conjugates such as phycoerythrin-Cy5™, green fluorescent protein, rhodamine, fluorescein isothiocyanate (FITC) and Oregon Green™, rhodamine and derivatives (*e.g*., Texas red and tetrarhodimine isothiocynate (TRITC)), biotin, phycoerythrin, AMCA, CyDyes™, 6-carboxyfhiorescein (commonly known by the abbreviations FAM and F), 6-carboxy-2',4',7',4,7-hexachlorofiuorescein (HEX), 6-carboxy-4',5'-dichloro-2',7'-dimethoxyfiuorescein (JOE or J), N,N,N',N'-tetramethyl-6carboxyrhodamine (TAMRA or T), 6-carboxy-X-rhodamine (ROX or R), 5-carboxyrhodamine-6G (R6G5 or G5), 6-carboxyrhodamine-6G (R6G6 or G6), and rhodamine 110; cyanine dyes, *e.g*. Cy3, Cy5 and Cy7 dyes; coumarins, e.g umbelliferone; benzimide dyes, *e.g.* Hoechst 33258; phenanthridine dyes, *e.g.* Texas Red; ethidium dyes; acridine dyes; carbazole dyes; phenoxazine dyes; porphyrin dyes; polymethine dyes, *e.g.* cyanine dyes such as Cy3, Cy5, *etc*.; BODIPY dyes and quinoline dyes. In some embodiments of any of the aspects, a detectable label can be a radiolabel including, but not limited to ³H, ¹²⁵I, ³⁵S, ¹⁴C, ³²P, and ³³P. In some embodiments of any of the aspects, a detectable label can be an enzyme including, but not limited to horseradish peroxidase and alkaline phosphatase. An enzymatic label can produce, for example, a chemiluminescent signal, a color signal, or a fluorescent signal. Enzymes contemplated for use to detectably label an antibody reagent include, but are not limited to, malate dehydrogenase, staphylococcal nuclease, delta-V-steroid isomerase, yeast alcohol dehydrogenase, alpha-glycerophosphate dehydrogenase, triose phosphate isomerase, horseradish peroxidase, alkaline phosphatase, asparaginase, glucose oxidase, beta-galactosidase, ribonuclease, urease, catalase, glucose-VI-phosphate dehydrogenase, glucoamylase and acetylcholinesterase. In some embodiments of any of the aspects, a detectable label is a chemiluminescent label, including, but not limited to lucigenin, luminol, luciferin, isoluminol, theromatic acridinium ester, imidazole, acridinium salt and oxalate ester. In some embodiments of any of the aspects, a detectable label can be a spectral colorimetric label including, but not limited to colloidal gold or colored glass or plastic (*e.g*., polystyrene, polypropylene, and latex) beads.

In some embodiments of any of the aspects, detection reagents can also be labeled with a detectable tag, such as c-Myc, HA, VSV-G, HSV, FLAG, V5, HIS, or biotin. Other detection systems can also be used, for example, a biotin-streptavidin system. In this system, the antibodies immunoreactive (*i.e.* specific for) with the biomarker of interest is biotinylated. Quantity of biotinylated antibody bound to the biomarker is determined using a streptavidin-peroxidase conjugate and a chromagenic substrate. Such streptavidin peroxidase detection kits are commercially available, *e.g*. from DAKO; Carpinteria, CA. A reagent can also be detectably labeled using fluorescence emitting metals such as ¹⁵²Eu, or others of the lanthanide series. These metals can be attached to the reagent using such metal chelating groups as diethylenetriaminepentaacetic acid (DTPA) or ethylenediaminetetraacetic acid (EDTA).

A level which is less than a reference level can be a level which is less by at least about 10%, at least about 20%, at least about 50%, at least about 60%, at least about 80%, at least about 90%, or less relative to the reference level. In some embodiments of any of the aspects, a level which is less than a reference level can be a level which is statistically significantly less than the reference level.

A level which is more than a reference level can be a level which is greater by at least about 10%, at least about 20%, at least about 50%, at least about 60%, at least about 80%, at least about 90%, at least about 100%, at least about 200%, at least about 300%, at least about 500% or more than the reference level. In some embodiments of any of the aspects, a level which is more than a reference level can be a level which is statistically significantly greater than the reference level.

In some embodiments of any of the aspects, the reference can be a level of the target molecule in a population of subjects who do not have or are not diagnosed as having, and/or do not exhibit signs or symptoms of infection, inflammation or diseases associated with these symptoms. In some embodiments of any of the aspects, the reference can also be a level of expression of the target molecule in a control sample, a pooled sample of control individuals or a numeric value or range of values based on the same. In some embodiments of any of the aspects, the reference can be the level of a target molecule in a sample obtained from the same subject at an earlier point in time, *e.g*., the methods described herein can be used to determine if a subject's sensitivity or response to a given therapy is changing over time.

In some embodiments of any of the aspects, the level of expression products of no more than 200 other genes is determined. In some embodiments of any of the aspects, the level of expression products of no more than 100 other genes is determined. In some embodiments of any of the aspects, the level of expression products of no more than 20 other genes is determined. In some embodiments of any of the aspects, the level of expression products of no more than 10 other genes is determined.

In some embodiments of the foregoing aspects, the expression level of a given gene can be normalized relative to the expression level of one or more reference genes or reference proteins.

In some embodiments, the reference level can be the level in a sample of similar cell type, sample type, sample processing, and/or obtained from a subject of similar age, sex and other demographic parameters as the sample/subject for which the level of SAA, CRP, Hp and/or MxA is to be determined. In some embodiments, the test sample and control reference sample are of the same type, that is, obtained from the same biological source, and comprising the same composition, *e.g*. the same number and type of cells.

The term "sample" or "test sample" as used herein denotes a sample taken or isolated from a biological organism, *e.g*., a blood or plasma sample from a subject. In some embodiments of any of the aspects, the present invention encompasses several examples of a biological sample. In some embodiments of any of the aspects, the biological sample is cells, or tissue, or peripheral blood, or bodily fluid. Exemplary biological samples include, but are not limited to, a biopsy, a tumor sample, biofluid sample; blood; serum; plasma; urine (and urine derivatives); sperm; mucus; tissue biopsy; organ biopsy; synovial fluid; bile fluid; cerebrospinal fluid; mucosal secretion; effusion; sweat; saliva; and/or tissue sample *etc.* The term also includes a mixture of the above-mentioned samples. The term "test sample" also includes untreated or pretreated (or pre-processed) biological samples. In some embodiments of any of the aspects, a test sample can comprise cells from a subject. In some embodiments of any of the aspects, the test sample can be whole blood, venous blood, arterial blood, capillary blood, serum, buffy coat (white blood cells and platelets), plasma from processed blood tubes (EDTA, K2 EDTA, sodium or lithium heparin, sodium citrate, potassium oxalate).

The test sample can be obtained by removing a sample from a subject but can also be accomplished by using a previously isolated sample (*e.g*. isolated at a prior timepoint and isolated by the same or another person).

In some embodiments of any of the aspects, the test sample can be an untreated test sample. As used herein, the phrase "untreated test sample" refers to a test sample that has not had any prior sample pre-treatment except for dilution and/or suspension in a solution. Exemplary methods for treating a test sample include, but are not limited to, centrifugation, filtration, sonication, homogenization, heating, freezing and thawing, and combinations thereof. In some embodiments of any of the aspects, the test sample can be a frozen test sample, *e.g*., a frozen tissue. The frozen sample can be thawed before employing methods, assays and systems described herein. After thawing, a frozen sample can be centrifuged before being subjected to methods, assays and systems described herein. In some embodiments of any of the aspects, the test sample is a clarified test sample, for example, by centrifugation and collection of a supernatant comprising the clarified test sample. In some embodiments of any of the aspects, a test sample can be a pre-processed test sample, for example, supernatant or filtrate resulting from a treatment selected from the group consisting of centrifugation, filtration, thawing, purification, and any combinations thereof. In some embodiments of any of the aspects, the test sample can be treated with a chemical and/or biological reagent. Chemical and/or biological reagents can be employed to protect and/or maintain the stability of the sample, including biomolecules (*e.g*., nucleic acid and protein) therein, during processing. One exemplary reagent is a protease inhibitor, which is generally used to protect or maintain the stability of protein during processing. The skilled artisan is well aware of methods and processes appropriate for pre-processing of biological samples required for determination of the level of an expression product as described herein.

In some embodiments of any of the aspects, the methods, assays, and systems described herein can further comprise a step of obtaining or having obtained a test sample from a subject. In some embodiments of any of the aspects, the subject can be a human subject. In some embodiments of any of the aspects, the subject can be a subject in need of treatment for (*e.g*., having or diagnosed as having an infection, a non-infectious disease, an inflammatory disease) an infection, a non-infectious disease, an inflammatory disease or a subject at risk of or at increased risk of developing an infection, a non-infectious disease, an inflammatory disease as described elsewhere herein.

In some embodiments of any of the aspects, the sample obtained from a subject can be a biopsy sample. In some embodiments of any of the aspects, the sample obtained from a subject can be a blood or serum sample.

### Sequences

SEQ ID NO: 1 (hSAA) (Accession No. AAA60297)



SEQ ID NO: 2 (hSAA) (Accession No. M1906.1)
ATGAAGCTTCTCACGGGCCTGGTTTTCTGCTCCTTGGTCCTGGGTGTCAGCAGCCGAAGCTTCTTTTCGT
TCCTTGGCGAGGCTTTTGATGGGGCTCGGGACATGTGGAGAGCCTACTCTGACATGAGAGAAGCCAATTA
CATCGGCTCAGACAAATACTTCCATGCTCGGGGGAACTATGATGCTGCCAAAAGGGGACCTGGGGGTGTC
TGGGCTGCAGAAGCGATCAGCGATGCCAGAGAGAATATCCAGAGATTCTTTGGCCATGGTGCGGAGGACT
CGCTGGCTGATCAGGCTGCCAATGAATGGGGCAGGAGTGGCAAAGACCCCAATCACTTCCGACCTGCTGG
CCTGTCTGAGAAATACTGA

SEQ ID NO: 3 (hSAA) (Accession No. NG_021330)
TTTTCACAGTTCATTTACGGCCCTCTTTCCAACACATTCACTGCCAATACTCTTATTGACAATAACTGTA
TTGTTGAACCTTCCAGTATCCTGCATTCCCGGATCAAGGCCCCCTCAAAGCCCTGATATGCAAATATCTG
GGAAAAGAATGTTCCAGAGGAAAGGAACAGCTAATCCGAGGCCCCTAGGGTAAGATGTGCCTGGGGGTTT
GGAGACCAGTGTGGCCAGAGCAAAATGAGCAGGAGGAGAGAATTGGATGATGAGGTACGAGAGGAAGGAG
TTAGGACAGTTTGAGTAAAGTTTGAAAACCATTATAAGGGCTTTGACTTCAACTATGAGTGGAAGTGGAA
TCCTCCGGAGAGTTTTGAATGGAGAGTGATAGAAGTTGTCTTGTGTTGTAACAGTCTGGCTGCTATACTG
AAAAGAGACTAGTTGGCGGCAAAGGGGGAAATGTGGAAGCCAGTTAAGAAGCCATCATAACCCAGAAGGT
GATGCCTAATAACATCTCTCTGGGAGCAGCGGAGAGATGATAAGGGTTTGCCTTCTGAATATGTTTTTTG
ACAATTAATGTAAACATTTCAAGTAGGCTGAGATTTTATTGCATATTAACAATGTCCATGTTCACTCGCG
GCAGCCGCCCCCTTCTGCGCGGTCATGCCGAGCCAGCACCTGGGCCTGGAACTGGGCCGCAGCCCCCAGC
TTCACCCACCACCTCCCTACCATGGACCCCTGCAAAGTGAACGAGCTTCGGGCCTTTGTGAAAATGTGTA
AGCAGGATCCGAGCGTTCTGCACACCGAGGAAATGCGCTTCCTGAGAGAGTGGGTGGAGAGCATGGGAGG
TAAAGTACCACCTGCTACTCAGAAGGCTAAATCAGAAGAAAATACCAAGGAAGAAAAACCTGATAGTAAG
AAGGTGGAGGAAGACTTAAAGGCAGACGAACCATCAACTGAGGAAAGTGATCTAGAAATTGATAAAGAAG
GTGTGATTGAACCAGACACTGATGCTCCTCAAGAAATGGGAGATGAAAATGTGGAGATAACGGAGGAGAT
GATGGATCAGGCAAATGATAAAAAAGTGGCTGCTATTGAAGTCCTAAATGATGGTGAACTCCAGAAAGCC
ATTGACTTATTCACAGATGCCATCAAGCTGAATCCTCGCTTGGCCATTTTGTATGCAAAGAGGGCCAGTG
TCTTCGTCAAATTACAGAAGCCAAATGCTGCCATCCAAGACTGTGACAGAGCCATTGAAATAAATCCTGA
TTCAGCTCAGCCTTACAAGTGGCGGGGGAAAGCACACAGACTTCTAGGCCACTGGGAAGAAGCAGCCCAT
GATCTTGCCTTTGCCTGTAAATTGGATTATGATGAAGATGCTAGTGCAATGCTGAAAGAAGTTCAACCTA
GGCACAGAAAATTGCAGAACATTGGAGAAAGTATGAGCGAAAACATGAAGAGCGAGAGATCAAAGAAAG
AATAGAACGAGTTAAGAAGGCTCAAGAAGAGCAGGAGAGAGCCCAGAGGGAGGAAGAAGCCAGACGACAG
TCAGGAGCTCACTATGGCCCTTTTCCAGGTGGCTTTCCTGGTGGAATGCCTGGTAATTTTCCCGGAGGAA
TGCCTGGAATGGGAGGGGACATGCCTGGAATGGCCGGAATGCCTGGACTCAATGAAATTCTTAGTGATCC
AGAGGCTCTTGCAGCCATGCAGGATCCAGAAGTTATGGTGGCCTTCCAGGATGTGGCTCAGAACCCAGCA

AATATGTCAAAATACCAGAGCAACCCAAAGGTTATGAATCTCATCAGTAAATTGTCAGCCAAATTTGGAG
GTCAAGCATAATGCCCTTCTGATAAATAAAGCCCTGCTGAAGGAAAAGCAACCTAGATCACCTTATGGAT
GTCGCAATAATACAAACCAACGTACCTCTGACCTTCTCATCAAGAGAGCTGGGGTGCTTTGAAGATAATC
CCTACCCCTCTCCCCCAAATGCAGCTGAAGCATTTTACAGTGGTTTGCCATTAGGGTATTCATTCAGATA
ATGTTTTCCTACTAGGAATTACAAACTTTAAACACTTTTTAAATCTTCAAATATTTAAAACAAATTTAAA
GGGTCTGTTAATTCTTATATTTTTCTTTACTAATCATTGTGGATTTTTCCTTAAATTATTGGGCAGGGAA
TATACTTATTTATGGAAGATTACTGCTCTAATTTGAGTGAAATAAAAGTTATTAGTGCGAGGCAAACATA
AAAAAAAAAAGTCCATGTTCATCTCTAAATGACATCATTGTTCCAAAGCTTTTCCATTCTTCTTAACCTT
CCACCTGTCAATCTATAGGAGATGACTTCTCCTACTTCACTCATGCATTGACTCCTTCAATCAATAAAAG
TGACTAAGAACCTGCTACAGGTGAGGTGCTGTGTTTGGTGTTAAAGTGACAACAGTTATCTGTCAATAAG
CCTGACAAGGTTCCTATCCCTGTGTTTTGTGCACTCTGGGTCAAACTCAGAAATGCAAACAGGTGGAGAG
CGATGAGTTCTATGACTGGTAAAGAAAAGGGCCTGCTGGTTTCCCTCAGGATCTCTGTCCTTCATCTCAA
AATGCATCTTCCTTGTTATCGTTCCTCTCCTTCCTGTCTCAGAGGAAGACCTGCTCCTGCTACACTCTGG
GCAACCTTGTCCCCGTGGCCCTGTGGCCCCTTGGTTGTTGAAGTCTATGTTATGCCCTATCTTTTACCCT
CAGTCACTCTCTCTGTTAACATTCTCCCTGTGCCCTGTAACCCTCCCTCATCTTTAAATAAATCCTCCTC
CTTTGACCTTCGCATGTATTCAGTCATGCAACTCAACAAGCATTTATTGCACAGTGATATTCAATTTGCC
ACTTGCTAAAAGTCTGAACCTTGGCAGCTGAATGTGATCAGAAAAAAAGCACGACTGCTATGACTAGTCT
CACTTTAAATTCATGGTCGTTGACCAAGAGCTACCATACAATCCACTACCTTTCTCAAGTTCAGTCACAT
TCTTCCTTTCCTAGATGTCTGCTTTCTACTTCTCTTCTCTTCTGAAACTTCCCACAACTCCTCGTTCATT
CTCTTCTCAGTTGACAACTTTGCTTCCTATTTCACTGAAAAATAGAAGCAATCAGATATGAACTTCTGGC
TGGGCATGGTAGCTCATGCCTATAATCTCAGCACTTTGGGAGGCCAAGGCAGGAGGACTGCAGGTTAGGA
ATTTGAGACCAGCCTGGGCAACATGGTGAAACTCCCACTGTACTAAAAATTTTAAAAATTACTCAAACAT
ATTGGCAAACAACTGCAGTCCCAGCTACTTGGGAGGTTGAGATGCAAGGATCACTTAAACCTGGGAGGCT
GAGGCTGCAGTGAGCCATGATTGCACCACTGCACTCCAGCTCAGGCAACAGAGCAAGACCCTGTCTTGAG
AGGAGAGGAGAAGAGAGGAGGGGAGGGGAGGGCAGGGGAGGGGAGGGGAGGGGAAGGGAGAGGGGAGGGG
AGAGGGGAGGAGAGAGGGGAGGGGAGGGGAGGGGAGGGGAGGGGAGGAGAGGAGGATCAGGTGAGGAGTA
TGCCAAGGAGTGTTTTTAAGACTTACTGTTTTCTCTTTCCCAACAAGATTGTCATTTCCTTTAAAAAGTA
GTTATCCTGAGGCCTATATTCATAGCATTCTGAAAGAAAGAAAAGAAAAGAGGAAAGAAAGAGAGAGGAA
GGAAGGAAGGAGAAAGAGAGAGGAAGGAAGGAGAAAGAGAGAGGAAGGAAGGGAGGAAGAGAAGAAGGGA
GGAAGAAAAGAAGGAAGGAAGGAGGGAGGGAGGGAAGGGAGGGAGGGAAAGAGGAAGAAAGGAGGGAAAG
AAGGAAGGAAGAGAGAGAGAGGAAGGAAGGAGGAAGAGAGAAGAAGGAAGGAGGAAGACAGAGAGGGAGTAA
GGAAGGAAGGAAGGAGAAAGAGAGAGGAAGGAAGAAATGAAGGAAGGAAGGAAAGAAAGAAAAAAATAAAA
GAGTGAAAACGGACTGGAGAAGAAGAAACCACAGTTGCTGCTATATCCACCAGCCTCTCTGCATGTCCTG
GCCTCAGCCCTGCTGGGCTCTGGTACTGACCACTTCCTTCCTTCCTAATTTCCTAATTGACTAGGCCAGC
TGAGCAGGGCTTTTCTGTGCTGAGGAGGTAAATCTCTGGATATCTAGACTGAGGGGTGGAAGGAGCCTTC
CAGGGCACACATGAGACATGGCAGGGGTAGGCTGCTAGTTTTATTTTGTTTTCTTTTAGACACAGGGTCT
TGCTCTGTTAACCAGGCTGGAGTGCAGTGGCGTGATTATAGCTCACTGCAGCCTTGACCTCCTGGGTCTC
CCACAATCCTTCCGCTTCAGCCTCTTGAGTAGCTGGGACTGCAGGTGCACACTACCACACCCGGTCCATT
TATTTTTATATTTCGTAGAGACAAGATCTTACAGTTTTGCACAGAGTGATCTTAAACTCTTGACCCCAAG
TGATCCTCCTGCCTTGGCCTCCAAAAGCATTGGGATTATAGGAGTGAGCCACTGTGCTGGACCTAGTCTG
TCAGCTTTGAAGCTTTAGATATGAACTCAGAGGGACTTCATTTCAGAGGCATCTGCCATGTGGCCCAGCA
GAGCCCATCCTGAGGAAATGACTGGTAGAGTCAGGAGCTGGCTTCAAAGCTGCCCTCACTTCACACCTTC
CAGCAGCCCAGGTGCCGCCATCACGGGGCTCCCACTCTCAACTCCGCAGCCTCAGCCCCCTCAATGCTGA
GGAGCAGAGCTGGTCTCCTGCCCTGACAGCTGCCAGGCACATCTTGTTCCCTCAGGTTGCACAACTGGGA
TAAATGACCCGGGATGAAGAAACCACTGGCATCCAGGAACTTGTCTTAGACCGTTTTGTAGGGGAAATGA
CCTGCAGGGACTTTCCCCAGGGACCACATCCAGCTTTTCTTCGCTCCCAAGAAACCAGCAGGGAAGGCTC
AGTATAAATAGCAGCCACCGCTCCCTGGCAGGCAGGGACCCGCAGCTCAGCTACAGCACAGATCAGGTGA
GGAGCACACCAAGGAGTGATTTTTAAAACTTACTCTGTTTTCTCTTTCCCAACAAGATTATCATTTCCTT
TAAAAAAAATAGTTATCCTGGGGCATACAGCCATACCATTCTGAAGGTGTCTTATCTCCTCTGATCTAGA
GAGGTAAGCAGGGTCGGGCCTGGTAGTACTTGGATGGGAGAACACCTGGGAATACCAGGTGCTAAAGGCT
TTAAGAATAAAAAATAATGATCCTGCTTTGTGTTTATCCCATGTTGAGTTCTGTGCGGGGCAGAGGGAAC
ACACGGTAAATGCGTTATGGGGAATTATAGGCTACTTGAGGGAGTGACAGTCTGGTGGTAACTCCTGCCT
TCCTCCATCAGTGCCACGTTGGCATCCTCTTATGCAGTCAGGCTTCAGGGCTGATGGGTTCAGAACCGAG
GGCTTCTGGCTCTGAGTGAGGTCCTGCTGCAAGGTTTCCTAGATGAGCCACTGAGACTCTAATAAGATCC
AGTGGAAATAACCAGGCTCTCGTCGGAATATAAGTCCCAAGGGAAGCTGTGCCAGTCTTGTGGGCGACTG
CCTGACTTCTCCTTTCATTTCAGCACCATGAAGCTTCTCACGGGCCTGGTTTTCTGCTCCTTGGTCCTGG
GTGTCAGCAGCCGAAGCTTCTTTTCGTTCCTTGGCGAGGCTTTTGATGGTAAGGCTTCAGAAGGTTTGCA
GGATTTCTGAAGAGAAACATCACCCTGGACCTGATAAACTGGGGAAAATGATGCTTTCGGAAGGCTGCTT
TTGAACCACAGAGTTGCTAGTGTCTGCGTTGCTGAGGCCTGCCAGGAACTAGGGTTTGCTGGGTTGCCTG
TCTCGAGTCTTTCAGAGCTGCTGGGAATATCCCCTTTCCCCGTAGTGCAGCTTCTCAGGATGTGTTAAGT

GGATGGATCACATTTCAGAAGCCGCTGCAAGGTGTATCAAAAACACATCTCCTGAGCCGTAAGGACGGG
GCATCCAGTAACAACGCACACGGGGTATTTTTGGGCTTCCTTAAGATTTGAGCCGCTGCCTTAGGTTGTG
CTGCCCAATGTGCCTGGGGAGCTGCTAAACAGATTAGAGAGTCGAGGATTGTTGTCAGTTACTCAGAGAA
AGAACAATCATCCTTTCCAGGAGCACCTGAGCTGTTTGTTTTGCGTAGAAGATGCAAAATAAGGCCTGCA
ATGGGTATAAAATGTCCCTCAGCATAAATCGCATAGGAGTATGACTAAGGCTGTTGACTCTTCTGTCTTC
TTTCTCCTTCCTCCTTCGATTTCCTAGTTGGATAATGTACAGGGCTCTTTAGCCTCGCTCTGTCAGGGGC
TCCCTTCCTGGTTTGTTCTGTTTCCATTCTTCCTTCTCCAGCCTTCTTGACAAGAGCTGGGAACTAACGT
GCCTCAAGCCCCCACAAGGACCACAGCATTTTCTCATTTAGTTTCAGAATGACTCTGTGACGCAATCTTC
CTCTCTTGGAAGGTGAGAAAGCTGATCTTGGAAGGTGAGAAAGCTGAGACTTAGAGCAGCTGAAGCCAAT
GCCCAGGGACTTACTGCCAGTCAGCAGGTGGCAGGGCAGAGGTTTGAGCCCGGCTGTGCTTGAGGTCAGG
GCTCTTGCCAGGTAGACGCATCACTGACCACCTCCTAGAGGTTGATGGTTATGAATCTCAGGCACACCTT
GGCATCACCTGAAATACCCATGCCTTCAACTCCCCAGCAGAGTCTGCAGAAACTGGCCTGGGGTGTGGCC
TGGGCACTGGGACTTTCAGTTTCTCTCTGGGTGATTAGAAAGTGCAGCCAAGGCTCACGCCTGTAATTCC
AGCACTTTGGGAGGCCAAGGTGGATGAATCACTTGAGGTCATGAGTTCCGGAGCAGCCTGGCCAACATGG
TGAAACCCCGTCTCTACTAAAAATACTAAAATGTAGCCAGGCGTGGTGGCAGGCACCTGTAATCCCAGCT
ACTCAGGAGGCTGAAGCACGAGAATCACTTGAACCCGAGAAGCAGAGGTTGCAGTGACTAGAGATCGCAC
CAGTGTCCTCCAACCTGGGTGACAGAGCGAGACTCCATCTAAAAAAAATGAAAAAGAAAGTGCAGCCAAG
GCAGAGCACCACTGCCCTATTGCTTCCTCAAGCAACCCACAGCATCAGTACAGCCTACTAAGAAAGTATT
TAGGGACTTTTATGCTCCTAACAGTCACTGGAACTCACGTCACAATGACGTGTATTCCATTTGCAAGAAT
ATATACTTTAGGTCGGGGTGCGGTGGCTCACGCCTGTAATCCCAGCACTTTGGGAGGCCAAGGCAGGGGG
ATCACGAGGTCAGGAGTTCGAGACCAGCCTGACCAACATGGTGAAATCCCCGTCTCTACTAAAAATACAA
AAATTAGCCAGGCGTGATGGCGCATGCCTGTAATCTCAGCTACTCAGGAGGCTGAGGCAGAAGAATCTCT
TGAACCTGGGAGGTGGAGGTTGCGATGAGCTGAGATAGCACCACTGCACTCCAGCCTGGGCGACAGAGCA
AGACTCTGTCTAAAAAAAAAAAAAAAAAAAAAAAAAAAAAAAAAGAATATAAACTTTAGTAGTCAGGGCAG
AAGTACTCTGTGTCTGCCACCTTTCTCAGCATCAGTATTCCATGTCACTACCTCATTCATACACACTCCT
GGATCTTATCATAGGCAGCTTCATTCTATAGCAGTGGCTCTTCACCAGGGCACTTGAAGAAGCCAACTAG
GATAAAGGAATGTGCTTCTCAACCCATGGTATCCAAGGCTGCTATGATCACAGGCTGAAAGCTTGAAGTC
AGTGGAAGATTTGTCCTTCCTCATTCCCCTCTAAGGTGTTGTTGGAGTCTTTATGTTCTCCTGATGTCCC
TTCTGCCTTTCCTTTCCTTTCCAGGGGCTCGGGACATGTGGAGAGCCTACTCTGACATGAGAGAAGCCAA
TTACATCGGCTCAGACAAATACTTCCATGCTCGGGGGAACTATGATGCTGCCAAAAGGGGACCTGGGGGT
GCCTGGGCTGCAGAAGTGATCAGGTAACTGGAGCTCCTGGGACGTTAGGGCTGGGTGAGCAGAGCTTGCC
TGCCTTGGACAGTCAGGAGGGAGACGAGCTCCTTGTGGAGAAGTTAGAGGCTGCGGCCCCTCCTCCTCTT
GCCCTCTCTCTGCCTCTGTGCTCAGTGTGAGGTCTGAGTGGATGGTAGGAGTGAGTGATTCCTCATCCTC
CCTCTCTGGGTGCTGTTCATCCAGCCTAGGGGTGCCCAGCCTGGCTGAATGGGGTGGTGCCCAGTGTTTT
CATCCCTCCTTCCTTGGCCTTTCTGGGCTCCTCTCTGAGCCCTCCCTTGGAACAGGGAGAATGGGAGGGT
GGGCTATTGCTCACTGGCCTGATTATTAATCTCCTTCTTGCCTGCCTTGATTACAGCGATGCCAGAGAGA
ATATCCAGAGATTCTTTGGCCATGGTGCGGAGGACTCGCTGGCTGATCAGGCTGCCAATGAATGGGGCAG
GAGTGGCAAAGACCCCAATCACTTCCGACCTGCTGGCCTGCCTGAGAAATACTGAGCTTCCTCTTCACTC
TGCTCTCAGGAGATCTGGCTGTGAGGCCCTCAGGGCAGGGATACAAAGCGGGGAGAGGGTACACAATGGG
TATCTAATAAATACTTAAGAGGTGGAATTTGTGGAAACTGGGTGTTATACTTTGTGGTATAGACTGCCTG
TTTAGTATGAAGGGGCGATCCATGCACATCTAAGTGAACGTGGAGGCTGGGTGGGTGGGAGACGACTCCT
GGGCACACAGGGCATCCTGGGCATCCCTGAGGCAAGGACATGATGAGTTCAGTGGCCACCCCCACAGGAT
CCCAGGGGCTTCAGCAGATCCCACCCCTTACCCCATGTGAGCAGCTGCCCAGTGAGTCTGTAGGAACCCG
AGCCACATTCCCAGTGAGTTCAACTGCACCCCGGCACGTTTTGCTAGCACCTCAATGGAGAGCTCCTTGC
TTGCAGCTTTGGCTTGTGGCACCCAGCAAAAGCTTCCTGCCACCCAGTGGCTACAGCCACACACTCTCCA
GCAAGATTTAATCTCAGCCTTGTGAGGAGCCCTTTCCCAAATTTATTTCTTTCTGTGTTTTTTATCCCTT
AGTAGCTAATCTCATGTTAGCCATTAATAACTCTCTATGTTAAACCCTTCCTTTTGTATCTGCGGCTACA
TTGATCAATTGTCTCACACCGCTCACCCACCCCCTCTCCCTGGTCATGCAGAGGCCTCACCAGTCATTTT
ATTGCTATTCCCAGGCCTCTGTGTGCCCAGATCTCTTCACTGCCCTGTCAGTTGTGTCCTGTCCCCTTCT
CGACCTCCTGGCCTTGTCCTCAATGATGTTTCTATGAGGCTTTGGAAAGCCTCATCCCAAGAGTCCTGGC
AACTGATACATTAGTCTCACCAACACTAGCCCCTACTCCTGATCTCATTTTAAAATTTTATTTTCTTATT
TTATTATTATTATTTTTAGGGACATCACCCTGCTTCTGACTGACCCAATTTTTAAAGTTTCTCTTTATTC
TCCTTAAAGAATGGCTCTCCCATTTGTTTCCCTCATCTTTCTTTTCACTGACTTAGAATTCAGGTCCAGA
CAAAAATTCCACTTCCTTGAAGAGCCTTCCCAGTAGCCATGAACCACACTCTGGGGCAGAGTTTGTGGCT
CCCAAGCACTTTGTTCACACCTGCTCGTTCATCTTTACCCCCTCCTCTCAGAATTGGTTTTGTATGTCAG
TTCCCCTGCTGGACTGGAAGCCCCTGTAACATAACTAGCATTTGAACAGTTCATAAAAACACTTTCATTT
CTATTGTCCTGTGTAACTGTTCACAAGATACATTAGTCTCACTCATTGTCGTTTGACAATGTTTATTGTT
CTAAAGAGAAAGCGAGATATGTAGGTAGACACAACACAATAAGAGCCTCAATACAGGCACACACTGGTGG
CAGTCCAAACTCTGCTGGAATATGGAAGGCCAGTGAATAGTAACTAGAATCGCTGCCATCTTTTACACTG
CAATTGCAAGACTGTGTCCTATACTTTACATGCTTTATCGGATTGACACTGCAGAGAAGCCTTTGATGTT

CTCGGTTGGATAAACAGGTTTAGAGGTATGTGTTCAGGCCTTCTCCAAGGTCACACATGTAACAGGTGCA
GAGCCAAGACTGCAGGCTGGTTTGTCTGACTCCAGAACCTGTGATACACTGAACAAGGCCAAGAGATTCA
GCAGTGGAGACCTCCTCTCTTCTTCAGGAAGATAGGGAACATGGTAAGAGCAGTGAATTCCATAAGCATG
GGACCCCGTTATACTTCATTTTTGTAAAATTAGTTCCTTGATCAGAAGCAACGCCTTTGGGTCTCTCGC
TATTGAGTTAAGTGTGGATGTGCCACTCTCCCTAGTACCCCAGAGTGTAGTTGGAGGCTTGCCTAGAGAT
TAGAGGTTGAGGATCCTTCAGTAGTTCCTATACCAAGCCATAAGTTGTGCTGCATCTGCAGGTAGGGTCA
GGGAGCTCCGCTGAACAGATGTGGAGGACATCAGAGTGGGAGGAAAAGGAAGCAAGTCGTGTGGGGAGAG
AAGACCCAGCCTGCAATGATGACTGGTTAACTACTGTTTCTTACCTAATACACACTCAGATAACAGACTA
AGTCTAG

SEQ ID NO: 4 (hCRP)(Accession No. AAA52075)
MEKLLCFLVLTSLSHAFGQTDMSRKAFVFPKESDTSYVSLKAPLTKPLKAFTVCLHFYTELSSTRGYSIF
SYATKRQDNEILIFWSKDIGYSFTVGGSEILFEVPEVTVAPVHICTSWESASGIVEFWVDGKPRVRKSLK
KGYTVGAEASIILGQEQDSFGGNFEGSQSLVGDIGNVNMWDFVLSPDEINTIYLGGPFSPNVLNWRALKY
EVQGEVFTKPQLWP

SEQ ID NO: 5 (hCRP) (Accession No. NM_001329057)
AAGGCAAGAGATCTAGGACTTCTAGCCCCTGAACTTTCAGCCGAATACATCTTTTCCAAAGGAGTGAATT
CAGGCCCTTGTATCACTGGCAGCAGGACGTGACCATGGAGAAGCTGTTGTGTTTCTTGGTCTTGACCAGC
CTCTCTCATGCTTTTGGCCAGACAGACATGTCGAGGAAGGCTTTTGTGTTTCCCAAAGAGTCGGATACTT
CCTATGTATCCCTCAAAGCACCGTTAACGAAGCCTCTCAAAGCCTTCACTGTGTGCCTCCACTTCTACAC
GGAACTGTCCTCGACCCGTGGGTACAGTATTTTCTCGTATGCCACCAAGAGACAAGACAATGAGATTCTC
ATATTTTGGTCTAAGGATATAGGATACAGTTTTACAGTGGGTGGGTCTGAAATATTATTCGAGGTTCCTG
AAGTCACAGTAGCTCCAGTACACATTTGTACAAGCTGGGAGTCCGCCTCAGGGATCGTGGAGTTCTGGGT
AGATGGGAAGCCCAGGGTGAGGAAGAGTCTGAAGAAGGATACACTGTGGGGGCAGAAGCAAGCATCATC
TTGGGGCAGGAGCAGGATTCCTTCGGTGGGAACTTTGAAGGAAGCCAGTCCCTGGTGGGAGACATTGGAA
ATGTGAACATGTGGGACTTTGTGCTGTCACCAGATGAGATTAACACCATCTATCTTGGCGGGCCCTTCAG
TCCTAATGTCCTGAACTGGCGGGCACTGAAGTATGAAGTGCAAGGCGAAGTGTTCACCAAACCCCAGCTG
TGGCCCTGAGGCCCAGCTGTGGGTCCTGAAGTGCTTTCTTAATTTTATGGCTCTTCTGGGAAACTCCTCC
CCTTTTCCACACGAACCTTGTGGGGCTGTGAATTCTTTCTTCATCCCCGCATTCCCAATATACCCAGGCC
ACAAGAGTGGACGTGAACCACAGGGTGTCCTGTCAGAGGAGCCCATCTCCCATCTCCCCAGCTCCCTATC
TGGAGGATAGTTGGATAGTTACGTGTTCCTAGCAGGACCAACTACAGTCTTCCCAAGGATTGAGTTATGG
ACTTTGGGAGTGAGACATCTTCTTGCTGCTGGATTTCCAAGCTGAGAGGACGTGAACCTGGGACCACCAG
TAGCCATCTTGTTTGCCACATGGAGAGAGACTGTGAGGACAGAAGCCAAACTGGAAGTGGAGGAGCCAAG
GGATTGACAAACAACAGAGCCTTGACCACGTGGAGTCTCTGAATCAGCCTTGTCTGGAACCAGATCTACA
CCTGGACTGCCCAGGTCTATAAGCCAATAAAGCCCCTGTTTACTTGA

SEQ ID NO: 6 (hCRP) (Accession No. M11725)
TTTGCTTCCCCTCTTCCCGAAGCTCTGACACCTGCCCCAACAAGCAATGTTGGAAAATTATTTACATAGT
GGCGCAAACTCCCTTACTGCTTTGGATATAAATCCAGGCAGGAGGAGGTAGCTCTAAGGCAAGAGATCTG
GGACTTCTAGCCCCTGAACTTTCAGCCGAATACATCTTTTCCAAAGGAGTGAATTCAGGCCCTTGTATCA
CTGGCAGCAGGACGTGACCATGGAGAAGCTGTTGTGTTTCTTGGTCTTGACCAGCCTCTCTCATGCTTTT
GGCCAGACAGGTAAGGGCCACCCCAGGCTATGGGAGAGTTTTGATCTGAGGTATGGGGGTGGGGTCTAAG
ACTGCATGAACAGTCTCAAAAAAAAAAAAAAAAAGACTGTATGAACAGAACAGTGGAGCATCCTTCATGGT
GTGTGTGTGTGTGTGTGTGTGTGTGTGGTGTGTAACTGGAGAAGGGGTCAGTCTGTTTCTCAATCTTA
AATTCTATACGTAAGTGAGGGGATAGATCTGTGTGATCTGAGAAACCTCTCACATTTGCTTGTTTTTCTG
GCTCACAGACATGTCGAGGAAGGCTTTTGTGTTTCCCAAAGAGTCGGATACTTCCTATGTATCCCTCAAA
GCACCGTTAACGAAGCCTCTCAAAGCCTTCACTGTGTGCCTCCACTTCTACACGGAACTGTCCTCGACCC
GTGGGTACAGTATTTTCTCGTATGCCACCAAGAGACAAGACAATGAGATTCTCATATTTTGGTCTAAGGA
TATAGGATACAGTTTTACAGTGGGTGGGTCTGAAATATTATTCGAGGTTCCTGAAGTCACAGTAGCTCCA
GTACACATTTGTACAAGCTGGGAGTCCGCCTCAGGGATCGTGGAGTTCTGGGTAGATGGGAAGCCCAGGG
TGAGGAAGAGTCTGAAGAAGGGATACACTGTGGGGGCAGAAGCAAGCATCATCTTGGGGCAGGAGCAGGA
TTCCTTCGGTGGGAACTTTGAAGGAAGCCAGTCCCTGGTGGGAGACATTGGAAATGTGAACATGTGGGAC
TTTGTGCTGTCACCAGATGAGATTAACACCATCTATCTTGGCGGGCCCTTCAGTCCTAATGTCCTGAACT
GGCGGGCACTGAAGTATGAAGTGCAAGGCGAAGTGTTCACCAAACCCCAGCTGTGGCCCTGAGGCCAGCT
GTGGGTCCTGAAGGTACCTCCCGGTTTTTTACACCGCATGGGCCCCACGTCTCTGTCTCTGGTACCTCCC
GCTTTTTTACACTGCATGGTTCCCACGTCTCTGTCTCTGGGCCTTTGTTCCCCTATATGCATTGAGGCCT
GCTCCACCCTCCTCAGCGCCTGAGAATGGAGGTAAAGTGTCTGGTCTGGGAGCTCGTTAACTATGCTGGG
AAATGGTCCAAAAGAATCAGAATTTGAGGTGTTTTGTTTTCATTTTTATTTCAAGTTGGACAGATCTTGG
AGATAATTTCTTACCTCACATAGATGAGAAAACTAACACCCAGAAAGGAGAAATGATGTTATAAAAAACT

CATAAGGCAAGAGCTGAGAAGGAAGCGCTGATCTTCTATTTAATTCCCCACCCATGACCCCCAGAAAGCA
GGAGCATTGCCCACATTCACAGGGCTCTTCAGTCTCAGAATCAGGACACTGGCCAGGTGTCTGGTTTGGG
TCCAGAGTGCTCATCATCATGTCATAGAACTGCTGGGCCCAGGTCTCCTGAAATGGAAGCCCAGCAATA
CCACGCAGTCCCTCCACTTTCTCAAAGCACACTGGAAAGGCCATTAGAATTGCCCCAGCAGAGCAGATCT
GCTTTTTTTCCAGAGCAAAATGAAGCACTAGGTATAAATATGTTGTTACTGCCAAGAACTTAAATGACTG
GTTTTTGTTTGCTTGCAGTGCTTTCTTAATTTTATGGCTCTTCTGGGAAACTCCTCCCCTTTTCCACACG
AACCTTGTGGGGCTGTGAATTCTTTCTTCATCCCCGCATTCCCAATATACCCAGGCCACAAGAGTGGACG
TGAACCACAGGGTGTCCTGTCAGAGGAGCCCATCTCCCATCTCCCCAGCTCCCTATCTGGAGGATAGTTG
GATAGTTACGTGTTCCTAGCAGGACCAACTACAGTCTTCCCAAGGATTGAGTTATGGACTTTGGGAGTGA
GACATCTTCTTGCTGCTGGATTTCCAAGCTGAGAGGACGTGAACCTGGGACCACCAGTAGCCATCTTGTT
TGCCACATGGAGAGAGACTGTGAGGACAGAAGCCAAACTGGAAGTGGAGGAGCCAAGGATTGACAAACA
ACAGAGCCTTGACCACGTGGAGTCTCTGAATCAGCCTTGTCTGGAACCAGATCTACACCTGGACTGCCCA
GGTCTATAAGCCAATAAAGCCCCTGTTTACTTGAGTGAGTCCAAGCTGTTTTCTGATAGTTGCTTTAGAA
GTTGTGACTAACTTCTCTATGACCTTTGAA

SEQ ID NO: 7 (hHp) (Accession No. AAA8808)
MSALGAVIALLLWGQLFAVDSGNDVTDIADDGCPKPPEIAHGYVEHSVRYQCKNYYKLRTEGDGVYTLND
KKQWINKAVGDKLPECEADDGCPKPPEIAHGYVEHSVRYQCKNYYKLRTEGDGVYTLNNEKQWINKAVGD
KLPECEAVCGKPKNPANPVQRILGGHLDAKGSFPWQAKMVSHHNLTTGATLINEQWLLTTAKNLFLNHSE
NATAKDIAPTLTLYVGKKQLVEIEKVVLHPNYSQVDIGLIKLKQKVSVNERVMPICLPSKDYAEVGRVGY
VSGWGRNANFKFTDHLKYVMLPVADQDQCIRHYEGSTVPEKKTPKSPVGVQPILNEHTFCAGMSKYQEDT
CYGDAGSAFAVHDLEEDTWYATGILSFDKSCAVAEYGVYVKVTSIQDWVQKTIAEN

SEQ ID NO: 8 (hHp) (Accession No. M69197)
TCTAGAAGCTGCCATCATACTGAAGGTAATCTTCTGAAACTTGCGGTTTTCTTTCAAAAATTATGTTTAT
AAGATGATCCATGTTCTTGCAGAGTTTATTCATTTATGCTGTATAATATTCCATTATATCCACATACAA
TGCAGTATTGACCCTTCCTCCTGTTGATGGGCATTTGTCTTGTTTCTAGTTACTTTGCTATTATATCAGT
GTCACCATGATTATCCAAAAGTAATTCTTTTGTACACTCTAATTTAAGAACAACTAACCCTTTTTAATGA
ATAAATCAACCTTGTATTGAGTTGCTACTAAGTTTCAGTTGACTAGTACCTGGGATACACACAGGTGCAG
ACATTTGACTGAGACATATTGATTTTTCTCATCTGCCTATTTAGGCTAATCACCAGACTATAAAACCATG
AGAACCACTGCCATTGAGTATAGTCTGTGTCAGTCTACACTATAGCTTTAACTAGTTGTGATTTCTTG
CAAAGAGCAATCAGAGAAGACACAATAAACACATTTACTGATTTCAGGCTGGAGAGCTTTTAAGCAATAG
GGAGATGGCCACACACAAGGTGGAGAAAATTACTGTGAAAAGGAAGTACTTTCTTTAGAGCCCCACCTAA
GCTAGGCTGCAGAAATGTCTACAATGGGTTGAAAAAACTCAAAATGAGCCTTTCTGCAGTGTGAAAATC
CTCCAAGATAAAGAGACAGATTGATGGTTCCTGCCGCCGCCCTGTCCTGCCCAGTTGCTGATTTCAGGAA
ATACTTTGGCAGGTTTGTGGGTCATAGAGTTGCCAGGTTTCTTGGGATTTGTAATAGAACATCACAAGAA
AATCAAGTGTGAAGCAAGAGCTCAACTCTTAACAGGGGTATTGTTTGTGGTTTTGTTACTGGAAAAGATA
GTGACCTTACCAGGGCCAAAGTTTGTAGACACAGGAATTACGAAATGGAGAAGGGGGAGAAGTGAGCTAG
TGGCAGCATAAAAAGACCAGCAGATGCCCCACAGCACTGCTCTTCCAGAGGCAAGACCAACCAAGATGAG
GTGGGTCCACAGCTTTCCCTCCTGCCTTTCCTCTGGTTCTTTATTTCAGTCTTTTTTGCATACATCGGTA
GAGATGCAGAAATAGAACAAAGAAACGGGCAAATGGGCTAAATTATAGTGAACCAAAGGGCTTAGTGTGT
TAAATCTTCTCCTTTTCTGCATCCATAGAAGACAGTGCTGCTGTCTTTCCCAGGAGATAAGATTTACTCT
CAGGAGTGTCTTTTTCCTTCAGGTTACATTTTTGACTTTATAGGGTATGTCATCAGCTCCCGTGGTAGGC
TTCCTGGCATCCTGAGTATATTTATTAGCAGATATTTTCCTCTTTAAAAATGTACAATAAGGAAGACTAA
TAGTAACACATTTGAATGACACAATTAATTGACTAGTACCTGGGATACACACTAATACCTGGGATACATC
TAATTAAGGCACTTAGATCTTATAAAAATAAACACTTTTTGAAATGTTGAAATAATAAGACTAGAAACTT
TTTTTTTTTTGAGATGGAGTCTCGCTTTGTCACCAGGCTGCAGTGCAGTGGCATGATCTCGGCTCACTG
CAACCTCCACCTCCCAGGTTCAAGTGATTCTCCTGCCTCAGCCTCCCAGGTAGCTGGGACTACAGGCGTG
CGCCACTATGCCCACCTAATTTTTGTATTTTTAGTAGAGACGGGCTTTCACCATGTTGGCCAGGATGACC
TCGATCTCTTGACCTCGTGATCTGCCTGCCTTGGCCTCCCAAAGTGCTGGGATTACAGGCATGAGCCACC
GTGTCTGGCCTAGAAACTATTTTAATAGAAGCAAGTAGTGCCCGAATGGTTGGCATTTGTTAGTGAGATG
GTGAACTGGCAGACGGCACCTGTGGGTCAATGCCCATGGCCACCGTCCTGCTTTTGGACACCAGTTCTCT
TCCAGGTAACCTTCTGGCATTTTGGGGTTTCAGATACCATTTCCTAAAGGTGAATTATTATAAAATACTA
GAAATACCCACGTGTTTAAAATTATATATTTAAGGAACCTTTTATTACGGAAAATATCAAGAAGTAGAGG
AATAGCACAGTAAGCCCAAAGTTCCCATCTCTGAGTTATCTACATTTTATTACCACTATCTTTGCGGTGT
CTGAGGGAGGTTTCTCTTTCCTGGAGGGCTCCTGTATTATTGCCAATGTACTTTCCTGAATGCAGCCAGA
AACTGAGCCCACCCCTCCACCTATGTGCCTTTCTATCCCTCTCTGAAGCTTCTGCAGAATTCCCAGCAGG
ACAGGGCTTGCTGGAAGCTTGGTATGCTCAGAAGCTGCTAAAGTGTGTATGGGCAGGTGTGGGGGCAATT
TCTTGGTCCTAGCACTTCCATATATCGACTTTCTTTTCTGGCTGCTAAGTGGGAGGAGTGTGTGTGTATG
CATGTGTGTGTGTGTGTGTGTACATGCATGTGTGTGTGGATGCATGCATGTGCTGTGAAGCAGGGAGACT

AGCTTTCCACTCCTCCTTGTCTTCTCTCTGCAGTGCCCTGGGAGCTGTCATTGCCCTCCTGCTCTGGGGA
CAGCTTTTTGCAGTGGACTCAGGCAATGATGTCACGGATATCGCAGGTCAGTCTTTGGTTGGGTAGGAGT
GTGCATCCCACTCTGACCCTCTCGGGTCTGCACTCTCTCTGAGAACACCCAATTCCCCCTTCTTATCTCG
ACCTCTGGGCTTTCAGGACCATAAAGAACATTGGGGTTCCTGCCAGAAATGAGGGGAGCTTGCCTTTCCA
TTGGCTTCTATTCGGGGTGGAAGGAGATTGATGTGCAGAGCAGCTCCCGCTCATCTGACTTTTCACGGTT
CACTGGGAACAATTTCCAAATAGCAAACTCTCTGGCTTCTCTCTCTTTGCAGATGACGGCTGCCCGAAGC
CCCCCGAGATTGCACATGGCTATGTGGAGCACTCGGTTCGCTACCAGTGTAAGAACTACTACAAACTGCG
CACAGAAGGAGATGGTAAGATGTGGACAACTGTCTCCATGCCCTACATACAACCCCCTTCTCTGACATTT
CCATGATGGGTGGTGCTGAGGTGATTCGCCAGAAAGTTCGTTGCTCTCCTTGGAGCCAGGAGATTTAGAT
TCTAATAAGCGTTTTGTCGCCAGTAGCCATGGCCCTTTGGGCAGACTAACTTTTGTCAGCCTCAAGTTTT
CTGTTTTGTTAAGGGGAGGCGATGCCATGCAGCCTACCTCATGTAAATCTCAGAGTCAGATTTACATCTC
CAGCAGATGTGGGAAAAGAAGGAATGCTGATGATGATGTCACCCTCACCTAGTGAGTCTTGCTGTCCTGG
CACTGCTCTAAGGGCTTTATACTTATTTGCTCACTTAGTCCTCACAGTATCCCTCTGAACAGAGTTTATT
GTTTTCACTTTGCTGATAAGGAAACTGAGGCACAGACAGGTTGAGTATCTTGCCCAAATTCAGGCAGCCT
GTAAGAGGCAGAGTCAGGATTTGAACCCTGAGCCCTCCCTGTACTGCTTGGCTGTGACCGCCATGACCAC
AGTGTGTTCTGCTGGGCTTAACTGGTGTCCAGGCACTTGGCTTCCAGCACAGCACTCTTTCCCTTCCTCC
TTCTCATATTCTCTCTCCTTTCTCCCTTCCTGTCTGCCTCCTTTCTTCTTCTTCTTTTTAATTCTTCTCC
TTAAATGCCTTCTCACTCTGCTCTGGGTGCAGACTTGACTTTTCCTTTGGCTCATTTCTTGCCTTTTGTT
TCAGGAGTATACACCTTAAATGATAAGAAGCAGTGGATAAATAAGGCTGTTGGAGATAAACTTCCTGAAT
GTGAAGCAGGTGGGTGCTGAGCACTGAGCACTTAAGAGAGCAGGCAGGCGTCCAGCGGGGAACGTCCTAG
AGGCACAGCCTTCCAGTGCGGCTTCCTCTGAGCACACAAGAGCCAGGAGGAGGGATGTGGGAGAACCGCA
GCTGGCCAGGGAGAGACTTAAGCAGTTAGGTGATGACTCCCTAAGGGTCACCAAGGGTCTTGTTCATTGG
GGCCTGAAGGGCACTGGCTGAATCCACTGTCGGCACTGCCCACAGATCAGGAGAGCCTGTGCATACAGAG
AGCCTGCTAGAAAGCCCTGGGTCTAAGGAGAAGCAAGCTCCAGGGAGAACAAGTCAAGGAATGACATAAA
ATCTTAATCCATGGAAGCCTAGCAGGAGGCTGGACATGGGCTGGAACTCCTGCTTCTCGTTATTAGGAGG
AGCTGTTGCTCTCTCCTTTCATTCTCAGAACCAGAGGCAAAGACCCAGCCTCTTCTGCTCTTACTGGTGT
GGAAATGCCAACCTGCCTCGTATTAACTGCACCATCTACAAAATCTGAGCTCCAGCCAGTGCTGCTCTAG
ATTCATCTTTCTTTAGAGAGAATGAATTATTGTAGCCCCTAGCCCTTTCAATGAATTTCAGGGAATTGTG
GAAATTCCTTTATTGGGATAATTGTTTAAATATAATACAGTTCGCGAGCTTCTATTCGGGGTGGAAGGAG
ATTGATGTGCAGAGCAGCTCCCGCTCATCTGACTTTTCACGGTTCACTGGGAACAATTTCCAAATAGCAA
ACTCTCTGGCTTCTCTCTCTTTGCAGATGACGGCTGCCCGAAGCCCCCCGAGATTGCACATGGCTATGTG
GAGCACTCGGTTCGCTACCAGTGTAAGAACTACTACAAACTGCGCACAGAAGGAGATGGTAAGATGTGGA
CAACTGTCTCCATGCCCTACATACAACCCCCTTCTCTGACATTTCCATGATGGGTGGTGCTGAGGTGATT
CGCCAGAAAGTTCGTTGCTCTCCTTGGAGCCAGGAGATTTAGATTCTAATAAGCGTTTTGTCGCCAGTAG
CCATGGCCCTTTGGGCAGACTAACTTTTGTCAGCCTCAAGTTTTCTGTTTTGTTAAGGGGAGGCGATGCC
ATGCAGCCTACCTCATGTAAATCTCAGAGTCAGATTTACATCTCCAGCAGATGTGGGAAAAGAAGGAATG
CTGATGATGATGTCACCCTCACCTAGTGAGTCTTGCTGTCCTGGCACTGCTCTAAGGGCTTTATACTTAT
TTGCTCACTTAGTCCTCACAGTATCCCTCTGAACAGAGTTTATTGTTTTCACTTTGCTGATAAGGAAACT
GAGGCACAGACAGGTTGAGTATCTTGCCCAAATTCAGGCAGCCTGTAAGAGGCAGAGTCAGGATTTGAAC
CCTGAGCCCTCCCTGTACTGCTTGGCTGTGACCGCCATGACCACAGTGTGTTCTGCTGGGCTTAACTGGC
ATCCAGGCACTTGGCTTCCAGCACAGCACTCTTTCCCTTCCTCCTTCTCATATACTCTCTCCTTTTCCCC
TTCCTTTTTGTCCCCTTTTCCTCTTCCTTTTAGTTCTTCTCTTTAAATGCCTTCTCACTCTGCACGGGGT
CTAGACTTGACTTCTCCTTTGGCTCACTTCTTGCCTTTTGTTTCAGGAGTGTACACCTTAAACAATGAGA
AGCAGTGGATAAATAAGGCTGTTGGAGATAAACTTCCTGAATGTGAAGCAGGTGGGTGCTGAGCACTTAA
GAGAGCAGGCAGGCGTCCAGCGGGGAACGTCCTAGAGGCACAGCCTTCCAGTGCGGCTTCCTCTGAGCAC
ACAAGAGCCAGGAGGAGGGATGTGGGAGAACCGCAGCTGGCCAGGGAGAGACTTAAGCAGTTAGGTGATG
ACTCCCTAAGGGTCACCAAGGGTCTTGTTCATTAGGGCCTGAAGGGCACTGGCTGAATCCATTGTCTACA
TCGCCCACAGATTAGGAGAGCCTGTGCATACAGAGAGCCTGCTAGAGAGCCCTGGGTCTAAGGAGAAGCA
AGCTCCAGGGAGAACAAGTCAAGGAATGACATAAAATCTTAATCCATGGAAGCCTAGCAGGAGGCTGGAC
ATGGGCTGGAACTCCTGCTTCTCGTTATTAGGAGGAGCTGTTGCTCTCTCCTTTCATTCTCAGAACAAGA
GGCAAAGGCCCAGCCTCTTCTGCTCTTACTGGTGTGGAAATGCCAACCTGCCTCGTATTAACTGCACCAT
CTACAAAATCTGAGCTCCAGCCAGTGCTGCTCTAGATTCATCTTTCTTTAGAGAGAATGAATTATTGTAG
CCCCTAGCCCTTTCAATGAATTTCAGGGAATTGTGGAAATTCCTTTATTGGGATAATTGTTTAAATATAA
TACAGTTCACCAGCCAGGGCTCAAAAATCTCAGTATTTCCCACTTCCTTTGTTAGAAAAGTGGGAAATAG
AGCTTTTTGTAATGTAAACAATTTAAAAAACAGAATTATTTTAAAACTGCAACTATTGGAAATGAGATCA
GCAGGTGGTAAGGGCAAAGCATTTAAATCTTTCTACTTTACGCAGCAGTGACAGCCGCCCATGCTTTCAC
CCCTTTCTCAGATGGAAAGGCTCTTGCACATTTCCACTCACGAGTGTCTTGCTCTCCTTGACAGTATGTG
GGAAGCCCAAGAATCCGGCAAACCCAGTGCAGCGGATCCTGGGTGGACACCTGGATGCCAAAGGCAGCTT
TCCCTGGCAGGCTAAGATGGTTTCCCACCATAATCTCACCACAGGTGCCACGCTGATCAATGAACAATGG
CTGCTGACCACGGCTAAAAATCTCTTCCTGAACCATTCAGAAAATGCAACAGCGAAAGACATTGCCCCTA

CTTTAACACTCTATGTGGGGAAAAAGCAGCTTGTAGAGATTGAGAAGGTTGTTCTACACCCTAACTACTC
CCAGGTAGATATTGGGCTCATCAAACTCAAACAGAAGGTGTCTGTTAATGAGAGAGTGATGCCCATCTGC
CTACCTTCAAAGGATTATGCAGAAGTAGGGCGTGTGGGTTATGTTTCTGGCTGGGGGCGAAATGCCAATT
TTAAATTTACTGACCATCTGAAGTATGTCATGCTGCCTGTGGCTGACCAAGACCAATGCATAAGGCATTA
TGAAGGCAGCACAGTCCCCGAAAAGAAGACACCGAAGAGCCCTGTAGGGGTGCAGCCCATACTGAATGAA
CACACCTTCTGTGCTGGCATGTCTAAGTACCAAGAAGACACCTGCTATGGCGATGCGGGCAGTGCCTTTG
CCGTTCACGACCTGGAGGAGGACACCTGGTATGCGACTGGGATCTTAAGCTTTGATAAGAGCTGTGCTGT
GGCTGAGTATGGTGTGTATGTGAAGGTGACTTCCATCCAGGACTGGGTTCAGAAGACCATAGCTGAGAAC
TAATGCAAGGCTGGCCGGAAGCCCTTGCCTGAAAGCAAGATTTCAGCCTGGAAGAGGGCAAAGTGGACGG
GAGTGGACAGGAGTGGATGCGATAAGATGTGGTTTGAAGCTGATGGGTGCCAGCCCTGCATTGCTGAGTC
AATCAATAAAGAGCTTTCTTTTGACCCATTTCTGTGTTGTGTTCAGTCTTGAGTCTTTTTTATTTGCTCC
TTTATGGTCCAGGGTAGTCAGAAGGTATAGAGTCTACTGGGAGTATGGCAGAAAACACCCTAAACCCACT
GGAAATCCCGAAGGTGATACAAACTCTTCCACCTTAGGGAATCATGCTCACTGATTGAGTGCCTATTGAA
TGCTAGGTCCCAGAAAGTTAACTGTTGTCCTTGTTTTACAGACAAGGAAACAGAGACTCAGAGATGGTAA
GTGAGTTGCTTAAGGTTACATAGCTATGAAACAGGGAAGCAGAACTTTGAACCCAGGTCTGTTTGATACA
AACTCAGAGGTCCTTTCACTGCATGCTGTTGCCTCCTCAAAGTGAATTAGGAGAAAAGGCATGGGCCTGG
TTGAGGAAGAGGCTAGCTCAAAATGGGATGGGGAAAAAGTGTTTTAACACAGACAGTAATTCAGAGTTTG
GGATCTAACCTACCAGCACTCCAGAAAACAAAAGACAATGATGTAAAGGCAGGATCTCTGGACTTACTGA
GTCCAAATCACAGAATGGCCCATAAATTTGCTAGGTAATTTAGGTTTCCTGGCCATCAGTTCCTGGTCTC
TTCCATGAAGGACTTGAGCCAGGCGATGGCTAATGTCCCTATCAGCAATAACCTTCTGGTATTCCATAAA
GAGTGGGGAGGTAGAGTCCCTGCATCAGTAGAAGCTGCTTTTCCTACTGGGAACAAACCCACCCTGCACC
AGGAATCAAATGCAGGCTGTCTGAGCCTCCTCTCCTGATGGCAGGTCATGCTATTAAGGGTCTCCCAGAA
GAAAGGTTCTGGAACTCTGAGAAACAGAAGCTCTTCTAACAGAACTTTTTCTTGCTATAAGCTTTCAATA
CCACCAATCACCAGTGCACCTTCTTCTCATGCCAAAATACGGCCCCTCCTTTGTAGGGTGTAAAACGACA
CTGATGTCACTCTCTCTCACCAAGGTCTGGCCATCTCTGTGAAGCTCTCCTCACAGGCACCCTGCAGATT
CATCAATAAACTTCAACAGGTTAGCGCCAGTAGGCCAATAAGATGCACACTTATATTCTAGAACCTGTGA
AAGAAGTAATAGCCAGAAACTGGAAAATAACAGTCAAACTCTTAAAAAAATGCCTCTCAGAACACAGTCA
CTTACACCTTTTCCTCTGGTAGTTCTCTTCAGCACCAAGTTTCCTGGAAGAACAAGCTACTCCCCAGTTT
TCTTAAAGAATTGTCTGATTGTATTAAGTAAGTGGCTTCTGGTAAAATAATGGTAAGAGAAAAGCTCTGG
CATTCTCTTGATCACTCATCTTCTCTGCCTTCCTCCCTAGCAACATCTCCTGGCCTCAGCCTGTGGGCTT
CAGCAACACCAAACTCCATGTTGCTCTCTGACAAGCCCGGCCGTCTCCAGTTCTTGGGCTTTATTTCCTC
GGGGGCCCTCTGCTGAAATTCCCTTCCCCTTGCTGTATTAGCACCTCCTACCCGCCTTCAACATGCAGGC
ACTGCCTCCTCTAAGGAGCCATCTGTACTTACGTAGCTGTGAGCATAGGATGGGGCATACAGCAGGCACT
TAACAAATACTTGTGGAATGGAAAGAAACTAAGATAGGATCAAAAATACAAATCAAGATGTGAGAGAAAG
ACGATCAAAAGCCCTCACGAGGGGGTCGGGCGTGATGGCTCACACCTGTAATCTCAACACTTTGGGAGGC
TAAGGCGGGTGGATCACTTGAGGGTGGGAGTTTGAGATCAGCCAGAGCAACATGGAGAAACCTCGTCTCT
ACTAAAAATACAAAAAATTAGCCAGGCATGGTGATATATGCCTGTAATCCCAGCTACTCGGGAGGCTGAG
GCAGGAGAATTGCTTGAACCTCGGAGGTAGAAGTTGTGGTGAGCTGAGATCATGCCATTGTACTCCAGCC
TGGGCAACAGGAGTGAAACTCTGTCTCAAAAAAAACAAAAAACAAAAAAGAACACCACGGGAAAATCAAG
TGTGAAGCAAGAGCTCAACTCTTAACAGGGGCAATGTTTGGAGTTTTGTTACTGGAAAAGATAGAGACCT
TACCAGGTCCAAAGTTTGTAGACACAGGAATTACGAAATGGAGAAGGGGGAGAAGTGAGCTAGTGGCAGC
ATAAAAAGACCAGCAGATGCCCCACAGCACTGCTCTTCCAGAGGCAAGACCAACCAAGATGAGGTGGGTC
CACAGCTTTCCCTCCTGCCTTTCCTCTGGTTCTTTATTTCAGTCTTTTTTGCATACATTGGTACAGATGC
AGAAATAGAACAAAGAAACAGGGCAAATGGGCTAAATTATAGTGAACCAAAGGGCTTAGTGTGTTAAATC
TTCTCCTTTTCTGCATCCATAGAAGACAGTGCTGCTGTCTTTCCCAGGAGATAAGATTTACTCTCAGGAG
TGTCTTTTTCCTTGAGGTTACGTTTTTGTCTTTGTAGGGTATGTCATCAGCTCCCGTGGTAGGCTTCCTG
GCATTCGGAATATATTTACTAGCAGATATTTTCCTCTTTAAAAATGTATAATAAGAAAGACTAATAGTAA
CACATTTGAATGACACAATTAATTGACTAGTACTTGGGATACACACTAGTACCTGGGATACATCTAATTA
AGACACTTAGATCTTAAAAAATAAAAAGACTTTTTGAAATGTTGAAATAATAAGACTAGAAACTTTTTTT
TTTGTGAGACAGAGTCTCACTCTGTCACCAGGATGGAGTGAGTGGCGCGATCTCAGCTCATCGCAACCTC
CCCCTCCCCGGTTCAAGCAATTCTCCTGCCTCAGCCTCCCAAGTAGCTAGGACTACAGGCGTGTGCCACC
ACGCCCAGCTAATTTTTGTATTTTTAGTAGCGATGTGGTTCACCATGTTGGCCCGGATGATCTCGATCTC
TTGACCTCGTGATCCACCTGTCTCGGCCTCCCAAAGTGTTGGGATTACAGGTGTGAGCCACCGCACCCTG
CCTAGAAACTATTTAATAGAAGCAAATAGTGCCTGAATGGTGGGCGTCTGTTAGCGAGATGGTGAACTG
GCAGATGGCGCCTGTGTGTCAATGCCCATGGCCACCGTCCTGCTTTCAGACACCAGTTCTCTTCCAGGTA
ACCTTCTGGCATTTTGGGGTTTGAGATACCATTTCCTAAAGGTGAATTATCACAAAATACTAGAAATAAC
CACATAAGTGTTTAAAATTATTGTTAAATACAGTAAGAAATTCTTCTTCAAAAGTTTAGCCTGCTTAAGT
TTCCTTGTCCTTTGTTTCCTGCTTTCAAGGCCAGACTTCCTTACTCTCTGTGTTTCCCCTACCCTGGTAA
ACAACCTTCCTGCCAGTCCTTACCCATACAGCCCACATTCCACATCTGCTACCCACTCTGTGATTTACCT
CTCCCGTCGCAATAGCCTCTCCCACCAAAACTGATCTTCCCGCCTTCCCACCAGTGCAACCACATTCCTG

CACTTTGCAAGTTAGCCAACCGGGTTCGGATTGTGCAGTCCAACTCCAGCCAATGGAGTCAGGACACAGT
AGCAGGGACAAGCTGCGTTAGACATAAAAACCTCTGCTTTCCTTTGTTTAGGGTGCTCTCGTGGCAACCA
GACTTACCAGGAGCTCTATTCTGCAAAAGTAAATTTGCCTTGCTGAGAGACCCTTTGTCCTTTGGCTCAG
TGTTGGTTCTTCTTTGCAGCACCGAGCATTTGTTTCCAACAAATTTGGTGGCCCATACAGGGAAAACATT
GTCCTCCGGGAAAGGGTTCTTTGATCATCCTCTTGAGAGGAGAACACATCCCACTGTCCTTGTTGCGGTG
GCCTCATGAGTAGGAATCGAGACCCACCTGTCTGATGAATAACCCCAGACTCTCAACAACGTGGGGAGAA
AAAGACTTGCAACACTATGGTGGCCAGGTAACTCTGTGCGCAGACCAAGGTAAGAAATGTCGCAGGAGTG
ACAAAGTACTTCCTTGGTGGTCACTATATTCTGGTGGCTGAAAGTTCATGAATGGTAACAAGTGCTACTG
CTGTGTGGAGTGAATGAGTCCAATCTGTGGGTCTATGGTTACCTCATACGGCTTAGCCTTCTCTGAAGGA
TCCTGATGTTGGGGTTTATATAGTCCTCCCAATGCTAAGCGGGACTTAAAATATTCCTAGGAGGAAAGTG
GCCAGAGTGGATGAAGCAAAAGGAGAAGAGTGTGAAGAACCTCCAGGAGGTGGGGCTAAAAGATAGGCAA
GAAATCTCTAATACGAGGGATTGAGCCACAGAAAGCTCCAGACAGATAAAAAAGAAATTCCTAATATGAG
GAACTGAGCCACAGCAAGCCTCCAGCAGGCAACAAATCCCTAATATGAGGGATTGAGCCTAGCTAAGACC
CAATATGGGAAATACCCCAAGAAAGACAGAGAATAAGAAGGATGAAAATAGTAACAAGGATATACCCCTT
GATAGTCCCCTAGGTTTCATGTTAAAATACTGGAAAGATAATGAGAGGACTAAGCATAAGAAGAAGGAGC
AAATGATAAAATATTGCTGTTTCATTTGGACCCAAGGTCCCATCCTCAAACCCTCAATCTTCTGGCCAAA
GTATGGGTCGAATGAGGGTGTAATGAGTCAACTCCTAATCCAATATGTTAATGATAAAAGTCTGGTTTCT
CAAGAAGAACTAGACTATGCTCTTTGTTGGAAGCAGGGACCTGTCCTCCTCTTTCCCTTAAAGACAACTA
GGGAAGAATCCGATCCAGCATCTCAAATTGAGAAGTCAGACAAGCCGACTCCCACACCTAAAGTCAGCAC
ATGGGATCCCCTAGACTGTCTTCCTTTGCTTACTGCCCCAATCCTAACCCCCCTACTCCTCAGGCAGCTG
CTGCTGCCCCAGATCCCATCCCAGATTCTTCCCCAACTCATGATGTTCCTCCCGCTTACAACCCTGACTC
TCAGGGGCAGTCGTCCCAAGAGCCTGTTCATTGCCAACCTAAATATCCTTCCTTAAAAGGGCTCCAACAT
GAAATAGAGCAGTGTAAAAAGGACATTCAGAACTCCCCTTTTCTTTCCACACCTAAGGAGTCAGCCATAA
CTTTCTTCCCTTTAAAAGGGGTATCACAAGGAGGGGAAGTCATTGGTTTTGTAAATGCTCTCTTGACTAT
TTCAGAAGTCTGAGGTCTGAAGAAAGAACTTAAGCCACTGCTAGATGACCCCTATGGAGTGGCAGATCAA
GTTGATCAATTCTTAGGGACCTCAGTTATACACTTGGGTCGAGCAAATGTCTATCCTAGGCTCTTCTTTT
CAGGGGAGGAAAGAAAGCATGATCCGTAGGGCTGCTATGGCAATTTGGGAACATGAACACCCTCCTGGTC
AAAACGTTCCTACTGTGGACCAAACATTTGCTGCCCAAGCAAGACTCCTGGTGGGACAACAGCAATGCAG
CCCACTGAGAAAACATGCAGGACCTAAGGGAAATAATAATAAAGGAATCAGTGAATCCATACCCAGAAC
TCAAAAGCTCTCTAAAGCATTTGATATACAACAGGAGAAAGATGAGGGACCTATAAGATTCCTAGACAGA
CTAAAGGAGCAAATGAAGCAATATACAGGTCTGAATTTAGAAGATCCCCTTGGGCAAAGGATGTTAAAGA
TCCATTTTGTCACTAAAGGCTGGCCAGATGTTTCAAAAAAGTTACAAAAATTGGAGGACTGAGAAAACCG
ACCTCTAAGAGAACTTCTCAGAGAAGCTCAAAAGGTGTATGTGAGGAGGGACAGGGAAAAACAAAAACAG
AAGTCAAAACCAATGTTATCTACTTTCCAGCAGGTGGCTCCAAACCCATATGCTACTAAATGAGGCTTCC
AGGGAGCCAGAAACTATAAAAGGTCCCAAGCCTCCCAAACCCAGTTTAGAGAAACCAAACCTTCAGCTAG
AGGACCCAAGTCTACATTTCCCAGGCCCCCTAAAGAGCATAGAAAAGCAAGACCAAAAAATCCCAAAACT
GAGAGAGGGGAAGGACAAGATAAGTGTTACAAATGTGGAAGGACAGCCCCCTTCAAAAGAGAATGTCCCA
AATTAGAAAAGGAGAGAGAAGCCCTTCCACTCACGACCTTTGAAGAGGAACAGGGAAGTCAGGGGCTCTG
TCTATATTATCTTGAGTCCCACCAGGAGCCCTTGATAAATTTGGAGGTGGGACCTACACATGAGCTTATC
ACATTTTTGGTTGATTCAGGAGCGGCCTGTTCCTCTGTTTGTTTCCCCTCATCTAATGTTGCCTGCTCTT
CAGAAGAACTTATAGTCTCTGAGATAAAAGGGGAAGGATTTACGGTGAGAATCTTAGAAAATACAGAAGT
CAAGTACCAAGACTAAACAACCCAGGTTCAATTTTTGTTAATCCCTGAAGCAGCAACTAGTTTGTTAGGA
AGAGACTTAATGTTAAAGTTAGGCATAGGCCTACAAGTCAGCCCAAAGGGATTCCTTACTTCATTAAACT
TACTCACCACGGCGGATGAGAAATACATTCATCCTGATGTTTGGTCAAGGGAAGAAAACTGAGGAAAGCT
TCGAATTCTCCCAATCCACATCAAGCTAAACACCCCGCACTGGGAAGTAGTGAGGAGGAAGCAATTCCCC
ATTCCCTTAGAGGGCATGCTAGGGCTAAAACCTATAATTGAAAGTCTCATTAATGATGGGCTTCTTGAAC
CCTGTATGTCTCCTTATAACACCCCAATACTGCCTGTCAAAAAATCAGATGGGTCATACCGGCTGGTGAA
AGACCTCAGAGCCATTAACCAAACAGTCCAGACCACTAACCCTGTTGTCCCCAACCCTTACACCATTCTC
AGCAAAATTCCATATAATCATCAATGGTTTACTGTAATAGATTTAAAGGATGCTTTTTGGGCATGTCCCT
GGCTGAAGAGAGCCGAGACACATTTGCCTTTGAGTGGGAAGATCCCCAGTTAGGGTGAAAACAATGGTAT
CAATGGACAGTCTTGCCTCAGGGGTTCATGGATTCACCCAACCTTTTTGGTCAAATTTTAGAACAAGTGC
TAGACAAAGTTTCTGTTCCAAAACAATTATGCCTGCTTCAATATGTCGATGATATTCTCATATCTGGTGA
GGATATAGAGAAAGAAGCTGGCTTCTCTACACATATTTTTGACCATCTACAGTTCGAGGGGTTACGGGTC
TCAAAGGGAAAGCTTCAGTGTATGGAACCTAAAGTTAAATATTTAGGCCGCTTAATAAGTGCGGGCAAGC
GAAGGATAGGGCCTGAATGGGTCGAAGGAATCGTGTCCTTACCCTTGCCTCAGACTAAACAGGAACTCAG
GAAATTTTTAGGGTTAGTTGGATACTGCCGCTTAAGGATTAACTCACATGCCCTAAACAGTAAACTTTTA
TACCAAAAACTTGCCCAGGGAAAACCTGAGCATCTCCTGTGGACTTCTAAAGAGGTCGATCAGGTCAAAG
AGCTAAAAGGAATAGCTTATAACTGCTCTTGCCCTAGCCTTACCTTCCCTAGAAAATACACTTCACCTTT
TCGTCAGCGTGAAAAATGGGGTGGCTTTAGGGGTGCTTATCCAAGAGCACAGAGGCTGCTGGCAGCCCAT
GGCCTTCCTGTCAAAAATTTTGGACCTGGTCACCTGTGGATGGCCTCAGTGCATCCAATCCATTGCAGCT

ACAGCAGTATTAGTTGAAGAGAGTAGAAAATTAACCTTTGGGTGGAGATTAACAGTAAGCACACCCCACC
AAGTTAGAGAGCTATTTTAAATAAAAAAGCAGGAAGGCGACTAACTGACTCCAGAATCTTAAAATATGGG
GCTATTCTACTAAAAAAAGATGAGAGAACACCTATGTCTAGATTTAATTGACTACCAAACAAAACTCAGG
CCAGATCTAGGAAAGATCCCTTTCAAAACAGGACGGCACTTATTTATAGATGGTTCCTCCCAGCTGATTG
AGGGAAAAAGACACAACGGGTATCCAGTAATCGATGGAGAAATTCTCTATGTATAAGAACAGAGTCAAGA
AAATTGCCTAATAATTGGTCTGCCCAAACTTGTGAACTGTTTGCACTCAGCCAAGCCTTAAAGCACTTGC
AAAACCAGGAAGGAACCATCTATACTGATTCTAAGTATGCCTTTGGAGTGGCTCATACATTTGGAAAAAG
TTGGACTGAATGTGGCCTCACTAATAGTAAAGGTCATGACCTTGTTCATAAGGAGTTAATCATCCAAGTA
CTGGATAACCTTCAGTTGCCAGAAGAAATAGCTATTGTCCGTGTACCCGGCACCAGAAAAGCCTTTCTT
TTGAAAGTCGAGGAAATAACCTAACAAACCAGATAGCCAAACAAGCTGCTGTTTCCTCCGAAACACCTAT
GTTTCACTTAACTCCTTGTCTTCCTTCCCCTACTGCAATTTCCTTTTTCTCTTCCATTGAAAAAGAAGAA
TAAAGATAGGAGCCAAAGGAGAAGACCAGAATGAAAACGGCTGTTACTAGACCAAAAGGAAATGTTATCC
AAGCCCGTTATGTGGAAGATCTAGTCTCAACTATATCTGAGGACACACTGGGGATCCCAAGCCATGTGCG
ATGCAGTTCTCAGGGTCTATGGATGTATAAGAATTTACACCCTAGCCAAACAAGTTACAGATAGTTGCTT
AATATGTAAAAAGACTAGTAAGCAGATTCTAAGAAAACCGCCCCTTGGAGAAAGAGATTCAGGGCTAAGA
CCATTTCCAAGTGTTCAATTAATTATACTGAAATGCCCCCAATTGGTCATTTAAAATACTTATTAGTAAT
AATAGACCACTTTACCTACTGGGTAGAGACTATCCCACACTCAAATGCAACCACCAGTAACGTAGTTAAG
GCATTAATTGAAAACATTGTACCCAGATTTGGACTAATACAAAGCACTGATTCAGACAATGGAACCCATT
TCACTGCATATGTCATTAAAAAGTTAGCCCAGGTACTAGACATAAAATGGAAAAACCATATCCCTTGGCA
TCTCTCCTCCTCAAGAAGAGTAGAAAGGATGAATCAGACTCTAAAAAGCCACTTAACTAAATTAGTTCTA
GAAACTTGATTGCCATGGACTAAATGTCTTCCTATTGCCTTGTTAAGAATCCGAACTGCTCCTCAGAGAG
ATACTGGCCTTTCCCCTTATGAGATGCTCTATGGATTGCCCTATTTATACTCCACTGCTAACATTCCTAC
ATTCAAAATAAAAGATCAGTTCCTCAAAAATTATATACTTGGTCCATACTCTACTTTCTCTTCCCTTAAG
ACTAAAGGTCTCCTAGCACAGGCGCCACCGCTGGAGTTTCCAGCACATTAGCGTCAGCCTGGAGACCATG
TCCTCATAAAAGGGTGGAAGGAAGGCAAACTCAAACCAGCTTGGGAAGGACCCTACTTGGTGCTCCTAAA
TACTAAGACTGCAGTCCGAACAGCAGAATGAGGATGGACTGATCACACCCGCGTCAAAAAGGCGCTGCCA
CCTCCAGGATCATAAACCGTCACTCCAGGGCTCACCCCAACCAAATTAACTCTAAAAAGGGCTTAATAAT
CACTTGTTTATTTTTTCTTTTCTTTCCAACAGAAGGTCATCTTGTCATCAATGTAACTTGGGCTAACCAT
CCTTTAATCCTTCAGTTTGATGCTTGTTCAGTCATCCTGTGTGGAGACAAGCAAGCTCAAAGGAAGCTGT
CTCATGTAGATAAGTACCTATGTCCATACCATAAAAAGTCAACCAAGTATAAGTATAGAACCTTAAAAAG
TCCCTGTGGTGACTGGACAGATGTTTGGAAACCACTCAGTATGGAGGGTGGACAGCCAGGCCCCCTTTTC
AAATAAGTTATGGGGACTAAAACAGAAACTCCAACTAATTCATGGTCCCACCCCACCAAACTGTAAGCCA
CTGCAGTGTAACCCCTCATTGCTAATTATAGCTAACCCCCAAACAATGGCCCAAGAACCCACTATATTCA
AACAGTATGGATTAGGAGCAAATGTTACAAAACAAAATCCCATAAAAATCTTCAACCACTGGTTAACAAT
GATAATTAAAAAAAAATCCACACCCAGAGTCTGGGAAACGAGTGGAATCTAACACTCTCTCCAAGTATAG
CGAGCTCAGCGTCCTCCTCCCATCTCCAAAACAACCCAACTAAAGTAACGGTTGTAAAGGTAAAAAATTT
AAAGCAGACTATAGCCCAACTAAAGTAACGGTTGTAAAGGTAAAAAATTTAAAGCAGACTATAGCCCTAG
AGACAGGATATCAAGATGCAAAGTCTTGGCTAAAATGGATTAAATATTCCATCTGCACTTTAAACAAATA
TGAATGTTATGCTTGTGCTCACGGTAGACCAGAGGCCCAGATTGTCCCCTTTCCACTCAGATGGTCTTCT
AACTGACCGGGCATAAGCTGTATGGTAGTTCTCTTCCAAGATCACACAGCCTGGGGTGACAAATCATGTC
AAGCTCTTTCTCTGCTGTTCCCTGAAGCTCAACACCCTGAGGGTCAGCCCCCGAGGGCCATCAGCTTCCG
TTTCCCAATGCCAATTTTACTTTGTGTCTCTCACAACAGGGAGAAAACTTGGTGTTCCTTGGAGCCTTAA
TGGGATGCAGTGAGCTTAAGTCCTTCCAAGAGCTTACCCATCAGTCTGCCCTTAGTCATCCTCGAGCGGA
TGTATGGTGGTATTGTGGTGGACCATTACTGGACACTGCCGAGTAACTGGAGCAGAACATGCACTCTAAT
TCCATTGGCTATCCCTTTCACCCTGGCATTTCATCAACCAGAAAAGATAGAAACCAAACACTGTAAAACA
AGAGAGGCCCCTCATGGGTCTGTTGACTCCCACATTTATATAGATTCCACTGGAGTCCAACGAGGAGTGC
CAGATGAATTTAAGGCCCAAAATCAGACAACTGCAGGATTTGAATCCACGTTCTTCTGGTGGTTAACTAT
AAATAAAAATGTGAATTAGATAAACTACATTTATTACAACCAATGATGATTTTTTTTTTTTGACGGAGT
TTTGCTCTTGTTGCCCAGGCTGGAATGCAACGGCATGATCTCGGCTCACCACAACTTCTGCCTCCTGGGT
CCAAGCAATTCTTCTGCCTCGGCCTCCCGAGTAGCTGGGATTATAAACATGTGCCACCACGCCTGGCTAA
TTTTGTATTTTTAGTAGAGACAGGGTTTCTCCATGATGGTCAGGCTGGTCTCAAACTCCCGACCTCAGGT
TATCTGCCTGCCTCGGCCTCCCAAAGTGCTGGGATTACAGGCATCAGCCACTGTGCCCAGCCTATGATTT
GTTAATTATACTAGAGATGCTCTTAAAGGGACAGCTGAACAATTAAGACCCACCAGTCAAATGGCCTGGG
AAAATAAAATAGCATTAGACATGATACCAGCGAAAGAAGGTGGAGATTGTGTCATTATCGGAACCCGATG
CTATACTTTTATCCCTAATAATACTACTCCAGGTGGGATCACACCAAAAGCACTACAAGGTCTTACCGCC
TTATCAAATGAGCTAGCCAAAAATTCTGGACTAAACGACCCCTTCACAAATTTAATGGGAAATTCTTTGG
CAAATGGAAGGGATTTATGTCCTCAATCCTCATGTCTCTTGCCATCAGAATAGGGTTGCTTATTCTTGTA
GGATGCTGTGTCATACCCTGTGCCCTAGGATTAATACAAAAGCTCATTGAAACAGCTCTCCCCAAAACCT
CCCTCAATCCTCCTCCTCCTCGTTCCAGTGAGCTTTTTCTTTTAGAAGACCCAAGTAGAATAACAGAGCC
AAATTATGTTAAAAGGGTTTAAGAAAAAATACTGTAAAATATTTAACAGGGGGATTRTGTTAAATACAGT

AAGAAATTCTTCTTCAAAGGTTCAGCTTGCTTAAGTTTCCTTGTTCTTTGCTCCCTGCTTTCAAGGCCAG
ACTTCCTTACTCTCTGTGTTCTCCCTGCGCTGGTAAACAACCTTCCCGCCAGTCGTTATCTATAGAGCCC
ACATTCCACATCTGCTACCCACTCTGTGAATTACCCCTCCCATCACAATGGCTTCTCCCGGCAAAACTGA
TCTTCCTGCCTTCCCAGCAGTGTAATCACATTCCTGCACTTTTCAAGTTAGCCAACCGGGTTCAGCTTAG
ATTGTACGGTCCAACTCTAGTCAATGGAGTCAGGATACAGTAGCAGGGACAAGCTGCGTTAGACATAAAA
ACCTCTGCTTTCCTTTGTTCGGGGTGCTCTCGTGGCAACCGAACCTATGAGGAGCAACCTTATGCAAAAG
TAAATTTGCCTTGCTGAGAGACCCTTTGTCCTTTGTCTCAGTGCTAGTTCTTCTTTGCAGCACCAAGCAT
TTGTTTTCAACAATTATATATTTAAGGAATCTTTGATTATGGAAAATTTCAAGAAGTAGAGGGAATAGCA
TACTAAGCCCTCTGTTCCCATCTTTGAGTTATCTACATTTTATTAGAAGTATCTTTGGGGTGACTGAGGG
AGGTTTCTCTTTCCTGGAGGGCTTCTGAATTATTGCCAATGTACCTTCCTGACTGCAGCCAGAAACTGAG
GCCCCTATACCTATGTACCTTTCTATCCCTCTCTGAAGCTTCTGCAGAATTCCCAGCAAGACAGAGCTTG
CTGGAAGCTTGGTATGCTCAGAAGCAGCTAAAGCGTGTATGTGGGGCGGAGGGTGGGGGCAACTTCTTGG
TCCTAGCACTTCCATATATTGATTTTCTTTTCTGGCTGCTTAGTGGGAGGAGTGTGTGTGTATGCATGTG
TGTGTGTGCGTGTGTGTGTGTGTGTACATGCCTGTGTGTGTGGATGCATGCATGTGCTGTGAAGCAGGGA
GACCAGCTTTCCGTTCCTTCTTGTTTTCTCTCTGCAGTGACCTGGGAGCTGTCATTTCCCTCCTGCTCTG
GGGACGACAGCTTTTTGCACTGTACTCAGGCAATGATGTCACGGATATTTCAGGTCAGTCTTTGAGTTGG
GTAGGAGCATGCATCCCTGGCACTGCCACATCCCACTCTGACTCTCTCGGGTCTGCATTCTTTCTTTGAG
AACACACAGTTCCCCATTCTTATCCTGACCTCTGGGCTTTCAGGACTGCCAAGAACATTGGGGATCCTGC
CAGAAATGAGGGGAGCTTGAGCTTTCGTTGGCTTCTATTTGGGGTAGAAGGAGATTGATATGCAGAGCAG
CTTCCACTCATCTGACTTTTCATGGGTCTCTGGGAACAATTTCCAAATGGTAAACTCTCTGGCTTCTCTC
TCTTTGCAGATGACCGCTTCCCGAAGCCCCCTGAGATTGCAAATGGCTATGTGGAGCACTTGTTTCGCTA
CCAGTGTAAGAACTACTACAGACTGCGCACAGAAGGAGATGGTAAGACCTGGACAACTATCTCCTGTGCT
CTACCTACAACCCCTGCTCTGACATTTCCATGATGGGTGGTGCTGAGGTGATTTGCCAGAAAGTTCGTTG
CTCTCCTTGGAGCCAGGAGATTTAGATTCTGATAAGCGTTTTGTCGCCAGTAGCCATGGCCCTTTGGGCA
GACTAACTTTTGTCAGCCTCAAGTTTTCTGTTTTGTTAAGGGGAGGTGATGCCATGCAGCCTACCTCATG
TAAATCTCAGAGTCAGATTTACATCTCCAGCAGATGTGGGAAAAGAAGGAATGCTGATGATGATGTCACC
CTCACCTAGTGAGTCTTGCTGTCCTGGCACTGCTCTAAGGGCTTTATATTTATTTGCTCACTTAGTCCTT
ACAGTTTACCTCTGAACAGAGGTTATTATTCTCACTTTGCCGATAAGGAAACAGAGGCACCGACAGGTTG
AGTATCTTGCCCAAATTCAGGCGGCCTGTAAGAGGCAGAGTCAGGATTTGAACCCTGAGCCCTCCCTGTA
CTGCTTGGCTGTGACCGCCATCACCACAGTGTGTTCTGCTGGGCTTAACTGGTGTCCAGGCACTTGGCTT
CCAGCACAGCACTCTTTCCCTTCCTCCTTCTCGTATTCTCTCTCCTTTCTCCCTTCCTGTCTGCCTCCTT
TCTTCTTCTTCTTTTTAATTCTTCTCCTTAAATGCCTTCTCACTCTGCTCTGGGTGCAGACTTGACTTTT
CCTTTGGCTCATTTCTTGCCTTTTGTTTCAGGAGTATACACCTTAAATGATAAGAAGCAGTGGATAAATA
AGGCTGTTGGAGATAAACTTCCTGAATGTGAAGCAGGTGGGTGCTGAGCACTGAGCACTTAAGAGAGCAG
GCAGGCGTCCAGCGGGGAACGTCCTAGAGGCACAGCCTTCCAGTGCGGCTTCCTCTGAGCACACAAGAGC
CAGGAGGAGGGATGTGGGAGAACCGCAGCTGGCCAGGGAGAGACTTAAGCAGTTAGGTGATGACTCCCTA
AGGATCACCAGGGTCTTGTTCATTGGGGCCTGAAGGGCACTGGCTGAATCCACTGTCGGCATTGCCACAG
ATCAGGAGAGCCTGTGCATACAGAGAGCCTGCTAGAGAGCCCTGGGTCTAAGGAGAAGCAAGCTCCAGGG
AGAACAAGTCAAGGGATAACATAAAATCTTAATCCATGGAAGCCTAGCAGGAGGCTGGACATGGGCTGGA
ACTCCTGCTTCTCGTTATTAGGAGGAACTGTTGCTCTCTCCTTTCCATCTCAGAACCAGAGGCAAAGGCC
CAGCCTCTTCTGCTCTTACTGGTGTGGAAAGGCCAGCCTCTTCTGCTGTGAGTGGTGTGGAACTACCAAC
CTGCCTCGTATTAACTGCAACATCTATAAAGTATGAGCTCCAGCCAATGCTGCTCTAGATTCCTCTTTCT
TCAGAGATGATGAATTATTGTAGCTCCTAGCCCTTTCTTTTTTCTTTCTTTCTTTTTTTTTTTTTTTGAG
ACAGAGTTTTGCTCTCGTCGCCTAGGCTGGAGTGCAGTGGTGCTAATTCCTGACCTCAGGTGATCCAACT
GCCTCAGCCTCCCAAAGTGCTGGGATTACAGGCGTGAGCCACCGCATCTGGCCCCTAGCCCTTTCAATGA
ATTTCAGGGAATTGTGAAAATTCCTTTGTTGAGATAATTGTTTAAATATAATATAGTTCACCAGCCAGGC
CTCAAAAAACTCAGTATTTCTCATTTCCTTATTTAGAAATAGAGCTTTTTGTAATGTAAACAATTAAAAA
AATTATTTTAAAACTGCAACTATTGGAAATGAGATCAGCAGGTGGTAAGGACAAAGCATTTAAATCTTTC
CAGTTTATGCAGCAGTGACAGCCGCCAATGCTTTCACCCCTTTCTCAGATGGAAAGGCTCTTGCACATTT
CCACTCACGAGTGTCTTGCTCTCCTTGACAGTATGTGGGAAGCCCAAGAATCCGGCAAACCCAGTGCAGC
GGATCCTGGGTGGACACCTGGATGCCAAAGGCAGCTTTCCCTGGCAGGCTAAGATGGTTTCCCACCATAA
TCTCACCACAGGGGCCACGCTGATCAATGAACAATGGCTGCTGACCACGGCTAAAAATCTCTTCCTGAAC
CATTCAGAAAATGCAACAGCGAAAGACATTGCCCCTACTTTAACACTCTATGTGGGGAAAAAGCAGCTTG
TAGAGATTGAGAAGGTGGTTCTACACCCTAACTACCACCAGGTAGATATTGGGCTCATCAAACTCAAACA
GAAGGTGCTTGTTAATGAGAGAGTGATGCCCATCTGCCTACCTTCAAAGAATTATGCAGAAGTAGGGCGT
GTGGGTTACGTGTCTGGCTGGGGACAAAGTGACAACTTTAAACTTACTGACCATCTGAAGTATGTCATGC
TGCCTGTGGCTGACCAATACGATTGCATAACGCATTATGAAGGCAGCACATGCCCCAAATGGAAGGCACC
GAAGAGCCCTGTAGGGGTGCAGCCCATACTGAACGAACACACCTTCTGTGTCGGCATGTCTAAGTACCAG
GAAGACACCTGCTATGGCGATGCGGGCAGTGCCTTTGCCGTTCACGACCTGGAGGAGGACACCTGGTACG
CGGCTGGGATCCTAAGCTTTGATAAGAGCTGTGCTGTGGCTGAGTATGGTGTGTATGTGAAGGTGACTTC

CATCCAGGACTGGGTTCAGAAGACCATAGCTGAGAACTAATGCAAGGCTGGCCGGAAGCCCTTGCCTGAA
AGCAAGATTTCAGCCTGGAAGAGGGCAAAGTGGACGGGAGTGGACAGGAGTGGATGCGATAAGATGTGGT
TTGAAGCTGATGGGTGCCAGCCCTGCATTGCTGAGTCAATCAATAAAGAGCTTTCTTTTGACCCATTTCT
GTGTTGTGTTCAGTCTTGAGTCTTTTTTATTTGCTCCTTTATGGTCCAGGGTAGTCAGAAGGTATAGAGT
CTACTGGGAGTATGGCAGAAAACACCCTAAACCCACTGGAAATCCCGAAGGTGATACAAACTCTTCCACC
TTAGGGAATCATGCTCACTGATTGAGTGCCTATTGAATGCTAGGTCCCAGAAAGTTAACTGTTGTCCTTG
TTTTACAGACAAGGAAACAGAGACTCAGAGATGGTAAGTGAGTTGCTTAAGGTTACATAGCTATGAAACA
GGGAAGCAGAACTTTGAACCCAGGTCTGTTTGATACAAACTCAGAGGTCCTTTCACTGCATGCTGTTGCC
TCTTTAAAGCGAATTAGGAGAAAAGGCATGGGCCTTGGTGAGGAAGAGGCTAGCTCAAAATGGGATGGGG
AAAAAGTGTTTTAACACAGACAGTACTTCAGAGTTTGGGATCTAACCTACCAGCACTCCAGAAAACAAAA
GATGATGATGTAAAGGCAGGATCTCTGGACTTGCTGAGTCCAAATCATGGACTTGGCCCAAACATTTGCT
AAGTAATTTAGGCTTCCTGGCCATTAGTTCCTGGTCTCTTCCATGAAGGACTTGAGCCAGGGTAACGTCC
CTATCAGCAATAAGCTTCTGGGACTCTGTAAAGAGTGGGGAGGTAGAGTCCCTGCCTCAGTAGAAGCTGC
TTTTCCTACTTGGAACAAACCCACCCTTCACCAGGAATCAAAAGCAAGCTGTCTGAGCCTCCTCTCCTGA
TGGCAGGTCATGCTATTAAGGGTCTCCCAGAAGAAAGCTTCTGGAGCTCTGAGAAACAGAAGCTCTTCTA
ACAGAACTTTTTCTTGCTATAAGCCTTCAATACCACCAATCACCAATGTGCCTTCTTCTCATGCGAAAAT
ACGGCCCCTCCTTTGCAGGGTGTAAAATGACACTGATGTCACTCTCTCTCACCAGGGTCTGGCCATCTCT
GTGAAGCTCTCCTCACAGGCACCCTGCAGATTCATCAATAAACTTCAACAGGTTAGCGCTAGTAGGCCAA
TAAGACCTATATTCTAGAACCTGTGAAAGAGATAATAGCCAGAAACTGGCAAACACCAGTCAAACTCTTT
AAAAAAATGCCTCTTAGAACACAGTCACTTACACATTTTCCTAGATCTCTTCAGCACCAAGTTTCTTGGA
AGAACAAGGCACTCTGCAGTTTTCTTAAAGAATTGTCTGATTGTATTAAGTGGCTTCTGGTAAAACAACA
GTAAGAGAAAAGCTCTGGCATTCTCTTGATCACTCATCTTCTCTGCCTTCCTCCCAGCACCACCTCCTG
GCCTCAGCCTGTGGGCTTCTGCAAAGTCAGGTATCAGGACTAATTAGCTAGCATCAAATTCCTGTTAATT
CCAGAAGCAGGGACAAACCTGTTGGGGAGGGACTTAATGACAAAATTAGGTATAGGTCTATACGTTAATC
AAGGAAAATTCTCAACTTCCTTAAACTTACTTACCACCCTAGAGGAGAGCCAAATCCACTCTGATGACGT
TTGGTCATAGGAAGGAAATTGGGGAAAATTACAAGTTTCTCCAATCCATGTAAAATTAAGAAATCCTGGG
GAAGTTGTAAAGAGAAAACAGTACCCTATTCCCTTGAAAGGCAGAATAGGTTTATAACTTATAATCGAAG
GTCTCCTTCAGGAAGGACTTCTTGAACCCCATATGTCCTCTTACAACACTCCAATATTGCCTGTAAAAAA
GTCGGATGGGTCATAGCGGCTACTGCGAGACCTTCGAGCCATTAACCAAATAGTCCAGACCACCCACCCT
GTCATTCCTAACCCTTACACCATTATCAAGCAAGATCCCTCATGACTGCCAGTGGTTCACAGTAATAGAC
CTCAATGATGACTTCTGGGCTTGCCCCTTAGCTGTGGATAGCTGGGACATATTTGCTTTTGAATGGGAAG
ACCCTCATTCCAGTTGGAAGCAATAGTAGTGATGGACAGTTCTACCCCAAGGGTTTACAGACTCTCCAAA
CCTATTAGTCAAAGTTTGGAACAAGTCCTAGAGAATTTCCCTCTTTCATCATCCTTATGTCTACTCCAAT
ATGTGGATGACCTGCTCATTTCCAGAGGCACCAAAGACCAAGTAACCGCAATTTTAATTAGCTTTCTAAA
TTTCCTAAGGGAACAATGGTTACGGGTCTCAAAAAGTAAACTCCAGCTTGTAGAACCTGAGGTAAAACAC
ATGGGGCACTTAATAAGCAAAGGTAAGCGGAAGATAGGGCCCGAACAAATGGAAGGGATCATATCCCTGC
CACTGCCTGAGACAAAACAAGAACTTAGAAAATTCTTCAGGCTAGCTGGAAATTATTGCCTGTGGATTGA
CTCTTATGCCTTAAAAACAAAACCTCTATATCTAAAACTTACCCAAGAAGGGCCTGACCCCCTTCTTTGG
ACCCCACAAGAAGTCCAGCAAGTTGAGGAACTAAAACATCTACTTATAACTGCCCCTGTCTTAGCTCTGC
CATCCTTAGAACAGCCATTTCACTTTTTTGTTAATATAAGCAATGGGGCAGCTATAGGAGTACTCACTCA
AAAGCATGGGGCCATTGCCAGCCCACAGCCTTTCTGTCAAACATTCTTGACGTGGTAACCTGTGGGTGGC
CCGAATGTATCCAATCTACAGCAGCAACTGTTTTATTGACAGAGGAAAGTAGAAAAATAACCTTTGGGGG
AAGTCTCATCCTAAGCACACCCCATCAGGTTAAAACCATTCTTAGCCAAAAAGCAGGGAGATGGCTTACA
GATTCAAGAATCCTAAAATATGAAGCTATCCTATTAGAAAAAGGTGATTTAACCCTAACCACTGACAATG
TGCTCAACCCCGCCACCTTCCTTTTTTTTTTTTTTTTTTGAGATGGAGTTTCACTCTTATTGCCCATGCT
AGAGTGCAATGGCACAATCTCGGCTCACCGCAACCTTTGCCTCCTGGGTTCAAGCAATTCTCCTGCCTCA
GCCTACCAAGTAGCTGGGATTACAGGCATGCTACCATGCCTGGCTAATTTTTTTGTATTTTTAGTAGAGA
TGGGGTTTCTCCATGTTGGTCAGGCTGGTCTTGAACTCCTGTCCTCAGGTGATCTGCCTGCCTTGGCCTC
CCAAAGTGTTGGGATTACAGGCGTGAGCCACTGTGCCCGGCCTACCCCGCCACCTTCCTGACAGGAAATC
CAAACGCTGGAGACCCCAAGCACAAATGTTTAGACTTAATCATCAAACTAAAGTAAGGCCTGATCTAAGT
GAGACCCCTTTAAAAACAGGGCACCATCTCTTTGTAGACAGCTCCTCTCGGGTAATTGAAGAGAAAAAAC
ATAATGGCTACTCAGTGGTTGATGGAGAAACTCTCACAGAAGTAGAATCAGGGAGACTTCCCAATAACTG
GTCTAACCAAACATGTGAGTTGTTCGCACTAAATCAAGCCTTAAAATAGCTGCAGAATCAAGAAGGGACT
ATTTACACCAATTCCAAATATGCTTTTGGAGTGGCCCATACCTTTGGGAAGATTTGGACTGAACAAGGCC
TTATTACTAGCAAGGGCCAAAACCTTGTCCACAAAGAGTTAATCATGCAAGTATTAGAAAACCTTCAGCT
ACCAGAGGACATAGCCATTGTTCATGTCCCAGGACACCATAAAGGTCTCTCTTTCAAGAGTTACTTCCCA
AGAAGCACCCATTTTCCACTGAACCCCTTGTCTCCCTTCCCCATCTGCAACCCCCATCTTCTCCCAAGCA
GACAAAGAAAAATTAAAGAAAATAGGGGCTAACAAAAACTCAGAAGAGAAACGGATACTGCCAGATAACA
AAGAAATGTTGTCCAAACCCCTAATGACAGAAATCTTAACACAACTTCACCAAGGGACCCATTGGGGTCC
CCAAGCCATGTGTGATGCAGTCCTTAGAACCTATGGGTGTACAGGGATTTATACCCTCACCAGGCAAGTC

ACAGACAGTTGTATGGTATGCAGAAAAACCAATAAACAAACTTTAAGAAAACTGCCCTTCGGCGGAAGAA
ATCCTGGGCTAAGGCATTCCAAAGTGTCCAAATTGACTTATACCGAAATGCCCCAAATAGGCTGCCTCAA
ATGTTTGTCAGTTATAGTGGACCATCTCACTCACTGGGTGGAAGCCATTCACCTGCCAAGTGCAACTGCC
AATAATGTAGTTAAAGTACTAATAGAAGATACCATACCCAGATTCAGATTAATAGAGAACATTGACTCAG
ACAATGGAACCCATTTTACTGCACAGGTCATTAAAGGATTAACCCAGACACTAGAGATAAAATGGGAATA
TCATACTCCCTGGCACCCACCCTCATCAGGAAAAGCAGAAAGAATGAATCAAAATAAATAAATCACCTAA
CTAAACTAATCTTGGAAACCCGACTGCCATGAACTAAGTACCTCCCTGTCGCCTTACTAAGAATTCGTAT
CGCCCCTTGAAAAGATATCGGCCTGTCCCCTTATGAAACGCTCTACAGATTGCCATGCTTAAATCCACCA
CTGACATCCCTACATTTGAAACAAAACATCAATTTCTTAAAAACTATATACGTGGTCTATCTTCCACCCT
TTTTTCCCTCAGGACCCAAGGCCTCTTAGCACAAATGCCGCCTCTAGAGTTTCCAGTACACCCACATCAG
CCTGGGGATTATGTGCTCATCAAAAGCTGGAGGGAGGAAAACTCGAACCATCGTGGGAAGGACCCTATCT
AATACTCCTAACAACTGAAACAGCAGCCCGAACGGCTGAACAGGGGTGGACTCATCACACTTGGGTAAAA
AGGACCCCACCCCTTATGGAATCATGGACCATCACTCCAGGACCAACTCCCTCAAAATTAACATTCAAAA
AGGTCTAATTTGTCTCTTCTTCCCCCTAATGCCCAGGGGCATTTCATTTTTTTTTTTTTTTGAGATGGAG
TCTCGCTCTGTGCCCAGGCTGGAGTGCAGTGGCAAGATCTCCACTCACTGCAAGCTCTGCCTCCTGGGTT
CACACCATTCTCCTGCCTCAGCCTCCCGAGTAGCTGGGACTACAGGTGCCTGCCACCATGCCCGGCTAAT
TTTTTGTATTTTTAGTAGAGACGGGGTTTCACAGTGTTAGCCAGGATGGTTGATCTCCTGACCTTGTGAA
CTGCCCGCCTCGGCCTCCCAAAGTCCTGGGATTACAGGCATGAGTCACCGTGCCCAGCCCGCATTTCATT
GTTAATGTAACCAGGTCAGCCTCCCCTCAAGCTGTTGTGTTTGATGCTTGCCTAGTCATACCTTGCAGAG
ACCTACAAAGTCAAAGGCAACTAGCCTCTTCAGAAAAGTATCTTTGCCCTTGGTACTCACCCCACCCTCA
ATCTGATCCTTGCAAGATTCCCTATAATTGGCTACCTTGCCATTCCTGGTACGATATTCTCTGGACCACT
CAGTCCCAAGGCTGGACTTCTTCAGCAGGCTGCACCTCCCTAAAACCTTATATCCATTTTACTAAACAAA
ATAACTCCTCTAACTGCCAACACCTCCGATGTAACCTGGTGCATATTTCTATTACTACTTCAACCTCCAC
TAACCATATTCCCACCCTAAGTCGCTTTTATGGTATGGGGCAGATGTTAGCGGAAAGACCCCATAGGGT
CCTTTGAGATTCGTTTTGTCGCCCCTCCAGCACCCCTTCCTTTCCGAAGATCTCCTCCAAATGAAACAAC
TATCTTGCCACTACCTAATGACAAAACTAAAGTGCACATAGTGGAAGTCAAAGACCTAAAACAAACCCTG
GCCATCGAGACCGGACACCAAGAAATGAATGCCTGGCTGAAATGGATCAAATATTCAGTTTGCACTTTAA
ATAAAAGCGACTGTTATGCCTGTGCAATGGGCAGGCCAGAAATCCAAATAGTTCCCTTCCTGCTTGGATG
GTCTAACCAGCCGGGCATGGACTGTACAGTAGCTCTCTTCCAGCACCCCACAGCCTGGGGTAATAAGTCA
TGCACCAGTCTCTCACTGCTTTTCCCAAAAGTCAAAGACTTCCCTGTGGGTCAGCCCCCAAGGCCATCTG
GCTCCTGGCCTCTGATGCCAATTTTACCTCTTGTCTCTCATGACGGGGAAAACTTACATCTTTCAGGAAT
CTAACGGGATGCAGTGAATCCGAGCCTTTTCGAGAGCTCACCAATCAGACTGCCCTTGTCTATCCACAAG
CAGATGTTTGGTGGTATTGTGGAGGACCTCTACTGGGTATGCTGCCAAATAATTGGAGCAGCACTTGCAC
TCTAATCCAACTGGCCATCTCTTTCACCCTGGCATTTCATCAGTCCGACAAAAAGTCACCCTCTTGGGAA
GAAAGAGAAGCTCCTCAAGGGTCTTTTGACCCCCATGTCTATATAGAGGCAATTGGAGTACCACGAGTAG
CCGCCAATGAATTTAAAGCTCGAAATCAAATAGCTGCCAGGTTTGAGTCAGTCTTATTTTGGTGGTCTAC
TATAAACAAAAACGTAGACTGGATAAACTATATATATTACAATCAACAAGGATTTGTTAATTATACCAGA
GATACCATTTGGGGATAGCCGAGCAATTTGGTCCCACCAGCCAAATGGCCTGGAAAACTGGATAGCTCT
TGACATGACATTAGCAGAAAACGGCAGAGTTTGTGTAATCATCAGAGCCCAATGTTGTACTTTGATCCCA
AATAACACAGCTCCCGATGGAACCACTACCAAGGCTCTACAAGGCCTCACCACCCTAGCAAATGAGCTGG
CTGAAAATTCTGGAATAGATGACCCCTTCACAGGTTTAACCAAAAGATGGTTTGGTAAATGGAAAGGACT
TATGGCCTCTATCCTCACTTCCCTTGCCATTGTTACAAGTGCACTCATCCTTGTAGGCTGTTGCATCCAC
CTTGTGTTTGTGGGTTAGTTCAAAGACTCATAGAAATGGCTTGCACTAAAACCTTCTCTGTCTCTCCCCC
TCCATATTCAGACCAACTTTTACTCCTAGACAATCAAGAAGAGCAACAAAGCCAAATCTTACTAAAATAA
TTTGAAGAGGAAGAATTATAACAACAAAAAGGGGGAAATTGTCAGAATAAAAGAAAGGTTCCTCTTCAAA
GATCCAACTTTCTTGTCATAGGCTGTAAACCGCCTTGCTCCAAACCTTAACCTGTAACCTTTACCCTCTG
CCTTATTTGAGAAAGATTCAAGCGCATAGCCAATTGGAGTCAGCCTAGACTGTGCGGTCCAACCCAGCCA
ATGGGGAAAGACAAAGAAGCAGGAACTGCGTTAGGGTTAGAAACCTGTTCTCCTTTATTAGGTGTGCTTT
TGCGACTGCTATAGGCGCGGGCAGCACCCTTCTGCAGAAGTAAACTTTGCCTTGCTGAGAAAACTTTTCT
TCTGAGTGCTCCTCTTCCTCTGCGGCACTCAGAAATAAGAGAACTTATTTCTAACAGGAGGAAGAACATG
ATAATTTTGCACACGCAGGTTCTGAGTTTTGGCTGGTGCGCTGAGTGCTTACCATACCGCAAATACTGTG
TGATCTGGGCTGAAGAACAGAAAACCCAACTAACAGTGACTTAGGCAAAAAGAAATTTTATTCTTTCACA
GAAGTCTGTGGCAGGCAGGTCCAGAGTTCATGCAGGAGCTCAATAACATCAGAGATTGGGCTCTTTTTGT
TCTTCTTAGCATATCGATGTTTTACGTGATGCTGGGCACCTCGGGGACCTAAGATGGCTGTTGTAGCTCC
ATGCATCATGATTAAACCAGAGACAGGAAGTGTACGATGGAAGAGGCCAGAGGTGAATGGCCTCCTCCTC
TCCTTTTGTCTTATTCCTGGAGTGACTCCCTCCTCTTATCAGCAGGGATACAAGCAGTGCCTGGCAGCCT
TCCCTCTCTCCTGTATGAGCCAAAAGCAAGACTCATGGCACCCCTCCCTGCAGGAAAGGGGTGGAATGGG
CCCCTGGCAAAGGGGGCTGGACTTGCAGTGACAGTGCTGCTCTGAACAGGACTAGGCTTCTGTTGGAGAG
GTGGTCAGCGGCTGCTAGGCAGGTCACAAGCTGCCTCACCCATGGCTCTCCAGCTCTGCACCTACCCTCT
CCTTCTAATAAATCTGTTTCAATAACCACCACGAGGTTTAAAGAGGAGCAGTAACTTGCCCACGGTTGCG

CCAGGCACCCCTAGAAGAGCAAGAATTCCTTACAAAGTTATTAAAACAGGCAAATCCCATATAGGTGACA
TTTCTATCCAACAATGCTGACATCTCTTTTAGTTTTGAGCCTTAGTAATCCAATTTTCTGCCTCAGGAAG
AGGCAGCAGCTTCTGGGAAAAATCTGCTCCTGCTAAGGTGGCTCATTCATTGTTTACCTGAATAGAGTG
GTGGATGGTGAAGGTTCAAATTCTCACTTTCCATATTAAATTTTATAACATAAAAAATAATTAAATGGGG
TGCGCGCGGTGGCTCACGCCTATAATCCCAGCACCTGGGAGGCTGAGGAGGGCAGATCACGAGGTCAGGA
GATCGAGACCATCTTGGCTAACACGGTGAAACTGCGCCTCTATAAAAATACAAAAAATTAGCTGGGCATG
GTGGCGGGCGCCTGTAGTCCCAGCTACTCAGGAGGCTGAAGCAGGAAAATGGCATGAACCCAGGAGGTGG
AGCTTGCAGTGAGCCGAGATCGCGCCACTGCACTCCAGCCTGGGTGACAAGAGTGACTCGGTCTCAAAAA
AAAAAAAAAAAATTAAATGAGCCAACCAGGACAATGTTTAAAAGCCAGAGCCAAAAAGGCTCCAAAATAA
AACAAACTTGTCTGCTCAATTAATTATTTGAAGAGAACCTACAGTAATACAGACTGGAGATGGGAAAGCA
CGAGGGATGTATGAGTCGAGGAACCGGCAAAGGGGTGTGGGGGTGTGTGTGTGTGTGTGTGCACACAC
ATGCACCCAGCTAGGGTAGAAAATAAGTACTTTTGCTTCTATGGGAAGTGACTTTCCAGTTCACTTGTGT
ATCAAATGAACATGATGAAATCAAGTTGTACTAGCCAATGTTTGGGAGGGTTCTGTATTAAAGGGATAAA
GAAAAACATTAGAAAGAGAAGTTACAAAAATCACTGTGGGGAAAATGAACACCAATGACTTGGCTGCTCT
GAGGCTTGCAGGGAGTAGACAGTTAGGCATCCCAGGTCATCAGAGAACCAGTGGGGTCTTTTCCTCAGGG
GCCGGGTTTTCTACACGCAAGTCAAACCTCTTCTCCTCTGGGACACATGTTTCCAAAGGACTCCAGAAAC
ACAGCACAGCTGGATTCAGGTACTGTGTCAAGATGTGCCTTCTTCTTCATCTTTGACAGCAATTAATGTA
CTAAATGTCTTGATAGAGAAGGTCATATTTGGGAATATTCTTGGGATCAATAGGACTTTGGACAATAAGC
TTCCCCCTCATTGCTCCTCGATAGATTACTTCAATCAAATCTATGAAGTCTTGTTTGGTTTTGAAGCTTC
CCACAAACTTAGTGTGATCTGGAGATCTAGACAGCATAGAAGAGAAACAACTGCTCTAAGAACTTTGAGT
AATCACTTGTTGAAGACTTTCAGGATAAACAAAATGAAAAACAGCTTTGTTATTTCTTCTTGTCAATGA
GACTTCAGAAATAAAGCCCAACATTAATTTTTCAATCATTTTTTTCCATTTAGTAAATGACTCAACCCAG
TGCTGTGGGCCACTAGCATGAAAGCTCTGTCATGTGCAAAGCTTTAGCTTCAAAAGCTATTGTAATCACA
AAATCTATTTTTATCACTTCTCAAATCATCAAGCAGCTTTCACCAAGTGTTGGAGTGCACATTGTTTCTT
CGTTTGCTGTTGTTGCTTTTGCCTGGGCATTGTGCAAAGCCCATTCAATATTCATGGCCTTTTGATGTTG
GTAGGTATTAAAAAATTGATTTTTAGAAAAGTATCTCAAACACCAGGTATGTTACTGTAAAATTTTAAA
GAAATAAATAAAATCACGGATGAGGCCAGAGGCCAGAGGTCACTTTTCAGTGTCTATCCTTCCAATCTTG
TGTGTGTGTGTGTGTGTGTGTGTGTGTGTGTGTGTGTGTGTATGTGCAGATACAAACTTCTTTTTTCCCA
ACATTGGGAATTATGTCATACAAATTATTCAGTAATATGCTTTTTTCTAGCTGACATATATTTCTTTCAT
TTGTTAAATGTGCATCTGCAACATCATATTAAAGTCCTGGAAGTGTAATTACTGGGTTAAAAAGTTTAAA
AACTTTAAACTATTTGATACATAATGTTGAACTGGCCCCCATAAAAGGTTGTACCAAATTAATACTTACA
TCAAATGTGTTCCAGTAGAATAAACAGCTTTAAAATACTTTTGTGCTTTTTTTTTGTTGTTGTTGTTGTT
GTTTTGCGATGGAGCCTTGCTCTGTCACCAGACTGGAGTGTAGTGGTGCGTTCTCTGCTCACTGCAACCT
CCACCTCCTGGGCTCAAGCGGAATCTCCTGCCTCAGCCTCCTGAGTAGTTGGGATTACAGGCACCCACCA
TCATGCCTGGCTAATCTTTTTTTTGAGACGGCAGCTCTGTTGCCCAGGATGGAGTGCAGTGGCGCGCTCT
CGGCTCACTGCAAGCTCCGCCTCCTGGGTTCATGCCATTTTTCTGCCTCAGCCTCCAGAGTAGCCGGGAC
TACATGCGCCCGGCCACCACGCCCAGCTAATTTTTTGTATTTTTAGTAGAGACAGGGTTTCACTGTGTTA
GCCAGGATGGTCTCGATCTGCTGACCTCATAATCTGCCTGCCTCGGCTTCCCAAAGTGCTGGGATTACAC
GCGTGAGCCACTGCGCCTGCCATGCCTGGCTAATTTTTGTATTTTTAGTAGAGATGGGGTTTCACTATGT
TGGCCATGCTGGTTTTGAACTCCTGACCTCAAATGATCCGCCTGCCTTGGCCTCCCAAAGTGCTGGGATT
ACAGGCGTGAGCCACTATGCCCAGCCTAAAACACTTTAAAACAGATAATATTTCACATACTTTCAAATAA
AGACAAAAAGGGTACACAGATCTAGATAAGTGGACACATCAACCACAGTAAGAAATGATCAGAATCATGG
ATGCACCCAATCATCAATATTAAGTATTGAGATGGTAATTGGATGGAGTGGAAGGCACTAAAGAGGACAT
CATGTTACTTATTCTTGACAGGCTTTGTTGAAGAATGATTTAAAATTCAATGGCGAAAAGCAGGAAACTT
TGCAAGAGAGAAGCACATCTTAGTTTGCTAGAAGGAATGTTTCTTTCATAAATGCCACTTACCAGTCCAA
AAGCTTAAGAAAAACTACATGTTTTGTTTCAAATGAAATTCCTGGGAAAATTTTAAGAGTTCTTTATTTA
GCTAATATGGCTATTTTATATTTTATAGACTTATGGATGGTAGTGCTTTGCCACCTGTACTATTTCTTCA
TACTTAAGTCGGAAATTAAAATTTGACAAACCATGTGTATGCACTATTAAAAAAATAAATAAAATTTGAC
AATCATACCAGAAGCACTCTCATTTTGTAGCAAGGTTTTGCATAAAAAATTAGAGAGTGCGTCAATTGGG
GAAACAGTACTGATATAAGACTCACAGAGCCAACATCACATGGAAATAGGCAAGCTACTGAAAATGCTTA
CCAAGGAAACTTACAAGGTTGCAATTAACTTCAGATCAACTGCACTTACCCATAATCCACTTTCATATGC
TGCCCATTGAAGAAAAAGACAGTAGATGGAATATAACTGATGTCAAAATACTGTGTATAAACTGCAGTTT
GGTCCACATCTACCAGGTATATAGCAGCCATTTTACTTAAGTCAGAAGAGGTCTTAGAAAGCTGCAAATG
ACGAGAGAGACAAAGGTTACAATATGAGAGGCAGCTTAATGTTTCTTCTGTGAAACTTGTTACTACAACA
GAGTGTTTTGTTTGAAAAAACTCCTGGGCCAACAATGCAGCCCATGTGGCTCCCACATTAGACAAAGCAT
AACCCCATCCTCCAGAGTCTCATTCACTGCAACGGGGTTAGGAGGGAATCTTGGGGAAAACTGTATGAGG
TTAGGAAGGGACCTTGGGGAAAACTGTGTGAGGTTAGAAGGGGACCTTGGGGAAAACTGTGTGAGTTTAG
GAGGGTGACAGCATGGGAGTATTCCTGTCTATTCATTGTGGAGAGAGACCTAACCCAGAACTAAGACCTC
TACCCGCCATTTCTACTGCTGCCATCCCGTGTTAAGAGCTGAGCATACAAATCCTTGGGAAAACTGTGTG
AATCTGTTTCTTCCTTTAATTTCTTTCTTTGAATGCTTTTATAAAAGTAGCCCATGCTCATTTAAAACAA

CACAACTTAGAAATATGTTATGTGGATACTGAAATTCCCTAACGTCTCCTTCCTCCCAACCCTCCCAGAA
ATGAAAACCACGATAATTAGTAGCAGACACTTATATAACATTACTATGTGGTAACTGTCCTAAGTGCTTA
ACATTTGTTAACTCATTTAATCCTTACATCATTCTTATAAGGTATATGCTATCAGAGTCCCCATTTTATA
GATAGGGAAACTGAGGCACAGGAAGGTAAAGTAATTTGCCCCAGGTCACACAGCTCCTGAGCAGTAGGGC
TGGGGTTTGAACTTTGGCAGTGTGGCTTCAGTATACCACTGACCTCTGTGGTATTTCCTCCCGCCTATGA
CTGAGGGTAGAGTTCTCTGGCCCTTTTTTCCCCAAGTTAACACACTGCACCTTCATTGCAATTATGGAAA
CGAGGATTTGTACGCTCAGCTCTTACACGGGATGGTAGCAGTAGAAATGGCGGTAAGAGGTCTTAGTTCT
GGGTTAGGTCTCTCTCTGCATCAGGCACATACTGGAGGCAGCACACTAGAAAAGATTATTTGCAGCTACA
GAAAACTGAGTTCAAATCTCAACTCTGCAATATAGTGGATCTGCACCTGTGGGAAATTACTTGACCTCTC
TGGTTTTTTTTTTCCTACTGGGAAATGGGGCTACTATTATCCCACTATTGCGAGCACTGAAAAGATAGTG
TTCACAGATGCTTGTTAATTAGTTTTCTGCTCTTCCATCACCTTGTTGGTGAAACAGCATTGACAGCTTC
TAGTGGGTAAGGACATCCAAGACAGATTCAGCACACACAGATTTGGGGGCTTGGTTCAGGGCAGGCACAC
AATCAGCTCTTAGTGAACTAACAAATGAACAGCTATATACCCAACCCTGACTACTCCCTCTCATGTACTA
CTCAGATGCTGAAGTGAGAATATACAGATTATTAATAAAAACCAATTATTTTAGACATTCACTTACAATA
TCATCTAGCTGCAGACAGACAGGATCTTCATCTCTCCCAAACCTGAGAACCAACACCTTCTCAGCAGTAC
TTTTTATCGCCTGGTCTACTTCCTTTTTGCTAGTCAGCTTGGGCAGTAGGAAGCTCATCTTGAAATAACC
CAAATGTGAATCCTCACTGTCACTCCTGAAATAAAGGTGCAATGTTTAAAGACAGTTTAGATTAAGTGCG
TGAGCCCTCATTAAAAAAAATTAATTCACAGAGTAGCTAATGTATTCACATGGTATGAAAGAAACATCAT
AGAAAGGTAACACTGTGGAGTTTCCCTCTCTTTGTCCCTGCTCCTTGGAACCAGTTTTCATTGTTCCCTC
CCCCGGGCCAGATAACTGCTTTCATTAGTTTCTCATGTATCTTTCAGAGTTTTGTTATGCATTCACCAAA
AAATACAAATATGCGTTCCGATATTTTTTTTCTCTCTCTCCCCACCCCCAAAAGATAGCCTATTGTATCA
GTGTTCCAGCAGGAAAGCGATGACACTCTCAACTTTGGTAATTTGAAGGCAGTTTAATTAAGGCAGGTGT
AGAAAAACAATACGGTAAAGTGTACTACTTTGGGGCTAGTAACAGCGGGATGCTGTACCCATCCCTAGGC
CATGGGAGGAAGAAGCCACGGTAGGAAGCAGTTATAAGAATTGTGTGGCAGAAAGAGGTGAGTGGAGAGG
ACAGTGTATAGGAGGGCAGCCTGCTGTTAGGTGTTGCAGCCACTCTCCTCCCTCCCTCTGACCTTTGCTC
ATGCAACCACCGGCTGACTCCAACTGGAACCAGAGGGCAGGGGCCTGTTGAGGCAGGCAAACCTCCCTTG
TCCCAGAGCAGTCTAAGAGGAGGTTGAAGAATGGCTGTGGAGGGACCAATGGCAGATGTTCAGCACACAC
ACTTTATCTACTCTTCAGCACTTTTCATTATTTCAAACCTAACCTATGAAGATTGCCCCATATCTCTATA
TAGAGAACATCCATATTCTCTGTCATGGCAGCATATTATTTCTTGCATGTGTTACACTTTTTAAACAGTC
CTTTATTGTATACTTTATATTATTACAAGTCCTGGCTTAAAGATTCTTCGTGTGCACCTTACTAGGTGCA
CCTCATTACACGGCAAACAGATCAATTAAGAGTGTGTGTGAAAACTACAATACCATAAACAAATAATAAA
ATACTGAGGTACAAGGGACCTAATACAACCCCGTCATTTTACAAACTAGCTTGCTTACAGTTGCACTTTC
TACTACAATGACAAGGACTAGAATACAGGCCACCAAATTTCTAGTCCAGGGGTCCTTCTACTACAATAAT
CTATTTTCATGATTGGTAGAAATTTCATTTTTGAGAAACTCTTTGACAAAGAAGCTTAGTTTTGTGGTCA
TCTATAAGAGACTCCATTGAGATGGTCGGTATTTTGGAATTC

SEQ ID NO: 9 (hHp) (Accession No. AH003344)
AACTGCCATCATACTGAAGGTAATCTTCTGAAACTTGCGGTTTTCTTTCAAAATTATGTTTATAAGATGA
TCCATGTTCTTGCAGAGTTTATTCATTTTATGCTGTATAATATTCCATTATATCCACATACAATGCAGTA
TTGACCCTTCCTCCTGTTGATGGGCATTTGTCTTGTTTCTAGTTACTTTGCTATTATATCAGTGTCACCA
TGATTATCCAAAAGTAATTCTTTTGTACACTCTAATTTAAGAACAACTAACCCTTTTTAATGAATAAATC
AACCTTGTATTGAGTTGCTACTAAGTTTCAGTTGACTAGTACCTGGGATACACACAGGTGCAGACATTTG
ACTGAGACATATTGATTTTTCTCATCTGCCTATTTAGGCTAATCACCAGACTATAAAACCATGAGAACCA
CTGCCATTGAGTATAGTCTGTGTCAGTCTACACTATAGCTTTAACTAGTTGTGTGATTTCTTGCAAAGAG
CAATCAGAGAAGACACAATAAACACATTTACTGATTTCAGGCTGGAGAGCTTTTAAGCAATAGGGAGATG
GCCACACACAAGGTGGAGAAAATTACTGTGAAAAGGATACGTACTTTTCTTTAGAGCCCCACCTAAGCTA
GGCTGCAGAAATGTCTACAATGGCTTTGAAAAAACTCAAAATGAGCCTTTCTGCAGTGTGAAAATCCTCC
AAGATAAAGAGACAGATTGATGGTTCCTGCCGCCGCCCTGTCCTGCCCAGTTGCTGATTTCAGGAAATAC
TTTGGCAGGTTTGTGGGCATAGAGTTGCCAGGTTTCTTGGGATTTGTAATAGAACATCACAAGAAAATCA
AGTGTGAAGCAAGAGCTCAACTCTTAACAGGGGTATTGTTTGTGGTTTTGTTACTGGAAAAGATAGTGAC
CTTACCAGGGCCAAAGTTTGTAGACACAGGAATTACGAAATGGAGAAGGGGGAGAAGTGAGCTAGTGGCA
GCATAAAAAGACCAGCAGATGCCCCACAGCACTGCTCTTCCAGAGGCAAGACCAACCAAGATGAGGTGGG
TCCACAGCTTTCCCTCCTGCCTTTCCTCTGGTTCTTTATTTCAGTCTTTTTTGCATACATCGGTAGAGAT
GCAGAAATAGAACAAAGAAACGGGCAAATGGGCTAAATTATAGTGAACCAAAGGGCTTAGTGTGTTAAAT
CTTCTCCTTTTCTGCATCCATAGAAGACAGTGCTGCTGTCTTTCCCAGGAGATAAGATTTACTCTCAGGA
GTGTCTTTTTCCTTCAGGTTACATTTTTGACTTTATAGGGTATGTCATCAGCTCCCGTGGTAGGCTTCCT
GGCATCCTGAGTATATTTATTAGCAGATATTTTCCTCTTTAAAAATGTACAATAAGGAAGACTAATAGTA
ACACATTTGAATGACACAATTAATTGACTAGTACCTGGGATACACACTAATACCTGGGATACATCTAATT
AAGGCACTTAGATCTTATAAAAATAAACACTTTTTGAAATGTTGAAATAATAAGACTAGAAACTTTTTTT
TTTTTTGAGATGGAGTCTCGCTTTGTCACCAGGCTGCAGTGCAGTGGCATGATCTCGGCTCACTGCAACC

TCCACCTCCCAGGTTCAAGTGATTCTCCTGCCTCAGCCTCCCAGGTAGCTGGGACTACAGGCGTGCGCCA
CTATGCCCACCTAATTTTTGTATTTTTAGTAGAGACGGGCTTTCACCATGTTGGCCAGGATGACCTCGAT
CTCTTGACCTCGTGATCTGCCTGCCTTGGCCTCCCAAAGTGCTGGGATTACAGGCATGAGCCACCGTGTC
TGGCCTAGAAACTATTTAATAGAAGCAAGTAGTGCCCGAATGGTTGGCATTTGTTAGTGAGATGGTGAA
CTGGCAGACGGCACCTGTGGGTCAATGCCCATGGCCACCGTCCTGCTTTTGGACACCAGTTCTCTTCCAG
GTAACCTTCTGGCATTTTGGGGTTTCAGATACCATTTCCTAAAGGTGAATTATTATAAAATACTAGAAAT
ACCCACGTGTTTAAAATTATATATTTAAGGAACCTTTTATTACGGAAAATATCAAGAAGTAGAGGAATAG
CACAGTAAGCCCAAAGTTCCCATCTCTGAGTTATCTACATTTTATTACCACTATCTTTGCGGTGTCTGAG
GGAGGTTTCTCTTTCCTGGAGGGCTCCTGTATTATTGCCAATGTACTTTCCTGAATGCAGCCAGAAACTG
AGCCCACCCCTCCACCTATGTGCCTTTCTATCCCTCTCTGAAGCTTCTGCAGAATTCCCAGCAGGACAGG
GCTTGCTGGAAGCTTGGTATGCTCAGAAGCTGCTAAAGTGTGTATGGGCAGGTGTGGGGGCAATTTCTTG
GTCCTAGCACTTCCATATATCGACTTTCTTTTCTGGCTGCTAAGTGGGAGGAGTGTGTGTGTATGCATGT
GTGTGTGTGTGTGTGTACATGCATGTGTGTGTGGATGCATGCATGTGCTGTGAAGCAGGGAGACTAGCTT
TCCACTCCTCCTTGTCTTCTCTCTGCAGTGCCCTGGGAGCTGTCATTGCCCTCCTGCTCTGGGGACAGCT
TTTTGCAGTGGACTCAGGCAATGATGTCACGGATATCGCAGGTCAGTCTTTGGTTGGGTAGGAGTGTGCA
TCCCACTCTGACCCTCTCGGGTCTGCACTCTCTCTGAGAACACCCAATTCCCCCTTCTTATCTCGACCTC
TGGGCTTTCAGGACCATAAAGAACATTGGGGTTCCTGCCAGAAATGAGGGGAGCTTGCCTTTCCATTGGC
TTCTATTCGGGGTGGAAGGAGATTGATGTGCAGAGCAGCTCCCGCTCATCTGACTTTTCACGGTTCACTG
GGAACAATTTCCAAATAGCAAACTCTCTGGCTTCTCTCTCTTTGCAGATGACGGCTGCCCGAAGCCCCCC
GAGATTGCACATGGCTATGTGGAGCACTCGGTTCGCTACCAGTGTAAGAACTACTACAAACTGCGCACAG
AAGGAGATGGTAAGATGTGGACAACTGTCTCCATGCCCTACATACAACCCCCTTCTCTGACATTTCCATG
ATGGGTGGTGCTGAGGTGATTCGCCAGAAAGTTCGTTGCTCTCCTTGGAGCCAGGAGATTTAGATTCTAA
TAAGCGTTTTGTCGCCAGTAGCCATGGCCCTTTGGGCAGACTAACTTTTGTCAGCCTCAAGTTTTCTGTT
TTGTTAAGGGGAGGCGATGCCATGCAGCCTACCTCATGTAAATCTCAGAGTCAGATTTACATCTCCAGCA
GATGTGGGAAAAGAAGGAATGCTGATGATGATGTCACCCTCACCTAGTGAGTCTTGCTGTCCTGGCACTG
CTCTAAGGGCTTTATACTTATTTGCTCACTTAGTCCTCACAGTATCCCTCTGAACAGAGTTTATTGTTTT
CACTTTGCTGATAAGGAAACTGAGGCACAGACAGGTTGAGTATCTTGCCCAAATTCAGGCAGCCTGTAAG
AGGCAGAGTCAGGATTTGAACCCTGAGCCCTCCCTGTACTGCTTGGCTGTGACCGCCATGACCACAGTGT
GTTCTGCTGGGCTTAACTGGTGTCCAGGCACTTGGCTTCCAGCACAGCACTCTTTCCCTTCCTCCTTCTC
ATATTCTCTCTCCTTTCTCCCTTCCTGTCTGCCTCCTTTCTTCTTCTTCTTTTTAATTCTTCTCCTTAAA
TGCCTTCTCACTCTGCTCTGGGTGCAGACTTGACTTTTCCTTTGGCTCATTTCTTGCCTTTTGTTTCAGG
AGTATACACCTTAAATGATAAGAAGCAGTGGATAAATAAGGCTGTTGGAGATAAACTTCCTGAATGTGAA
GCAGGTGGGTGCTGAGCACTGAGCACTTAAGAGAGCAGGCAGGCGTCCAGCGGGGAACGTCCTAGAGGCA
CAGCCTTCCAGTGCGGCTTCCTCTGAGCACACAAGAGCCAGGAGGAGGGATGTGGGAGAACCGCAGCTGG
CCAGGGAGAGACTTAAGCAGTTAGGTGATGACTCCCTAAGGGTCACCAAGGGTCTTGTTCATTGGGGCCT
GAAGGGCACTGGCTGAATCCACTGTCGGCACTGCCCACAGATCAGGAGAGCCTGTGCATACAGAGAGCCT
GCTAGAAAGCCCTGGGTCTAAGGAGAAGCAAGCTCCAGGGAGAACAAGTCAAGGAATGACATAAAATCTT
AATCCATGGAAGCCTAGCAGGAGGCTGGACATGGGCTGGAACTCCTGCTTCTCGTTATTAGGAGGAGCTG
TTGCTCTCTCCTTTCATTCTCAGAACCAGAGGCAAAGACCCAGCCTCTTCTGCTCTTACTGGTGTGGAAA
TGCCAACCTGCCTCGTATTAACTGCACCATCTACAAAATCTGAGCTCCAGCCAGTGCTGCTCTAGATTCA
TCTTTCTTTAGAGAGAATGAATTATTGTAGCCCCTAGCCCTTTCAATGAATTTCAGGGAATTGTGGAAAT
TCCTTTATTGGGATAATTGTTTAAATATAATACAGTTCGCGAGCTTCTATTCGGGGTGGAAGGAGATTGA
TGTGCAGAGCAGCTCCCGCTCATCTGACTTTTCACGGTTCACTGGGAACAATTTCCAAATAGCAAACTCT
CTGGCTTCTCTCTCTTTGCAGATGACGGCTGCCCGAAGCCCCCCGAGATTGCACATGGCTATGTGGAGCA
CTCGGTTCGCTACCAGTGTAAGAACTACTACAAACTGCGCACAGAAGGAGATGGTAAGATGTGGACAACT
GTCTCCATGCCCTACATACAACCCCCTTCTCTGACATTTCCATGATGGGTGGTGCTGAGGTGATTCGCCA
GAAAGTTCGTTGCTCTCCTTGGAGCCAGGAGATTTAGATTCTAATAAGCGTTTTGTCGCCAGTAGCCATG
GCCCTTTGGGCAGACTAACTTTTGTCAGCCTCAAGTTTTCTGTTTTGTTAAGGGGAGGCGATGCCATGCA
GCCTACCTCATGTAAATCTCAGAGTCAGATTTACATCTCCAGCAGATGTGGGAAAAGAAGGAATGCTGAT
GATGATGTCACCCTCACCTAGTGAGTCTTGCTGTCCTGGCACTGCTCTAAGGGCTTTATACTTATTTGCT
CACTTAGTCCTCACAGTATCCCTCTGAACAGAGTTTATTGTTTTCACTTTGCTGATAAGGAAACTGAGGC
ACAGACAGGTTGAGTATCTTGCCCAAATTCAGGCAGCCTGTAAGAGGCAGAGTCAGGATTTGAACCCTGA
GCCCTCCCTGTACTGCTTGGCTGTGACCGCCATGACCACAGTGTGTTCTGCTGGGCTTAACTGGCATCCA
GGCACTTGGCTTCCAGCACAGCACTCTTTCCCTTCCTCCTTCTCATATACTCTCTCCTTTTCCCCTTCCT
TTTTGTCCCCTTTTCCTCTTCCTTTTAGTTCTTCTCTTTAAATGCCTTCTCACTCTGCACGGGGTCTAGA
CTTGACTTCTCCTTTGGCTCACTTCTTGCCTTTTGTTTCAGGAGTGTACACCTTAAACAATGAGAAGCAG
TGGATAAATAAGGCTGTTGGAGATAAACTTCCTGAATGTGAAGCAGGTGGGTGCTGAGCACTTAAGAGAG
CAGGCAGGCGTCCAGCGGGGAACGTCCTAGAGGCACAGCCTTCCAGTGCGGCTTCCTCTGAGCACACAAG
AGCCAGGAGGAGGGATGTGGGAGAACCGCAGCTGGCCAGGGAGAGACTTAAGCAGTTAGGTGATGACTCC
CTAAGGGTCACCAAGGGTCTTGTTCATTAGGGCCTGAAGGGCACTGGCTGAATCCATTGTCTACATCGCC

CACAGATTAGGAGAGCCTGTGCATACAGAGAGCCTGCTAGAGAGCCCTGGGTCTAAGGAGAAGCAAGCTC
CAGGGAGAACAAGTCAAGGAATGACATAAAATCTTAATCCATGGAAGCCTAGCAGGAGGCTGGACATGGG
CTGGAACTCCTGCTTCTCGTTATTAGGAGGAGCTGTTGCTCTCTCCTTTCATTCTCAGAACAAGAGGCAA
AGGCCCAGCCTCTTCTGCTCTTACTGGTGTGGAAATGCCAACCTGCCTCGTATTAACTGCACCATCTACA
AAATCTGAGCTCCAGCCAGTGCTGCTCTAGATTCATCTTTCTTTAGAGAGAATGAATTATTGTAGCCCCT
AGCCCTTTCAATGAATTTCAGGGAATTGTGGAAATTCCTTTATTGGGATAATTGTTTAAATATAATACAG
TTCACCAGCCAGGGCTCAAAAATCTCAGTATTTCCCACTTCCTTTGTTAGAAAAGTGGGAAATAGAGCTT
TTTGTAATGTAAACAATTTAAAAAACAGAATTATTTTAAAACTGCAACTATTGGAAATGAGATCAGCAGG
TGGTAAGGGCAAAGCATTTAAATCTTTCTACTTTACGCAGCAGTGACAGCCGCCCATGCTTTCACCCCTT
TCTCAGATGGAAAGGCTCTTGCACATTTCCACTCACGAGTGTCTTGCTCTCCTTGACAGTATGTGGGAAG
CCCAAGAATCCGGCAAACCCAGTGCAGCGGATCCTGGGTGGACACCTGGATGCCAAAGGCAGCTTTCCCT
GGCAGGCTAAGATGGTTTCCCACCATAATCTCACCACAGGTGCCACGCTGATCAATGAACAATGGCTGCT
GACCACGGCTAAAAATCTCTTCCTGAACCATTCAGAAAATGCAACAGCGAAAGACATTGCCCCTACTTTA
ACACTCTATGTGGGGAAAAAGCAGCTTGTAGAGATTGAGAAGGTTGTTCTACACCCTAACTACTCCCAGG
TAGATATTGGGCTCATCAAACTCAAACAGAAGGTGTCTGTTAATGAGAGAGTGATGCCCATCTGCCTACC
TTCAAAGGATTATGCAGAAGTAGGGCGTGTGGGTTATGTTTCTGGCTGGGGGCGAAATGCCAATTTTAAA
TTTACTGACCATCTGAAGTATGTCATGCTGCCTGTGGCTGACCAAGACCAATGCATAAGGCATTATGAAG
GCAGCACAGTCCCCGAAAAGAAGACACCGAAGAGCCCTGTAGGGGTGCAGCCCATACTGAATGAACACAC
CTTCTGTGCTGGCATGTCTAAGTACCAAGAAGACACCTGCTATGGCGATGCGGGCAGTGCCTTTGCCGTT
CACGACCTGGAGGAGGACACCTGGTATGCGACTGGGATCTTAAGCTTTGATAAGAGCTGTGCTGTGGCTG
AGTATGGTGTGTATGTGAAGGTGACTTCCATCCAGGACTGGGTTCAGAAGACCATAGCTGAGAACTAATG
CAAGGCTGGCCGGAAGCCCTTGCCTGAAAGCAAGATTTCAGCCTGGAAGAGGGCAAAGTGGACGGGAGTG
GACAGGAGTGGATGCGATAAGATGTGGTTTGAAGCTGATGGGTGCCAGCCCTGCATTGCTGAGTCAATCA
ATAAAGAGCTTTCTTTTGACCCATTTCTGTGTTGTGTTCAGTCTTGAGTCTTTTTTATTTGCTCCTTTAT
GGTCCAGGGTAGTCAGAAGGTATAGAGTCTACTGGGAGTATGGCAGAAAACACCCTAAACCCACTGGAAA
TCCCGAAGGTGATACAAACTCTTCCACCTTAGGGAATCATGCTCACTGATTGAGTGCCTATTGAATGCTA
GGTCCCAGAAAGTTAACTGTTGTCCTTGTTTTACAGACAAGGAAACAGAGACTCAGAGATGGTAAGTGAG
TTGCTTAAGGTTACATAGCTATGAAACAGGGAAGCAGAACTTTGAACCCAGGTCTGTTTGATACAAACTC
AGAGGTCCTTTCACTGCATGCTGTTGCCTCCTCAAAGTGAATTAGGAGAAAGGCATGGGCCTGGTTGAG
GAAGAGGCTAGCTCAAAATGGGATGGGGAAAAAGTGTTTTAACACAGACAGTAATTCAGAGTTTGGGATC
TAACCTACCAGCACTCCAGAAAACAAAAGACAATGATGTAAAGGCAGGATCTCTGGACTTACTGAGTCCA
AATCACAGAATGGCCCATAAATTTGCTAGGTAATTTAGGTTTCCTGGCCATCAGTTCCTGGTCTCTTCCA
TGAAGGACTTGAGCCAGGCGATGGCTAATGTCCCTATCAGCAATAACCTTCTGGTATTCCATAAAGAGTG
GGGAGGTAGAGTCCCTGCATCAGTAGAAGCTGCTTTTCCTACTGGGAACAAACCCACCCTGCACCAGGAA
TCAAATGCAGGCTGTCTGAGCCTCCTCTCCTGATGGCAGGTCATGCTATTAAGGGTCTCCCAGAAGAAAG
GTTCTGGAACTCTGAGAAACAGAAGCTCTTCTAACAGAACTTTTTCTTGCTATAAGCTTTCAATACCACC
AATCACCAGTGCACCTTCTTCTCATGCCAAAATACGGCCCCTCCTTTGTAGGGTGTAAAACGACACTGAT
GTCACTCTCTCTCACCAAGGTCTGGCCATCTCTGTGAAGCTCTCCTCACAGGCACCCTGCAGATTCATCA
ATAAACTTCAACAGGTTAGCGCCAGTAGGCCAATAAGATGCACACTTATATTCTAGAACCTGTGAAAGAA
GTAATAGCCAGAAACTGGAAAATAACAGTCAAACTCTTAAAAAAATGCCTCTCAGAACACAGTCACTTAC
ACCTTTTCCTCTGGTAGTTCTCTTCAGCACCAAGTTTCCTGGAAGAACAAGCTACTCCCCAGTTTTCTTA
AAGAATTGTCTGATTGTATTAAGTAAGTGGCTTCTGGTAAAATAATGGTAAGAGAAAAGCTCTGGCATTC
TCTTGATCACTCATCTTCTCTGCCTTCCTCCCTAGCAACATCTCCTGGCCTCAGCCTGTGGGCTTCAGCA
ACACCAAACTCCATGTTGCTCTCTGACAAGCCCGGCCGTCTCCAGTTCTTGGGCTTTATTTCCTCGGGGG
CCCTCTGCTGAAATTCCCTTCCCCTTGCTGTATTAGCACCTCCTACCCGCCTTCAACATGCAGGCACTGC
CTCCTCTAAGGAGCCATCTGTACTTACGTAGCTGTGAGCATAGGATGGGGCATACAGCAGGCACTTAACA
AATACTTGTGGAATGGAAAGAAACTAAGATAGGATCAAAAATACAAATCAAGATGTGAGAGAAAGACGAT
CAAAAGCCCTCACGAGGGGGTCGGGCGTGATGGCTCACACCTGTAATCTCAACACTTTGGGAGGCTAAGG
CGGGTGGATCACTTGAGGGTGGGAGTTTGAGATCAGCCAGAGCAACATGGAGAAACCTCGTCTCTACTAA
AAATACAAAAAATTAGCCAGGCATGGTGATATATGCCTGTAATCCCAGCTACTCGGGAGGCTGAGGCAGG
AGAATTGCTTGAACCTCGGAGGTAGAAGTTGTGGTGAGCTGAGATCATGCCATTGTACTCCAGCCTGGGC
AACAGGAGTGAAACTCTGTCTCAAAAAAACAAAAAACAAAAAAGAACACCACGGGAAAATCAAGTGTGA
AGCAAGAGCTCAACTCTTAACAGGGGCAATGTTTGGAGTTTTGTTACTGGAAAAGATAGAGACCTTACCA
GGTCCAAAGTTTGTAGACACAGGAATTACGAAATGGAGAAGGGGGAGAAGTGAGCTAGTGGCAGCATAAA
AAGACCAGCAGATGCCCCACAGCACTGCTCTTCCAGAGGCAAGACCAACCAAGATGAGGTGGGTCCACAG
CTTTCCCTCCTGCCTTTCCTCTGGTTCTTTATTTCAGTCTTTTTTGCATACATTGGTACAGATGCAGAAA
TAGAACAAAGAAACAGGGCAAATGGGCTAAATTATAGTGAACCAAAGGGCTTAGTGTGTTAAATCTTCTC
CTTTTCTGCATCCATAGAAGACAGTGCTGCTGTCTTTCCCAGGAGATAAGATTTACTCTCAGGAGTGTCT
TTTTCCTTGAGGTTACGTTTTTGTCTTTGTAGGGTATGTCATCAGCTCCCGTGGTAGGCTTCCTGGCATT
CGGAATATATTTACTAGCAGATATTTTCCTCTTTAAAAATGTATAATAAGAAAGACTAATAGTAACACAT

TTGAATGACACAATTAATTGACTAGTACTTGGGATACACACTAGTACCTGGGATACATCTAATTAAGACA
CTTAGATCTTAAAAAATAAAAAGACTTTTTGAAATGTTGAAATAATAAGACTAGAAACTTTTTTTTTTGT
GAGACAGAGTCTCACTCTGTCACCAGGATGGAGTGAGTGGCGCGATCTCAGCTCATCGCAACCTCCCCCT
CCCCGGTTCAAGCAATTCTCCTGCCTCAGCCTCCCAAGTAGCTAGGACTACAGGCGTGTGCCACCACGCC
CAGCTAATTTTTGTATTTTTAGTAGCGATGTGGTTCACCATGTTGGCCCGGATGATCTCGATCTCTTGAC
CTCGTGATCCACCTGTCTCGGCCTCCCAAAGTGTTGGGATTACAGGTGTGAGCCACCGCACCCTGCCTAG
AAACTATTTTAATAGAAGCAAATAGTGCCTGAATGGTGGGCGTCTGTTAGCGAGATGGTGAACTGGCAGA
TGGCGCCTGTGTGTCAATGCCCATGGCCACCGTCCTGCTTTCAGACACCAGTTCTCTTCCAGGTAACCTT
CTGGCATTTTGGGGTTTGAGATACCATTTCCTAAAGGTGAATTATCACAAAATACTAGAAATAACCACAT
AAGTGTTTAAAATTATTGTTAAATACAGTAAGAAATTCTTCTTCAAAAGTTTAGCCTGCTTAAGTTTCCT
TGTCCTTTGTTTCCTGCTTTCAAGGCCAGACTTCCTTACTCTCTGTGTTTCCCCTACCCTGGTAAACAAC
CTTCCTGCCAGTCCTTACCCATACAGCCCACATTCCACATCTGCTACCCACTCTGTGATTTACCTCTCCC
GTCGCAATAGCCTCTCCCACCAAAACTGATCTTCCCGCCTTCCCACCAGTGCAACCACATTCCTGCACTT
TGCAAGTTAGCCAACCGGGTTCGGATTGTGCAGTCCAACTCCAGCCAATGGAGTCAGGACACAGTAGCAG
GGACAAGCTGCGTTAGACATAAAAACCTCTGCTTTCCTTTGTTTAGGGTGCTCTCGTGGCAACCAGACTT
ACCAGGAGCTCTATTCTGCAAAAGTAAATTTGCCTTGCTGAGAGACCCTTTGTCCTTTGGCTCAGTGTTG
GTTCTTCTTTGCAGCACCGAGCATTTGTTTCCAACAAATTTGGTGGCCCATACAGGGAAAACATTGTCCT
CCGGGAAAGGGTTCTTTGATCATCCTCTTGAGAGGAGAACACATCCCACTGTCCTTGTTGCGGTGGCCTC
ATGAGTAGGAATCGAGACCCACCTGTCTGATGAATAACCCCAGACTCTCAACAACGTGGGGAGAAAAAGA
CTTGCAACACTATGGTGGCCAGGTAACTCTGTGCGCAGACCAAGGTAAGAAATGTCGCAGGAGTGACAAA
GTACTTCCTTGGTGGTCACTATATTCTGGTGGCTGAAAGTTCATGAATGGTAACAAGTGCTACTGCTGTG
TGGAGTGAATGAGTCCAATCTGTGGGTCTATGGTTACCTCATACGGCTTAGCCTTCTCTGAAGGATCCGG
GNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNAGCGTCCTCCTCCCATCTCCAAAACAACCCAACTAAAGT
AACGGTTGTAAAGGTAAAAAATTTAAAGCAGACTATAGCCCTAGAGACAGGATATCAAGATGCAAAGTCT
TGGCTAAAATGGATTAAATATTCCATCTGCACTTTAAACAAATATGAATGTTATGCTTGTGCTCACGGTA
GACCAGAGGCCCAGATTGTCCCCTTTCCACTCAGATGGTCTTCTAACTGACCGGGCATAAGCTGTATGGT
AGTTCTCTTCCAAGATCACACAGCCTGGGGTGACAAATCATGTCAAGCTCTTTCTCTGCTGTTCCCTGAA
GCTCAACACCCTGAGGGTCAGCCCCCGAGGGCCATCAGCTTCCGTTTCCCAATGCCAATTTTACTTTGTG
TCTCTCACAACAGGGAGAAAACTTGGTGTTCCTTGGAGCCTTAATGGGATGCAGTGAGCTTAAGTCCTTC
CAAGAGCTTACCCATCAGTCTGCCCTTAGTCATCCTCGAGCGGATGTATGGTGGTATTGTGGTGGACCAT
TACTGGACACTGCCGAGTAACTGGAGCAGAACATGCACTCTAATTCCATTGGCTATCCCTTTCACCCTGG
CATTTCATCAACCAGAAAAGATAGAAACCAAACACTGTAAAACAAGAGAGGCCCCTCATGGGTCTGTTGA
CTCCCACATTTATATAGATTCCACTGGAGTCCAACGAGGAGTGCCAGATGAATTTAAGGCCCAAAATCAG
ACAACTGCAGGATTTGAATCCACGTTCTTCTGGTGGTTAACTATAAATAAAAATGTGAATTAGATAAACT
ACATTTATTACAACCAATGATGATTTTTTTTTTTTTGACGGAGTTTTGCTCTTGTTGCCCAGGCTGGAAT
GCAACGGCATGATCTCGGCTCACCACAACTTCTGCCTCCTGGGTCCAAGCAATTCTTCTGCCTCGGCCTC
CCGAGTAGCTGGGATTATAAACATGTGCCACCACGCCTGGCTAATTTTGTATTTTTAGTAGAGACAGGGT
TTCTCCATGATGGTCAGGCTGGTCTCAAACTCCCGACCTCAGGTTATCTGCCTGCCTCGGCCTCCCAAAG
TGCTGGGATTACAGGCATCAGCCACTGTGCCCAGCCTATGATTTGTTAATTATACTAGAGATGCTCTTAA
AGGGACAGCTGAACAATTAAGACCCACCAGTCAAATGGCCTGGGAAAATAAAATAGCATTAGACATGATA
CCAGCGAAAGAAGGTGGAGATTGTGTCATTATCGGAACCCGATGCTATACTTTTATCCCTAATAATACTA
CTCCAGGTGGGATCACACCAAAAGCACTACAAGGTCTTACCGCCTTATCAAATGAGCTAGCCAAAAATTC
TGGACTAAACGACCCCTTCACAAATTTAATGGGAAATTCTTTGGCAAATGGAAGGGATTTATGTCCTCAA
TCCTCATGTCTCTTGCCATCAGAATAGGGTTGCTTATTCTTGTAGGATGCTGTGTCATACCCTGTGCCCT
AGGATTAATACAAAAGCTCATTGAAACAGCTCTCCCCAAAACCTCCCTCAATCCTCCTCCTCCTCGTTCC
AGTGAGCTTTTTCTTTTAGAAGACCCAAGTAGAATAACAGAGCCAAATTATGTTAAAAGGGTTTAAGAAA
AAATACTGTAAAATATTTAACAGGGGGATTGTTAAATACAGTAAGAAATTCTTCTTCAAAGGTTCAGCTT
GCTTAAGTTTCCTTGTTCTTTGCTCCCTGCTTTCAAGGCCAGACTTCCTTACTCTCTGTGTTCTCCCTGC
GCTGGTAAACAACCTTCCCGCCAGTCGTTATCTATAGAGCCCACATTCCACATCTGCTACCCACTCTGTG
AATTACCCCTCCCATCACAATGGCTTCTCCCGGCAAAACTGATCTTCCTGCCTTCCCAGCAGTGTAATCA
CATTCCTGCACTTTTCAAGTTAGCCAACCGGGTTCAGCTTAGATTGTACGGTCCAACTCTAGTCAATGGA
GTCAGGATACAGTAGCAGGGACAAGCTGCGTTAGACATAAAAACCTCTGCTTTCCTTTGTTCGGGGTGCT
CTCGTGGCAACCGAACCTATGAGGAGCAACCTTATGCAAAAGTAAATTTGCCTTGCTGAGAGACCCTTTG
TCCTTTGTCTCAGTGCTAGTTCTTCTTTGCAGCACCAAGCATTTGTTTTCAACAATTATATATTTAAGGA
ATCTTTGATTATGGAAAATTTCAAGAAGTAGAGGGAATAGCATACTAAGCCCTCTGTTCCCATCTTTGAG
TTATCTACATTTTATTAGAAGTATCTTTGGGGTGACTGAGGGAGGTTTCTCTTTCCTGGAGGGCTTCTGA
ATTATTGCCAATGTACCTTCCTGACTGCAGCCAGAAACTGAGGCCCCTATACCTATGTACCTTTCTATCC
CTCTCTGAAGCTTCTGCAGAATTCCCAGCAAGACAGAGCTTGCTGGAAGCTTGGTATGCTCAGAAGCAGC
TAAAGCGTGTATGTGGGGCGGAGGGTGGGGGCAACTTCTTGGTCCTAGCACTTCCATATATTGATTTTCT

TTTCTGGCTGCTTAGTGGGAGGAGTGTGTGTGTATGCATGTGTGTGTGTGCGTGTGTGTGTGTGTGTACA
TGCCTGTGTGTGTGGATGCATGCATGTGCTGTGAAGCAGGGAGACCAGCTTTCCGTTCCTTCTTGTTTTC
TCTCTGCAGTGACCTGGGAGCTGTCATTTCCCTCCTGCTCTGGGGACGACAGCTTTTTGCACTGTACTCA
GGCAATGATGTCACGGATATTTCAGGTCAGTCTTTGAGTTGGGTAGGAGCATGCATCCCTGGCACTGCCA
CATCCCACTCTGACTCTCTCGGGTCTGCATTCTTTCTTTGAGAACACACAGTTCCCCATTCTTATCCTGA
CCTCTGGGCTTTCAGGACTGCCAAGAACATTGGGGATCCTGCCAGAAATGAGGGGAGCTTGAGCTTTCGT
TGGCTTCTATTTGGGGTAGAAGGAGATTGATATGCAGAGCAGCTTCCACTCATCTGACTTTTCATGGGTC
TCTGGGAACAATTTCCAAATGGTAAACTCTCTGGCTTCTCTCTCTTTGCAGATGACCGCTTCCCGAAGCC
CCCTGAGATTGCAAATGGCTATGTGGAGCACTTGTTTCGCTACCAGTGTAAGAACTACTACAGACTGCGC
ACAGAAGGAGATGGTAAGACCTGGACAACTATCTCCTGTGCTCTACCTACAACCCCTGCTCTGACATTTC
CATGATGGGTGGTGCTGAGGTGATTTGCCAGAAAGTTCGTTGCTCTCCTTGGAGCCAGGAGATTTAGATT
CTGATAAGCGTTTTGTCGCCAGTAGCCATGGCCCTTTGGGCAGACTAACTTTTGTCAGCCTCAAGTTTTC
TGTTTTGTTAAGGGGAGGTGATGCCATGCAGCCTACCTCATGTAAATCTCAGAGTCAGATTTACATCTCC
AGCAGATGTGGGAAAAGAAGGAATGCTGATGATGATGTCACCCTCACCTAGTGAGTCTTGCTGTCCTGGC
ACTGCTCTAAGGGCTTTATATTTATTTGCTCACTTAGTCCTTACAGTTTACCTCTGAACAGAGGTTATTA
TTCTCACTTTGCCGATAAGGAAACAGAGGCACCGACAGGTTGAGTATCTTGCCCAAATTCAGGCGGCCTG
TAAGAGGCAGAGTCAGGATTTGAACCCTGAGCCCTCCCTGTACTGCTTGGCTGTGACCGCCATCACCACA
GTGTGTTCTGCTGGGCTTAACTGGTGTCCAGGCACTTGGCTTCCAGCACAGCACTCTTTCCCTTCCTCCT
TCTCGTATTCTCTCTCCTTTCTCCCTTCCTGTCTGCCTCCTTTCTTCTTCTTCTTTTTAATTCTTCTCCT
TAAATGCCTTCTCACTCTGCTCTGGGTGCAGACTTGACTTTTCCTTTGGCTCATTTCTTGCCTTTTGTTT
CAGGAGTATACACCTTAAATGATAAGAAGCAGTGGATAAATAAGGCTGTTGGAGATAAACTTCCTGAATG
TGAAGCAGGTGGGTGCTGAGCACTGAGCACTTAAGAGAGCAGGCAGGCGTCCAGCGGGGAACGTCCTAGA
GGCACAGCCTTCCAGTGCGGCTTCCTCTGAGCACACAAGAGCCAGGAGGAGGGATGTGGGAGAACCGCAG
CTGGCCAGGGAGAGACTTAAGCAGTTAGGTGATGACTCCCTAAGGATCACCAGGGTCTTGTTCATTGGGG
CCTGAAGGGCACTGGCTGAATCCACTGTCGGCATTGCCACAGATCAGGAGAGCCTGTGCATACAGAGAGC
CTGCTAGAGAGCCCTGGGTCTAAGGAGAAGCAAGCTCCAGGGAGAACAAGTCAAGGGATAACATAAAATC
TTAATCCATGGAAGCCTAGCAGGAGGCTGGACATGGGCTGGAACTCCTGCTTCTCGTTATTAGGAGGAAC
TGTTGCTCTCTCCTTTCCATCTCAGAACCAGAGGCAAAGGCCCAGCCTCTTCTGCTCTTACTGGTGTGGA
AAGGCCAGCCTCTTCTGCTGTGAGTGGTGTGGAACTACCAACCTGCCTCGTATTAACTGCAACATCTATA
AAGTATGAGCTCCAGCCAATGCTGCTCTAGATTCCTCTTTCTTCAGAGATGATGAATTATTGTAGCTCCT
AGCCCTTTCTTTTTTCTTTCTTTCTTTTTTTTTTTTTTTGAGACAGAGTTTTGCTCTCGTCGCCTAGGCT
GGAGTGCAGTGGTGCTAATTCCTGACCTCAGGTGATCCAACTGCCTCAGCCTCCCAAAGTGCTGGGATTA
CAGGCGTGAGCCACCGCATCTGGCCCCTAGCCCTTTCAATGAATTTCAGGGAATTGTGAAAATTCCTTTG
TTGAGATAATTGTTTAAATATAATATAGTTCACCAGCCAGGCCTCAAAAAACTCAGTATTTCTCATTTCC
TTATTTAGAAATAGAGCTTTTTGTAATGTAAACAATTAAAAAAATTATTTAAAACTGCAACTATTGGAA
ATGAGATCAGCAGGTGGTAAGGACAAAGCATTTAAATCTTTCCAGTTTATGCAGCAGTGACAGCCGCCAA
TGCTTTCACCCCTTTCTCAGATGGAAAGGCTCTTGCACATTTCCACTCACGAGTGTCTTGCTCTCCTTGA
CAGTATGTGGGAAGCCCAAGAATCCGGCAAACCCAGTGCAGCGGATCCTGGGTGGACACCTGGATGCCAA
AGGCAGCTTTCCCTGGCAGGCTAAGATGGTTTCCCACCATAATCTCACCACAGGGGCCACGCTGATCAAT
GAACAATGGCTGCTGACCACGGCTAAAAATCTCTTCCTGAACCATTCAGAAAATGCAACAGCGAAAGACA
TTGCCCCTACTTTAACACTCTATGTGGGAAAAAGCAGCTTGTAGAGATTGAGAAGGTGGTTCTACACCC
TAACTACCACCAGGTAGATATTGGGCTCATCAAACTCAAACAGAAGGTGCTTGTTAATGAGAGAGTGATG
CCCATCTGCCTACCTTCAAAGAATTATGCAGAAGTAGGGCGTGTGGGTTACGTGTCTGGCTGGGGACAAA
GTGACAACTTTAAACTTACTGACCATCTGAAGTATGTCATGCTGCCTGTGGCTGACCAATACGATTGCAT
AACGCATTATGAAGGCAGCACATGCCCCAAATGGAAGGCACCGAAGAGCCCTGTAGGGGTGCAGCCCATA
CTGAACGAACACACCTTCTGTGTCGGCATGTCTAAGTACCAGGAAGACACCTGCTATGGCGATGCGGGCA
GTGCCTTTGCCGTTCACGACCTGGAGGAGGACACCTGGTACGCGGCTGGGATCCTAAGCTTTGATAAGAG
CTGTGCTGTGGCTGAGTATGGTGTGTATGTGAAGGTGACTTCCATCCAGGACTGGGTTCAGAAGACCATA
GCTGAGAACTAATGCAAGGCTGGCCGGAAGCCCTTGCCTGAAAGCAAGATTTCAGCCTGGAAGAGGGCAA
AGTGGACGGGAGTGGACAGGAGTGGATGCGATAAGATGTGGTTTGAAGCTGATGGGTGCCAGCCCTGCAT
TGCTGAGTCAATCAATAAAGAGCTTTCTTTTGACCCATTTCTGTGTTGTGTTCAGTCTTGAGTCTTTTTT
ATTTGCTCCTTTATGGTCCAGGGTAGTCAGAAGGTATAGAGTCTACTGGGAGTATGGCAGAAAACACCCT
AAACCCACTGGAAATCCCGAAGGTGATACAAACTCTTCCACCTTAGGGAATCATGCTCACTGATTGAGTG
CCTATTGAATGCTAGGTCCCAGAAAGTTAACTGTTGTCCTTGTTTTACAGACAAGGAAACAGAGACTCAG
AGATGGTAAGTGAGTTGCTTAAGGTTACATAGCTATGAAACAGGGAAGCAGAACTTTGAACCCAGGTCTG
TTTGATACAAACTCAGAGGTCCTTTCACTGCATGCTGTTGCCTCTTTAAAGCGAATTAGGAGAAAAGGCA
TGGGCCTTGGTGAGGAAGAGGCTAGCTCAAAATGGATGGGGAAAAAGTGTTTTAACACAGACAGTACTT
CAGAGTTTGGGATCTAACCTACCAGCACTCCAGAAAACAAAAGATGATGATGTAAAGGCAGGATCTCTGG
ACTTGCTGAGTCCAAATCATGGACTTGGCCCAAACATTTGCTAAGTAATTTAGGCTTCCTGGCCATTAGT
TCCTGGTCTCTTCCATGAAGGACTTGAGCCAGGGTAACGTCCCTATCAGCAATAAGCTTCTGGGACTCTG

TAAAGAGTGGGGAGGTAGAGTCCCTGCCTCAGTAGAAGCTGCTTTTCCTACTTGGAACAAACCCACCCTT
CACCAGGAATCAAAAGCAAGCTGTCTGAGCCTCCTCTCCTGATGGCAGGTCATGCTATTAAGGGTCTCCC
AGAAGAAAGCTTCTGGAGCTCTGAGAAACAGAAGCTCTTCTAACAGAACTTTTTCTTGCTATAAGCCTTC
AATACCACCAATCACCAATGTGCCTTCTTCTCATGCGAAAATACGGCCCCTCCTTTGCAGGGTGTAAAAT
GACACTGATGTCACTCTCTCTCACCAGGGTCTGGCCATCTCTGTGAAGCTCTCCTCACAGGCACCCTGCA
GATTCATCAATAAACTTCAACAGGTTAGCGCTAGTAGGCCAATAAGACCTATATTCTAGAACCTGTGAAA
GAGATAATAGCCAGAAACTGGCAAACACCAGTCAAACTCTTTAAAAAAATGCCTCTTAGAACACAGTCAC
TTACACATTTTCCTAGATCTCTTCAGCACCAAGTTTCTTGGAAGAACAAGGCACTCTGCAGTTTTCTTAA
AGAATTGTCTGATTGTATTAAGTGGCTTCTGGTAAAACAACAGTAAGAGAAAAGCTCTGGCATTCTCTTG
ATCACTCATCTTCTCTGCCTTCCTCCCCAGCACCACCTCCTGGCCTCAGCCTGTGGGCTTCTGCAAAGTC
AGGTATCAGGACTAATTAGCTAGCATCAAATTCCTGTTAATTCCAGAAGCAGGGACAAACCTGTTGGGGA
GGGACTTAATGACAAAATTAGGTATAGGTCTATACGTTAATCAAGGAAAATTCTCAACTTCCTTAAACTT
ACTTACCACCCTAGAGGAGAGCCAAATCCACTCTGATGACGTTTGGTCATAGGAAGGAAATTGGGGAAAA
TTACAAGTTTCTCCAATCCATGTAAAATTAAGAAATCCTGGGGAAGTTGTAAAGAGAAAACAGTACCCTA
TTCCCTTGAAAGGCAGAATAGGTTTATAACTTATAATCGAAGGTCTCCTTCAGGAAGGACTTCTTGAACC
CCATATGTCCTCTTACAACACTCCAATATTGCCTGTAAAAAAGTCGGATGGGTCATAGCGGCTACTGCGA
GACCTTCGAGCCATTAACCAAATAGTCCAGACCACCCACCCTGTCATTCCTAACCCTTACACCATTATCA
AGCAAGATCCCTCATGACTGCCAGTGGTTCACAGTAATAGACCTCAATGATGACTTCTGGGCTTGCCCCT
TAGCTGTGGATAGCTGGGACATATTTGCTTTTGAATGGAAGACCCTCATTCCAGTTGGAAGCAATAGTA
GTGATGGACAGTTCTACCCCAAGGGTTTACAGACTCTCCAAACCTATTAGTCAAAGTTTGGAACAAGTCC
TAGAGAATTTCCCTCTTTCATCATCCTTATGTCTACTCCAATATGTGGATGACCTGCTCATTTCCAGAGG
CACCAAAGACCAAGTAACCGCAATTTTAATTAGCTTTCTAAATTTCCTAAGGGAACAATGGTTACGGGTC
TCAAAAAGTAAACTCCAGCTTGTAGAACCTGAGGTAAAACACATGGGGCACTTAATAAGCAAAGGTAAGC
GGAAGATAGGGCCCGAACAAATGGAAGGGATCATATCCCTGCCACTGCCTGAGACAAAACAAGAACTTAG
AAAATTCTTCAGGCTAGCTGGAAATTATTGCCTGTGGATTGACTCTTATTCTTAAAAACAAAACCTCTAT
ATCTAAAACTTACCCAAGAAGGGCCTGACCCCCTTCTTTGGACCCCACAAGAAGTCCAGCAAGTTGAGGA
ACTAAAACATCTACTTATAACTGCCCCTGTCTTAGCTCTGCCATCCTTAGAACAGCCATTTCACTTTTTT
GTTAATATAAGCAATGGGGCAGCTATAGGAGTACTCACTCAAAAGCATGGGGCCATTGCCAGCCCACAGC
CTTTCTGTCAAACATTCTTGACGTGGTAACCTGTGGGTGGCCCGAATGTATCCAATCTACAGCAGCAACT
GTTTTATTGACAGAGGAAAGTAGAAAAATAACCTTTGGGGGAAGTCTCATCCTAAGCACACCCCATCAGG
TTAAAACCATTCTTAGCCAAAAAGCAGGGAGATGGCTTACAGATTCAAGAATCCTAAAATATGAAGCTAT
CCTATTAGAAAAAGGTGATTTAACCCTAACCACTGACAATGTGCTCAACCCCGCCACCTTCCTTTTTTTT
TTTTTTTTTTGAGATGGAGTTTCACTCTTATTGCCCATGCTAGAGTGCAATGGCACAATCTCGGCTTATC
GTAATCTTTGCCTCCTGGGTTCAAGCAATTCTCCTGCCTCAGCCTATTAAGTAGCTGGGATTACAGGCAT
GCTACCATGCCTGGCTAATTTTTTTGTATTTTTAGTAGAGATGGGGTTTCTCCATGTTGGTCAGGCTGGT
CTTGAACTCCTGTCCTCAGGTGATCTGCCTGCCTTGGCCTCCCAAAGTGTTGGGATTACAGGCGTGAGCC
ACTGTGCCCGGCCTACCCCGCCACCTTCCTGACGGAAATCCAAACGCTGAAGACCCCAAGCACAAATGTT
TGAACTTAATCATCAAACTAAAGTAAGGCCTGATCTAAGTGAGACCCCTTTAAAAACAGGGCACCATCTC
TTTGTAGACAGCTCCTCTCGGGTAATTGAAGAGAAAAAACATAATGGCTACTCAGTGGTTGATGGAGAAA
CTCTCACAGAAGTAGAATCAGGGAGACTTCCCAATAACTGGTCTAACCAAACATGTGAGTTGTTCGCACT
AAATCAAGCCTTAAAATAGCTGCAGAATCAAGAAGGACTATTTACACCAATTCCAAATATGCTTTTGGA
GTGGCCCATACCTTTGGGAAGATTTGGACTGAACAAGGCCTTATTACTAGCAAGGGCCAAAACCTTGTCC
ACAAAGAGTTAATCATGCAAGTATTAGAAAACCTTCAGCTACCAGAGGACATAGCCATTGTTCATGTCCC
AGGACACCATAAAGGTCTCTCTTTCAAGAGTTACTTCCCAAGAAGCACCCATTTTCCACTGAACCCCTTG
TCTCCCTTCCCCATCTGCAACCCCCATCTTCTCCCAAGCAGACAAAGAAAAATTAAAGAAAATAGGGGCT
AACAAAAACTCAGAAGAGAAACGGATACTGCCAGATAACAAAGAAATGTTGTCCAAACCCCTAATGACAG
AAATCTTAACACAACTTCACCAAGGGACCCATTGGGGTCCCCAAGCCATGTGTGATGCAGTCCTTAGAAC
CTATGGGTGTACAGGGATTTATACCCTCACCAGGCAAGTCACAGACAGTTGTATGGTATGCAGAAAAACC
AATAAACTTTAAGAAAACTGCCCTTCGGCGGAAGAAATCCTGGGCTAAGGCCATTTCCAAAGTGTCCATA
TTGACTTATACCGAAATGCCCCAAATAGGCTGCCTCAAATGTTTGTCAGTTATAGTGGACCATCTCACTC
ACTGGGTGGAAGCCATTCACCTGCCAAGTGCAACTGCCAATAATGTAGTTAAAGTACTAATAGAAGACTC
CATACCCAGATTCAGATTAATAGAGAACATTGACTCAGACAATGGAACCCATTTTACTGCACAGGTCATT
AAAGGATTAACCCAGACACTAGAGATAAAATGGGAATATCATACTCCCTGGCACCCACCCTCATCAGGAA
AAGCAGAAAGAATGAATCAAAATAAATAAATCACCTAACTAAACTAATCTTGGAAACCCGACTGCCATGA
ACTAAGTACCTCCCTGTCGCCTTACTAAGAATTCTCATGTTTGTCCTTATCATCGATAAG

SEQ ID NO: 10 (hMxA) (Accession No. NP_001171517)
MVVSEVDIAKADPAAASHPLLLNGDATVAQKNPGSVAENNLCSQYEEKVRPCIDLIDSLRALGVEQDLAL
PAIAVIGDQSSGKSSVLEALSGVALPRGSGIVTRCPLVLKLKKLVNEDKWRGKVSYQDYEIEISDASEVE
KEINKAQNAIAGEGMGISHELITLEISSRDVPDLTLIDLPGITRVAVGNQPADIGYKIKTLIKKYIQRQE

TISLVVVPSNVDIATTEALSMAQEVDPEGDRTIGILTKPDLVDKGTEDKVVDVVRNLVFHLKKGYMIVKC
RGQQEIQDQLSLSEALQREKIFFENHPYFRDLLEEGKATVPCLAEKLTSELITHICKSLPLLENQIKETH
QRITEELQKYGVDIPEDENEKMFFLIDKVNAFNQDITALMQGEETVGEEDIRLFTRLRHEFHKWSTIIEN
NFQEGHKILSRKIQKFENQYRGRELPGFVNYRTFETIVKQQIKALEEPAVDMLHTVTDMVRLAFTDVSIK
NFEEFFNLHRTAKSKIEDIRAEQEREGEKLIRLHFQMEQIVYCQDQVYRGALQKVREKELEEEKKKKSWD
FGAFQSSSATDSSMEEIFQHLMAYHQEASKRISSHIPLIIQFFMLQTYGQQLQKAMLQLLQDKDTYSWLL
KERSDTSDKRKFLKERLARLTQARRRLAQFPG

SEQ ID NO: 11 (hMxA) (Accession No. NM_001178046)
ATCTGATTCAGCAGGCCTGGGTTCGGGCCCGAGAACCTGCGTCTCCCGCGAGTTCCCGCGAGGCAAGTGC
TGCAGGTGCGGGGCCAGGAGCTAGGTTTCGTTTCTGCGCCCGGAGCCGCCCTCAGCACAGGGTCTGTGAG
TTTCATTTCTTCGCGGCGCGGGGCGGGGCTGGGCGCGGGGTGAAAGAGGCGAAGCGAGAGCGGAGGCCGC
ACTCCAGCACTGCGCAGGGACCGGAATTCTGTGGCCACACTGCGAGGAGATCGGTTCTGGGTCGGAGGCT
ACAGGAAGACTCCCACTCCCTGAAATCTGGAGTGAAGAACGCCGCCATCCAGCCACCATTCCAAGCTTAC
TTTGCAAAGAAGGAAGATGGTTGTTTCCGAAGTGGACATCGCAAAAGCTGATCCAGCTGCTGCATCCCAC
CCTCTATTACTGAATGGAGATGCTACTGTGGCCCAGAAAAATCCAGGCTCGGTGGCTGAGAACAACCTGT
GCAGCCAGTATGAGGAGAAGGTGCGCCCCTGCATCGACCTCATTGACTCCCTGCGGGCTCTAGGTGTGGA
GCAGGACCTGGCCCTGCCAGCCATCGCCGTCATCGGGGACCAGAGCTCGGGCAAGAGCTCCGTGTTGGAG
GCACTGTCAGGAGTTGCCCTTCCCAGAGGCAGCGGGATCGTGACCAGATGCCCGCTGGTGCTGAAACTGA
AGAAACTTGTGAACGAAGATAAGTGGAGAGGCAAGGTCAGTTACCAGGACTACGAGATTGAGATTTCGGA
TGCTTCAGAGGTAGAAAAGGAAATTAATAAAGCCCAGAATGCCATCGCCGGGGAAGGAATGGGAATCAGT
CATGAGCTAATCACCCTGGAGATCAGCTCCCGAGATGTCCCGGATCTGACTCTAATAGACCTTCCTGGCA
TAACCAGAGTGGCTGTGGGCAATCAGCCTGCTGACATTGGGTATAAGATCAAGACACTCATCAAGAAGTA
CATCCAGAGGCAGGAGACAATCAGCCTGGTGGTGGTCCCCAGTAATGTGGACATCGCCACCACAGAGGCT
CTCAGCATGGCCCAGGAGGTGGACCCCGAGGGAGACAGGACCATCGGAATCTTGACGAAGCCTGATCTGG
TGGACAAAGGAACTGAAGACAAGGTTGTGGACGTGGTGCGGAACCTCGTGTTCCACCTGAAGAAGGGTTA
CATGATTGTCAAGTGCCGGGGCCAGCAGGAGATCCAGGACCAGCTGAGCCTGTCCGAAGCCCTGCAGAGA
GAGAAGATCTTCTTTGAGAACCACCCATATTTCAGGGATCTGCTGGAGGAAGGAAAGGCCACGGTTCCCT
GCCTGGCAGAAAAACTTACCAGCGAGCTCATCACACATATCTGTAAATCTCTGCCCCTGTTAGAAAATCA
AATCAAGGAGACTCACCAGAGAATAACAGAGGAGCTACAAAAGTATGGTGTCGACATACCGGAAGACGAA
AATGAAAAAATGTTCTTCCTGATAGATAAAGTTAATGCCTTTAATCAGGACATCACTGCTCTCATGCAAG
GAGAGGAAACTGTAGGGGAGGAAGACATTCGGCTGTTTACCAGACTCCGACACGAGTTCCACAAATGGAG
TACAATAATTGAAAACAATTTTCAAGAAGGCCATAAAATTTTGAGTAGAAAAATCCAGAAATTTGAAAAT
CAGTATCGTGGTAGAGAGCTGCCAGGCTTTGTGAATTACAGGACATTTGAGACAATCGTGAAACAGCAAA
TCAAGGCACTGGAAGAGCCGGCTGTGGATATGCTACACACCGTGACGGATATGGTCCGGCTTGCTTTCAC
AGATGTTTCGATAAAAAATTTTGAAGAGTTTTTTAACCTCCACAGAACCGCCAAGTCCAAAATTGAAGAC
ATTAGAGCAGAACAAGAGAGAGAAGGTGAGAAGCTGATCCGCCTCCACTTCCAGATGGAACAGATTGTCT
ACTGCCAGGACCAGGTATACAGGGGTGCATTGCAGAAGGTCAGAGAGAAGGAGCTGGAAGAAGAAAAGAA
GAAGAAATCCTGGGATTTTGGGGCTTTCCAGTCCAGCTCGGCAACAGACTCTTCCATGGAGGAGATCTTT
CAGCACCTGATGGCCTATCACCAGGAGGCCAGCAAGCGCATCTCCAGCCACATCCCTTTGATCATCCAGT
TCTTCATGCTCCAGACGTACGGCCAGCAGCTTCAGAAGGCCATGCTGCAGCTCCTGCAGGACAAGGACAC
CTACAGCTGGCTCCTGAAGGAGCGGAGCGACACCAGCGACAAGCGGAAGTTCCTGAAGGAGCGGCTTGCA
CGGCTGACGCAGGCTCGGCGCCGGCTTGCCCAGTTCCCCGGTTAACCACACTCTGTCCAGCCCCGTAGAC
GTGCACGCACACTGTCTGCCCCCGTTCCCGGGTAGCCACTGGACTGACGACTTGAGTGCTCAGTAGTCAG
ACTGGATAGTCCGTCTCTGCTTATCCGTTAGCCGTGGTGATTTAGCAGGAAGCTGTGAGAGCAGTTTGGT
TTCTAGCATGAAGACAGAGCCCCACCCTCAGATGCACATGAGCTGGCGGGATTGAAGGATGCTGTCTTCG
TACTGGGAAAGGGATTTTCAGCCCTCAGAATCGCTCCACCTTGCAGCTCTCCCCTTCTCTGTATTCCTAG
AAACTGACACATGCTGAACATCACAGCTTATTTCCTCATTTTTATAATGTCCCTTCACAAACCCAGTGTT
TTAGGAGCATGAGTGCCGTGTGTGTGCGTCCTGTCGGAGCCCTGTCTCCTCTCTCTGTAATAAACTCATT
TCTAGCAGACAAAAAAAAAAAAAAAAAA

SEQ ID NO: 12 (hMxA) (Accession No. NG_027788)
ATCATTCTTCACAGAACTAGAAAAAACATTCTTAACATTCATATGGAACCAAAAAAGGAGCCCGCATAGC
CAAAGCAAAACTAAGCAAAAAGAATAAACCTGGAGGCATCATATTACCCAACTTCAAACTATACTACAAG
GCTATAGTCCCCAAAACAGCATGATACTGATATAAAAATAGGCACATAGACCAATGGAACAGAATACATA
ACCCAGAAATAAAGCCAAATACTTATAGCTGACTGAACTTTGACAAAGCAAACAAAAGCATAAAGTGGGG
AAAGGACACCCTATTCAACACATGGTGCTGGGATAATTGGCAAGCTACATGTAGAAGAATGAAACTGGAT
CCTCATATCTTGCCTTATACAAAAGTCAACTCAAGATGGATCAAAGACTTAAATCTAGGACCTAAAACCA
TAAAAATTCTAGAAGATAACATTGGAAAAACCCTTGTAGACATTGGCTTAGGCAAAGAGTTCATGACCAA
GAACCCAAAAGCAAATGCAACAAAAATGAAGACAAATGGATCGGACTTAATTAAGCTGAAAAGCTTCTAC

ACAGCAAAAGAAATAATTAGCAGAGTAAATAGACAACATACAGAGTGGGAGAAAATTTTGCAAACTATGC
AGCCAACGAAGGGCTAATATCCAGAATCTACAAGGAACTCAAACAAATCAGCAAGAAGAAAACAAATACT
CCCTCACAAAGTGGGCTAAGGATGTGAATAGACAATTCTCAAAAGAAGATATACAAATGGCCAACATATA
AAAAAAAATCAACATCACTAATTATTAGGAAAATGCAAATCAAAACCACAATGGGATACCACCTTACTCC
TGCAAGAAGGGCCACAATCAAGAAATCAAAAAATAATAGATGTTGGCATTAATGTGGTGAAAGGGATCAC
TTTTACACTGCTGGCAGGAATGTAAACTAGTACAACCAATGGAAAACAGTAGAGTTTCCTTAAATAACTG
AAAGTAGATCTACCATTTGATCCAACAATCCCACTAGTGGGTATTTAGCCAGTGGAAAATAAGTCATTAT
ATGAAAAAGACACTTGCACACACATGTTTATAGCAGCACAATTCCCCAAGTGCCCACCAATCAACAAGTG
GACAAAATGTTATATATATATGTGTATATATATGTATATATATATAATGTGATATATATGTATATATATG
TATATATATATAATGTGATATATATGTATATATATAATGTGATATATATGTATATATAATAATGTGATAT
ATATGTGTATATATAATGTGATATATATGTATATATATATAATGTGATATATATGTATATATATATAATG
TGATATATATGTGTGTGTGTATATATATAATGTGATATATATATGTGAATACTACTACTACTACTACTAC
TACTACTAATACTCAGCCATAAAAAGGAATGAAATAATGGCATTCACAGCAACCTGGATGGAGTTGGAGA
CCATTATTCTAAGTGAAGTAACTCGGGAATAAAAACCAAACATTGTATATTCTCACTCATAAGTGAGATT
ATACAAGCTATGAGGATGCAAAGTCATAAGAATGATACAATAAACATTGGGAACTCAGGGGAAGGGTGA
GGGTGGGGTGAGGGATAAAAGACTAGACATCGGGTACAGTATACACTGCTTGGGTGATGGGTGCACCAAA
ATCCCAGAAATCACGATTAAAGAACTTGTCAATGTAACCAAACACCACCTGTTCCTCAAAAACTATTGAA
ATAAAAAATAAATACATAAAAGAAGTTGAGGTTAAATTAATCTAAGTAGGGAGTGCATTTGGGCCAAGCT
TGAGGATTGCAACGTTGGAGCACAGATTTAAGTTGCCCTGAATCTACACTCTGATAAGTGGCATTTATAA
GTGGATTTTTAAAGGCAAAAGGGGAGACAGGGAGAGGGCTGATACAAAGCTGTTTGTCAGGAATTCTTA
TTGGTTTACAGAAATAACATTGATAAGTGCTTATTAGTCCATTTTCATGCTGCTGATAAAGACATACCTC
AGACTGGGCAATTTACAAAAGAAAGAGGTTTATTGGACCTATAGTTCCACATGGCTGAGGAGGCCTCACA
ATCATGGTGGAAGGCAAGGAGGAGCAAGTCACATCTTACATGGATGACAGCAGGAAAAAAGAGAGAGCTT
GTGCAGAGAAACTCCCTTTTTAAAACCATCAGATCTTGTGAGACCCGTTCAGGATCATGAGAACAGCAC
AGGAAAGACCCACCCCCAAGATTCAATCATCTCCCACCGGGTCTCTCCCACAACATGAGTGAATTATGGG
AGCTACAGGATGAGATTTGGGTGAGGACACAGAGCCAAACCATATCAGAGTGATTGACTATACATTGTTA
AGCTACAGGGTGTGGATTACAGCGTCCAGTGTGGCATTATTAGGTTAATCCATAGCTATTAATACCTGTG
GCAATAGTAAGAGGTTTCAAGAGATGAACACACAGCTGAAAGTGGGGAGTAGGGAGTGGTTGCTGTCTTA
TTTTAATGTCTCTCTGGGCCTGATAATTTTAAAAGCCTTGCATTCTTCGGATACAAGTTCCTTTCTTTTT
TTCAAGAACAACAGAGAAAGGCTGCCTGGCTACCTTTACCTGCAGCACCTCTGATAGTGGCTGCTTGGCA
GCTGGTTAGGTAAACCCAATTTCTACTGGATAAACTTCATGGACATTCACCCATGTTAGTACTGGGGAAT
GCTCTGAAATAGCAAACTTGTCCATACAGCTCCCTTGCTGCTCAGAGGGTCTGGAGAGGAACAGCAGAGG
CCCAGCCCCAAATACTTCTTGTACCGGCTTCTATTATGAGAGGTGATGGCACCTTTAGGCACTTATATTT
CAGGTAGAAGGAGCAGGAATAAAGAAGAGAGGGCCACAATCTGAAATATCAGTTCTGGATCTCCCTGGGG
GCAAGACACTGGCCCTTAGCTGAGCTATTCTGTCATCACTGTGAGCCCTCATGCTCGACCTCACCGGAGC
ACCCCTCGATCATGTGCTGAATGCCTAGCACGCGCCTGGCACTGTGGGGTACAGAAATCTAGAGCACAGA
GCTGAGTTTTCAGGGCTTAGCTGTCCTCACATCTGTGACACAAACAAGGATCAAGGAAAGTTTTGTTTTT
GTTATTTTTATTTTTTTTTAAGTTGGGGCCTTACTCTGTAGCCCAAGCTAGAGTGCAGTGGTGCAAACAT
AGCTCATTGCACCCTCGAACTCCTGGGCTCAAGTCATCCTCCCACCTTGGCCTCCAGAGTAGCTGGAAGC
CCAGCTCAAGGAAAGCATTAACATGTTCTACTTTTCACCATAACTCCTGTGTATTGGGAAATGGAAAATG
AGCTTTTGAAGTGGCCTATTCGCTTCCTCCATGAGTACTTCCTGAACACTTTCCTCTTGGCCACTGCTCC
CAAGACCCCCTCAAAGTCTCTCTCCTGGACTTCCAGCTTCTGGAATGAGCCCCAAGAGTGGCACTTAGCT
CCAGCTCCTAGTAGCTCACTCCTTCCTTGCTCACCTGTGCCCCCCACCTGTCTCCTTAGGGGCAGTGGCT
TTGTCTTACTCATTTGCATATCACAAATGTCCAGCATAGAAGCCAGCATGGGGCCCAGTAAGTGTTTGTG
GAGAAAGAAAGGGAAGAGGTGAAGAAGGGAGGAGACGGAGGGAGAAAAGGAGGGCAAGAGAGGGGATGTC
ATGTCATGTCATGTCATGTCATGTCAGTTCTCCCTTCCTAGATCTGGACAAGGGAAAGAAAAGCAAGCAA
GCCTGACCGCCTCAGTGCAGACCCTCTAAGATGCAAGCCTTCCCCAGGTAAGACAGTTCCGCAGGGTGCT
TCTGTGGGCTGGCTTTCTGAGCAGCCATCTCAAAGTATGCGAAAGAAATAGATTTTGGGCCGGGCACGGT
GGCTCATGCCTGTAATCCCAGCACTTTAGGAGGCCGAGGCGTGTGGATCACCTGAAGTCAGGAGTTCAAG
ACCAGCCTGGCCAACATGGTGAGACCTTGTATCTACTAAAAATACAAAAACTAGCCGGGCTTGGTGGCGC
ATGCCTGTAGTCCCAGCTACTCAGGAGGCTGAGGCAGGAGAATCGCTTGAACCCAGGAGGTAAATGTTGC
AGTAAGCCGAGATTGCCCCACTGCACTCCAGCCTGGATGACAAGAGTGAGACTCCATCTCAAAAAAAAAA
AAAAAAAAAGATTTTAGGGGTAAAATATTTTGCTTTCCTTCAGTGGCTGGGCCACCTGTACACAATGCCT
GAACCCAGGTCCTGCAGCCCCCAGCTTGGCTCTCCAAAGCTCACCAGTATCAAAGGATTTCAGCTTCTGC
TCCTCTCCGCAGGGCTCTCTGGTGGGCCCCAGACAGAGATGAAGGAAAAGTCTGGCCTTGACCTTGAGGA
CCAAAAGCGACCACGTCAGACGCCAGCAGTCTCGTGCCCAGTTGCCAAGGGATCACCTGAGGAAGGCCCT
TAAAATACAGGTGGTAATGCAGGGGTCTTGGGATGGCCCCGGACCGTTCATTTCTAATAATTTCCCAGGT
GAAGCCAATGCTGCTGTTCCAGGAACCACCCTTTGAGAAGCACGGGTCTATACCACACGCACAGAAGAGG
AAACGAAACGAGGCAAAGGAAACCAGTCCCTTTACTCGCGCCCAGGCAGGACAAAGCGGGCAAGGGTGGG
GGCGGGGGCGCGGGCGCGGGCGCGGGCAGGCGGACGGACTTGCAGGAAATGCAGCCGGGAGGATGGGCAG

GTTGTGGGATGCTCCGCCGTTCCGCGACCACCCGCCTCCCCGGCCCAGCAGAACCGGGTCCAGGCTGGGG
GAGGTGCTAGCGGTGTATGTGAGTAACACTGCCCCCTCGTCGTGGCACCGCCCCCTTCTTCCCTGCCCCC
TTCCCCGCCCCCTGGATTCTGAAGTCTGAAGTCATGCAATTTGACTTTTCCTTTCTGGAAACCAGCGAGC
AGAAATGAAACCGAAACTGAATTGTCCGGGAAATTCGCGGTGGGGGCGGAGAGCGCAGGGAGAAGTAAGC
CCAGTGCAGGATCCTGAGGCCCGTGTTTGCAGGACCAGGGCCGGCCTTCCGATTCCCCATTCATTCCAGA
AGCACCGAACCACGCTGTGCCCGGATCCCAAGTGCAGCGGCACCCAGCGTGGGCCTGGGGTTGCCGGTTG
ACCCGGTCCTCAGCCTGGTAGCAGAGGCCAGGCCAGTGCCACAAGGTCAGGCTCCACTGGGCCCTGCCCG
CCCAGGAGCCTGTGGTGTCAGGCACCTGAAGGGGTGGGTTGCCCCTCCACACCTGTGGGTGTTTCTCGTT
AGGTGGAAGGAGAGACTTGGAAAAGAAAAAGACACAGAGACAAAGTATAGAGAAAGAAATAAGGGGACCC
AGGGAACCAGCGTTCAGCATATGGAGGATCCCGCCAGCCTCTGAGTTCCCTTAGTATTTATTGATCATTT
GTGGGTGTTTCTCCGAGAGGGGGATGTGTCAGGGTCACAAGACAATAGTGGGGAGAGAGTCAGCAGACAA
ACACGTGAACAAAGGTCTTTGCATCATAGACAGGGTAAAGAATCAAGTGCTGTGCTTTTAGATATGCATA
CACATAAACGTCTCAATGCTTTACAAAGCAGTATTGCTGCCTGCATGTCCCACCTCCAGCCTTAAAGTGG
TTTTTCCCTATCTCAGTAGATGGAACGTACAATCGGGTTTTATACCGAGACATTCCATTGCCCAGGGACG
GGCAGGAGACAGATGCCTTCCTCTTGTCTCAACTGCAAGAGGCATGCCTTCCTCTTATATTAATCCTCCT
CAGCACAGACCCTTTATGGGTGTCGGACTGGGGGACGGTCAGGTCTTTCCCTTCCCACGAGGCCGTATTT
CAGACTATCACATGGGGAGAAACCTTGGACAATACCTGGCTTTCCTAGGCAGAGGTCCCTGCGGCCTTCC
GCAGTGTTTGTGTCCCTGGGTACTTGAGATTAGGGCGTGGTGATGACTCTTAAGGAGCATGCTGCCTTCA
AGCATCTGTTTAACAAAGCACATCTTGCACCGCCCTTAATCCATTTAACCCTGAGTGGACACAGCACATG
TTTCAGAGAGCATGGGGTTGGGGGTAAGGTTATAGATTAACAGCATCCCAAGGCAGAAGAATTTTTCTTA
GTACAGAACAAAATGGAGTCTCCTATGTCTACTTCTTTCTACACAGACACAGTAACAATCTGATCTCTCT
TGCTTTTCCCCACAGGCACCTAAGTCCACCTGGGCCTGGAGCAGGACAGGTTGCAAAAGAAAATATCTCG
GGACCCCCAAACTCCTTATGCTAAGGGAAACATCGAGCCTGGGAACTGAGCCATCAACGCTGCCATTCTT
TTTCCCAAACAGAACCCTGTTGTCAGAGGTACACCCAGAGCAACTCCACACCGGGTGCATGCCACAGCAA
CTCCATCTTAAATAGGAGCTGGTAAAACGAGGCTGATACCTACTGGGCTGCATTCCCAGACGGGTAAGGC
ATTCTAAGTCACAGGGTGAGATAGGAGGTCAGCACAAGGTCATAAAGGTCATAAAGACCTTGCTCATAAA
ACAGGTTGCAATAAAGAAGCTGGTTAAAACTCACCACAACCAAGATGGCCACGAGAGTGACCTGTGGTTG
TCCTCATTTTTACACTCCCACCAGCGCATGACAGTTTACAAATGCCATGGCAATGTCAGGAAGTTACCCT
ACATGTATGTCTAAAACGGGGAGGCATGAATAATCCACCCCGTGTTTAGCATATCATCAAGAATAACCAC
AAAAATGGGCAACCAGCAGCCCTCAGGGTTGCTCTGTGGAGTAGCCATTCTCTTACTCCTTCACTTCAGG
CAGGGGAGATGAATTTCCCTTTGAGCCTGTGGACAGAGGGCAGCCTGGCAGAGGGACAGGCATCAACAAA
GCCTCTGAGTGTGACCTGGGTCCTGAGTTCAGGGATGGCAATGGGGTAACTGAGACTGGATCAGAAGTTG
CCTGGAAGAAGAGAGGGCCATTATCAGAGTATAAGAGTGTGAAACTTATCTGGTAGGAAAAAAAAAAGAG
TAACTGCTGTTCGTTTAATAGAGCACACATCCTCTGCAATTCAGGTGGGAGGTTGTGAGCTTCTTAAGTA
CAGAAGCTGCCTCTTGATTTCAGCATAGCGAGGAGGTGCTGAAGAGCGCAGGTTTGGAGAATGATCACCT
GGATTGGAACCATAGCTCTACCAATATGGAACCCAGCTCCTTAGGCCTCGGTCTTCTCATGGAGAACATG
GTGTGATAATCCTACTCCTCTGGGAGGGTGGCTGTTAAGGTGAGTGATTGCACACGAGTCCCTCTAGCTG
ATGTTACCGAGCGGTCCTTGTTTTTAGAGCTCCCAAGAGGGTGGCGGGCCACTTCCAAGATGGCGGCAAG
CCTCTTGTTCTCTGACCTGGGGTTCTTGGCCTCACAGATTCCAAGGAATGGAATCTTGGGGCATGTGGTG
AGTGTTCTCATTAGGACGAACCCAGGCACTTAGACATGCGGGAACAATGGCGAGCCTTTAGCCCGAATGG
GAGCGGCAATGGGTGCCTCGCTGGATCAGGAGCACAGCGGACACACTGCCGGATCCAGAGGGGTGGACAT
CAGTGGTGGGTCTGTGATGGCGGCAAACAACAGCAGACAGCAAATGGCAGTGGTGGATGGCAAGCGAAAG
CTCAGCTCCAGCCATAACAAACACGGACCAGAAGAGTGTGCAGTTGCAAGATTTAACAGAGTGAAAACAG
ACGTCCCATACAAAGGGAGGGAACCCAAAGGGGGTTGCCATTGCTGGGTCGAATGCCTGGGTTTCTGTCT
CAATCACTGTCCCTCCCCCTGTGCTCTCAGGCAATAGATGATTGGCTATTTCTTTACTTCCTGTTTTTGC
CTAATTAGTATTTTAGTGAGCTCTCTTTACTACCTGATTGGTCGGGTGTGAGCTAAGTTGCAAGCCCTGT
GTTTAAAGGTGGATGTGGTCACCTTCCCAGCTAGGCTTAGGGATTCTTAGTCGGCCTGGGACATCCAGCT
CGTCCCGTCTCTCACTGACACCTAACACATCCTACATGCTCAGTAGACAGGAGCTATTATTGTATGACTG
ACTGCTGAATTACACCCCAAAACAACCACCTATACTCCCCAAGGCCCTGCCATATCATATGCATATTAGA
GGCAATGAAGCACCAAAATCTAAGGCCAGGGAAGGACTGCGGTGGACAAGGGAAGTGCCTAGGGCGTTAA
AACTTAAGGAGGCATTGCACCAACTGGGAGTGAGCTGTGTGTGCTGTGTTTCCTGGCAGGGCGCCTCAC
TGCCCTTCCGAGTCCTGCCCTGGCTGAGGTTGTGCCTCTATCTGGTTATCAGTTGCCTGCAGCTCTTTCT
GATTTTGTTCGTGAACAAGGGCCCTTAGGGAAGCCTCCCGCTGTTGGAAACAGCAGCAGCATCACCCAGA
GTGAAGTCTCTGTTCACATGAGTGCCAGCTCTGCTCACACCTACCCTGCGTTCCCAAGGCACTGTTCCTG
CTTCAGAAAGCATCAGCCCAGCCTGCTCACAGTCCCCCAGGATGTGCTGTTGCCTGGGTTCTTGACTAGG
AAGTAAAAGGCCCAGAGGGGTTGAGTGTGTGTGTGTGTGTGTGTGTGTGTGTGTGTGTGTTAAAGTGAGG
TCATTGCTAATTCCTGGGAGAACTTGTGTTCCCCTTGGATGAACCCCTTGGATGAAGAACCCCTTGGATG
GGGTTGCCCAGCCCTAGGAGTGGATCCTGCAAAGCAAAATAGATTTCAGCACAGGGAGCTGGCTGGGGGG
CTCCTCCCCCTCTTGTAGGTGCAGCAGTATTGAATTTGATTCCATCCAGGTTTCCCCTGACCCCAGGACC
TAAAGCTGGGGCAGGTTCTGGGCCTTGAGGTTGGTTGGGGGTGACCCTTCCTAATCATGGAAGTCTTTAG

CGGGGGCTTGTAAGGGACTGTGAGCTGAGCTAAGGAGAATGGAGGTGGGGTGCCAACCGCTCCCAAAGGG
AAATGCCATCTCCCACTCACAGCCAGTTAGCCGAGAATGGGAGCTCCCAAAGGGAGGACCACCCATGGGT
CTGCTTGACTCAGCCCTCCCCAACCCCTTTCACCTTTGCAGTAAAACTCTAGCCAAGGAAGACAAAGAGA
CCTTTGGAGACCAAAACAGAACTTTTAATTCGGGCCAACAGCAGGCTCATGCCCAAAATGGCTGCCAACC
CCAACAAAGAAAGCAGCTAGCTTATATGTCGTTTGAGATGGGAAAAACAAGGTAGGATACAGGTTTCAGA
CAAAGACAGTAAATTACTTAACCCGTGACAATTCTGAGGAAACTGGCAATTCAGTTATATTGACTAGTCA
TCCTCCAAGCTGGACTAGGGTTGGAGGCTGGGGTCCCGAGGCAGGTGATAAGCTTTTGAGATAAGCTTGC
ATCTGCAACTTGTTACAATGCTGGGAGGGGCTGCTTAAAATTTTAGCCTATGTGTTACTTCTAAATAGCT
TATACTAAATGTTAACTGTTTTCATGTGCGTCCATGTGAAGAGACCACCAAACAGGCTTTGTGTGAGCAA
CATGGCTGTGTATTTCACCTGGGTGCAGGCGGGCTGAGTCCGAAAAGAGAGTCAGCGAAGGGAGATAGGG
GTGGGGCCGTTTTATAGGATTTGGGAAGGTAATGGAAAATTACAGTCAAAGGGGGTTGTTCTCTGGTGGG
CAGGGGTGAATCTCACAAAGTACATTCTCAAGGGTGGGGAGAATTACAAATAACCTTCTTAAGGGTGGGG
AAGATTACAAAGTACATTGATCAGTTAGGGTGGGGCAGGAACAAATCACAATGGTGGAATGTCATCAGTT
AAGGCTGTTTTTACTTCTTTTGTGGATCTTCAGTTACTTTAGGCCATCTGGATGTATACCTGCAAGTCAC
AGGGGATGCGATGGCCTGGCCTGGGATGCGATGGCCTGGCCTGACAACTATTACCTATGTTATGTTTATT
ATTTTAAGCTTTATTATTACTATTTTATTTATTTTATTTTATTTTCCTTCCACACACCCGTTTCCACCCT
GGAGAGGCCAGATGAGCCAGACTCCAGGGAGGCCTAGAAGTGGGCAAGGGGAAACGGAAAGGAGGAAGA
TGGTATGGGTGTGCCTGGTTAGGGGTGGGAGTGCTGGACGGAGTTCGGGACAAGAGGGGCTCTGCAGCCA
TTGGCACACAATGCCTGGGAGTCCCTGCTGGTGCTGGGATCATCCCAGTGAGCCCTGGGAGGGAACTGAA
GACCCCCAATTACCAATGCATCTGTTTTCAAAACCGACGGGGGGAAGGACATGCCTAGGTTCAAGGATAC
GTGCAGGCTTGGATGACTCCGGGCCATTAGGGAGCCTCCGGAGCACCTTGATCCTCAGACGGGCCTGATG
AAACGAGCATCTGATTCAGCAGGCCTGGGTTCGGGCCCGAGAACCTGCGTCTCCCGCGAGTTCCCGCGAG
GCAAGTGCTGCAGGTGCGGGGCCAGGAGCTAGGTTTCGTTTCTGCGCCCGGAGCCGCCCTCAGCACAGGG
TCTGTGAGTTTCATTTCTTCGCGGCGCGGGGCGGGGCTGGGCGCGGGGTGAAAGAGGCGAAGCGAGAGCG
GAGGCCGCACTCCAGCACTGCGCAGGGACCGGTGAGTGTCGCTTCTGGGGGCAGCGCCCAGTAACCGCGC
TAGGAGCGCGGAGAAGGGCATTGGGAGAGCGGCGTTCGTGGCGGAGACTAGCGCTCCGGAGCACGGGCAC
GACGGGGGCACCTTCTCGGCTGCTAGTAACTAACAATAATAATAATCATAATCATAGCAAGGGCGCTGAT
GGGCGGGCTCGGAGCACGCCTGATTCTGGTTCCCACCAGGCTGCCCAGGCTCCTGATGACGCATCAGAAA
CATCCCCCTAACCCGCGGCCTTCCTGCAGGAGAGGTTGGGAAGGGGTGGGGGACGGGGCTCGGGGGAGGT
CTCCGAGGGACTCTAGTAAGCGGGGAAGGGCGCCGGGAAAGTTTCAGATCCACGGCTGCGCGGGCCACGA
GCCCACCCGAACGCCGACCACTGCTTTCCGTCGACTTCTATTTCCTGGGAACGCGCGAAAGCAAACCCAA
GTCAGACTGCGGAGGTCGCTGGGGAGGGAAGGTTCAAGGAGTTCTCGCCGATCCTGCTGAATAAAGGGGG
TTCCGAGCTGGGCCGAGATGGGGCATGCGCGGGAAGACCCCTGCCCGCTGTTCCCCCCACCGCCCCAGT
GGATGCCATGCCTGGGGCCTCCCCGGCGCGTGGGGCTGACGCACCCTCGGGGTCCATCGTAGTTGGCCGG
GATCGTGGAGTGGGTGCGGTGGACGAAGGGAGGCAGGACAGTCCCGGGGGTGGCAGAAGGAGCCCGGGCA
CAGCTGAGACCTGCGCTCCCATCCCACCAACACTCACAGCAGGTGCTGCCGAGCTGGGCAATTGGGATGG
CCCAAGTTATTTGGTTAAATTTTAAATCACGTTTGTTACTGGGAAGTAGAGTCCAGTGATGCTAACCGCG
CCTCTACCTCCACCACCGGTGTCAGTCCCAAAGGGCTCCTAAAATGGCTGTGTCATCTTTCAGCCTTGGA
CCGCAGTTGCCGGCCAGGAATCCCAGTGTCACGGTGGACACGCCTCCCTCGCGCCCTTGCCGCCCACCTG
CTCACCCAGCTCAGGGGCTTTGGTAGGTAGCAGTGCATTTGGTCTAAAGGGCAAGATGTTCTCTCTTTTA
TTCATAACAAATTTAAATACCAGCAGGGTTTGGGGGGAAAAACGCTTTCAGAAGAAAAGGTGAATGTCAG
TCCTGCAAGAGTTAGTTTTAAAACTAGACTGAATTGGCACATGTATACCTATGTAACAAACCTGCACGTT
CTGCACATGTACCCCAGAACTTAAAAGCTTATAAAAAAAGAAAAAACTAGACTGGATTATGTTGGGAAAG
TGTAGCCTCTTCCATCTTAGGCATTTCCTAGAACGTAGGCAGTAGGTGGTCCTTATTAGGAGTTTTGGGA
GAGGAAGGGGGCTGAATCCTACCTCCCATCCCTGCTCCTCTATGGGGTCTGAGCTGAGGAAGCTTCACCA
CAAGGAGAGAACCCCCTGACAACCCTGGATGCCACCTTTACCCTCACTGCAGGAATTCTGTGGCCACACT
GCGAGGAGATCGGTTCTGGGTCGGAGGCTACAGGAAGACTCCCACTCCCTGAAATCTGGAGTGAAGAACG
CCGCCATCCAGCCACCATTCCAAGGTAAGGCAGAAATGAAGTGGGCCGTTGGGTTCTTTCTTTTCTTTCT
TTCTTTTTTTTGAGACAAGGTCTCACTCTGTCGCCCAGGCTGGAGTGCAGTGGCGCCATCTCAGCTCACT
GCAACCCCCGCCTCCCAGGTTCAAGCGATTCTGGTGCCTCAGTCTCCTGAGTAGCTGGGATTACAGGCAC
ACATCACCACACCTGGCTAATTTGTATGTGTTTAGTAGAGACAGCATTTCACCATATTGGCCAGGCTGGT
TTCAAACTCCTGGCCTCAAATGATCCACCCGCCTTGGCTTCCCAAAGTACTGGGATTACAGGCACGAGCC
ACTGCACCCAGTCAGGTTCATTTTAGTTGTTATGTTAACCAGGTTTCCTGCACCTGTGCGCTAACTTTCA
CTTTCCCAAAAGGTTTCAGGGTGACCCAGCAGGCAATGAGTGATTCTCAAATTCAGGATTTATTGTGAGA
GATTCACACACACAATTGAGCAGACATTCACAGTACAATGATTAAAGGGAGTGATAGGGTAAGGACCCAC
AGTGGAGGCTCTGGAGGCCAGCCCACTGACAGCCACTCCAGGGAGTCCAGAAGTCCCGCTCTAGTGCTGG
GTGGTGGAGGGAAATCTGTTCCTCCAGGGACCTCGTCCTCGGCTGCCCAGCTGCCAAAGTCAGGAATAAG
CTTTCAGAAATCTCACTGCCAAGATTCCGAAAACGCTTCAGACATTGCTAGTCCCTTGTCGCTTTTGCGA
TCCTCCACAGGTGTGCGTGCCACTGGGTCCTTATTCACTGGGGTCTCTGGTGGCATTGGGCCACAGCAAG
TGTTCCCTCATCCCCTTAGTCTACCACACACATGCTTACCACTTTGAAGAAAAACCCCTTTACTATGAGC

GAAAGTGAGAAACACGTATGTTTATTGTTTCTAAAGAAAGAAACTTAATATGGGCTTAATGCTACCTAGT
GAGTGCCTCCATTTTGAGACATTAGGGTCACAAGTCATTATTATATATCATGGGCACAAACCTGCCCTGG
GCAGGGACGGAAGGAAGCCCCTGCACAGGGGCAGTTGCTCAGGATGTGAGAAGAGCCTGGTGCATAACCC
CATCCATGCCCACCTAACATCTCAGGCTCTGACCAGTGGGGCTGTGCAGTAGCGAGTGGATGGAGGGCTG
GAACCCTGCAGCCTCCTCTCCAAACACAGGGTGCAGCCAAGACATTTTAGGAGCAATTTGGGATGGAGAG
CTAGGAGTCGCCACCTCTTGGCTCTTCCAAGGCCGGAACTGGTGCCTGCACTCAGTTCAGTTTGAAGACT
GCAGCTGGATGCCAAGTTCCATGGAGGAGTAAGAAACCGGTTTGAACTCCCGAGATTGCCCTGCCCCTGA
AATCCAAACTGATGTTCCGAATGATCAGGGAAAGGTACAAACGTTTATGGTTTACAGACAAAACCCATAA
GGTTTAGCTTTCAGAGAATCTCATTTTATGAAGCAAATTAGGGAAGGGAATCTACTCACCAAGTCCTGTT
TCAGCTGATTGAGTGGAACCTGTGGTCATGTGGTACAAGTCCTGGTCTCAATGATGCTCCTTATCTGGCT
GCAGAAAGGCCAACTGAGGCAACCATAGCCCAGAAGACTGGTACTCCTGAGAGGCAGATGAAGTGGTGGT
CTTTGATATCGAGCCTGGGATGCCCTGGGCACATGAGGTATTTCCAAAGGCATGGGAGTTTTAGGGAATA
AATTCCCAGATTGTCAGACTCCATAAGTACCGTTTACAATGGATTACCTTTTATAACCATCCCAATCCTA
CCTGACAAAAGAGGTGGGCAGATTACGAGGTCAGGAAATTGAGACCATCCTGGCTAACACTGTGAGACCC
CATCTCTACTAAATAAATACAAAAAATTAGCCGGGTCTGGTGGCGTGCACCTGTAGTCCCAGCTACTTGG
GAGCCTGAGGCAGGATAATCGCTTGAACCCGGGAGGTGGAGGTTGCAGTGAGCCAAGATTGCGCCACTGC
TCTCCAGCCTGGTGACAAAGCGAGACTGTCTCAAAAAAAAAAAAAAAAAGAAAGAAATACATCCCTTTCT
TCCCTTCCAAATCGAGCAAGGATGCCTGCCCTGGAAGTGTATAAACCCGGGGAAGGGAGACAGAAAAGGA
TAGTTTTAAGTATTGGTGTTGGGGACGTGTTCTTTAGCCAAGGCAGCATGAACCCATGGCAGCACTTCCC
AACCTTCCTGACATGGGCGTTTCTGTGAACTCCAGTGTGATGGAGAAATGGCATTGGCTCAGGTGTGCAG
CTAGATATGTTACAGAGCAGGGTGACAGGCAGGGGTGATGAGTTTTGTTTTAACAACCTGTCCCTTCAAC
CCTCATGGTACTGACAAAGATCACATGGCTCTCGGGGGAGATTCCTGCGAGGGGAAGCAAGGAGAGCATC
CTTACATATTATTGATCCAGGCAGCAGATTTGCAGCAAAGCTCTGTGCTTTATTCATCTGTTAAAATAGT
TAAAATAGTCAAAACATAGGAAAAGGATTCTGGGAAGTCAGAATCGGCTTCAGAGCACACCCCTCCTGCA
CTTGCCCGGTTCTCAGACTTGGGAATGGGACTGGGTGGGTGGGTACTCTCGGGTGTTCCGCGGGTTTGGG
TCTTACTTGTACACTTTGCTTGATTTCAAGGAGGTGCAGGAGAACAGCTCTGTGATACCATTTAACTTGT
TGACATTACTTTTATTTGAAGGAACGTATATTAGAGGTAAGTTGGTGCATGCTATTTTCTGTAACATTTA
TTTTGAGTCATAGGAGAAAGATTTTCAGTTACTTTTATCCAAGATTATTAGACACTGTAAAATTTCATAT
TTAGGCACTTGTCCTACAACATTTTAAAAATGAATTTCAAATACATACGTGTGTATTTGTAATGCAGACA
AGTATAAGGCAGTCAGTTACATGCTTTCAAGAGTAAAATGAATGACATTTCATTTCCCCCATTTGTGGGA
GTAAAAGAATGACAATATGAAATTGATGATCAAAAGAAAGAGCATAAAAGATTTAGAGCTCACGTGTTTT
TTAAACTAAAGGTTTGGGTATCAAATTACCGTAATATTTGGATTCTCTTGGCTACATTGGAAACAGTTCT
ATAACAATTTTATTTTTAAATGTAAAGTTTTTGTTTGTTGTTGTTTTTAAGACGGGGTCTCGCTCTGTCG
CCCAGGCTGGAGTGCAGTGGTGCGATCTCGGCTCACTGCAACCTTCACCTCCCAGGTTCAAGTGATTCTC
CTGCCTCAGCCTCCCAAGTAGCTGGGACTACAAACACACACCACCACTTCCAGCTAATTTTTGTATATTT
AGTAGAGATGGGGTTTCACTATGTTGGCCAAGCTCGCTTCGAACTCCTGACCTCAGGTGATCCACTCACC
TTGGTCTCCCAAAGTGCTGGGTGGGATACAGGCGTGAGCTACTGTGCCCAGCCTTTAAATGTAAACTTTT
TAATTGTATTACAACTGCATCAGAAGTTGTATACTTTGCAACTATTCAAATTTATACTGAAAACGTTTTT
GAAGTTCAACCTAAAATTATGACAGGAGATAGTTTTAGAAAATATTTTGGGGAACAGAGGCATATTCTAT
TTTTTTTTTTGAGACAGAGTCTTGCTCTGTTGCCCAGACTGGGGTGCAGTGGCGTGATCTCAGCTGACT
GCATCCTCTGCCTGCCAGGTTCAAGCAATTCTTTGCCTCAGCCTCCTGAGTAGTTGGGATTACAGGTGCC
CGCCACCATGCCTGGCTAATTTTTTTGTATTTTTAGTAGAGACAAGTTTTACCATCTTGGCCAGGGTGGT
ATTGAACTCCTGACCTCATGATCTACCTGCCTCAGCCTCCCAAAGTGCTGAGATTACAGGCGTGAGCCCC
GGGGCCCGGCCTGGAGGGATATTCTTGAAGCGCCTTGAGCAGGGAGGCAGCGTTTGTCTTTATCTGACCT
TGGCTTCTTTGGGTCACTCTGTTTCTCTTTCCGTGAATAAAAAGCCAGTGAGCACACACTGTGTCCCAGG
CACTCTTCTACGCTCTGGGGACATCACCATGAACAACTAGTCAGAGTCCCCACCTCCAGGGGCCTTCCGT
TCTGGTGGTGGGTTTGGTTCCATGGTTGAATGCACCCAGCTGCTTATCTGTTCAATAGGCATCTGCTTTA
TTTTAAGCTTACTTTGCAAAGAAGGAAGATGGTTGTTTCCGAAGTGGACATCGCAAAAGCTGATCCAGCT
GCTGCATCCCACCCTCTATTACTGAATGGAGATGCTACTGTGGCCCAGAAAAATCCAGGCTCGGTAAGTT
GCTCTCTGAAAGTCGCTATCCATGTGACATGAGCCATGCCCATTCGAGGCTGCCCTTTTCTACAGCGGTG
CTACTGCTGCCCAGATATGCCTGCTCTCTCGCCTCTCCTGTGCCAGGACGCAGATCCTGACCCTCTACTT
GCCAGCTGACAACCATGTATAGACTCGTTGCCTTAGCTCACCGTGGGTGGAGGTGCTGCTGGGGTTGGGG
ACACTGGCTGAGCTGTCGATGGGTTCAGCTCATCTATACTAGAAGGAACTGGCCCAGGCCCTGGGTTCAA
GGAGCACTAGGTTTGTTTTTCCTGCCCACATCATGATTCAGTCTCAAGCTACAAACCCTGGGGCATCATT
AAGATATTATCTTTGTAGGGAAGCCACGTGGTGAATTTTTTGCCCAGAATCAAGACATATTTTTGGTGGG
AACCAATCATGGCCCCTGGAATCAGTCCAACCTCAGTTGGTCCCGACGCTGACACTCTCAGCTGTGTGAT
TGTGGGTAGTTATTCCTCCTGTTATAAGCCTGGTTTTCCATATCTAAAATGAGAATAATGGTCTTTGCTT
TATATGGCTGAGAGCAAAGTGCAGGGAGTGTCCAGGTACTCAGAGTGCTCAGTTTCTTATTACCGTGGAT
CACTGGTCTGTTTCAGGCTGGCTCTGTTTTCCTAGGCAATGTTAAACAATTTTTCAAACAATATTCAAAA
AACATTGAAGCGTTTAGAAAAATACAGAGGACCACCACCTTTCCACCACCCTGATACCACCGTTTACATT

TTTCTGCATTTCCGTCCCTGGTGCGTCTCTTGTCTGGCTGTAAGAGATGTAATTATGTCAGGGTATGGGG
CAGGAGGAACTCTCACCCACTCATACTTGCGTGCTTTGGAAGCAGCTTGGCCTTTTCCAGTCAAGGAGAA
CAGAGTGTGTCTCGTGATCTGGCAGTTCCACTTCTGGGGATAGACTGTGGTTCTCAAAGTGTGGCCCCCA
GCCCAGCCCATTAGCATCACCTGGGAAGTTGACTGAAATGCAAATGACCAGCCCCTCCTCCCACTCTTAG
ACCTGGTGAATTGGACACTCTGGGGCAGGGCCCACCCCCTGTGCTTTACCAGGCTCTCCAGGTGATTCTG
CTGTGTGCTGGAGTTTGAGGACCATGTGTAAACCCTTGCACATCCCCCTGGGAGCCACATGCATAGCAGC
ACTGTTTATAACAGCAAAACTCAGACCCTGTCTACGTGCCTGTCATGGTGGAATGGATACTGGGAGTGTG
GTATGTTCATGTAGCAGAATTCTATACAGTAGTAAAGAGGAATGAACTAGAGTTCCAGGTATCAAAATGG
CTGCATCAAATGAACAACGTTGAACCAAAGAATCAAGTTACAGGAAATATACAAAATGATTCCACTTACG
TAAAATTCCCCAACAGGCAGCAATAGGCAATATACCATTGTGGAAAAACATATAGGTGGCAAAACTATA
AAGAAAAGCAAGGATGTGATGATTGCAAGCCTCAGGATAGAGGGACCCTCTGGAGGTGAAGGAGTTGACC
CAGGGAGGTGTGTGCAATGTCCTAAGTACTGGGAGTGTTTGTGTTCTTAACCCAAGTGGTTGGTACATGC
ATATTTGCTTTATTATGTTTGACACACTTTTGTAAATAGATAGTATAAATAAATGAAAACAAACAAAAAG
TTATAAGGTGAACTAAGACCGAGGCTACCAACTGTATTCATGCATTTGGTAAGGCTGTGGTTCTTTCTCA
GTCAGGGCCCATTTTGCTCCCTGGAACTTGTGGCCAGGTCTAGAGACATCTTGGTTGTCACAACTCAGGG
TGAATAGTGAATAGAGGCCAGGTATTTGGCTAAACCTCCTACAATGTGTAGGGTAGCCCCTGCAACAAAC
AATCTTCTAACCCCAAATATGAATAGCTTCTTGTCCTGTTATAAAGAAGCTATTCTAGTAAAAACGTCTG
TCTATGATGAAGCATGCACAAAAATAGTCATTAGAAAGAGGTAAAAGACAAAATGATTTTCTCATATTTT
CTTCCTGAACCTCAATCAGCCCACTTTAGGAAAATTGCACCCAGCTGCTGGTAGGTAGGCAGGACCGAGT
GTGAAGTCTGCTGCTTTCTCTGTTTTTATGCAAGTACTTCACTATTTTGATTACTTTAGTTTTATAGTAA
TTATGGAAATTAGGTAATGCTAGTCCTCTGACTTTGTTCTTCTTTTTCAAAGTCATTTTGACTAAGAATA
TTTAGATCATTTCTATTTAATGTGACTGGTAATATAATTAGATTTGAGAATATCATCTTGCTATTTGTTT
TCTATTTGTCCCGTCTGTTCTTCGTTCCCCCTTTCGTCTTTTTCTGCCTTCTCTTGGATTATTTTTTATG
ATTCCATCATGTCTCCTTTGTTGTCTTATTAAGTATAACTCTTGGTGTTTTTAGTATATATCTTTAACTT
AATAAGTCAACCTTCAAGTGATAGTCTGTCACTTCATGTATAGTGCGAGAACCTTAGAATAGTGTATTTC
CATCTCTCTGCTCTGAGCCTTCATACTATTTCTATCATGCATCTTTTACATACATCATAAACCCCACAAT
ACATTGTTATTATTGATGTTCAAACATTCAATTATCTTTTAAATAAAGATAGCAAAGGAATACAAAAAAG
TGTAGTAGTTACCCCTTCTAGTATAGACTCCTTTGTATAGATCCAGATTTCCATTCAGTATCATTTTCCT
TCTACCTAAAGAACTTCTTTAACATTTCCTGTAGTGCAGGTCTGCTGGTAATGAATTAGTTAAGCTTTTG
AATGGCTAAAAAAGTCTTTGTTTTGCCTTCATTTTTAAAAGTTATTTTTGCTGGGTATAGAATTCTAGAT
TGATGGTGTTTTTCAGTACTTTAAAAATACTGCTTCACTGTCTTCTCGCTTGTTATTGCTTCTGATAAGA
TTGACAGCAGATTTCTCATTTGTGTCCCTCTGCTCACACTGTATCATTCTCTGGCTGCTCCTAACATTTT
CTCTTTATTACTGGTTTTGAGCAATTTGACCCTCTTATGGCTTGATGATGTTTATGTTTGCTGTGCTTAA
TGTCTGTTGAGTTTCTGGGATCTTTGGGTTTATGGTTTTCATTAAGTTTGAGGGAATTGTATGTATTATT
TCTTCAAATATTTTTTTCTGTCTCTCTTCCATTCTCTTTTGGGGATTCCAGTAACCTGTGTATTAGACTT
ATTGAAGTTGGCCGTCTTTAATGGAGTGTATTGGTTCATTCTCACACTGCTATAAAGAACTGCCTGAAAC
TAGGTAATTTATAAAGAAAAGAGGTTTAATTGACTCACAGTCCACATGGCTGGGGAGGCCTCAGGAAACT
TACAATCATGGAGGAAGGCATCTCTTCACAAGGTGGCAGGAGAGAGAATGACTGAAGGAGGAACTTGCCA
AACACTTATAAAACCATCAGACCTCATGAAAACTCACTATCATGAGAACAGCATGGGGGAAACCTCCCCC
ACAATCCAATTACCTCCACCTGGTCTCTCCCTTGACACGTAGGGATTATGGGGATTACAATTCGAGATGA
GATTTGGGTAGGGACACAGAACCAAACCATATCATGAGCATGATTTGCAGGCCATGAAGAATTCTCCATT
TTTGTTTCCTCCAGGTGGCTGAGAACAACCTGTGCAGCCAGTATGAGGAGAAGGTGCGCCCCTGCATCGA
CCTCATTGACTCCCTGCGGGCTCTAGGTGTGGAGCAGGACCTGGCCCTGCCAGCCATCGCCGTCATCGGG
GACCAGAGCTCGGGCAAGAGCTCCGTGTTGGAGGCACTGTCAGGAGTTGCCCTTCCCAGAGGCAGCGGTA
AGAACTTACATTCTGTGTTAGTCTGCTCAGGCTGCCATAACAAAATACCACAGACAGGGTGGCTTATACA
ACAAAAGTTTATTTTCTCACAGTTCTGGAGACTGGAAGTCCAAAATCAGGGTTTAGCTTCTCCTGAGGCC
TTTCTCCATGGCTTGCAGATGGCCACCTCCTCACCGCTCCCCCATGCGGCCTTCCTTCCACACACAAGCA
TCTCTGCTGTCTCTTCCCCTTCTTCATAAGGGCACCAGTCATATTGGATTGGGGCCTACCCTAATGACCT
CATTTAACCTTAATTGCCTCTTTAAAGGCCTTATCTTCAAATACAGTCCCATTAGGGGTTAGGGCTTCAA
CATAGGAATTTGGAGGGAACACCATTTCATAACGCTATCTCATTGCACATTTTTTTCACATAAGTCATAT
GTAATCTACAGTTTGAGGAGAATCTGAATAAACACATTTGGGTCCCCCAGTTCAGAACTATATCAGTGAG
GTCTCAAAGAGTTCAGGCCTGGGACGGGTCTTGCAATACAGATCAGGTGTGGTAGGAAGAATATGGAAGG
AGTTTACAGTAGGAGGACTGTTGTAAGGTGGCCTGGTAGCAGCAGGATGCTTTCCTAATGGGGGTAGAGT
GTGTATGCTGGGGGGATAATGGAAGATGTTCGGATGAGTTTCGGGGGATTCCCAATGTGGTCTGCCACCT
GAGCTGATGGCAGAACACTGGGATGAGGCAGGAAGCCAAAAGTGGTGGCTTTCAAGCGTTAGTAAGCAAA
AACTCACCTGGGTTACATACTCAGAATGCATGTTCTTGAGGTCACCCAGACACAGTGCGATGTCCCCGCA
TATCAGAGGGTAAGACCAGAAAGTTTCCAGTTTTAAATGTCTCCCCATATGATTGTATAAAAGTTTGAGA
ACCATGGGCCTAAGGCGCTATGTAGGTCTTTTAAGAGCAAAGTGGAGCACTGATGTGGGCGTGGCCTCCT
AGGGATCGTGACCAGATGCCCGCTGGTGCTGAAACTGAAGAAACTTGTGAACGAAGATAAGTGGAGAGGC
AAGGTCAGTTACCAGGACTACGAGATTGAGATTTCGGATGCTTCAGAGGTAGAAAAGGAAATTAATAAAG

GTGAGTACCCCCTGTTTGGATGCCTGGTCAAGCCTTCTGACATGCATGGGGTCTGTTTGTAACTGTTCAT
ACTCCCACCTCCCTGGGCCTGTGCTGTCAGGACACCTTTCTCCTGCACATCAGGCCACGGTTCCTTCTAC
TTCTTTTACCTCATTATGACCAGCACGCTTGGATATCAGCATCTGATAGCAATCATTTATTTCTGGCCAG
GCACAGTGGCTTGTGCCTGTAATCCCAGCTCTTTGGGAGGCTGACACGGGGGGATTGCTTGAGCCTAGGA
GTTCAAGACCAGCCAGGGCAACATAGTGAGACCCCGTCTCTTAAAAAAAAAAAAATTAAAATAGCTGGGCA
GGGTGGCATGCACCTGTGGTCCCAGCTATTCAGGAGGCTGAGGTGGGAGAGTTGCTTGAGCCTGGGAAGT
CAAGGCTGCAGTGAGCCGTGATCTTGCTACGGCACTCTAGCCTGACAACAGAGTGAGACCCTGTCTCAAA
AACACATGTATTGCTTATTATGTAAGTATCTAGAATAATGTGAATTTTAAAATGTCCCCACATATGGATG
ATCTGTCCCTTATTCAAGGGCTTCCCTACTTAGATCTGGCAGGAAGAGGAGCCAGATATGGGGGTGAGGG
AGCTCCTCCCCCTGTTCCTTTGTACAAGGAACTCCACATTGTGGACAGGATCGTCACTGAACCCCACTCA
GAACCAGCACCCTTTTCTAAGAAAGAGGAGTGACTGTGTTTGCATAATCCCAGCTTAGGCTAATTCATGG
CAGGCCTCCATAAATGCAAACCACAAGGATCAATTTGAAGTGTCTGCAAGGGGAAATGACACCAGCAGTG
TAGACAGGGTAGAGTAGCTGACAAAGAACAGCCTCTGTGAGATGCATGGATAACATCTTCCTATCGACCT
TCATGTTTTTCTGGCATGTCACATGTTTAAGTTTCATTCACACTGGGAAGGTACTGAAGAGACATGAACT
AATGCCCAGCAGTAGGAAGGGACGGGTTTAGCATTTGTAAAGATGGAGCATTAATCACATTTGTTGACTG
TTTAAAGAAAGATAAATAATGTTATTGACAAACCGTGATTTTGAATTAGTTGGGATTAGGTTGGCTGCTT
GCAGCAGAAAACTCAAAATAACTGTGTGCCTTAGCCCTTGCTGTATAACAAACCCATCTTAAAACTAATG
GCTTGACCACTTTTATTTCTCATGATTTGATGGACCAGCTGGGCAGTTCTTCTCTGGGCTAGCTGGGCTG
GGGCTGATGGTCCAGGATGGCCCTTGGCTGGAGTGACCGGGCCTTCTGTCTGTGTGGTCTCTCACCCTCC
AGAAGGCTAAGCTGGACTTATCAATGTGGGTGGAGGGGTTCCCAGCAGCAAGAGAGGGCAAGACCCGACA
TCTAACACCTTTCGGTCTCTCCTGACGTGGCACTGTGTAACATCCCCTTAGCCAGAGAAAGTCACGTGGC
CAAGCCCAATTTCAAGGGACCGATTTCTCTCTCTCCATGGAAGTGACGAAGGCACCCTGTAAAGTAGCG
TGCATACAGGGATGGAAGGGAATGTGGATGCCATTGTGCCAGCAAGTTGCTGAGACAGCGGTCTAAACAA
TGTAGAGGCTTTCTGTCCCTCTCATATAAGTCTGAAGGCGGGCAGACCAGAGCTGATTGGGGATTCCAAT
GTCAGGAACCAAGATTTATTCTTCTCCCATTTCTTCATTGGGTGTGGCCTCTGTTTCCAAGGCCCCCCCG
TGACTTAGTCAGCAAGTCCGCTTTGAAGCCAGCTGGACCATGGCAAGGGGCATATCTCTGCCTTTGTAAG
CACACTTTCTGGAAGTTGCACATAACATTTTCACATGGCCCATTGGCCAGAACCCGATCTCATGACCACA
TGGCAGGTACATGGATATTGGGGGAACAATTAGTGGACCATAACCACTGATATTTCCTAAGTTCTAAATT
GATATCAAACATCCCAAAAAGGCATTCTAGATTTAGAAAAGAGTAAAGTGGTGTTAGCCAACAATTTGAT
GAAACAAATTCATATCCTAAAATTCATTAAGGAGGAAGGAGCAAAATAAAATCTCTTAATGGGATGTTAA
CAGCCAGTGCTTATCTTAGCTAAAATAAGCACATTTCCCCATATAATTTTCCAGTTTATATTTTAGGCAT
TTCCATATATTTTTATTTGTTTTTATTTTGCTTGGTTGCTAATTTCCTACTGACATCAATGAGAAGGATT
TAGGAATGCTACCAGGAAGAACTTCTTGCCTCCGCCCAGCTTTGGACTGGTCTAAGTGGGTGTCACTCAT
GGTGACGTTCTCACAAGGTCTCTCTACACACAGTGCTGGCCAACAGCAGGGAAAATACTGAGTTATCCTT
TGAGATCTCTTTTATCCCAATCACAGAAAATTGAATCTGCTCCAAATATGCTTTTATCCATGACTCGCAG
AGAGGAGAAGATGCTTTCAGAGTATTCACCATCATGAGATCCGTTTATCCTAAGCTCTGTTTGGGTTTGA
TTTTCCCTGTCTCTTTTCTAGCCCAGAATGCCATCGCCGGGGAAGGAATGGGAATCAGTCATGAGCTAAT
CACCCTGGAGATCAGCTCCCGAGATGTCCCGGATCTGACTCTAATAGACCTTCCTGGCATAACCAGAGTG
GCTGTGGGCAATCAGCCTGCTGACATTGGGTATAAGGTCAGACTTCAGACCCATTCTGACCTTGGCCGTG
GCGTGGGGATGGGGGAGTGGAGGGGTGGGAGGAGAAAGAGGGTACTGTATTAGAGTAACCGTGAGTCCAG
AGCTGAGTTTTGGAGTTAGTATTTGGAGGTGTGAGTGGGGAATTTAGAGAGCCCGTTGGTCACAGTCTGT
TCTGTCAAGTTGAATGGAAGCTTCTTTGGAGAAAGTGAGGCCAGTGGGCACAGTTGGAAATGTGTTCTGT
GTATTTGTTTTATGTTTTATGCAATGACTTGTTTTTGGTTATATACATTTTGCAGCATATCTAAAGTGCT
GTGTATTAGGAAGGGGTCTTATGTGGGAAGAGAGCATTAAAAATAAGTATAATGGGCCACACACAGTGGC
TCAGTCCTATAATCCCAGCACTTTGGGAGGCTGAGGCAGGAGGATTCCTTGAGCCCAAGAGTTTGACAGA
AACCTGAGCAACATAGTGAGACCCCCTTCTCTATAAAAGAAAGGTTAAAAAATTAGCCAGGTATGGTGGC
GTGCACCTGTCAGCTACTAGGAGGATTGCTTGAACCAGGGAGGCTGTGATGAGCCGTGATTGTGCCACTG
CACTCCAGCCTGGGCAACAGAGCAAGAATCTGTCTCAAAACAAAAAACAAAACAAAACAAGCAAGAAAGA
AATAGGTATAATGATATTTTAGTATCAGTGAATCTCACTTTACAGATTAAAGATTTAGGGGTGAAGTGGG
GTTTTTTGGCCACCATTTTTCATTGTGACCATCAGATCTGAGGTCTTAGGGGTTAATTATCTGAAACTTC
ATGGTTTTCCCTGAGCCTATAGCTCTGCTTCTGCCACAGATAATTTATTTCTCATAATTCCAGCTTGGT
ACCTCCAGGGTTGTGTTTGTGGGTTCATTTCTCCAAAGTTACTTCTTTTGGGGGAAATACCCCTGGGACT
CTTAGGGCCTAAAGCAAGTGCAAGGTCAGGACTTGTCTCACCTCTCACTTGCCTTTGCCATACTCACGAG
TCACCTCCTCTCATTTCCTTACAGATCAAGACACTCATCAAGAAGTACATCCAGAGGCAGGAGACAATCA
GCCTGGTGGTGGTCCCCAGTAATGTGGACATCGCCACCACAGAGGCTCTCAGCATGGCCCAGGAGGTGGA
CCCCGAGGGAGACAGGACCATCGGTGAGAGTGGGGGAGCCCCACTGTGCTCAGTGAGAATGGGGGAGCCC
GCCTGTGCTCGGTGAGAATGGGGGAGCCCACCTGTGCTCGGTGAGAATGGGGGAGCCCGCCTGTGCTCGG
TGAGAATGGGGGAGCCCGCCTGTGCTCGGTGGTCTGCCAGTGGGCAAGCGTCCCTCCAGTCTCCATGGGC
TTTGCTCAGTGGGGACCTGCCTCCACTAAGACCTGCTAAGGGAGCAGGTTTGGTGCCCACCAAGGCCAAG
TGAAATGAGCTGCTTTTGACTCTCACTGGCTAGGTTGCCTTGTAAGCCTTATCTACTTGCTCAGAAAGGC

ACAGTGGGCTCGGAAGCAGGTCAAACTCAGGAGGCACATGGTACTCATTAAGAATGCATTTGAGATGGGA
TGTCCATAACTCAAGGGATAAACAAAACGTGGCGTGTTCTACAGTGGACCCGGGTGAAGGAGCTTGGGGA
GAGCCACATGCTGTTCTGGGAGGCATCCCTGCCTTCACGCGGCTTGTCGTGGAGTTCTTTTCTGGAGCGG
GGCTCCACTGCCCCCATGGTTCTGCAGGGGCTATGGCCTGTCCTCAAGCAAGGATGGGAGGAAACCCTGG
GAGGCCGGGGGCGTGAGCAGTTGTTCGTTCACCTCTGCCTCGTGACTGAGCACGTTCTCTCCCCAAATAC
ATCTGGCTCGCAGGAATCTTGACGAAGCCTGATCTGGTGGACAAAGGAACTGAAGACAAGGTTGTGGACG
TGGTGCGGAACCTCGTGTTCCACCTGAAGAAGGGTTACATGATTGTCAAGTGCCGGGGCCAGCAGGAGAT
CCAGGACCAGCTGAGCCTGTCCGAAGCCCTGCAGAGAGAGAAGATCTTCTTTGAGAACCACCCATATTTC
AGGTGCGCTTGCCTGGGTTTCATCATGGATCAGTCCAAGCCCAGGATGTCAGGCCTTCCAGGGGACAGTG
GCAGCCGTCCCACAGATGTGTGGAGTGTGTGTGTGTGTGTGCGTGTGTGTGTGTGCGCGTGTGTGTGT
GACTATGCTTGTTCCCCAACAAGGACTATGGAATTCACCTAGAAGAATAGGAAGGGGATTACAAAATACT
GCCAAGAAAAAAAAAACTAAAAACCAATCAAAATAGGGAGAGAACAATGTACAATAATTTACGTAGCATG
GTGCTGGAACCATATTTTATAAAAACATAAATAGAAGAGAATAGGAAAAAAGTAGAAAGCCCAGAAATAG
ACCTAGATATATATATATTTGACACATGATAAATGCAGCATTTCAAATCAAATAGTGGACTATATCAGCTTG
AGTATCTATTAGTGGTGTTAGGATAATTAATATTTGGGAAAAAACTAAAATACTGCCCTTACTTATCTCA
TTATACCAAAATAGTTAAAGTTTAAAGTTAAATATTAAGAGGAAGCAATAAGCATCTCAGAAGAAAGGTA
GGTGAATAGTTTATAAGATTTTTCTATCCCTCCTACCAAAAGTGACATTTTTTAAAAGGAAAAGACTGAC
AAATTGGTAAGATTTAAAATGATGAGACTATGTAGAGTTGTAAACATTCTTACATTCAGTTCTCCCAGAA
GCCTACAGAGAGCCATTACTCAGAATTCCAGGAATATCAAATGGAAACTTACATCCTGTTCTGCACATTC
ACAATTGCCAGAAGATGAGATGATTCAGTGTCCATTGATGGATGGATGCAGAAAGCAATGTGGTCTGTAC
AAAAACATGGAATACTTTCAGCCTTAGAAAGGAAAGACATTCTGACACATGCTACAACATGGATGAAGCT
TGGGAACATTCTACTAAGTGAAAGAACCCAGTCGTAAGAGGACGGATACTGTCTGATTCCACTTAGCTGA
GGTCCCTGGAGTAGTCAGATTCATAGAGACAGAAAGAATGATGGGCACCAGGGGCTGGGAGAGAGAGAAT
GGGCAGGTAGTGTTTAATGGGGCCATTGTTTCAGTTTGGGAATATGAAAAGTTCTGAAGACAGACAGTGG
TGATGGTTGCATAATAGTGTGAATGTACTTAATGCCACTCAAGTGTACTCTTAAAGATGGTTAAATGGTC
AATTTTATATGTAGTTTACCACAATTTAGAAAAATTGACAGAGAAACTGAAGCTTAGGTATGAGTATACT
CACAAAAAGGCACAGAAACTCATGCTTCACTGCTGCCTTTATCCTAAAATGTCCTATAAAATGTGGGAAA
CCCTGTAATAACTCACTCTGTGAGCACAAATTTGGATCGAGTGAGAAGATACTTGACTTCCTTCCTCCAG
GCAGCCCATGGTTTAAGTTTTTATCTTGGACAAGATATCTTGTGTCTCTTCTCCTCAGTGTTCTGCTACC
CATTTATCTCAATATGCTTCAATGTATTTGTATGAAGATATGTCTGTATCCATTATGATCACCTACACAT
ATTACACATAGAAGGGGGTATGTGTTATAAAAACATATCTATACATGTCTGTGTATTTTTGTGATGACCA
AGTCTATAGTCAGACACCATGATACACATTTATTATATCAGCTGGAAGAGCTCATTCCATATCATTGTGG
AAATATCCTAGATTGCTAAAATTCAGTCATAATCCTATTCAATCCAGTTCTGAGTATTTGTTGGGTACCA
ACTGCAAGACATTCCATCCAGTTGTAAGCAACTGAAATTTGCCTTGACTTTCCCCAACAGCAAAAAGGCA
GACATGCGTGTTCTGGCTACATCAAGGTGGAAATCGGTCCTGTGTTCTCTTCTAGGGATCTGCTGGAGGA
AGGAAAGGCCACGGTTCCCTGCCTGGCAGAAAAACTTACCAGCGAGCTCATCACACATATCTGTGTAAGC
ACGGGCAGAGCTGTGGGTTCTCTAAAAAGAATACTACGACCGCAGAGCTGAACCTTGCTGGCTTCTTAAA
CATCACTGTACACACAGATCTTCTGAGGATCTTGTTAAGATGCAGTTTCAGATTCTGTGGGTCTGGAGTG
GGGCCTAGAATTCTGCATTTCCAGCAAGCCCCCAGACAATGTGGATATTCCTTTTCAGGGGACCACAGTC
AGGGGGAATGCTGATAGACTATATCTACTGGGCCAAAATAAAAATTAAAATCTTATGCACAAGCTACTAA
CTCTTCCTTTCTCATTGACAACCACTATTATAATGTCTTAGTCATTCTAATGAACATATTTTTTAACTTC
TAAAAGCTTTGTAAAAGCTCTCTGTGGTTCTTTTTAAAAGTCTGCCTGAATATAGTGTCTTCCTTTTTCA
AATTTTCTTTTCTTTTCTTTTCTTTCTTTTTTTTTTTTTTTTTTTTTTTTTTGAGACAGAGTCTCACTCT
GTTGCCCAGGCTGGAGTGCAGTGGTGTGATCTCAGCTCACTGCAACCTCTGCCTCCTGGGTTCAAGCAAT
TCTCCTACCTCAGCCTCCTGAGTAGCTGGGATTACAGGTGCCCACCACCATGCCTGGCTAATTTTTGTAT
TTTTAGTAGAGACAGGGTTTCACCATGTTGACCAGACTGGCCTTTTTCAAATTTTCAACTCAGCACCAGA
GTGCAAGGTCTTCCACGTGGTCCCCAGGAATGCGGGTGCATAACAGGGTTGTTTCCAGCCGACCATGATG
AGTGCAGAGCTCTCTGGGGTCCCACTGTATGCAGAAAGAGGATGCTTCCTTATTAGATTCCCCACCTCGA
GCAAGCCCATGGGGATTGATTTTTTGCCTCTGCACCAAGTCAGGTTCATAAGTTCCCGTTCGAATTTTCT
TACCTAGACAGATGCCCTTGTGGCTGAGCCGGGCTTCATTGCTGCCTTCTCCTTGAGCCCCTGCCTGGCC
ACTGTTACTGGGGCTGGCCTCTGACTACCCCTCACTAACTTGTGAGTCCACCGATACATTTAAAGGTGCA
GCTTTCACATGTCAGCTGGCATTTTTAGATGTTTGCCGTGGAAGGGTGAGCCAGCATATGGCGTCAACCG
TATTGTTAAAAACATAAGTCTCTGATCACTTTTTATTGATTGCAAGCAACATAAAAGTTGTTGAATCTCA
AATTGCTCCAAATGCCACTTTTTCAGAACCTACTAGACAAGTGGATCTCTCCAGTCTCCCTCCAGAGAGT
TTACCTAATATGACCACAGAGGAACTGCTCCCGGGTCACTCTGCCGGGGCCTAGGACCCATGCACAGTGG
GTGCCACAGTGCTGCTCATGAGGCTGCTGTCGCAGGAGTGGGGAAGGGGAAGACCTGGGCAGAAAACAG
TGCCCCCAGTGTGTGCCCCCCTGCACCTCCCCCGGGTCTGGAAAAGCTTCCTTTTAGAGGAAGCCAGGAA
GTCAAATGGCCCACACAACTCCTCTGCAGAGGGAGGCCCGGGACCTCCTTTTCATTCTCTGTTCATCTTT
ACACATTTCCATTATTTTCTCTCCATTTTCCTCAGAAATCTCTGCCCCTGTTAGAAAATCAAATCAAGGA
GACTCACCAGAGAATAACAGAGGAGCTACAAAAGTATGGTGTCGACATACCGGAAGACGAAAATGAAAAA

ATGTTCTTCCTGATAGATGTGAGTGTTGCCAGCTGCATGGAGCTGGAGAAGCACATGTCATGGTCAAAAA
AGGGACCCTGGGCCTTATGCACTTCCTTCTTCACTCCCCCAAGGCTGATCCAAAGACATCTGGCCCGTAG
CACTCAAAGGGTGGACAGGGCTGAGGGAGGCAGGGCAGGGAGTGCAGATGTGGGGGTGGAGTCAGCAGCG
AGGGATGCTCAGGCTGCGTTGTGCCTACTCTGTCACGAGCATCACCCAGATCCCTAAGGCAGTAAGGGGT
GGGATAGGATTTTCTAGTGCCAAAACCTCTTCTTTCCCCTGATCCACAGTGTCCCATAAGAAAGCAAAGA
ATGACTCCCCACCCTCCACATAGGCACGGCCTCCAAATGACCTTGACACTTGGATTTGAAGTCTATCCAC
TTATACTGATGTTTTTCTTCTTGACAGAAAGTTAATGCCTTTAATCAGGACATCACTGCTCTCATGCAAG
GAGAGGAAACTGTAGGGGAGGAAGACATTCGGCTGTTTACCAGACTCCGACACGAGTTCCACAAATGGAG
TACAATAATTGAAAACAATTTTCAAGAAGGTGAGTGTCTTAGTCCCTTCTTTTGGGCTGCTACAACCGAA
TACCTGAGACTGGGTCATTTATAAACAGTAGAAACTTATTGCTCATTGTTCTGGAGGTGAGAAATCTATT
CTTAAGGAATCAGGAAATTTGGTGTCTGGTGAGAGCTTGTTCTCTGCTTCAAAGATGGCACCTTCTAGCT
GTGTCCTCTCATGGGATAAGGGACGAACAAGCTTCCTCGGACCTCTTTTTTACAGGGGTACCACAGGCAT
ACCTCAGAGATATTGTGGGTTCAGTTCCAGACAAAAAGAATATTGCAATAATGCAAGTCATATAAACTTC
TTGGTTCCTGGTGCATAAACAGTTCATTTATGCCCTACTGCAGTCTATTAAGTATGTAATAGCATTAGGC
CTAAAAAATATGTATGTACCTTAGTTTAAAACACCTTATTGCTAAAAAATTGCTGGTACAGAAACAAAAA
GTGAGCATGTGCTACTGGAAAAAAATGGTGCTGATTTGCTTGACATGGGATTGCCACAGACTTTCAATTT
GTAAAAAATACGGTATCAGTGAAGTGCAATAAAACAAGATATGCCTGGAATGCCATTATGCGGGCAGAGT
GCTCATAACCCAATCCTTCCTAAAGGTCTCCTCTGTTGATACCATCACACTGGGGATTAAGTTTCAACAT
AGGAATTTTTAGGGGACACCAACATGTAGACCATAGCAATGAGTCAATACCGTGGTAAACCTGATACGTT
GGCTTAAGACAGAGAAGAGTGGGGCAGTTGGGGAGGATGGTCAGGATAAGGAGCTAGTGACAACTAAAGC
CATGTTTGCTCTCTTCTATATCACTGAACCCAAATGACCATCCACTGATGAATTGATAAACCAACTGGCT
TGTGTCTGTGTGTAGCGGTTGGCTTTGGCTGTCATAACAAAGTACCACAGACTTGGGGGGGCTTAAACAG
TAGAAATTTATTTCTTACAGTTCTGGAGGCTGGAAGTCCAAGATCAAGATGTTGGTGGAGCTGGTTCCT
TCTAAGGCCTCTCTCCTTGCCTTGCAGATGGCCTCTTCTCATTGGGTCCTCATGTGGTTGTCCCTCAGTG
TGTGTGTCCTCATCTCCTCCCACAAGGACACTAGGCAGATGGGATGAGGGCCCACCCTAGTGACCTGATT
TCAATTTAATTACCTCTTTGCCTGTCTCCAAACACAGTCAGATTCTGAGTTTCTGAGGGTTAGGACTTCA
ACATTGGAGTTTGAAGAGGTCACAACTCAAGCTCAGCCCGTAACACCAAGTCCTGGAATATTTCCAGCCA
CAGACAGGCACAGAGTGCTGGTCCACAGCACCCCATGGATGAACCTTGAAAATGTCATGCTGAGTGAAAG
AAGCCAGCCACAAAGGCCACACGGTCTATGATTCCATTGATAGAAAATGGCTAGAACAGGCAAACCCAGG
CAGGCAGAAAGCAGAATAGTGGCTGCCAGGGGCTGGGGAGGGAAAAGTGGGAAGTTATCACTGATGGGTG
ATGGATGTGGGGTTTGGGAGTTATGTCTGGGGATGGTCGCACAACTTTGTGAATATACTAAAATTCACTC
ACCCATACACTTTTTTTTTTCTTTTTCTTTGGAGACAGGGTCTCACTCTGTTGCCCAGGCTGGGGTGCAG
TGGCTCAGTCTCGGCTCACTGCACCCTCTGCCTCCCAGATTCAAGCGATTCTCCTGCTTCAGCCTCCACC
TCCCTAGTAGCTGGGATTATAGGCACCTGTCTAATTTTTGTATTTTTAGTAGAGATGGGGTTTCACCATG
TTGGCCAGGCTGGTCTCGACCTCCTGACCTCAGGTGATCACTGGCCTCAGCCTCCCAGAGTGTTGGGATT
ACGGGCGTGAGCCACTGTGCCTGGCCTGAACCATATATTTTTAACAGAGTGAATGTTATACTATGTAAAT
GACATCTCAATTAGAAAAATCCTTATGGGAAAATATTTCCTGACTAAAAAAAGTGTTCTAGATTACCACT
CAAAAAGGAACTCAAACCCTCTGAACTTCTGATGGGGCTAACTCTCTCTAGTGTGGATTGTTGGGAGTAC
AAATCATTCCAAAAGTTTAAAGAAAAATGCAGCATCTTACACAGTGAACAGTGCTACTGTATCACATTCA
TACAAGTTGATGTGCCTGGTTTACTCTGTTATCCCATTTGACTTGTAAACACTTTCTACACATGGCAATA
CTTTCGACACATGAATATGTGATGTATTGTTAATTCCAGAAAGTGTTCATGCTCATTTCTAATGGGCATG
CAGTTGAGGGCAAGGAGTGTATTATGGTACAATTTCTTTGGTAAAACTAAAATTGGATTCACAAAACTTC
ATACTCGAGTACGTTTTTAAGAAGGGGTCTTGGCCGGGCACGGTGGCTCACGCCTGTAATCCCAGCACTT
TGGGAGGCCGAGGCAGGTGGATCATGAGGTCAGGAAATTGAGACCATCCTGGCTAACACGGTGAAACCCC
GTCTCTACTAAAAATACAAAAAAATTAGCCGGGCGTGGTGGCGGGTACCTGTAGTCCCAGCTACTTGGGA
GGCTGAGGCAGAAGAATGGCGTGAACCCGGGAGGCAGAGCTTGCAGTGAGCTGAGATCACGCCACTGCAC
TCCAGCCTGGGTGACAGAGCGAGACTCTGTCACAAACAAACAAATGAAAAAAAAGGGTCTTACTCGAAGT
TTCTGCGTATGTGGGTTCCTGGCATCGTACCTGGCTCTGCACTCCCCTTCCTGAGATGACTAAGGAAAAT
TATCTTCAGATCTGGTTTTGTGTGTGTGTGTGTGTGTGTGTGTGTGTGTGTGTGTGTGTGTGTGTGTAAT
CCCTGGATATTTTTAGTTTACCAGTTAGATTTGATTTGATACCACTTTTTCTTGCCATTTATATTTTCAG
AAAATTTAGAATGGTATTGTGTTTAGAAAAATGTGCAAGATTATTTTTGTAAAATAATTTAGAGGGTTTT
TTTTCCTGCTATAGGCCATAAAATTTTGAGTAGAAAAATCCAGAAATTTGAAAATCAGTATCGTGGTAGA
GAGCTGCCAGGCTTTGTGAATTACAGGACATTTGAGACAATCGTGAAACAGCAAATCAAGGCACTGGAAG
AGCCGGCTGTGGATATGCTACACACCGTGACGGGTGAGTGCTCAGTTTCACCTCTGAGCATTGATTTCTA
AAGAAAGGAAAGGTTCGAACCAAAGCCAGCACCAAACTTCAGCACTTTCCTCCTGGGGTGCATCCCACAC
CAACGAGCAAACCTCTCATTCTCCAGATGCCAAGTTGGTATTCAACAATTCAATTCAATTCTGACACTAA
CTACCCTCAGTCAGTGTGGACCCCATAGCTTAAGGGCTCAGTTCCACAACACTGGCCCCAACTACAAATG
CCGGTCACAAGTCCCAGACCTCCTATTCTTCTGATGGACTGTTTATAAATCAAGGTTCTTGCGACCCATT
CCTCAGGTCAACCAAGAACTCTGGAACACACTTCACTGACATTTACTGGTCTATTAGAAAGGATTTGATA
AGGGGCACAAATGAAGCTGTTGGAGAGGCACATAGTAGGGGCCTGAACACAGAAGCTTCTGTCCCCACGG

GGTTGGGGGCACCATCCTCATGGCACAGAGATGTGGTCATCAACCAGGGAGCTCTTGGAACCTCACCGCG
GAGAAGGTTTTATGGAGGCCTCATCATGAAGGCATGATGGAGGATTGACTCAATCTCCAGGCCCTCCCTC
CTCTGTGGAGCTGGAAGTTCTAAGTTTCTAGCCAAGGCTTGGTCTTTCTAGTGCCCGGCCCCAATCCTGA
AGCTATGTAGGGGCCCACCAGGCATCATCTCTTCAAAACACGAGATACTCCTAAGGCTGGACATTCCAAG
AGATGTAGGGGCTCTATGTTAGGAAATGGGGACAAAGACAAAATATTTATATTTTTCCTATCACACCACA
CCTCCCCCCTGCTCCACTGCTATGCTGGCTTCACACTCAAAATCGGCTGTTTATTTGAAATCTCCGAGGA
GTAAAGCCAATGGTTCCATAACTGCACGTGTAGATGTGTTTGGAACCTTTTGGAGTGCTGTAGGAATCTA
GGTGTGTCACGGATAGGTAGGAAACTAGATCCTACTGTGGATCCACTCCCTTCTTGAAATGCTTTGCTTT
CTTGGTTTTCCAGGTATTAAATCTCTATTCTTCATCCTCTCCTTGACTGACAGTATCCTTACTCACACTT
CAGCTGCCTCATCTTAGCAGTAATTAATAATCACTCATGGATCCATGAACTAAGGAGCTGGAGATAGCCT
CAGAACAGCTCATTCAGAGGTGTATTTCCAGTAAAATTGACCTTTTAGCCCTGATAATCATATACCAAAA
CCTGCAATCATGTTGTTTTGGTCCATTGTAGACTCTTAACTCATTCCAGAGGAAAGTTTATAATACTTAG
AGCCTTATAGTCATAAAAATCAACATAGATATACCTATTTCTTTTTCAGAAATGTATGACATGGAGATCA
ATAAGAGGTTTTCAATCATAAAGATACTATACCTTGTATTACAATAAAATTCTGTGAGGAAGTAGAATAG
AAATGAGTTTCAAAAATAAAAGATAAATAATATAAATTTTTAATCTAAGAGCTTGTTCTTGTATTTTTT
TCAAATGGATAATGTAGACACTCAAATTCCATTGATATATTTAAGAGTGATTTGACTTATATTAAGAGTT
GTATTATAAAATATTAATATTTATAATTTAAAAGAAATTACATTCTTTGCAGCTATTTAGGATAAAAAGT
TTAAATATCAAATAAATGTATGCCAGGGGTCATTTGCTTTTAAGATTCTTCCAGCAAATTATTAAGCAAA
AAGAGCATGCCTTGCTTTTTCATGGTAAAGAGAAGAAGGGAGCGGGGAGAGGGGAAACTTTACTTCATAC
CATTTGATCCTCATATTTTTTTGCATCTTAAGAAGAGAACAAATGATCCTACCAATATTGAACTATTTTT
CTCTCTTTGATTAGATATGGTCCGGCTTGCTTTCACAGATGTTTCGATAAAAAATTTTGAAGAGTTTTTT
AACCTCCACAGAACCGCCAAGGTAAAACCAACCATGTGTTGTTTAAAAAAAAAAAAAGAAAAGAAATTAAG
CTTGACACTAGAAAATAGATTTCTTGGATGAGGATTATTTCAACTTTATTGTATACTTTTAGAACAGCAA
ATAACATCACTCACTAGTGCTTCTTCTGATGTTACCGGTGATGTCTGGTTAAAAGCAATAAAGGAGGGAG
TGCTTAAACGCACAGAACAAGAGATCCACAGTTAGCGGAGAAGATTATCACATCTAAGGGCAATGGCTCC
AAATCCAGAAACTCACTGAGGAAACTACATATAAAAATAGAATATTTCTGGCCCGAGTGGGCATGATGAG
CCTGTAATCCCAGCACTTTGGGAGGCTGAGGCCGGTAGATGACTTAAAGCCAGGAGTTTGAGACCAGCCT
GGCCCACATGGCAAAACCCCATCTCTACTAAAAATACAAAAAAGTAGCTGGACGTGGTGGTGCATGCCTG
TAATCCCAGCTACTTGGGAGGCTGACACTTTAGAATTGATTGAGCCCAGGAGGTGGAAGTTGCAGTGAGC
CAACATTGCATCACTGCACTCTAGCCTAGGCGATGGAGCGAGACCCCGTCTCAAAAAAAAAAAAAAAAAC
AAACAAAAAAACTTTCCATCCAGAGTGAGGAAAGAGCCTACAGGAAATGAGCCTGGGGGACAGACTGGGC
CAAGAGACCAGACTTAGCCACTCTTAGAAATAGGTGTCCCCGGCACAGATGAGGAGCCTGGCCCCATGAT
TCACCAGCTGGAGGCCTTGGGATGTGCCACTTCCAGCCTGTGCCCCTGACTCCTCATTCATAAAAGAAGA
CTGATAAGGCCTTCCTCAGAAGGTTGAGATGGACGTGGAGTAAGATGTTTAGGATGCACCTGCCACTGTG
CACTGTGCCTCTCCTCAAGGCCTGGAGGGTCCAGGGGTGAAGTTTCTCCTCCTCAGGTTTTGGCAACCAG
TTTCTCTAAACCCCGGGAACATAAAACATAATTTTCTGACTTAAACATGGCTTTCCTGCTCATCCCTGTG
GATTATCTGATGGATATGACAATCCTCGCCATCAGATATAGAAGCCCCTAAAAGAGAAAGGAAAGAAGCT
GAGTTACGGGGCCTGAAAGCAAGCCTGTGCAGGTCCCCAGGCCCCGGGATGGGGGTCCGGCCCATCTGTG
GCTCAAGCCTCCTGGGAAGCTCTGACCCTCAGCCAGGGCTAGAAACCTGCCTTAGATACACCAGGGCGCG
GCCCAGAGGGCTGTTCCAGGAAACGTGCTGTTTCACTCACGTTGGGTAACCTGGTATTTACGGACTTCTT
ACCTACTTTCCTGTGACTCAGGAATTTGTGTCTTGAGGGAAACTGTATTTATTTATTTTTTACTGTAGTC
CAAAATTGAAGACATTAGAGCAGAACAAGAGAGAGAAGGTGAGAAGCTGATCCGCCTCCACTTCCAGATG
GAACAGATTGTCTACTGCCAGGACCAGGTATACAGGGGTGCATTGCAGAAGGTCAGAGAGAAGGAGCTGG
AAGAAGAAAAGAAGAAGAAATCCTGGGATTTTGGGGCTTTCCAGTCCAGCTCGGCAACAGACTCTTCCAT
GGAGGAGATCTTTCAGCACCTGATGGCCTATCACCAGGTACGTCTTCGCGTGGTTCAGGATGCCAGCTTC
CATTCTTTCCTTTTCTTCTGAACGCCTCTCTCTTTAGTCTTGCTCTCTCTGTAGGTGACGTTGGTCAGCT
CTGTCGTTTACCTCCTTGTTAGCCTCCTGTATTAGTCCATTTTCATGCTGCTGATAAAGACATACCTGAG
ACTGGGCAATTTACAAAAGAAAGAGGTTTAACGGACTTACAGTTCCACATGGCTGGGGAGGCCTTCTACC
ATCACGGCAGAAGGCAATGGGCACTTCTTACCTGGCGGCGGTGGCAAGAGAGAGAATGAGAGCCAAGTGA
AAGGGGTTTCCCCTTATCAAACCATCAGATCTCATGAGAGTTACTCACTACCATGAGAACAGTATTGGAG
AAACTGCCCCCATGATTCAGTTATCTCCCCCTGAGTCCCTCCCACAACAGGTGGGAATTATGGGAGTACA
ATTCAAGATGAGATTTGGGTGGGGACACAGAGCCAAACCATATCGCCTTCGTAGAAGCAGCTCAACCTCA
GACAGAGAGATGGTGGCTTAGAGCCAGTGACATCTGGTTTTGATGGCTGTCTAGCTCTGGCCAAGTTACT
TAACCTCTCTGAGCCTCAGCTTTCTTTGTAAAATGGTGTCTCCTCATAGATTCTAGTGCATATTCCAGGA
GACGAGTGTGGATGATGATAATGGATTGCTAATGGAAAAACCAAACTCTGTTAAAATATTTGAAAGAGGT
TTATTCTGAGCCAAATATGAGGGACCATGGCTCTGGGAACAGTCTCAGGAGGTCCTGAGGAAGTGTGCCT
GAGGCTGTCAGGATGCAGTTTGATTTTATACATTTCAGAGAGGCAGGAATTGTAGGTAAAATCATAAATC
AATACATGGGAGGTGTACTTGCCCTCCCTAAAGAGGCAGGACACCTTGAAGGAGGGGGAGCTTACCGGTC
ATAGGTGGGTTCAGAGATTTTCTGGTTGACGATTGCTTGAAAGAGTTAAACTTTGTCTACAAACTTGACA
TCAATAGAAAGAAATGCCTGAGTTAAGGCAGTGTTAGAGGCCAAAGGTATGTAGATGAAGACTCTGGGTA

GCAGCCTTCAGAGAGAATAAATGGTAAATGTTTCTTTTCAGGCCTTAGAGGCAGCAGGCTCTCAGTTAAT
CTCTCCTAGATTCAGGGAAGGCCTAGAAGGGGAGAGGTCTGACTGCATTAATGGAGATTCTCTACAGGTG
CAAATTCCCCCCCACAAAACATGGCAGGGCCATTTCAATCTGTTGGTCCTGTTACAGCCGTTTCAAAAT
ATGTCCACAAAATATATTTTTAGGTAAAATATTTGTATTTCCTTTAGGGTCTGCAATCTGTCTTGTGATG
CTATACCAGAGTCGGGTTGGAAAGTAAGCCATTTTATACTGAGTTCATGGAAACTCATCCAAGGAGATTT
CATGGTTTGTGGGGTGTGTGTGACTTAACCCCTGCCTCACATGACTTTATAATATGGTATCTTACTACTC
CAGAGTCTTTTTGGCCAACCTTATGATCTCAATTTCAACCTAAACTCCAAAAGGGCCTGGCTTCTCTTCC
TGTTACGGCCAGGAATTCAGATTTTCAGGTTTCTCTGGGGTCCACTTGGCCAAGAGGGGGTCTGTTGAGT
TGGCTGGAAGGCATAGGATTTTATTTCTGGTTTACAACAATTTCCTTAGTGCAGCATTGGAATGCAATGG
TAGCAGACTAAATGGAAGCTATCGCGTAGACACATGCTTTGGTTGATACTGCACGATTCAGTTAACCTGA
AGTACAATCTAATTCATCCTAGGGAAGGAGGCAGTGAACACAGACACAACTCAGGTAGAGCCCTTGGGAT
GTGTAAACACCTGAGGAGGTAAAGCAAATTGTAATCTCTCGGTTTATCAGATGTCCCCATTGCCTTACTA
TTTGGATGCTTTAAAGCAGGGCCTCTCAAACTCCACCCAGCACAGAGGCCTCCTGGGATCTTGTGGAAAT
GCAGATGCTGATTGCAGGTCAGGATGAAGCTGAGATTCTGCCTTTCTTTTTTTTTTTTTTTGAAACCGA
GTCTCACTCCATTACCCAGGCTGGAGTGCAGTGGCACAATCTCAGCTCACTGCAACCTCTACATCCTGGG
TTCAAGCAATTCACCTGCCTCAGCCTCCCAAGTAGCTGGGATTACAGGCTTACCTTGCCACCATGCCTAG
CTATTTTTTCTATTTTAGTAGAGATGGACTTTTACCATGTTGGCCAGGCTGGTCTTGAACTCCTGACCT
CAAGTGATCTGCCTGCCTCGGCCTCCCAAAGTGCTGGGATTACAGGCGTGAGCCACCATGCCTGGCCTCT
GCATTTCTAACGGGCTCTCAGGGGTCACCATACTACTGGATAGAGGCCACACTTGGAGGAGCAAGGCTCT
AAACCGAGGGTCAACATCCATTCCTCCAGACACTGGGAGCTGCATGCACGTGAGTGAAGCCAGTTAAGGG
GAAGACAGGCATGCACATCAGCTTCTCCTGCAGCCAAGCTCACACCTGTCCGCTGCTTCCACTGCCTCCT
AGAATGAACAGTTACCTTGAGAGTAGGTGAGGCATATACATGCACAGAATCCAAACAATAGGATGAGTGA
CAATGGCAGAGGAGTCTCCGAGCCAAGCAGCTCCCTGGACAGAAGCAGCCCTTCTCCGGGTTCATTTCTG
TCCTCCGAGGCTGACTCATGCACTCAAAAGCTCCCATGCATATACATTTTATAATGGTTTTTACACAAAG
GTTAGCAGAGGAGTGGACGTGCTGCTCTGTACCCTGCCTCTTTTGCTGTACCTGGGAGATTGTTCTGCCT
CAGTTCTGATGGGGCTGCCTTGTTCTTTTCAATGGCTGCTGAGTATCCCATTTTATGGATGTGGTATATT
GAGCCAGCTCCCTTTAAGCGAACAGTTTGTTTGCAGTCTTTTGCTAATGCAGGTGTGTTGCTGTGAATAG
GTTTGTTTGTATATCATGTATCTGGAAGCATCAATTCCTAGAAATGAGATTCCTGGTATATTAGGATTGT
GCAGGGAAACAGAACCACAGATATATGTATGTAAAGAAGTATATTTCAGCCAGGCATGGTGGCTCATGCC
TGTAATCCCGGCACTCTGGAAGGCTGAGGTGGGTGGATCTCTTGAGGCCAGGAGTTTGAGACCAGCCTGG
CCAACATGGCGAAACCCGGTCTCTACTAAAAATACAAAAAAATTAGCTGTGCATGGTGGCCCATGCCTAT
AGTCCCAGCTACTTGGGAAGCTGAGATATGAGAATTGCTCGAACCTGGGAGGCAGAGGTTGCAGTGAGCC
AAGATCACACCACTGCACTCCAGCCTGGGTGACAGAGCGAGACTCCATCTCAAAACAAACAAACAAACAA
AAAACAAAACAAAACAAAAAAACAGAAGGAAAGAAAGAAATATATGTATATTTCAAGGAATTGGCTTCTGC
CATTTTGGGAGCTGGCAAGTCCAAAATCCCAGGGCAGGCCAGCAGGAAGAGCAGGCCAGAAATTGCAGCA
GGAGCTAAGGCTGAGTCCACAGGCGGAATTTCTTCTTTTTAGGGAAACCTCATTTTTCTTCTTAAGACCC
TCAACTGATTGGATGAGGCCCATCCACATCATTGAGAATGGTCTCCTTCACTTAAAGTCAGTGGGTTACA
CATGTTACCCACATCTACAGAATACCTCCGCAGCAATACCTAGATTCGTGTTTGATGGAATCACTGGGGA
CTCGAGCCTAGCCAAGCTAACACATGAAACACACCATCACAGCTGGGGAAAGGATGGCTTATTTTAGACT
GATAAAGATGACCCAGAGAAGGCCTGCTCCATCCACACTGGCCGCTTTAGTCTGCACTAAAGTTGTTGGT
TTTTTTTGTTTGTTTGGTTTTTTTTTGGTGACAGAGTCTCACTCTGTCGCCCAGGCTGGAGTGCAGTGGC
GCAGTCTCAGCTCACTGCAACCTCTGCATCCTGAGATCAAGCGATTCTCCTGCCTCAGCCTCCTGAGTAG
CTGGGACTACAGGCACGTGCCACCACACTCGGCTAATTTTTGTTTTTTCAGTAGAGACGGGGTTTCACCA
TATTGGCCAGGCTGGTCTTGAACGCCTGACCATGTGATCCATCCGCCTCAGTCTACCAAAGTGCTGGGTT
TACAGGCGTGAGCCACCACGCCCGGCCTTGTTGGGGTTTTTTGACAGCCTAATAGGTGAAAATGACATCT
CATTACAATCTTAATTGGCATTCTCTTATGACAACAAGCTGGTACATCTTTTTGTGTGTTGAGGGTTATT
TCTATTTCTTGCTCAGCAAACAGTTCATCCAGGAAGAGCTTCTTGGTGAGATAGTAGACCTCTGCGATTT
CTGTTGCAGACGATCTACATTTTGTCATTTGCTTTGTCATTTTTGTCTATGGTGGTTTTAGACTATGCGT
AAGTTTTCTAGAGCAGAAACTCAAGTTGGATTTGGGCCTCAGTGGTTATTGCCATACTTTAAAAGGACTT
TGTCTCCCTGAGATGATAAATGAGGTGGACAATATTTTCTTTAAGTAATTTCTTATTTTAACTGTTACAT
GATACCTTTGGCCCATTTGGAGTTCTTTGATGTCAAGAATGAGGCAGGATCCAGATGGCAGCAGAGGTCC
CAGTCCCATCCTGGAAGGGTCGTCTAGTTCCCACTGGTACTCCACACGCCCACTCAGGCACTCACTTCCC
CTCTGCGTTGGGTCTTGTCTGCAAGACTCTCTTATGTTTTACCATCTAGTGCAGCCAGCACCCCCACATC
ACCCTCACTTTTTCTTTCTTTAAATTGTGCAGAAATATTCATCATGTCTATTTTGCCATCTTAACCATTT
GGGGGTACATAGTTCAGTGGCATTAAGTACATTCATATTGTGCCAGCATCACCAGCAGCCATCTCCAGGA
CCCTATCACCTTCCCACACTGAAACTCTGTCCCCATTAAACACATTCCCCATTCCCCGCCCCTGAATCCC
TGACAGCTACCATCCTACTGTCTGTCTCTGTGAATTCAACTAACCTAAGTACCTCATAGGAGTTGTGACT
GGCTTGTTTCATGCAGTATGATGTCCTCATCCAGGTGGTAGCAAGTGTCAGAGTTTCACGCCTATTTATT
TATTATTATGAGACAGAGTCTTGCTCTGTCGCCCAGCCTGGAGTACAATGGCGCGATCCCAGCTCACTGC
AGCCTCCCCCTGCCTGGGTTCAAACAATTCTCCTGCCTCAGCCTCCCATGGTGTGCCGCCACACCTGGCT

ATTTTTTGTATTTTTAGTAGAGACGCGGTTTCACCACGTTGACCAGGCTGGTCTGGAAATGCAGTTTTTG
CACTGTCTGCCTGCTTACCTTTATAGAGCATATTTTGCCCTCTTCCATCAGAATTACCCATTTAATGGTC
AGGAAAAGCTGCTGGGAATATGACTCATAGCTGGGACATTCTCTGCACTGTGCATAGTTCCTCTCTGCCA
CCACCATGGAGGAGATTGATGGGTTTGAAACCCAGGGGAAGGTCATTGCCCTGCGAGGGTCTCCCTCATT
GAGAATCTGGATCCCCTCATGTGCACATGGTGAGGTCAGAGTCCCCTCCTCACAGTGTCCCCTCCACCCT
CCCGTGAACTGTTCTTTCCTTCCAGGAGGCCAGCAAGCGCATCTCCAGCCACATCCCTTTGATCATCCAG
TTCTTCATGCTCCAGACGTACGGCCAGCAGCTTCAGAAGGCCATGCTGCAGCTCCTGCAGGACAAGGACA
CCTACAGCTGGCTCCTGAAGGAGCGGAGCGACACCAGCGACAAGCGGAAGTTCCTGAAGGAGCGGCTTGC
ACGGCTGACGCAGGCTCGGCGCCGGCTTGCCCAGTTCCCCGGTTAACCACACTCTGTCCAGCCCCGTAGA
CGTGCACGCACACTGTCTGCCCCCGTTCCCGGGTAGCCACTGGACTGACGACTTGAGTGCTCAGTAGTCA
GACTGGATAGTCCGTCTCTGCTTATCCGTTAGCCGTGGTGATTTAGCAGGAAGCTGTGAGAGCAGTTTGG
TTTCTAGCATGAAGACAGAGCCCCACCCTCAGATGCACATGAGCTGGCGGGATTGAAGGATGCTGTCTTC
GTACTGGGAAAGGGATTTTCAGCCCTCAGAATCGCTCCACCTTGCAGCTCTCCCCTTCTCTGTATTCCTA
GAAACTGACACATGCTGAACATCACAGCTTATTTCCTCATTTTTATAATGTCCCTTCACAAACCCAGTGT
TTTAGGAGCATGAGTGCCGTGTGTGTGCGTCCTGTCGGAGCCCTGTCTCCTCTCTCTGTAATAAACTCAT
TTCTAGCAGACACTGCTCTGCCATGTTTTGTATTTTGGCGAGAAGCCTGAAACTAGCAGGTAGGGTGCAG
TGGAGCAGTGGACGTAAAGCTGCCCTCTGTGGCGGGGCCAGGTAGGAGCAAGCAAAAGAACAGGGTCTGA
TGATTTCCTAGAGACTGGAGGGAAATGGGGGAACACACTTTAAAAAATGAAACAATATGAAGCCAACGGT
GCTAACTTAATAATGTGCATTGATCAAATGTCCAATTTGTTGAGTCACTGGGCACCTGGCATGGAGTTGA
AAAGCACAAGGCCCAGTCAGGAAGCGGTTGCCAGGCCAGGTGCGATGGCTCACGCTGTAATCCCTTCACT
TTGGGAGGCCGAGGCGGGTGGATCACCTGAGGTCAGGAGTTCGGGACCAGCCTGACCAATATGGTGAAAC
CCTGTCTCTACTAAAAATACAAAAATTAGCTGGGTGTGGTGGCGTGTGCCTATAGTCCCAGCTACTCAGG
AGGCTGAGACATGAGAATTGCTTAAACCCGGGAGGTGGAGGTTGCAGTGAGCCCAGATCGTGCCACTGCA
CTCCAGCCTGGGCTACAGAGCAAGACTCCATCTCAGGGAAAAAAAAAGGTTTCCCAGTGGGACTTTACCT
GCAGAGGCTGTGGCTGGGGGAGCGGTCTGGGAAGCAGAGAAGGCGGAACACTAACCTTGGAGGAGAACCT
GACTGAAGTTCTCTGGTCTAGCCTGGTTGGGGGAGACTCGAGCCACACTCCTACGCCCTGACTCTGAGAA
CCATGGCTCCCAATCAGTGCTGCTGCTGGGCCTCCTCACCATGAGGCCAGGGCAGGCTCAAGGGCAGGAA
ATCGAACAGTGGACTAGATGGATGGGGCAGGAGAGGACCGGACACTCTGTCCCTTAAGGGGAGAGTTGAA
GGATCAGGATTGCCCCAGCCTAGGAAAGAGAAGGCCAAAAGGCAACAAGAGAGATGAAGTGTGAGCACCA
AGGTGTGACATCCTCTGGGGAGGTATATTCTAGATGATCAGTGTGGGGAATGGGGGTACTTTCTACAGAG
GGGCCCCCGGATGGCATGGCTTCCTCAAGAGCTGTGAGGTCCCAGGCAGTGGGGGCATCAGGCCAAGCTG
CACAGCTGCTTGTCAGGTTGGGGGTGGGGGCTGGACCAGCAACACCATCCTCTCCCCTCTGGACCACGCC
CCCCCATCCCAGATCTCTTCTGACTCTTGGGACTGTGTAATTTTCAATTCTTCGGGAAGTTTGAGAATAC
ATTTATAAGAGAGGATGTCCGGAGAGCAGTGGACATTTCACAAGCCCAGGTCAGCAGCTGCCGCTGGGCA
GCAACTTTATGGTTCCCCCAGGTGCTGAAGTGCACCTGCAGGTTGACTTGATCTACTGTTAACAGAGGGT
GATCAGTTTGACTCATGCTTAAGGGAGAGACGACACCAACACACACACACAGAGGTTTGCAAGAGTAAGGCA
GGGTGGATCCAAACATTCTTAATTCAATACTAGGCACGCGTTCTCGCAGTGGACATGGCCCAGGTCCTCT
GGCTGTGTTGGTGCATCAGACAGCTCGGCCCTTCCATGAGGCAGGTGCCCTCTCGTGGCAGGCCTGGGAA
ACTTCTCCACTACAAGACGTCCCTTCCTTCCACCACCACCCAAATCAGTCTTCCACTTGCCTTAAGTTTC
TGCTGCACGGGAAACAGAGTCAGAGGTGTCAAAGTGTTGCACAGGATTCTATTTTGCTGTTGTTAGAATT
TTGCTAGTGATAACCAGAGAGGATGGAGGAAGAAAGTTGACAGTATGTGTCCCAGAGAACAGCAATTTCA
ATTTCCCCAGCAGAGTGAACCTGCTGGGGATGCCCCAAGGTGCCCGCAAGGATGTTCCTTTCAGCCAAAA
TGAACATGTGTGGTGATCCCCCAGGCCTGTCCTAACTAGTCATCCAGAGCCGAGAGGGGAGTGGCCCCAG
CGGCCAGAACTGGGAGAGAGCCCTGGAGACCACTTTGTGTGACTCCTTCCTG

### Brief Description of the Drawings

The invention will be more clearly understood from the following description of an embodiment thereof, given by way of example only, with reference to the accompanying drawings, in which:-
**Figure 1** illustrates distribution plots of (A) SAA, (B) CRP and (C) Hp for healthy, infectious and inflammatory disease.
**Figure 2** illustrates mean SAA, CRP and Hp trends over time for septic arthritis.
**Figure 3** illustrates a receiver operating characteristic (ROC) curve showing the area under the curve (AUC) plot for Hp when evaluating biomarker ability to classify healthy and non-healthy patients.
**Figure 4** illustrates a ROC curve showing an AUC for SAA when evaluating biomarker ability to classify healthy and non-healthy patients.

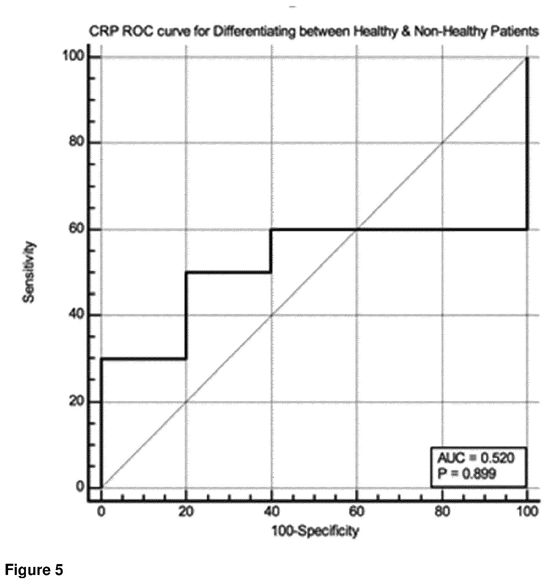

**Figure 5** illustrates a ROC curve showing an AUC for CRP when evaluating biomarker ability to classify healthy and non-healthy patients.
**Figure 6** illustrates a ROC curve showing an AUC for SAA when evaluating biomarker ability to classify healthy and Inflammation groups.
**Figure 7** illustrates a ROC curve showing an AUC for SAA when evaluating biomarker ability to classify infectious and non-infectious groups.
**Figure 8** illustrates a ROC curve showing an AUC for Hp when evaluating biomarker ability to classify Infectious and Non-Infection groups.
**Figure 9** illustrates a ROC curve showing an AUC for CRP when evaluating biomarker ability to classify Infection and Non-infection groups.
**Figure 10** illustrates a ROC curve showing an AUC for SAA when evaluating biomarker ability to classify Infectious and Inflammatory groups.
**Figure 11** illustrates a ROC curve showing an AUC for Hp when evaluating biomarker ability to classify Infectious and Inflammatory groups.
**Figure 12** illustrates a ROC curve showing an AUC for CRP when evaluating biomarker ability to classify Infectious and Inflammatory groups.
**Figure 13** illustrates a ROC curve showing an AUC for Hp when differentiating between healthy and inflammatory conditions in an individual.

### Detailed Description of the Drawings

### Materials and Methods

In this study SAA, CRP and Hp levels were retrospectively assessed in repository equine serum samples (n= 162) with clinical records available. Samples were included in the study if they had a healthy, infectious or inflammatory clinical record at the time of collection and divided into five groups: Healthy, Inflammation, Infection, Non-Healthy (inflammation and Infection combined) and Non-Infection (Healthy and Inflammation combined). The Kruskal Wallace test was engaged on all samples in the Healthy, Inflammatory and Infectious groups to determine differences in levels across the groups for each biomarker, where P<0.05 was considered statistically significant. Receiver operating curves were used on a subset of data across groups where data for all three biomarkers was available for each sample to identify the diagnostic ability of the biomarkers to differentiate between healthy and disease states. Diagnostic ranges were determined by Youden index (a single statistic that captures the performance of a dichotomous diagnostic test) and in Microsoft Excel using diagnostic value statistics, sensitivity, specificity, positive predicative values and negative predictive values. The biomarkers were also assessed in cases where serial sampling across a time course was available to compare performance across time.

Protein levels were determined using immunoassays. CRP and Hp levels were measured using species appropriate enzyme linked immunosorbent assays (ELISA). SAA concentrations were determined using a commercial lateral flow test. Statistical manipulations were performed using Microsoft Excel and MedCalc Software. Other methods can be used to detect nucleic acids, such as PCR, quantitative (or real time) PCR (qPCR), reverse transcriptase (RT) PCR (RT-PCR), RT-qPCR, Photometry, Fluorescence, lateral flow methods, and those methods described above. Such techniques can give quantitative outputs such as molar, ng/ul, ng/ml, copies/ul, and optical density (OD) readings.

### C Reactive Protein (CRP)

Serum CRP protein concentrations were determined using a CRP Horse ELISA kit and performed using antibodies and standards obtained from Abcam, Cambridge, UK. All reagents were equilibrated to room temperature. Serum samples were diluted before use with 1X Diluent (supplied as 5X diluent concentrate and diluted 1/5 using deionized water). 100µL of each standard, including zero control, and each sample was pipetted, in duplicate or dilution, into predesignated wells on a 96 well micro titer plate and covered with an adhesive plate cover. The plate was left to incubate at room temperature for thirty (30 ± 2) minutes. The plate was then aspirated and washed four times with wash buffer (supplied as 20X concentrate and diluted 1/20 with deionized water). Following this, 100µL CRP Horse HRP conjugate (10µL enzyme-antibody conjugate to 990µL of 1X diluent per test strip) was added to each well and the plate covered with an adhesive plate cover. The plate was left to incubate at room temperature for thirty (30 ± 2) minutes in the dark. The plate was then aspirated and washed four times with wash buffer. Following this, 100µL of TMB Substrate was added to each well, the plate covered with an adhesive plate cover and incubated in the dark at room temperature for precisely ten minutes. After ten minutes, 100µL of Stop Solution was added to each well. Absorbance was then read at 450nm on a microplate reader (Benchmark microplate reader, Bio-Rad, US). CRP levels were extrapolated from standard curve of CRP level (ng/mL) versus optical density at 450nm (Excel).

### Haptoglobin (Hp)

Serum samples were diluted before use with 1X Diluent (supplied as 5X diluent concentrate and diluted 1/5 using deionized water). 100µL of each standard, including zero control, and each sample was pipetted, in duplicate or dilution, into pre-designated wells on a 96 well micro titer plate and covered with an adhesive plate cover. The plate was left to incubate at room temperature for thirty (30 ± 2) minutes. The plate was then aspirated and washed four times with wash buffer (supplied as 20X concentrate and diluted 1/20 with deionized water). Following this, 100µL Haptoglobin Horse HRP conjugate (10µL enzyme-antibody conjugate to 990µL of 1X diluent per test strip) was added to each well and the plate covered with an adhesive plate cover. The plate was left to incubate in the dark at room temperature for thirty (30 ± 2) minutes. The plate was then aspirated and washed four times with wash buffer. Following this, 100µL of TMB Substrate was added to each well, the plate covered with an adhesive plate cover and incubated in the dark at room temperature for precisely ten minutes. After ten minutes, 100µL of Stop Solution was added to each well. Absorbance was then read at 450nm on a microplate reader (Benchmark microplate reader, Bio-Rad, US). Haptoglobin levels were extrapolated from standard curve of Haptoglobin level (ng/mL) versus optical density at 450nm (Excel).

### Serum Amyloid A (SAA)

Serum SAA samples were analysed utilising a commercial lateral flow immunoassay test, Equine SAA, Stablelab. Assay comprised of immunoassay strip housed in a cartridge, mix solution for dilution and propriety EQ1 reader device for SAA quantitation. Samples were allowed to come to room temperature and allowed to mix before use. Samples required a dilution whereby 5µL of sample (serum/plasma) was pipetted into a mix solution. 4 drops of sample (approx. 100µL) was then added to the sample well of the lateral flow cartridge. A timer was then set for a total run time of ten minutes, following which the cartridge was inserted into associated reader device for SAA quantitation. Readout were shown in µg/ml.

### Myxovirus Resistance Protein A (MxA)

Serum samples were diluted before use with 1X Diluent. 100µL of each standard, including zero control, and each sample was pipetted, in duplicate or dilution, into pre-designated wells on a 96 well micro titre plate. The plate was left to incubate at room temperature for 1 hour, shaking at ca. 300 rpm on an orbital microplate shaker. The plate was then aspirated and washed three times with wash buffer (supplied as 10X concentrate and diluted 1/10 with deionized water). Following this, 100µL Biotin Labelled Anti-MxA Antibody Solution was added to each well and left to incubate at room temperature for 1 hour, shaking at ca. 300 rpm on an orbital microplate shaker. The plate was then aspirated and washed three times with wash buffer. Following this, 100µL of Streptavidin-HRP Conjugate was added to each well and the plate incubated at room temperature for 30 minutes, shaking at ca. 300 rpm on an orbital microplate shaker. The plate was then washed five times with wash buffer. 100µL of Substrate Solution was added to each well, the plate covered with tin foil and incubated in the dark for 20 minutes at room temperature (no shaking carried out during this incubation). 100µL of Stop Solution was then added to each well to stop colour development. Absorbance was then read at 450nm on a microplate reader (Benchmark microplate reader, Bio-Rad, US). MxA levels were extrapolated from standard curve of the log of MxA level (ng/mL) versus optical density at 450nm (Excel).

### Results

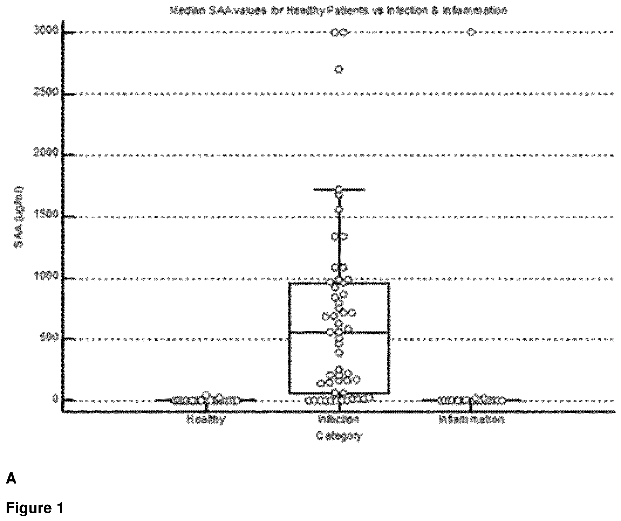

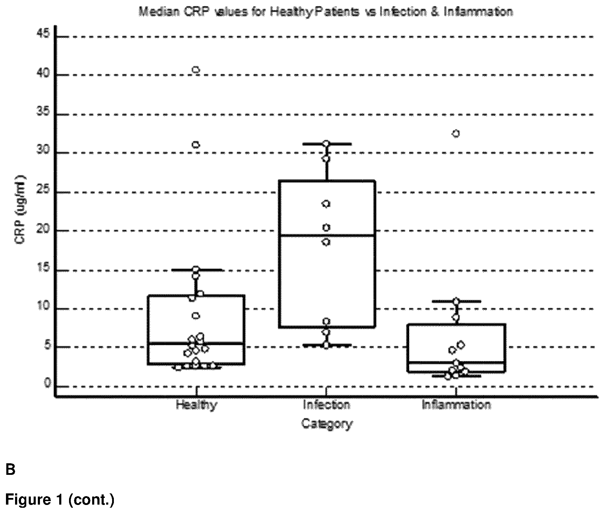

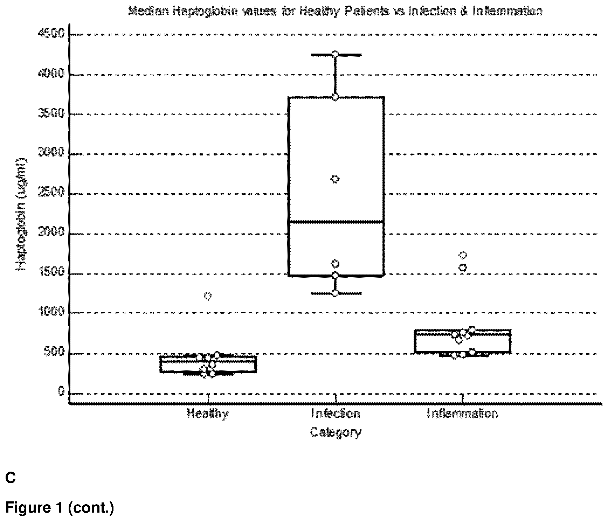

In single time point analysis levels of SAA (n = 99) and CRP (n=39) there was a significant difference in biomarker values between the Healthy, Inflammation and Infection groups, p < 0.000001 and p = 0.010325, respectively (see **Figure 1A****,** **1B**). SAA had a median value of 558ug/ml for infection compared to median values of 0ug/ml for both inflammatory disease and healthy controls. There was no significant difference between the Healthy group and the Inflammation group for SAA. CRP demonstrated a median value of 19.45ug/ml for the infection group, approximately three times normal reference levels compared to 5.45ug/ml and 2.98ug/ml for the Healthy and Inflammation groups respectively which are within normal limits. CRP was slightly more elevated in the Healthy group than in the Inflammation group, but this was not considered statistically significant. There was a statistically significant distribution of Hp levels (n=24) across all three groups (p = 0.000345) (**Figure 1C**). The median Hp value was 2153ug/ml for the Infection group, 731ug/ml for the inflammation group and 397ug/ml for the Healthy group.

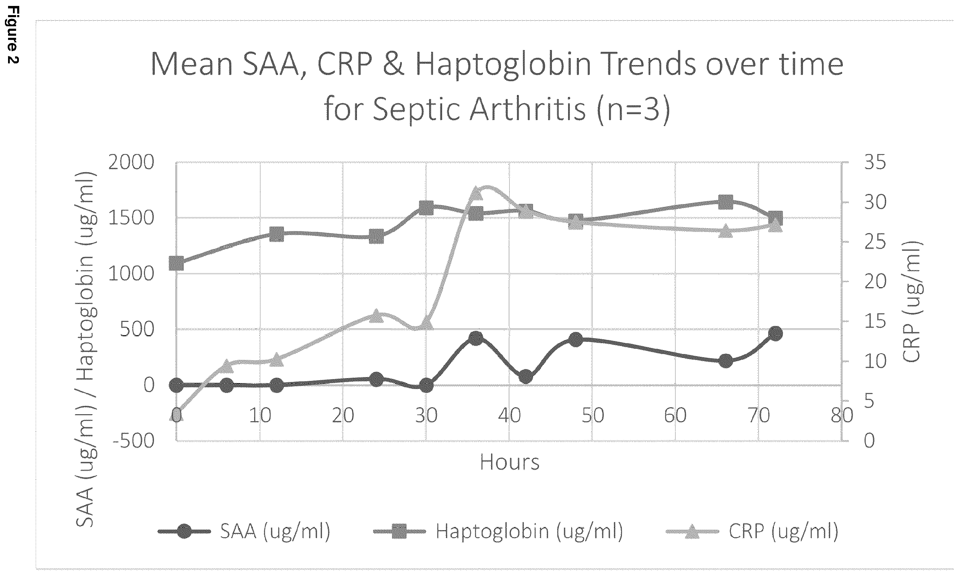

In a time course assessment for patients with a confirmed infection (n=3) (see **Figure 2**), CRP was elevated from baseline levels within 12 hours of infectious challenge. SAA started to elevate rapidly at 24 hours for cases of septic arthritis. Hp levels started to elevate immediately to just above the reportedly normal reference level (200-1000ug/ml) within 10 hours, reaching a peak at 30 hours. None of the biomarkers returned to normal levels during the time course (**Figure 2**).

ROC analysis was undertaken on a subset of data for which SAA, CRP and Hp (n=24) levels were available to identify the diagnostic ability of the biomarkers to differentiate between healthy and disease states.

### Distinguishing Healthy and Non-Healthy (Infection and Inflammation combined)

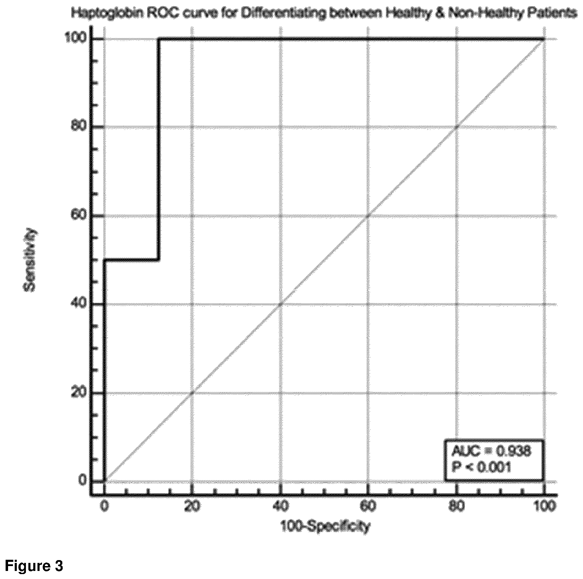

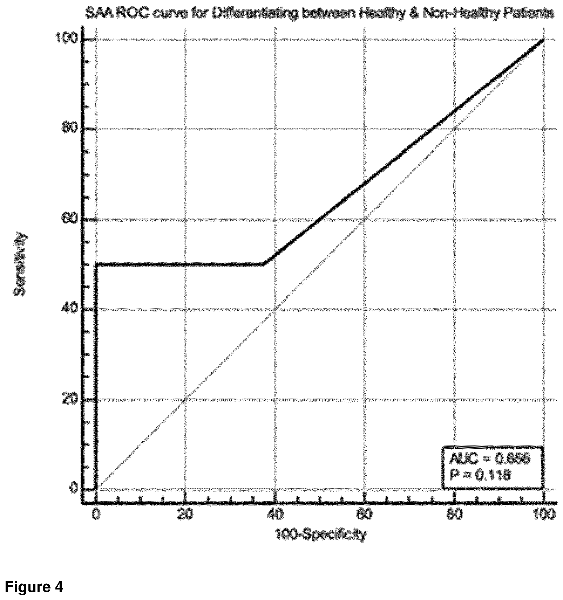

The most significant area under the curve (AUC) for the Healthy and Non-Healthy Group was observed for Hp (see **Figure 3**) with an optimum cut off value of >473ug/ml reported by Youden Index. Sensitivity was 100%, demonstrating all Non-Healthy patients are differentiated from the Healthy group (**Figure 3** and Table 3 below) and specificity was 87.5% with an overall accuracy of 95.8%. AUC value for SAA was not significant for the same group at 0.656 and p=0.118 (see **Figure 4**). Sensitivity was reported at 37.5% and specificity was 100%. The AUC for CRP was also not significant at 0.520 and P = 0.8992.

**Table 3: showing diagnostic performance statistics for a range of Hp cut-off values, including Youden Index associated criterion of greater than 473ug/ml, to distinguish between healthy and non-healthy patients (infection and inflammation combined).**

| **Cut-off (ug/ml) ->** | **400** | **473** | **500** | **600** | **700** |
|---|---|---|---|---|---|
| **Sensitivity** | 100.0 | 100.0 | 87.5 | 81.3 | 75.0 |
| **Specificity** | 50.0 | 87.5 | 87.5 | 87.5 | 87.5 |
| **Pos. Predictive Value** | 80.0 | 94.1 | 93.3 | 92.9 | 92.3 |
| **Neg. Predictive Value** | 100.0 | 100.0 | 77.8 | 70.0 | 63.6 |
| **Accuracy** | 83.3 | 95.8 | 87.5 | 83.3 | 79.2 |

### Distinguishing Healthy and Inflammation

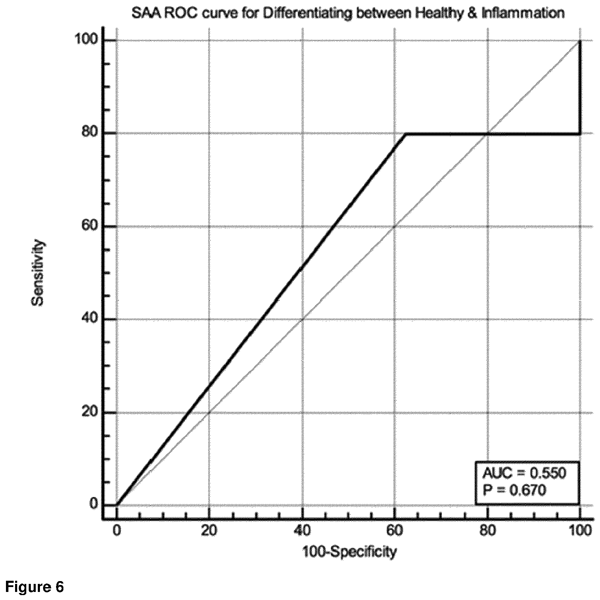

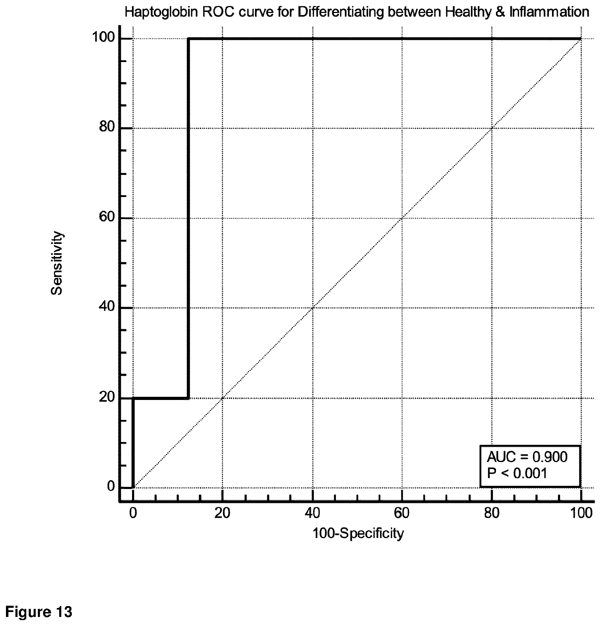

The most significant AUC was observed for Hp at 0.900 and p = 0.0001 with a cut off value of 473ug/ml (**Figure 13** and Table 4) SAA reported an AUC of 0.550, p = 0.66 (**Figure 6**). CRP had an AUC of 0.800 and P= 0.13.

**Table 4: showing diagnostic performance statistics for a range of Hp cut-off values, including Youden Index associated criterion of greater than 473ug/ml, to distinguish between healthy and inflammation patients**

| **Cut-off (ug/ml)** | **473** | **600** | **796** | **1217** |
|---|---|---|---|---|
| **Sensitivity** | 100.0 | 70.0 | 20.0 | 20.0 |
| **Specificity** | 87.5 | 87.5 | 87.5 | 88.9 |
| **Pos. Predictive Value** | 90.9 | 87.5 | 66.7 | 66.7 |
| **Neg. Predictive Value** | 100.0 | 70.0 | 46.7 | 50.0 |
| **Accuracy** | 94.4 | 77.8 | 50.0 | 52.6 |

### Distinguishing Infection and Non-Infection (Healthy and Inflammation)

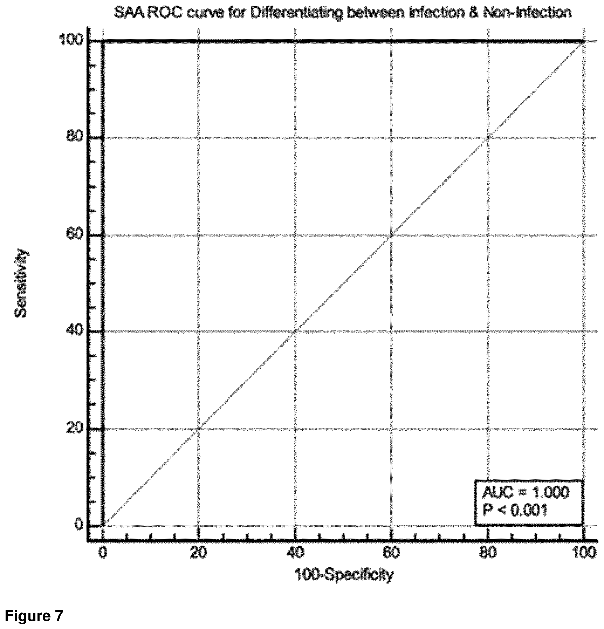

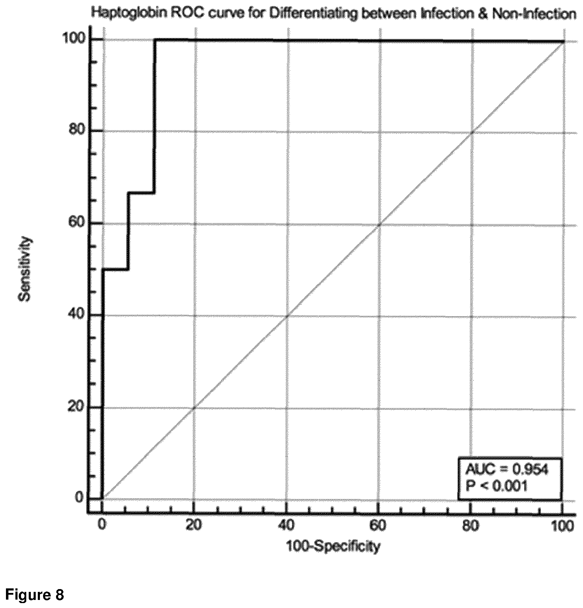

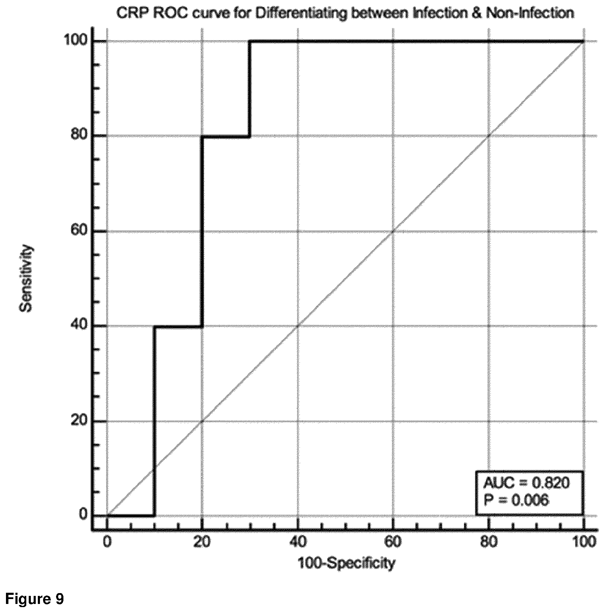

The most significant AUC was observed for SAA when differentiating between the Infection and Non-Infection groups. AUC was 1.000 (P <0.0001) (**Figure 7**) and optimum cut off value was identified as 18.5ug/ml (Table 5). Hp reported an AUC of 0.917, p<0.001 at Youden Index criterion >1217ug/ml (**Figure 8** and **Table 6**). AUC for CRP was 0.820 (p=0.0056) at Youden Index criterion >4.7ug/ml (**Figure 9** and **Table 7**)

**Table 5: showing diagnostic performance statistics for a range of SAA cut-off values, including Youden Index associated criterion of greater than 18.5ug/ml, to distinguish between infection and non-infection patients (healthy & inflammation combined)**

| **Cut-off (ug/ml)->** | **15** | **18.5** | **50** | **100** |
|---|---|---|---|---|
| **Sensitivity** | 100.0 | 100.0 | 100.0 | 100.0 |
| **Specificity** | 94.4 | 94.4 | 100.0 | 100.0 |
| **Pos. Predictive Value** | 85.7 | 85.7 | 100.0 | 100.0 |
| **Neg. Predictive Value** | 100.0 | 100.0 | 100.0 | 100.0 |
| **Accuracy** | 95.8 | 95.8 | 100.0 | 100.0 |

**Table 6: showing diagnostic performance statistics for a range of Hp cut-off values, including Youden Index associated criterion of greater than 1217ug/ml, to distinguish between infection and non-infection patients (healthy & inflammation combined)**

| **Cut-off (ug/ml)->** | **400** | **473** | **500** | **600** | **700** | **1217** |
|---|---|---|---|---|---|---|
| **Sensitivity** | 100.0 | 100.0 | 100.0 | 100.0 | 100.0 | 100.0 |
| **Specificity** | 22.2 | 38.9 | 50.0 | 55.6 | 61.1 | 88.9 |
| **Pos. Predictive Value** | 30.0 | 35.3 | 40.0 | 42.9 | 46.2 | 75.0 |
| **Neg. Predictive Value** | 100.0 | 100.0 | 100.0 | 100.0 | 100.0 | 100.0 |
| **Accuracy** | 41.7 | 54.2 | 62.5 | 66.7 | 70.8 | 91.7 |

**Table 7: showing diagnostic performance statistics for a range of CRP cut-off values, including Youden Index associated criterion of greater than 4.7ug/ml, to distinguish between infection and non-infection patients (healthy and inflammation combined)**

| **Cut-off (ug/ml)** | **2** | **4.7** | **6** | **8** | **10** |
|---|---|---|---|---|---|
| **Sensitivity** | 100.0 | 100.0 | 80.0 | 60.0 | 40.0 |
| **Specificity** | 20.0 | 70.0 | 80.0 | 80.0 | 90.0 |
| **Pos. Predictive Value** | 38.5 | 62.5 | 66.7 | 60.0 | 66.7 |
| **Neg. Predictive Value** | 100.0 | 100.0 | 88.9 | 80.0 | 75.0 |
| **Accuracy** | 46.7 | 80.0 | 80.0 | 73.3 | 73.3 |

### Infection and Inflammation

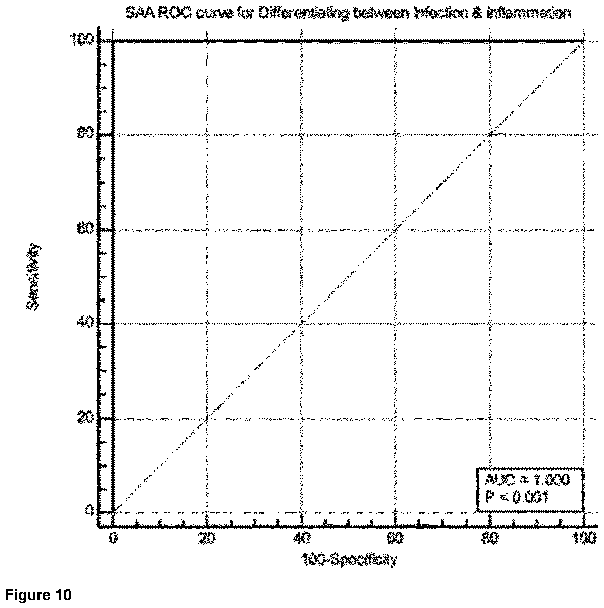

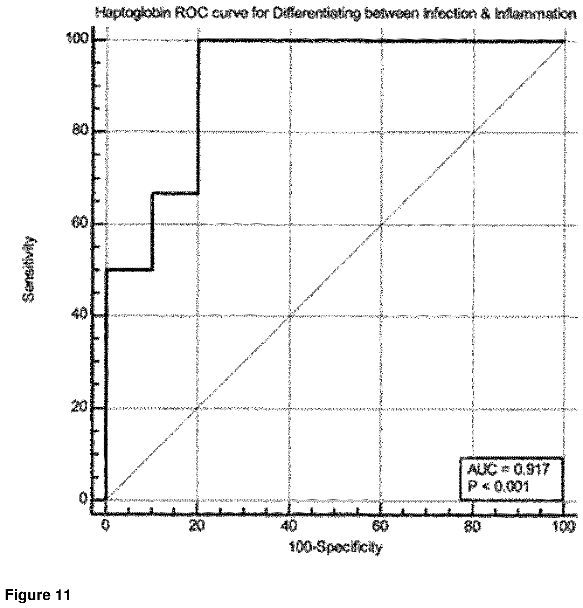

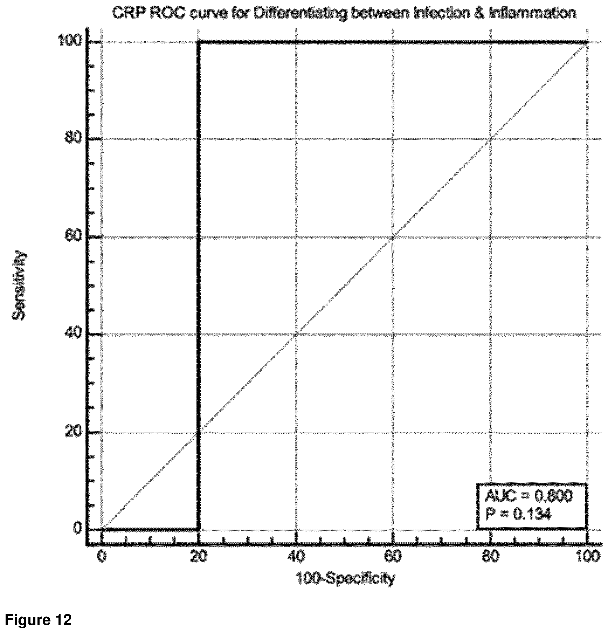

SAA AUC was 1.000, P<0.0001 and Youden Index criterion was >18.5ug/ml (**Figure 10**, Table 8). AUC for Hp was 0.917, P<0.0001 and Youden Index criterion was >796ug/ml (**Figure 11**, Table 9). CRP AUC was 0.800, P = 0.1336 and Youden Index criterion >2.456ug/ml (**Figure 12**, Table 10).

**Table 8: showing diagnostic performance statistics for a range of SAA cut-off values, including Youden Index associated criterion of greater than 18.5ug/ml, to distinguish between infection and inflammation patients**

| **Cut-off (ug/ml)** | **15** | **18.5** | **50** | **100** |
|---|---|---|---|---|
| **Sensitivity** | 100.0 | 100.0 | 100.0 | 100.0 |
| **Specificity** | 94.4 | 94.4 | 100.0 | 100.0 |
| **Pos. Predictive Value** | 85.7 | 85.7 | 100.0 | 100.0 |
| **Neg. Predictive Value** | 100.0 | 100.0 | 100.0 | 100.0 |
| **Accuracy** | 95.8 | 95.8 | 100.0 | 100.0 |

**Table 9: showing diagnostic performance statistics for a range of Hp cut-off values, including Youden Index associated criterion of greater than 796ug/ml, to distinguish between infection and inflammation patients.**

| **Cut-off (ug/ml)->** | **473** | **500** | **600** | **796** | **1217** |
|---|---|---|---|---|---|
| **Sensitivity** | 100.0 | 100.0 | 100.0 | 100.0 | 100.0 |
| **Specificity** | 38.9 | 50.0 | 55.6 | 80.0 | 80.0 |
| **Pos. Predictive Value** | 35.3 | 40.0 | 42.9 | 75.0 | 75.0 |
| **Neg. Predictive Value** | 100.0 | 100.0 | 100.0 | 100.0 | 100.0 |
| **Accuracy** | 54.2 | 62.5 | 66.7 | 87.5 | 87.5 |

**Table 10: showing diagnostic performance statistics for a range of CRP cut-off values to distinguish between infection and inflammation patients.**

| **Cut-off (ug/ml)** | **2** | **4.7** | **6** | **8** | **10** |
|---|---|---|---|---|---|
| **Sensitivity** | 100.0 | 100.0 | 80.0 | 60.0 | 40.0 |
| **Specificity** | 20.0 | 70.0 | 80.0 | 80.0 | 90.0 |
| **Pos. Predictive Value** | 38.5 | 62.5 | 66.7 | 60.0 | 66.7 |
| **Neg. Predictive Value** | 100.0 | 100.0 | 88.9 | 80.0 | 75.0 |
| **Accuracy** | 46.7 | 80.0 | 80.0 | 73.3 | 73.3 |

### Discussion

The main objective of this study is to evaluate serum amyloid A (SAA), C reactive protein (CRP), haptoglobin (Hp) and myxovirus resistance protein A (MxA) to assess their usefulness in determining an individual's health status by distinguishing between healthy, infectious, and inflammatory conditions.

The evaluation of CRP, SAA, Hp, and MxA in this study demonstrated a number of surprising findings. SAA, CRP and Hp and MxA are all elevated in the presence of infection. However, only Hp is consistently elevated in the presence of non-infectious inflammation. Elevated SAA and CRP are not associated with healthy controls or non-infectious inflammatory disease but are positively correlated with the presence of infection. This is a departure from previous findings in the literature which report SAA and CRP as being non-specific markers associated with inflammatory processes. Elevated Hp is positively correlated with both inflammatory conditions and infectious conditions however at different cut off levels and can therefore be used to distinguish inflammatory cases from both healthy and infectious cases. In time course analysis, SAA and CRP are earlier, more dynamic indicators of the onset of infection and can be used to monitor disease progression and response to therapy.

These findings are meaningful and present the possibility of a unique method for the differentiation of healthy, inflammatory and infectious groups by combining the biomarker levels at particular reference points. This could be extended to biomarker levels at particular reference points over particular periods of time. SAA is shown here to be a sensitive and specific marker of infection which can classify groups into infection vs non-infectious, thereby identifying infectious cases which may be candidates for antimicrobial treatment. However, SAA is not a good indicator of healthy or inflammatory groups by itself, and so those patients in inflammatory groups who have an inflammatory disease, which may progress to a more aggressive or infectious condition, or those with sterile inflammatory conditions, are not classified/detected when SAA is used, alone and so are at risk of being either untreated or mistreated. This poses a significant problem as in the absence of a method to definitively differentiate healthy, inflammatory and infectious conditions, clinicians may feel the need to treat a patient to avoid the risk associated with withholding treatment.

The data promotes SAA as a definitive marker of infection which could be used to differentiate/identify infection from inflammatory conditions. The data also strongly indicates that SAA in combination with Hp can be used to both detect infection and differentiate non-infectious inflammation from a healthy status.

Though the biomarkers are useful as single biomarkers for detecting groups *i.e.* individual groups, alone they lack the required sensitivity and specificity to differentiate between all groups in the study. However, when considered together it is possible to correctly identify the groups to a high degree of accuracy. The combination of the biomarkers as described here is also useful to monitor response to therapy in both inflammatory and infectious cases, whereby a decrease in the relevant biomarkers is associated with an improved health status. The method may also be engaged to assess the efficacy of anti-inflammatory or anti-microbial therapies. It may also be engaged as an ongoing screen in chronic inflammatory cases.

In practical use, when SAA (and/or CRP) values are below the threshold levels reported here, the presence of an elevated Hp is a positive indicator for inflammatory disease without an infectious process. This is a significant improvement upon existing methods of differentiating infectious and inflammatory conditions.

A testing method sequence is proposed for assessing the efficacy of the biomarkers together when differentiating between the three groups (healthy, inflammatory, and infectious) (see Table 11). An SAA cut off value of <20ug/ml and Hp cut off value of <475ug/ml (rounded up from Youden Index (J) for convenience) was used to identify and eliminate the healthy group. A second parameter, <20ug/ml SAA and >475ug/ml Hp, was used to detect inflammatory samples in the remaining group. The final parameter, > 20ug/ml SAA and >475ug/ml Hp, was applied to the groups to identify the infectious group. Using this method, 23 of the 24 samples were correctly identified giving an accuracy of 95.8% across all the groups which is a significant improvement upon values achievable by individual biomarkers to differentiate the groups.

This method could be further developed through the addition of CRP and/or MxA into the sequence to further improve one or more of the parameters of sensitivity, specificity, positive predictive value and negative predictive value.

**Table 11: A testing sequence for differentiating between Healthy, Inflammation and Infection groups.**

| **Status** | **SAA (ug/ml)** | **Hp (ug/ml)** | **n** | **Identified correctly** |
|---|---|---|---|---|
| **Healthy** | < 20 | < 475 | 8 | 7 of 8 |
| **Inflammation** | < 20 | > 475 | 10 | 10 of 10 |
| **Infection** | > 20 | > 475 | 6 | 6 of 6 |

This study has surprisingly found that despite all the biomarkers assessed being reportedly pro-inflammatory markers, they do not all behave in the same way in response to inflammation and are elevated or at normal levels based on the type of inflammation involved. The Applicant also presents a method of combining the biomarkers at particular reference levels to achieve greater levels of accuracy than currently available methods. This method can differentiate between healthy and inflammatory patients as well as inflammatory and infectious patients potentially leading to a reduction in the number of non-infectious cases treated unnecessarily with antimicrobials and an increase in the number of infections identified and treated correctly. This method also identifies inflammatory conditions as distinct from infectious conditions, and so patients with inflammatory symptoms without infection can also be treated and/or monitoring appropriately. Furthermore, the study shows that the biomarkers may be engaged together in a system whereby infection and/or inflammation can be detected early and corresponding treatment efficacy can be measured using the biomarkers over hours and/or days.

The identification of a biomarker or a panel of biomarkers which provides additional information on whether a condition is infectious or inflammatory in nature would present a number of positive outcomes, including a foreseeable reduction in unnecessary use of the already limited arsenal of antimicrobials available, rapid intervention, better clinical decision making and improved health management at home and in a clinical setting through monitoring regimes.

SAA, Hp, CRP, and MxA have been studied here as potential diagnostic and prognostic indicators that provide superior differentiation than currently available methods and which will aid clinicians in the treatment decision making process.

In the specification the terms "comprise, comprises, comprised and comprising" or any variation thereof and the terms "include, includes, included and including" or any variation thereof are considered to be totally interchangeable and they should all be afforded the widest possible interpretation and vice versa.

The invention is not limited to the embodiments hereinbefore described but may be varied in both construction and detail.

## Claims

1. A method of determining a health status of an individual, the method comprising the steps of assaying a biological sample from the individual for the positive expression of at least two genes, or proteins encoded by said genes, selected from SAA, CRP, Hp and MxA, and wherein the positive expression of the at least two genes, or proteins encoded by said genes, above or below a specified threshold level correlates with the health status of the individual.

2. A method of monitoring the effectiveness of treatment of an infectious disease in an individual with an infectious disease, the method comprising the step of assaying a biological sample from the individual with an infectious disease for the positive expression of at least two genes, or proteins encoded by said genes, selected from SAA, CRP, Hp, and MxA, wherein the positive expression of the at least two genes, or proteins encoded by said genes, above or below a specified threshold level correlates with the effectiveness of treatment of the infectious disease in the individual.

3. A method of identifying an individual with an infection or an infectious disease that is suitable for treatment with a therapy for treating the infectious disease, the method comprising the steps of assaying a biological sample from the individual with the infection or infectious disease for the positive expression of at least two genes, or proteins encoded by said genes, selected from SAA, CRP, Hp and MxA, wherein the positive expression of the at least two genes, or proteins encoded by said genes, above or below a specified threshold level correlates with the effectiveness of treatment of the infection or infectious disease in the individual

4. A method according to any one of Claims 1 to 3, wherein the at least genes, or proteins encoded by said genes, are SAA one selected from CRP, Hp and MxA.

5. A method according to any one of Claims 1 to 3, wherein the at least two genes, or proteins encoded by said genes are CRP and one selected from Hp and MxA.

6. A method according to any one of the preceding Claims, wherein the threshold levels for SAA protein expression levels are 20 ug/ml, the threshold levels for Hp protein expression levels are 475 ug/ml and the threshold levels for CRP protein expression levels are 4.7 ug/ml.

7. A method according to any one of the preceding Claims, wherein when the protein expression levels of SAA are less than 20 ug/ml and wherein when the protein expression levels of Hp are less than 475 ug/ml, the individual is healthy.

8. A method according to any one of Claims 1 to 6, wherein when the protein expression levels of SAA are less than 20 ug/ml and wherein when the protein expression levels of Hp are greater than 475 ug/ml, the individual is experiencing an inflammatory event or is suffering from an inflammatory condition.

9. A method according to any one of Claims 1 to 6, wherein when the protein expression levels of SAA are greater than 20 ug/ml and wherein when the protein expression levels of Hp are greater than 475 ug/ml, the individual is experiencing an infection.

10. A method according to any one of Claims 1 to 6, wherein when the protein expression levels of SAA are about 19 ug/ml to about 21 ug/ml, and wherein when the protein expression levels of Hp are about 470 ug/ml to 480 ug/ml, the individual is at risk of onset of an autoimmune and/or an autoinflammatory condition.

11. A method according to any one of Claims 1 to 6, wherein when the protein expression levels of SAA are less than 20 ug/ml and wherein when the protein expression levels of Hp are less than 475 ug/ml, the individual is responding to treatment; and wherein when the protein expression levels of SAA are greater than 20 ug/ml and wherein when the protein expression levels of Hp are greater than 475 ug/ml, the individual still has the infection and is not responding to treatment.

12. A method of determining the progression of treatment of an individual with an inflammatory disease, the method comprising the step of assaying a biological sample from the individual with the inflammatory disease for the positive expression of at least two genes, or proteins encoded by said genes, selected from CRP, Hp and MxA, and negative expression of SAA, wherein the positive expression of the at least two genes, or proteins encoded by said genes, above or below a specified threshold level, and the negative expression of SAA, correlates with the effectiveness of treatment of the inflammatory disease or disorder in the individual.

13. A method according to Claim 12, wherein the at least two genes, or proteins encoded by said genes, are CRP and one selected from Hp and MxA; and wherein the threshold levels for Hp protein expression levels are optionally 475 ug/ml and the threshold levels for CRP protein expression levels are optionally 4.7 ug/ml.

14. A method according to Claim 12 or Claim 13, wherein when the protein expression levels of Hp are less than 475 ug/ml, the individual is responding to treatment; and wherein when the protein expression levels of Hp are greater than 475 ug/ml, the individual is experiencing an inflammatory response and is not responding to treatment..

15. A method according to any one of the preceding claims, in which the individual is a mammal, and optionally a human.
